# NEW EUROPEAN PATENT SPECIFICATION

(11) **EP 1 144 454 B2**
(45) Date of publication and mention of the opposition decision: **12.12.2012**
(45) Mention of the grant of the patent: 12.12.2007
(21) Application number: 99971003.1
(22) Date of filing: 25.10.1999
(51) Int. Cl.: C07K 19/00, C12N 15/62, C12N 15/70, C12N 1/21

(54) **MODIFIED PEPTIDES AS THERAPEUTIC AGENTS**
MODIFIZIERTE PEPTIDE ALS THERAPEUTISCHE MITTEL
PEPTIDES MODIFIES UTILISES COMME AGENTS THERAPEUTIQUES

(30) Priority: 23.10.1998 US 105371 P; 22.10.1999 US 428082
(43) Date of publication of application: 17.10.2001
(73) Proprietor: AMGEN INC., Thousand Oaks, CA 91320-1799 (US)
(72) Inventor: FEIGE, Ulrich, Newbury Park, CA 91320 (US); LIU, Chuan-Fa, Longmont, CO 80503 (US); CHEETHAM, Janet, Montecito, CA 93108 (US); BOONE, Thomas, Charles, Newbury Park, CA 91320 (US)
(74) Representative: Grünecker, Kinkeldey, Stockmair & Schwanhäusser
(86) International application number: PCT/US1999/025044
(87) International publication number: WO 2000/024782

(56) References cited:
- WO-A-95/09917
- WO-A-96/18412
- WO-A-96/40772
- WO-A-97/28828
- WO-A-97/44453
- WO-A-98/24477
- WO-A-98/31820
- WO-A-98/46257
- WO-A1-01/02440
- WO-A1-91/11497
- WO-A1-96/18412
- WO-A1-97/28828
- WO-A1-97/30074
- WO-A1-97/34631
- WO-A1-97/43316
- WO-A1-98/03551
- WO-A1-98/28427
- WO-A1-98/46257
- WO-A2-01/01748
- WO-A2-91/08298
- WO-A2-93/22332
- WO-A2-96/05309
- WO-A2-98/30590
- US-A- 5 349 053
- US-A- 5 767 234
- US-A- 5 955 300
- H. LOETSCHER ET AL.: "Efficacy of a chimeric TNFR-IgG fusion protein to inhibit TNF activity in animal models of septic shock." INTERNATIONAL CONGRESS SERIES, vol. 2, 1993, pages 455-462, XP002067659 Amsterdam, The Netherlands
- B. BROCKS ET AL.: "A TNF receptor antagonistic scFv, which is not secreted in mammalian cells, is expressed as a soluble mono- and bivalent scFv derivative in insect cells." IMMUNOTECHNOLOGY, vol. 3, no. 3, October 1997 (1997-10), pages 173-184, XP002147314 Amsterdam, The Netherlands
- S. CWIRLA ET AL.: "Peptide agonists of the thrombopoietin receptor as potent as the natural cytokine." SCIENCE, vol. 276, no. 5319, 13 June 1997 (1997-06-13), pages 1696-1699, XP002142424 Washington, DC, USA cited in the application
- D. JOHNSON ET AL.: "Identification of a 13 amino acid peptide mimetic of erythropoietin and description of amino acids critical for the mimetic activity of EMP1." BIOCHEMISTRY, vol. 37, no. 11, 1998, pages 3699-3710, XP002147315 Washington, DC, USA
- CWIRLA ET AL: ' Peptide Agonist of the Thrombopoietin Receptor as Potent as the Natural Cytokine' SCIENCE 1997, pages 1696 - 1699
- ' PNAS' CHENG ET AL no. 94, 1997, pages 14120 - 14125
- CHOI ET AL MOL CELLS vol. 7, no. 5, 1977, pages 575 - 581
- LAUVRAK ET AL BIOL CHEM vol. 378, 1997, pages 1509- - 1519
- CHAMOW & ASHKENAZI TIBTECH vol. 14, 1996, pages 52 - 60
- ASHKENAZI & CHAMOW IMUN TECH vol. 9, 1997, pages 195 - 199
- FEHMANN & HABENER ENDOCRINOLOGY vol. 130, no. 1, 1992, pages 159 - 166
- LI ET AL BLOOD 98 2001, pages 3241 - 3248
- KUTER ET AL LANCET vol. 371, 2008, pages 395 - 403
- JAVA ET AL: ' Abstract 3425' 50TH ASH MEETING 2008,
- BUSSEL ET AL BLOOD vol. 113, 2009, page 2161

### SEQUENCE LISTING

<110> AMGEN INC.
<120> MODIFIED PEPTIDES AS THERAPEUTIC AGENTS
<130> A-527
<140> PCT/US 99/25044
   <141> 1999-10-25
<150> US 09/428,082
   <15i> 1999-10-22
<150> US 60/105,371
   <151> 1998-10-23
<160> 1143
<170> Patent In version 3.3
<210> 1
   <211> 684
   <212> DNA
   <213> HUMAN
<220>
   <221> CDS
   <222> (1)..(684)
<400> 1
   atg gac aaa act cac aca tgt cca cct tgt cca gct ccg gaa ctc ctg     48
   Met Asp Lys Thr His Thr Cys Pro Pro Cys Pro Ala Pro Glu Leu Leu
   1               5                   10                  15
   ggg gga ccg tca gtc ttc ctc ttc ccc cca aaa ccc aag gac acc ctc     96
   Gly Gly Pro Ser Val Phe Leu Phe Pro Pro Lys Pro Lys Asp Thr Leu
               20                  25                  30
   atg atc tcc cgg acc cct gag gtc aca tgc gtg gtg gtg gac gtg agc     144
   Met Ile Ser Arg Thr Pro Glu Val Thr Cys Val Val Val Asp Val Ser
           35                  40                  45
   cac gaa gac cct gag gtc aag ttc aac tgg tac gtg gac ggc gtg gag     192
   His Glu Asp Pro Glu Val Lys Phe Asn Trp Tyr Val Asp Gly Val Glu
       50                  55                  60
   gtg cat aat gcc aag aca aag ccg cgg gag gag cag tac aac agc acg     240
   Val His Asn Ala Lys Thr Lys Pro Arg Glu Glu Gln Tyr Asn Ser Thr
   65                  70                  75                  80
   tac cgt gtg gtc agc gtc ctc acc gtc ctg cac cag gac tgg ctg aat     288
   Tyr Arg Val Val Ser Val Leu Thr Val Leu His Gln Asp Trp Leu Asn
                   85                  90                  95
   ggc aag gag tac aag tgc aag gtc tcc aac aaa gcc ctc cca gcc ccc     336
   Gly Lys Glu Tyr Lys Cys Lys Val Ser Asn Lys Ala Leu Pro Ala Pro
               100                 105                 110
   atc gag aaa acc atc tcc aaa gcc aaa ggg cag ccc cga gaa cca cag     384
   Ile Glu Lys Thr Ile Ser Lys Ala Lys Gly Gln Pro Arg Glu Pro Gln
           115                 120                 125
   gtg tac acc ctg ccc cca tcc cgg gat gag ctg acc aag aac cag gtc     432
   Val Tyr Thr Leu Pro Pro Ser Arg Asp Glu Leu Thr Lys Asn Gln Val
       130                 135                 140
   agc ctg acc tgc ctg gtc aaa ggc ttc tat ccc agc gac atc gcc gtg     480
   Ser Leu Thr Cys Leu Val Lys Gly Phe Tyr Pro Ser Asp Ile Ala Val
   145                 150                 155                 160
   gag tgg gag agc aat ggg cag ccg gag aac aac tac aag acc acg cct     528
   Glu Trp Glu Ser Asn Gly Gln Pro Glu Asn Asn Tyr Lys Thr Thr Pro
                   165                 170                 175
   ccc gtg ctg gac tcc gac ggc tcc ttc ttc ctc tac agc aag ctc acc     576
   Pro Val Leu Asp Ser Asp Gly Ser Phe Phe Leu Tyr Ser Lys Leu Thr
               180                 185                 190
   gtg gac aag agc agg tgg cag cag ggg aac gtc ttc tca tgc tcc gtg     624
   Val Asp Lys Ser Arg Trp Gln Gln Gly Asn Val Phe Ser Cys Ser Val
           195                 200                 205
   atg cat gag gct ctg cac aac cac tac acg cag aag agc ctc tcc ctg     672
   Met His Glu Ala Leu His Asn His Tyr Thr Gln Lys Ser Leu Ser Leu
       210                 215                 220
   tct ccg ggt aaa                                                     684
   Ser Pro Gly Lys
   225
<210> 2
   <211> 228
   <212> PRT
   <213> HUMAN
<400> 2
   Met Asp Lys Thr His Thr Cys Pro Pro Cys Pro Ala Pro Glu Leu Leu
   1 5 10 15
   Gly Gly Pro Ser Val Phe Leu Phe Pro Pro Lys Pro Lys Asp Thr Leu
   20 25 30
   Met Ile Ser Arg Thr Pro Glu Val Thr Cys Val Val Val Asp Val Ser
   35 40 45
   His Glu Asp Pro Glu Val Lys Phe Asn Trp Tyr Val Asp Gly Val Glu
   50 55 60
   Val His Asn Ala Lys Thr Lys Pro Arg Glu Glu Gln Tyr Asn Ser Thr
   65 70 75 80
   Tyr Arg Val Val Ser Val Leu Thr Val Leu His Gln Asp Trp Leu Asn
   85 90 95
   Gly Lys Glu Tyr Lys Cys Lys Val Ser Asn Lys Ala Leu Pro Ala Pro
   100 105 110
   Ile Glu Lys Thr Ile Ser Lys Ala Lys Gly Gln Pro Arg Glu Pro Gln
   115 120 125
   Val Tyr Thr Leu Pro Pro Ser Arg Asp Glu Leu Thr Lys Asn Gln Val
   130 135 140
   Ser Leu Thr Cys Leu Val Lys Gly Phe Tyr Pro Ser Asp Ile Ala Val
   145 150 155 160
   Glu Trp Glu Ser Asn Gly Gln Pro Glu Asn Asn Tyr Lys Thr Thr Pro
   165 170 175
   Pro Val Leu Asp Ser Asp Gly Ser Phe Phe Leu Tyr Ser Lys Leu Thr
   180 185 190
   Val Asp Lys Ser Arg Trp Gln Gln Gly Asn Val Phe Ser Cys Ser Val
   195 200 205
   Met His Glu Ala Leu His Asn His Tyr Thr Gln Lys Ser Leu Ser Leu
   210 215 220
   Ser Pro Gly Lys
   225
<210> 3
   <211> 36
   <212> PRT
   <213> Artificial Sequence
<220>
   <223> TPO MIMETIC PEPTIDE CONSTRUCT
<220>
   <221> misc_feature
   <222> (18)..(18)
   <223> Methoxy-polyethylene glycol (5000 Dalton)-sulfoacetyl group attached to the sidechain.
<400> 3
   Ile Glu Gly Pro Thr Leu Arg Gln Trp Leu Ala Ala Arg Ala Gly Gly
   1 5 10 15
   Gly Lys Gly Gly Gly Gly Ile Glu Gly Pro Thr Leu Arg Gln Trp Leu
   20 25 30
   Ala Ala Arg Ala
   35
<210> 4
   <211> 36
   <212> PRT
   <213> Artificial Sequence
<220>
   <223> TPO MIMETIC PEPTIDE CONSTRUCT
<220>
   <221> misc_feature
   <222> (18)..(18)
   <223> Methoxy-polyethylene glycol (5000 Dalton)-succinimidyl group attached to the sidechain.
<400> 4
   Ile Glu Gly Pro Thr Leu Arg Gln Trp Leu Ala Ala Arg Ala Gly Gly
   1 5 10 15
   Gly Cys Gly Gly Gly Gly Ile Glu Gly Pro Thr Leu Arg Gln Trp Leu
   20 25 30
   Ala Ala Arg Ala
   35
<210> 5
   <211> 794
   <212> DNA
   <213> Artificial Sequence
<220>
   <223> Fc-TMP
<220>
   <221> CDS
   <222> (39)..(779)
<400> 5
   tctagatttg ttttaactaa ttaaaggagg aataacat atg gac aaa act cac aca      56
                                            Met Asp Lys Thr His Thr
                                            1               5
   tgt cca cct tgt cca gct ccg gaa ctc ctg ggg gga ccg tca gtc ttc       104
   Cys Pro Pro Cys Pro Ala Pro Glu Leu Leu Gly Gly Pro Ser Val Phe
               10                  15                  20
   ctc ttc ccc cca aaa ccc aag gac acc ctc atg atc tcc cgg acc cct       152
   Leu Phe Pro Pro Lys Pro Lys Asp Thr Leu Met Ile Ser Arg Thr Pro
           25                  30                  35
   gag gtc aca tgc gtg gtg gtg gac gtg agc cac gaa gac cct gag gtc       200
   Glu Val Thr Cys Val Val Val Asp Val Ser His Glu Asp Pro Glu Val
       40                  45                  50
   aag ttc aac tgg tac gtg gac ggc gtg gag gtg cat aat gcc aag aca       248
   Lys Phe Asn Trp Tyr Val Asp Gly Val Glu Val His Asn Ala Lys Thr
   55                  60                  65                  70
   aag ccg cgg gag gag cag tac aac agc acg tac cgt gtg gtc agc gtc       296
   Lys Pro Arg Glu Glu Gln Tyr Asn Ser Thr Tyr Arg Val Val Ser Val
                   75                  80                  85
   ctc acc gtc ctg cac cag gac tgg ctg aat ggc aag gag tac aag tgc       344
   Leu Thr Val Leu His Gln Asp Trp Leu Asn Gly Lys Glu Tyr Lys Cys
               90                  95                  100
   aag gtc tcc aac aaa gcc ctc cca gcc ccc atc gag aaa acc atc tcc       392
   Lys Val Ser Asn Lys Ala Leu Pro Ala Pro Ile Glu Lys Thr Ile Ser
           105                 110                 115
   aaa gcc aaa ggg cag ccc cga gaa cca cag gtg tac acc ctg ccc cca       440
   Lys Ala Lys Gly Gln Pro Arg Glu Pro Gln Val Tyr Thr Leu Pro Pro
       120                 125                 130
   tcc cgg gat gag ctg acc aag aac cag gtc agc ctg acc tgc ctg gtc       488
   Ser Arg Asp Glu Leu Thr Lys Asn Gln Val Ser Leu Thr Cys Leu Val
   135                 140                 145                 150
   aaa ggc ttc tat ccc agc gac atc gcc gtg gag tgg gag agc aat ggg       536
   Lys Gly Phe Tyr Pro Ser Asp Ile Ala Val Glu Trp Glu Ser Asn Gly
                   155                 160                 165
   cag ccg gag aac aac tac aag acc acg cct ccc gtg ctg gac tcc gac       584
   Gln Pro Glu Asn Asn Tyr Lys Thr Thr Pro Pro Val Leu Asp Ser Asp
               170                 175                 180
   ggc tcc ttc ttc ctc tac agc aag ctc acc gtg gac aag agc agg tgg       632
   Gly Ser Phe Phe Leu Tyr Ser Lys Leu Thr Val Asp Lys Ser Arg Trp
           185                 190                 195
   cag cag ggg aac gtc ttc tca tgc tcc gtg atg cat gag gct ctg cac       680
   Gln Gln Gly Asn Val Phe Ser Cys Ser Val Met His Glu Ala Leu His
       200                 205                 210
   aac cac tac acg cag aag agc ctc tcc ctg tct ccg ggt aaa ggt gga       728
   Asn His Tyr Thr Gln Lys Ser Leu Ser Leu Ser Pro Gly Lys Gly Gly
   215                 220                 225                 230
   ggt ggt ggt atc gaa ggt ccg act ctg cgt cag tgg ctg gct gct cgt       776
   Gly Gly Gly Ile Glu Gly Pro Thr Leu Arg Gln Trp Leu Ala Ala Arg
                   235                 240                 245
   gct taatctcgag gatcc                                                  794
   Ala
<210> 6
   <211> 247
   <212> PRT
   <213> Artificial Sequence
<220>
   <223> Synthetic Construct
<400> 6
   Met Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
   35 40 45
   His Glu Asp Pro Glu Val Lys Phe Asn Trp Tyr Val Asp Gly Val Glu
   50 55 60
   Val His Asn Ala Lys Thr Lys Pro Arg Glu Glu Gln Tyr Asn Ser Thr
   65 70 75 80
   Tyr Arg Val Val Ser Val Leu Thr Val Leu His Gln Asp Trp Leu Asn
   85 90 95
   Gly Lys Glu Tyr Lys Cys Lys Val Ser Asn Lys Ala Leu Pro Ala Pro
   100 105 110
   Ile Glu Lys Thr Ile Ser Lys Ala Lys Gly Gln Pro Arg Glu Pro Gln
   115 120 125
   Val Tyr Thr Leu Pro Pro Ser Arg Asp Glu Leu Thr Lys Asn Gln Val
   130 135 140
   Ser Leu Thr Cys Leu Val Lys Gly Phe Tyr Pro Ser Asp Ile Ala Val
   145 150 155 160
   Glu Trp Glu Ser Asn Gly Gln Pro Glu Asn Asn Tyr Lys Thr Thr Pro
   165 170 175
   Pro Val Leu Asp Ser Asp Gly Ser Phe Phe Leu Tyr Ser Lys Leu Thr
   180 185 190
   Val Asp Lys Ser Arg Trp Gln Gln Gly Asn Val Phe Ser Cys Ser Val
   195 200 205
   Met His Glu Ala Leu His Asn His Tyr Thr Gln Lys Ser Leu Ser Leu
   210 215 220
   Ser Pro Gly Lys Gly Gly Gly Gly Gly Ile Glu Gly Pro Thr Leu Arg
   225 230 235 240
   Gln Trp Leu Ala Ala Arg Ala
   245
<210> 7
   <211> 861
   <212> DNA
   <213> Artificial Sequence
<220>
   <223> Fc-TMP-TMP
<220>
   <221> CDS
   <222> (39)..(842)
<400> 7
   tctagatttg ttttaactaa ttaaaggagg aataacat atg gac aaa act cac aca      56
                                             Met Asp Lys Thr His Thr
                                             1               5
   tgt cca cct tgt cca gct ccg gaa ctc ctg ggg gga ccg tca gtc ttc       104
   Cys Pro Pro Cys Pro Ala Pro Glu Leu Leu Gly Gly Pro Ser Val Phe
               10                  15                  20
   ctc ttc ccc cca aaa ccc aag gac acc ctc atg atc tcc cgg acc cct       152
   Leu Phe Pro Pro Lys Pro Lys Asp Thr Leu Met Ile Ser Arg Thr Pro
           25                  30                  35
   gag gtc aca tgc gtg gtg gtg gac gtg agc cac gaa gac cct gag gtc       200
   Glu Val Thr Cys Val Val Val Asp Val Ser His Glu Asp Pro Glu Val
       40                  45                  50
   aag ttc aac tgg tac gtg gac ggc gtg gag gtg cat aat gcc aag aca       248
   Lys Phe Asn Trp Tyr Val Asp Gly Val Glu Val His Asn Ala Lys Thr
   55                  60                  65                  70
   aag ccg cgg gag gag cag tac aac agc acg tac cgt gtg gtc agc gtc       296
   Lys Pro Arg Glu Glu Gln Tyr Asn Ser Thr Tyr Arg Val Val Ser Val
                   75                  80                  85
   ctc acc gtc ctg cac cag gac tgg ctg aat ggc aag gag tac aag tgc       344
   Leu Thr Val Leu His Gln Asp Trp Leu Asn Gly Lys Glu Tyr Lys Cys
               90                  95                  100
   aag gtc tcc aac aaa gcc ctc cca gcc ccc atc gag aaa acc atc tcc       392
   Lys Val Ser Asn Lys Ala Leu Pro Ala Pro Ile Glu Lys Thr Ile Ser
           105                 110                 115
   aaa gcc aaa ggg cag ccc cga gaa cca cag gtg tac acc ctg ccc cca       440
   Lys Ala Lys Gly Gln Pro Arg Glu Pro Gln Val Tyr Thr Leu Pro Pro
       120                 125                 130
   tcc cgg gat gag ctg acc aag aac cag gtc agc ctg acc tgc ctg gtc       488
   Ser Arg Asp Glu Leu Thr Lys Asn Gln Val Ser Leu Thr Cys Leu Val
   135                 140                 145                 150
   aaa ggc ttc tat ccc agc gac atc gcc gtg gag tgg gag agc aat ggg       536
   Lys Gly Phe Tyr Pro Ser Asp Ile Ala Val Glu Trp Glu Ser Asn Gly
                   155                 160                 165
   cag ccg gag aac aac tac aag acc acg cct ccc gtg ctg gac tcc gac       584
   Gln Pro Glu Asn Asn Tyr Lys Thr Thr Pro Pro Val Leu Asp Ser Asp
               170                 175                 180
   ggc tcc ttc ttc ctc tac agc aag ctc acc gtg gac aag agc agg tgg       632
   Gly Ser Phe Phe Leu Tyr Ser Lys Leu Thr Val Asp Lys Ser Arg Trp
           185                 190                 195
   cag cag ggg aac gtc ttc tca tgc tcc gtg atg cat gag gct ctg cac       680
   Gln Gln Gly Asn Val Phe Ser Cys Ser Val Met His Glu Ala Leu His
       200                 205                 210
   aac cac tac acg cag aag agc ctc tcc ctg tct ccg ggt aaa ggt gga       728
   Asn His Tyr Thr Gln Lys Ser Leu Ser Leu Ser Pro Gly Lys Gly Gly
   215                 220                 225                 230
   ggt ggt ggt atc gaa ggt ccg act ctg cgt cag tgg ctg gct gct cgt       776
   Gly Gly Gly Ile Glu Gly Pro Thr Leu Arg Gln Trp Leu Ala Ala Arg
                   235                 240                 245
   gct ggt ggt gga ggt ggc ggc gga ggt att gag ggc cca acc ctt cgc       824
   Ala Gly Gly Gly Gly Gly Gly Gly Gly Ile Glu Gly Pro Thr Leu Arg
               250                 255                 260
   caa tgg ctt gca gca cgc gcataatctc gaggatccg                          861
   Gln Trp Leu Ala Ala Arg
           265
<210> 8
   <211> 268
   <212> PRT
   <213> Artificial Sequence
<220>
   <223> Synthetic Construct
<400> 8
   Met Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Lys Phe Asn Trp Tyr Val Asp Gly Val Glu
       50                  55                  60
   Val His Asn Ala Lys Thr Lys Pro Arg Glu Glu Gln Tyr Asn Ser Thr
   65                  70                  75                  80
   Tyr Arg Val Val Ser Val Leu Thr Val Leu His Gln Asp Trp Leu Asn
                   85                  90                  95
   Gly Lys Glu Tyr Lys Cys Lys Val Ser Asn Lys Ala Leu Pro Ala Pro
               100                 105                 110
   Ile Glu Lys Thr Ile Ser Lys Ala Lys Gly Gln Pro Arg Glu Pro Gln
           115                 120                 125
   Val Tyr Thr Leu Pro Pro Ser Arg Asp Glu Leu Thr Lys Asn Gln Val
       130                 135                 140
   Ser Leu Thr Cys Leu Val Lys Gly Phe Tyr Pro Ser Asp Ile Ala Val
   145                 150                 155                 160
   Glu Trp Glu Ser Asn Gly Gln Pro Glu Asn Asn Tyr Lys Thr Thr Pro
                   165                 170                 175
   Pro Val Leu Asp Ser Asp Gly Ser Phe Phe Leu Tyr Ser Lys Leu Thr
               180                 185                 190
   Val Asp Lys Ser Arg Trp Gln Gln Gly Asn Val Phe Ser Cys Ser Val
           195                 200                 205
   Met His Glu Ala Leu His Asn His Tyr Thr Gln Lys Ser Leu Ser Leu
       210                 215                 220
   Ser Pro Gly Lys Gly Gly Gly Gly Gly Ile Glu Gly Pro Thr Leu Arg
   225                 230                 235                 240
   Gln Trp Leu Ala Ala Arg Ala Gly Gly Gly Gly Gly Gly Gly Gly Ile
                   245                 250                 255
   Glu Gly Pro Thr Leu Arg Gln Trp Leu Ala Ala Arg
               260                 265
<210> 9
   <211> 855
   <212> DNA
   <213> Artificial Sequence
<220>
   <223> TMP-TMP-Fc
<220>
   <221> CDS
   <222> (39)..(845)
<400> 9
   tctagatttg ttttaactaa ttaaaggagg aataacat atg atc gaa ggt ccg act      56
                                            Met Ile Glu Gly Pro Thr
                                            1               5
   ctg cgt cag tgg ctg gct gct cgt gct ggc ggt ggt ggc gga ggg ggt       104
   Leu Arg Gln Trp Leu Ala Ala Arg Ala Gly Gly Gly Gly Gly Gly Gly
               10                  15                  20
   ggc att gag ggc cca acc ctt cgc caa tgg ctt gca gca cgc gca ggg       152
   Gly Ile Glu Gly Pro Thr Leu Arg Gln Trp Leu Ala Ala Arg Ala Gly
           25                  30                  35
   gga ggc ggt ggg gac aaa act cac aca tgt cca cct tgc cca gca cct       200
   Gly Gly Gly Gly Asp Lys Thr His Thr Cys Pro Pro Cys Pro Ala Pro
       40                  45                  50
   gaa ctc ctg ggg gga ccg tca gtt ttc ctc ttc ccc cca aaa ccc aag       248
   Glu Leu Leu Gly Gly Pro Ser Val Phe Leu Phe Pro Pro Lys Pro Lys
   55                  60                  65                  70
   gac acc ctc atg atc tcc cgg acc cct gag gtc aca tgc gtg gtg gtg       296
   Asp Thr Leu Met Ile Ser Arg Thr Pro Glu Val Thr Cys Val Val Val
                   75                  80                  85
   gac gtg agc cac gaa gac cct gag gtc aag ttc aac tgg tac gtg gac     344
   Asp Val Ser His Glu Asp Pro Glu Val Lys Phe Asn Trp Tyr Val Asp
               90                  95                  100
   ggc gtg gag gtg cat aat gcc aag aca aag ccg cgg gag gag cag tac     392
   Gly Val Glu Val His Asn Ala Lys Thr Lys Pro Arg Glu Glu Gln Tyr
           105                 110                 115
   aac agc acg tac cgt gtg gtc agc gtc ctc acc gtc ctg cac cag gac     440
   Asn Ser Thr Tyr Arg Val Val Ser Val Leu Thr Val Leu His Gln Asp
       120                 125                 130
   tgg ctg aat ggc aag gag tac aag tgc aag gtc tcc aac aaa gcc ctc     488
   Trp Leu Asn Gly Lys Glu Tyr Lys Cys Lys Val Ser Asn Lys Ala Leu
   135                 140                 145                 150
   cca gcc ccc atc gag aaa acc atc tcc aaa gcc aaa ggg cag ccc cga     536
   Pro Ala Pro Ile Glu Lys Thr Ile Ser Lys Ala Lys Gly Gln Pro Arg
                   155                 160                 165
   gaa cca cag gtg tac acc ctg ccc cca tcc cgg gat gag ctg acc aag     584
   Glu Pro Gln Val Tyr Thr Leu Pro Pro Ser Arg Asp Glu Leu Thr Lys
               170                 175                 180
   aac cag gtc agc ctg acc tgc ctg gtc aaa ggc ttc tat ccc agc gac     632
   Asn Gln Val Ser Leu Thr Cys Leu Val Lys Gly Phe Tyr Pro Ser Asp
           185                 190                 195
   atc gcc gtg gag tgg gag agc aat ggg cag ccg gag aac aac tac aag     680
   Ile Ala Val Glu Trp Glu Ser Asn Gly Gln Pro Glu Asn Asn Tyr Lys
       200                 205                 210
   acc acg cct ccc gtg ctg gac tcc gac ggc tcc ttc ttc ctc tac agc     728
   Thr Thr Pro Pro Val Leu Asp Ser Asp Gly Ser Phe Phe Leu Tyr Ser
   215                 220                 225                 230
   aag ctc acc gtg gac aag agc agg tgg cag cag ggg aac gtc ttc tca     776
   Lys Leu Thr Val Asp Lys Ser Arg Trp Gln Gln Gly Asn Val Phe Ser
                   235                 240                 245
   tgc tcc gtg atg cat gag gct ctg cac aac cac tac acg cag aag agc     824
   Cys Ser Val Met His Glu Ala Leu His Asn His Tyr Thr Gln Lys Ser
               250                 255                 260
   ctc tcc ctg tct ccg ggt aaa taatggatcc                               855
   Leu Ser Leu Ser Pro Gly Lys
           265
<210> 10
   <211> 269
   <212> PRT
   <213> Artificial Sequence
<220>
   <223> Synthetic Construct
<400> 10
   Met Ile Glu Gly Pro Thr Leu Arg Gln Trp Leu Ala Ala Arg Ala Gly
   1               5                   10                  15
   Gly Gly Gly Gly Gly Gly Gly Ile Glu Gly Pro Thr Leu Arg Gln Trp
   20 25 30
   Leu Ala Ala Arg Ala Gly Gly Gly Gly Gly Asp Lys Thr His Thr Cys
   35 40 45
   Pro Pro Cys Pro Ala Pro Glu Leu Leu Gly Gly Pro Ser Val Phe Leu
   50 55 60
   Phe Pro Pro Lys Pro Lys Asp Thr Leu Met Ile Ser Arg Thr Pro Glu
   65 70 75 80
   Val Thr Cys Val Val Val Asp Val Ser His Glu Asp Pro Glu Val Lys
   85 90 95
   Phe Asn Trp Tyr Val Asp Gly Val Glu Val His Asn Ala Lys Thr Lys
   100 105 110
   Pro Arg Glu Glu Gln Tyr Asn Ser Thr Tyr Arg Val Val Ser Val Leu
   115 120 125
   Thr Val Leu His Gln Asp Trp Leu Asn Gly Lys Glu Tyr Lys Cys Lys
   130 135 140
   Val Ser Asn Lys Ala Leu Pro Ala Pro Ile Glu Lys Thr Ile Ser Lys
   145 150 155 160
   Ala Lys Gly Gln Pro Arg Glu Pro Gln Val Tyr Thr Leu Pro Pro Ser
   165 170 175
   Arg Asp Glu Leu Thr Lys Asn Gln Val Ser Leu Thr Cys Leu Val Lys
   180 185 190
   Gly Phe Tyr Pro Ser Asp Ile Ala Val Glu Trp Glu Ser Asn Gly Gln
   195 200 205
   Pro Glu Asn Asn Tyr Lys Thr Thr Pro Pro Val Leu Asp Ser Asp Gly
   210 215 220
   Ser Phe Phe Leu Tyr Ser Lys Leu Thr Val Asp Lys Ser Arg Trp Gln
   225 230 235 240
   Gln Gly Asn Val Phe Ser Cys Ser Val Met His Glu Ala Leu His Asn
   245 250 255
   His Tyr Thr Gln Lys Ser Leu Ser Leu Ser Pro Gly Lys
   260 265
<210> 11
   <211> 789
   <212> DNA
   <213> Artificial Sequence
<220>
   <223> TMP-Fc
<220>
   <221> CDS
   <222> (39)..(779)
<400> 11
   tctagatttg ttttaactaa ttaaaggagg aataacat atg atc gaa ggt ccg act      56
                                             Met Ile Glu Gly Pro Thr
                                             1               5
   ctg cgt cag tgg ctg gct gct cgt gct ggt gga ggc ggt ggg gac aaa       104
   Leu Arg Gln Trp Leu Ala Ala Arg Ala Gly Gly Gly Gly Gly Asp Lys
               10                  15                  20
   act cac aca tgt cca cct tgc cca gca cct gaa ctc ctg ggg gga ccg       152
   Thr His Thr Cys Pro Pro Cys Pro Ala Pro Glu Leu Leu Gly Gly Pro
           25                  30                  35
   tca gtt ttc ctc ttc ccc cca aaa ccc aag gac acc ctc atg atc tcc       200
   Ser Val Phe Leu Phe Pro Pro Lys Pro Lys Asp Thr Leu Met Ile Ser
       40                  45                  50
   cgg acc cct gag gtc aca tgc gtg gtg gtg gac gtg agc cac gaa gac       248
   Arg Thr Pro Glu Val Thr Cys Val Val Val Asp Val Ser His Glu Asp
   55                  60                  65                  70
   cct gag gtc aag ttc aac tgg tac gtg gac ggc gtg gag gtg cat aat       296
   Pro Glu Val Lys Phe Asn Trp Tyr Val Asp Gly Val Glu Val His Asn
                   75                  80                  85
   gcc aag aca aag ccg cgg gag gag cag tac aac agc acg tac cgt gtg       344
   Ala Lys Thr Lys Pro Arg Glu Glu Gln Tyr Asn Ser Thr Tyr Arg Val
               90                  95                  100
   gtc agc gtc ctc acc gtc ctg cac cag gac tgg ctg aat ggc aag gag       392
   Val Ser Val Leu Thr Val Leu His Gln Asp Trp Leu Asn Gly Lys Glu
           105                 110                 115
   tac aag tgc aag gtc tcc aac aaa gcc ctc cca gcc ccc atc gag aaa       440
   Tyr Lys Cys Lys Val Ser Asn Lys Ala Leu Pro Ala Pro Ile Glu Lys
       120                 125                 130
   acc atc tcc aaa gcc aaa ggg cag ccc cga gaa cca cag gtg tac acc       488
   Thr Ile Ser Lys Ala Lys Gly Gln Pro Arg Glu Pro Gln Val Tyr Thr
   135                 140                 145                 150
   ctg ccc cca tcc cgg gat gag ctg acc aag aac cag gtc agc ctg acc       536
   Leu Pro Pro Ser Arg Asp Glu Leu Thr Lys Asn Gln Val Ser Leu Thr
                   155                 160                 165
   tgc ctg gtc aaa ggc ttc tat ccc agc gac atc gcc gtg gag tgg gag       584
   Cys Leu Val Lys Gly Phe Tyr Pro Ser Asp Ile Ala Val Glu Trp Glu
               170                 175                 180
   agc aat ggg cag ccg gag aac aac tac aag acc acg cct ccc gtg ctg       632
   Ser Asn Gly Gln Pro Glu Asn Asn Tyr Lys Thr Thr Pro Pro Val Leu
       185                     190                 195
   gac tcc gac ggc tcc ttc ttc ctc tac agc aag ctc acc gtg gac aag      680
   Asp Ser Asp Gly Ser Phe Phe Leu Tyr Ser Lys Leu Thr Val Asp Lys
       200                 205                     210
   agc agg tgg cag cag ggg aac gtc ttc tca tgc tcc gtg atg cat gag      728
   Ser Arg Trp Gln Gln Gly Asn Val Phe Ser Cys Ser Val Met His Glu
   215                 220                     225                 230
   gct ctg cac aac cac tac acg cag aag agc ctc tcc ctg tct ccg ggt      776
   Ala Leu His Asn His Tyr Thr Gln Lys Ser Leu Ser Leu Ser Pro Gly
                   235                     240                 245
   aaa taatggatcc                                                       789
   Lys
<210> 12
   <211> 247
   <212> PRT
   <213> Artificial Sequence
<220>
   <223> Synthetic Construct
<400> 12
   Met Ile Glu Gly Pro Thr Leu Arg Gln Trp Leu Ala Ala Arg Ala Gly
   1               5                   10                  15
   Gly Gly Gly Gly Asp Lys Thr His Thr Cys Pro Pro Cys Pro Ala Pro
               20                  25                  30
   Glu Leu Leu Gly Gly Pro Ser Val Phe Leu Phe Pro Pro Lys Pro Lys
           35                  40                  45
   Asp Thr Leu Met Ile Ser Arg Thr Pro Glu Val Thr Cys Val Val Val
       50                  55                  60
   Asp Val Ser His Glu Asp Pro Glu Val Lys Phe Asn Trp Tyr Val Asp
   65                  70                  75                  80
   Gly Val Glu Val His Asn Ala Lys Thr Lys Pro Arg Glu Glu Gln Tyr
                   85                  90                  95
   Asn Ser Thr Tyr Arg Val Val Ser Val Leu Thr Val Leu His Gln Asp
               100                 105                 110
   Trp Leu Asn Gly Lys Glu Tyr Lys Cys Lys Val Ser Asn Lys Ala Leu
           115                 120                 125
   Pro Ala Pro Ile Glu Lys Thr Ile Ser Lys Ala Lys Gly Gln Pro Arg
       130                 135                 140
   Glu Pro Gln Val Tyr Thr Leu Pro Pro Ser Arg Asp Glu Leu Thr Lys
   145 150 155 160
   Asn Gln Val Ser Leu Thr Cys Leu Val Lys Gly Phe Tyr Pro Ser Asp
   165 170 175
   Ile Ala Val Glu Trp Glu Ser Asn Gly Gln Pro Glu Asn Asn Tyr Lys
   180 185 190
   Thr Thr Pro Pro Val Leu Asp Ser Asp Gly Ser Phe Phe Leu Tyr Ser
   195 200 205
   Lys Leu Thr Val Asp Lys Ser Arg Trp Gln Gln Gly Asn Val Phe Ser
   210 215 220
   Cys Ser Val Met His Glu Ala Leu His Asn His Tyr Thr Gln Lys Ser
   225 230 235 240
   Leu Ser Leu Ser Pro Gly Lys
   245
<210> 13
   <211> 14
   <212> PRT
   <213> Artificial Sequence
<220>
   <223> TMP
<400> 13
   Ile Glu Gly Pro Thr Leu Arg Gln Trp Leu Ala Ala Arg Ala
   1 5 10
<210> 14
   <211> 36
   <212> PRT
   <213> Artificial Sequence
<220>
   <223> TMP-TMP
<400> 14
   Ile Glu Gly Pro Thr Leu Arg Gln Trp Leu Ala Ala Arg Ala Gly Gly
   1 5 10 15
   Gly Gly Gly Gly Gly Gly Ile Glu Gly Pro Thr Leu Arg Gln Trp Leu
   20 25 30
   Ala Ala Arg Ala
   35
<210> 15
   <211> 812
   <212> DNA
   <213> Artificial Sequence
<220>
   <223> EMP-Fc
<220>
   <221> CDS
   <222> (39)..(797)
<400> 15
   tctagatttg ttttaactaa ttaaaggagg aataacat atg gac aaa act cac aca      56
                                             Met Asp Lys Thr His Thr
                                             1               5
   tgt cca cct tgt cca gct ccg gaa ctc ctg ggg gga ccg tca gtc ttc       104
   Cys Pro Pro Cys Pro Ala Pro Glu Leu Leu Gly Gly Pro Ser Val Phe
               10                  15                  20
   ctc ttc ccc cca aaa ccc aag gac acc ctc atg atc tcc cgg acc cct       152
   Leu Phe Pro Pro Lys Pro Lys Asp Thr Leu Met Ile Ser Arg Thr Pro
           25                  30                  35
   gag gtc aca tgc gtg gtg gtg gac gtg agc cac gaa gac cct gag gtc       200
   Glu Val Thr Cys Val Val Val Asp Val Ser His Glu Asp Pro Glu Val
       40                  45                  50
   aag ttc aac tgg tac gtg gac ggc gtg gag gtg cat aat gcc aag aca       248
   Lys Phe Asn Trp Tyr Val Asp Gly Val Glu Val His Asn Ala Lys Thr
   55                  60                  65                  70
   aag ccg cgg gag gag cag tac aac agc acg tac cgt gtg gtc agc gtc       296
   Lys Pro Arg Glu Glu Gln Tyr Asn Ser Thr Tyr Arg Val Val Ser Val
                   75                  80                  85
   ctc acc gtc ctg cac cag gac tgg ctg aat ggc aag gag tac aag tgc       344
   Leu Thr Val Leu His Gln Asp Trp Leu Asn Gly Lys Glu Tyr Lys Cys
               90                  95                  100
   aag gtc tcc aac aaa gcc ctc cca gcc ccc atc gag aaa acc atc tcc       392
   Lys Val Ser Asn Lys Ala Leu Pro Ala Pro Ile Glu Lys Thr Ile Ser
           105                 110                 115
   aaa gcc aaa ggg cag ccc cga gaa cca cag gtg tac acc ctg ccc cca       440
   Lys Ala Lys Gly Gln Pro Arg Glu Pro Gln Val Tyr Thr Leu Pro Pro
       120                 125                 130
   tcc cgg gat gag ctg acc aag aac cag gtc agc ctg acc tgc ctg gtc       488
   Ser Arg Asp Glu Leu Thr Lys Asn Gln Val Ser Leu Thr Cys Leu Val
   135                 140                 145                 150
   aaa ggc ttc tat ccc agc gac atc gcc gtg gag tgg gag agc aat ggg       536
   Lys Gly Phe Tyr Pro Ser Asp Ile Ala Val Glu Trp Glu Ser Asn Gly
                   155                 160                 165
   cag ccg gag aac aac tac aag acc acg cct ccc gtg ctg gac tcc gac       584
   Gln Pro Glu Asn Asn Tyr Lys Thr Thr Pro Pro Val Leu Asp Ser Asp
               170                 175                 180
   ggc tcc ttc ttc ctc tac agc aag ctc acc gtg gac aag agc agg tgg      632
   Gly Ser Phe Phe Leu Tyr Ser Lys Leu Thr Val Asp Lys Ser Arg Trp
           185                 190                 195
   cag cag ggg aac gtc ttc tca tgc tcc gtg atg cat gag gct ctg cac      680
   Gln Gln Gly Asn Val Phe Ser Cys Ser Val Met His Glu Ala Leu His
       200                 205                 210
   aac cac tac acg cag aag agc ctc tcc ctg tct ccg ggt aaa ggt gga      728
   Asn His Tyr Thr Gln Lys Ser Leu Ser Leu Ser Pro Gly Lys Gly Gly
   215                 220                 225                 230
   ggt ggt ggt gga ggt act tac tct tgc cac ttc ggc ccg ctg act tgg      776
   Gly Gly Gly Gly Gly Thr Tyr Ser Cys His Phe Gly Pro Leu Thr Trp
                   235                 240                 245
   gtt tgc aaa ccg cag ggt ggt taatctcgtg gatcc                         812
   Val Cys Lys Pro Gln Gly Gly
               250
<210> 16
   <211> 253
   <212> PRT
   <213> Artificial Sequence
<220>
   <223> Synthetic Construct
<400> 16
   Met Asp Lys Thr His Thr Cys Pro Pro Cys Pro Ala Pro Glu Leu Leu
   1 5 10 15
   Gly Gly Pro Ser Val Phe Leu Phe Pro Pro Lys Pro Lys Asp Thr Leu
   20 25 30
   Met Ile Ser Arg Thr Pro Glu Val Thr Cys Val Val Val Asp Val Ser
   35 40 45
   His Glu Asp Pro Glu Val Lys Phe Asn Trp Tyr Val Asp Gly Val Glu
   50 55 60
   Val His Asn Ala Lys Thr Lys Pro Arg Glu Glu Gln Tyr Asn Ser Thr
   65 70 75 80
   Tyr Arg Val Val Ser Val Leu Thr Val Leu His Gln Asp Trp Leu Asn
   85 90 95
   Gly Lys Glu Tyr Lys Cys Lys Val Ser Asn Lys Ala Leu Pro Ala Pro
   100 105 110
   Ile Glu Lys Thr Ile Ser Lys Ala Lys Gly Gln Pro Arg Glu Pro Gln
   115 120 125
   Val Tyr Thr Leu Pro Pro Ser Arg Asp Glu Leu Thr Lys Asn Gln Val
   130 135 140
   Ser Leu Thr Cys Leu Val Lys Gly Phe Tyr Pro Ser Asp Ile Ala Val
   145 150 155 160
   Glu Trp Glu Ser Asn Gly Gln Pro Glu Asn Asn Tyr Lys Thr Thr Pro
   165 170 175
   Pro Val Leu Asp Ser Asp Gly Ser Phe Phe Leu Tyr Ser Lys Leu Thr
   180 185 190
   Val Asp Lys Ser Arg Trp Gln Gln Gly Asn Val Phe Ser Cys Ser Val
   195 200 205
   Met His Glu Ala Leu His Asn His Tyr Thr Gln Lys Ser Leu Ser Leu
   210 215 220
   Ser Pro Gly Lys Gly Gly Gly Gly Gly Gly Gly Thr Tyr Ser Cys His
   225 230 235 240
   Phe Gly Pro Leu Thr Trp Val Cys Lys Pro Gln Gly Gly
   245 250
<210> 17
   <211 > 807
   <212> DNA
   <213> Artificial Sequence
<220>
   <223> EMP-Fc
<220>
   <221> CDS
   <222> (39)..(797)
<400> 17
   tctagatttg ttttaactaa ttaaaggagg aataacat atg gga ggt act tac tct      56
                                             Met Gly Gly Thr Tyr Ser
                                             1               5
   tgc cac ttc ggc ccg ctg act tgg gta tgt aag cca caa ggg ggt ggg       104
   Cys His Phe Gly Pro Leu Thr Trp Val Cys Lys Pro Gln Gly Gly Gly
               10                  15                  20
   gga ggc ggg ggg gac aaa act cac aca tgt cca cct tgc cca gca cct       152
   Gly Gly Gly Gly Asp Lys Thr His Thr Cys Pro Pro Cys Pro Ala Pro
           25                  30                  35
   gaa ctc ctg ggg gga ccg tca gtt ttc ctc ttc ccc cca aaa ccc aag       200
   Glu Leu Leu Gly Gly Pro Ser Val Phe Leu Phe Pro Pro Lys Pro Lys
       40                  45                  50
   gac acc ctc atg atc tcc cgg acc cct gag gtc aca tgc gtg gtg gtg       248
   Asp Thr Leu Met Ile Ser Arg Thr Pro Glu Val Thr Cys Val Val Val
   55                  60                  65                  70
   gac gtg agc cac gaa gac cct gag gtc aag ttc aac tgg tac gtg gac        296
   Asp Val Ser His Glu Asp Pro Glu Val Lys Phe Asn Trp Tyr Val Asp
                   75                  80                  85
   ggc gtg gag gtg cat aat gcc aag aca aag ccg cgg gag gag cag tac        344
   Gly Val Glu Val His Asn Ala Lys Thr Lys Pro Arg Glu Glu Gln Tyr
               90                  95                  100
   aac agc acg tac cgt gtg gtc agc gtc ctc acc gtc ctg cac cag gac        392
   Asn Ser Thr Tyr Arg Val Val Ser Val Leu Thr Val Leu His Gln Asp
           105                 110                 115
   tgg ctg aat ggc aag gag tac aag tgc aag gtc tcc aac aaa gcc ctc        440
   Trp Leu Asn Gly Lys Glu Tyr Lys Cys Lys Val Ser Asn Lys Ala Leu
       120                 125                 130
   cca gcc ccc atc gag aaa acc atc tcc aaa gcc aaa ggg cag ccc cga        488
   Pro Ala Pro Ile Glu Lys Thr Ile Ser Lys Ala Lys Gly Gln Pro Arg
   135                 140                 145                 150
   gaa cca cag gtg tac acc ctg ccc cca tcc cgg gat gag ctg acc aag        536
   Glu Pro Gln Val Tyr Thr Leu Pro Pro Ser Arg Asp Glu Leu Thr Lys
                   155                 160                 165
   aac cag gtc agc ctg acc tgc ctg gtc aaa ggc ttc tat ccc agc gac        584
   Asn Gln Val Ser Leu Thr Cys Leu Val Lys Gly Phe Tyr Pro Ser Asp
               170                 175                 180
   atc gcc gtg gag tgg gag agc aat ggg cag ccg gag aac aac tac aag        632
   Ile Ala Val Glu Trp Glu Ser Asn Gly Gln Pro Glu Asn Asn Tyr Lys
           185                 190                 195
   acc acg cct ccc gtg ctg gac tcc gac ggc tcc ttc ttc ctc tac agc        680
   Thr Thr Pro Pro Val Leu Asp Ser Asp Gly Ser Phe Phe Leu Tyr Ser
       200                 205                 210
   aag ctc acc gtg gac aag agc agg tgg cag cag ggg aac gtc ttc tca        728
   Lys Leu Thr Val Asp Lys Ser Arg Trp Gln Gln Gly Asn Val Phe Ser
   215                 220                 225                 230
   tgc tcc gtg atg cat gag gct ctg cac aac cac tac acg cag aag agc        776
   Cys Ser Val Met His Glu Ala Leu His Asn His Tyr Thr Gln Lys Ser
                   235                 240                 245
   ctc tcc ctg tct ccg ggt aaa taatggatcc                                 807
   Leu Ser Leu Ser Pro Gly Lys
               250
<210> 18
   <211> 253
   <212> PRT
   <213> Artificial Sequence
<220>
   <223> Synthetic Construct
<400> 18
   Met Gly Gly Thr Tyr Ser Cys His Phe Gly Pro Leu Thr Trp Val Cys
   1               5                   10                  15
   Lys Pro Gln Gly Gly Gly Gly Gly Gly Gly Asp Lys Thr His Thr Cys
   20 25 30
   Pro Pro Cys Pro Ala Pro Glu Leu Leu Gly Gly Pro Ser Val Phe Leu
   35 40 45
   Phe Pro Pro Lys Pro Lys Asp Thr Leu Met Ile Ser Arg Thr Pro Glu
   50 55 60
   Val Thr Cys Val Val Val Asp Val Ser His Glu Asp Pro Glu Val Lys
   65 70 75 80
   Phe Asn Trp Tyr Val Asp Gly Val Glu Val His Asn Ala Lys Thr Lys
   85 90 95
   Pro Arg Glu Glu Gln Tyr Asn Ser Thr Tyr Arg Val Val Ser Val Leu
   100 105 110
   Thr Val Leu His Gln Asp Trp Leu Asn Gly Lys Glu Tyr Lys Cys Lys
   115 120 125
   Val Ser Asn Lys Ala Leu Pro Ala Pro Ile Glu Lys Thr Ile Ser Lys
   130 135 140
   Ala Lys Gly Gln Pro Arg Glu Pro Gln Val Tyr Thr Leu Pro Pro Ser
   145 150 155 160
   Arg Asp Glu Leu Thr Lys Asn Gln Val Ser Leu Thr Cys Leu Val Lys
   165 170 175
   Gly Phe Tyr Pro Ser Asp Ile Ala Val Glu Trp Glu Ser Asn Gly Gln
   180 185 190
   Pro Glu Asn Asn Tyr Lys Thr Thr Pro Pro Val Leu Asp Ser Asp Gly
   195 200 205
   Ser Phe Phe Leu Tyr Ser Lys Leu Thr Val Asp Lys Ser Arg Trp Gln
   210 215 220
   Gln Gly Asn Val Phe Ser Cys Ser Val Met His Glu Ala Leu His Asn
   225 230 235 240
   His Tyr Thr Gln Lys Ser Leu Ser Leu Ser Pro Gly Lys
   245 250

<210> 19
   <211> 881
   <212> DNA
   <213> Artificial Sequence
<220>
   <223> EMP-EMP-Fc
<220>
   <221> CDS
   <222> (41)..(871)
<400> 19
   tctagatttg agttttaact tttagaagga ggaataaaat atg gga ggt act tac      55
   tct tgc cac ttc ggc cca ctg act tgg gtt tgc aaa ccg cag ggt ggc     103
   ggc ggc ggc ggc ggt ggt acc tat tcc tgt cat ttt ggc ccg ctg acc     151
   tgg gta tgt aag cca caa ggg ggt ggg gga ggc ggg ggg gac aaa act     199
   cac aca tgt cca cct tgc cca gca cct gaa ctc ctg ggg gga ccg tca     247
   gtt ttc ctc ttc ccc cca aaa ccc aag gac acc ctc atg atc tcc cgg     295
   acc cct gag gtc aca tgc gtg gtg gtg gac gtg agc cac gaa gac cct     343
   gag gtc aag ttc aac tgg tac gtg gac ggc gtg gag gtg cat aat gcc     391
   aag aca aag ccg cgg gag gag cag tac aac agc acg tac cgt gtg gtc     439
   agc gtc ctc acc gtc ctg cac cag gac tgg ctg aat ggc aag gag tac     487
   aag tgc aag gtc tcc aac aaa gcc ctc cca gcc ccc atc gag aaa acc     535
   atc tcc aaa gcc aaa ggg cag ccc cga gaa cca cag gtg tac acc ctg     583
   ccc cca tcc cgg gat gag ctg acc aag aac cag gtc agc ctg acc tgc     631
   ctg gtc aaa ggc ttc tat ccc agc gac atc gcc gtg gag tgg gag agc     679
   aat ggg cag ccg gag aac aac tac aag acc acg cct ccc gtg ctg gac 727
   tcc gac ggc tcc ttc ttc ctc tac agc aag ctc acc gtg gac aag agc 775
   agg tgg cag cag ggg aac gtc ttc tca tgc tcc gtg atg cat gag gct 823
   ctg cac aac cac tac acg cag aag agc ctc tcc ctg tct ccg ggt aaa 871
   taatggatcc 881
<210> 20
   <211> 277
   <212> PRT
   <213> Artificial Sequence
<220>
   <223> Synthetic Construct
<400> 20
<210> 21
   <211> 885
   <212> DNA
   <213> Artificial Sequence
<220>
   <223> Fc-EMP-EMP
<220>
   <221> CDS
   <222> (39)..(869)
<400> 21
   tctagatttg ttttaactaa ttaaaggagg aataacat atg gac aaa act cac aca      56
                                            Met Asp Lys Thr His Thr
                                            1               5
   tgt cca cct tgc cca gca cct gaa ctc ctg ggg gga ccg tca gtt ttc      104
   Cys Pro Pro Cys Pro Ala Pro Glu Leu Leu Gly Gly Pro Ser Val Phe
               10                  15                  20
   ctc ttc ccc cca aaa ccc aag gac acc ctc atg atc tcc cgg acc cct      152
   Leu Phe Pro Pro Lys Pro Lys Asp Thr Leu Met Ile Ser Arg Thr Pro
           25                  30                  35
   gag gtc aca tgc gtg gtg gtg gac gtg agc cac gaa gac cct gag gtc      200
   Glu Val Thr Cys Val Val Val Asp Val Ser His Glu Asp Pro Glu Val
       40                  45                  50
   aag ttc aac tgg tac gtg gac ggc gtg gag gtg cat aat gcc aag aca      248
   Lys Phe Asn Trp Tyr Val Asp Gly Val Glu Val His Asn Ala Lys Thr
   55                  60                  65                  70
   aag ccg cgg gag gag cag tac aac agc acg tac cgt gtg gtc agc gtc      296
   Lys Pro Arg Glu Glu Gln Tyr Asn Ser Thr Tyr Arg Val Val Ser Val
                   75                  80                  85
   ctc acc gtc ctg cac cag gac tgg ctg aat ggc aag gag tac aag tgc      344
   Leu Thr Val Leu His Gln Asp Trp Leu Asn Gly Lys Glu Tyr Lys Cys
               90                  95                  100
   aag gtc tcc aac aaa gcc ctc cca gcc ccc atc gag aaa acc atc tcc      392
   Lys Val Ser Asn Lys Ala Leu Pro Ala Pro Ile Glu Lys Thr Ile Ser
           105                 110                 115
   aaa gcc aaa ggg cag ccc cga gaa cca cag gtg tac acc ctg cct cca      440
   Lys Ala Lys Gly Gln Pro Arg Glu Pro Gln Val Tyr Thr Leu Pro Pro
       120                 125                 130
   tcc cgg gat gag ctg acc aag aac cag gtc agc ctg acc tgc ctg gtc      488
   Ser Arg Asp Glu Leu Thr Lys Asn Gln Val Ser Leu Thr Cys Leu Val
   135                 140                 145                 150
   aaa ggc ttc tat ccc agc gac atc gcc gtg gag tgg gag agc aat ggg      536
   Lys Gly Phe Tyr Pro Ser Asp Ile Ala Val Glu Trp Glu Ser Asn Gly
                   155                 160                 165
   cag ccg gag aac aac tac aag acc acg cct ccc gtg ctg gac tcc gac      584
   Gln Pro Glu Asn Asn Tyr Lys Thr Thr Pro Pro Val Leu Asp Ser Asp
               170                 175                 180
   ggc tcc ttc ttc ctc tac agc aag ctc acc gtg gac aag agc agg tgg      632
   Gly Ser Phe Phe Leu Tyr Ser Lys Leu Thr Val Asp Lys Ser Arg Trp
           185                 190                 195
   cag cag ggg aac gtc ttc tca tgc tcc gtg atg cat gag gct ctg cac      680
   Gln Gln Gly Asn Val Phe Ser Cys Ser Val Met His Glu Ala Leu His
       200                 205                 210
   aac cac tac acg cag aag agc ctc tcc ctg tct ccg ggt aaa ggt gga      728
   Asn His Tyr Thr Gln Lys Ser Leu Ser Leu Ser Pro Gly Lys Gly Gly
   215                 220                 225                 230
   ggt ggt ggc gga ggt act tac tct tgc cac ttc ggc cca ctg act tgg      776
   Gly Gly Gly Gly Gly Thr Tyr Ser Cys His Phe Gly Pro Leu Thr Trp
                   235                 240                 245
   gtt tgc aaa ccg cag ggt ggc ggc ggc ggc ggc ggt ggt acc tat tcc      824
   Val Cys Lys Pro Gln Gly Gly Gly Gly Gly Gly Gly Gly Thr Tyr Ser
               250                 255                 260
   tgt cat ttt ggc ccg ctg acc tgg gta tgt aag cca caa ggg ggt          869
   Cys His Phe Gly Pro Leu Thr Trp Val Cys Lys Pro Gln Gly Gly
           265                 270                 275
   taatctcgag gatcca                                                    885
<210> 22
   <211> 277
   <212> PRT
   <213> Artificial Sequence
<220>
   <223> Synthetic Construct
<400> 22
   Met Asp Lys Thr His Thr Cys Pro Pro Cys Pro Ala Pro Glu Leu Leu
   1 5 10 15
   Gly Gly Pro Ser Val Phe Leu Phe Pro Pro Lys Pro Lys Asp Thr Leu
   20 25 30
   Met Ile Ser Arg Thr Pro Glu Val Thr Cys Val Val Val Asp Val Ser
   35 40 45
   His Glu Asp Pro Glu Val Lys Phe Asn Trp Tyr Val Asp Gly Val Glu
   50 55 60
   Val His Asn Ala Lys Thr Lys Pro Arg Glu Glu Gln Tyr Asn Ser Thr
   65 70 75 80
   Tyr Arg Val Val Ser Val Leu Thr Val Leu His Gln Asp Trp Leu Asn
   85 90 95
   Gly Lys Glu Tyr Lys Cys Lys Val Ser Asn Lys Ala Leu Pro Ala Pro
   100 105 110
   Ile Glu Lys Thr Ile Ser Lys Ala Lys Gly Gln Pro Arg Glu Pro Gln
   115 120 125
   Val Tyr Thr Leu Pro Pro Ser Arg Asp Glu Leu Thr Lys Asn Gln Val
   130 135 140
   Ser Leu Thr Cys Leu Val Lys Gly Phe Tyr Pro Ser Asp Ile Ala Val
   145 150 155 160
   Glu Trp Glu Ser Asn Gly Gln Pro Glu Asn Asn Tyr Lys Thr Thr Pro
   165 170 175
   Pro Val Leu Asp Ser Asp Gly Ser Phe Phe Leu Tyr Ser Lys Leu Thr
   180 185 190
   Val Asp Lys Ser Arg Trp Gln Gln Gly Asn Val Phe Ser Cys Ser Val
   195 200 205
   Met His Glu Ala Leu His Asn His Tyr Thr Gln Lys Ser Leu Ser Leu
   210 215 220
   Ser Pro Gly Lys Gly Gly Gly Gly Gly Gly Gly Thr Tyr Ser Cys His
   225 230 235 240
   Phe Gly Pro Leu Thr Trp Val Cys Lys Pro Gln Gly Gly Gly Gly Gly
   245 250 255
   Gly Gly Gly Thr Tyr Ser Cys His Phe Gly Pro Leu Thr Trp Val Cys
   260 265 270
   Lys Pro Gln Gly Gly
   275
<210> 23
   <211> 1546
   <212> DNA
   <213> Artificial Sequence
<220>
   <223> pAMG21
<400> 23
   gcgtaacgta tgcatggtct ccccatgcga gagtagggaa ctgccaggca tcaaataaaa      60
   cgaaaggctc agtcgaaaga ctgggccttt cgttttatct gttgtttgtc ggtgaacgct     120
   ctcctgagta ggacaaatcc gccgggagcg gatttgaacg ttgcgaagca acggcccgga     180
   gggtggcggg caggacgccc gccataaact gccaggcatc aaattaagca gaaggccatc     240
   ctgacggatg gcctttttgc gtttctacaa actcttttgt ttatttttct aaatacattc     300
   aaatatggac gtcgtactta acttttaaag tatgggcaat caattgctcc tgttaaaatt     360
   gctttagaaa tactttggca gcggtttgtt gtattgagtt tcatttgcgc attggttaaa     420
   tggaaagtga ccgtgcgctt actacagcct aatatttttg aaatatccca agagcttttt     480
   ccttcgcatg cccacgctaa acattctttt tctcttttgg ttaaatcgtt gtttgattta     540
   ttatttgcta tatttatttt tcgataatta tcaactagag aaggaacaat taatggtatg     600
   ttcatacacg catgtaaaaa taaactatct atatagttgt ctttctctga atgtgcaaaa     660
   ctaagcattc cgaagccatt attagcagta tgaataggga aactaaaccc agtgataaga     720
   cctgatgatt tcgcttcttt aattacattt ggagattttt tatttacagc attgttttca     780
   aatatattcc aattaatcgg tgaatgattg gagttagaat aatctactat aggatcatat     840
   tttattaaat tagcgtcatc ataatattgc ctccattttt tagggtaatt atccagaatt     900
   gaaatatcag atttaaccat agaatgagga taaatgatcg cgagtaaata atattcacaa     960
   tgtaccattt tagtcatatc agataagcat tgattaatat cattattgct tctacaggct    1020
   ttaattttat taattattct gtaagtgtcg tcggcattta tgtctttcat acccatctct    1080
   ttatccttac ctattgtttg tcgcaagttt tgcgtgttat atatcattaa aacggtaata    1140
   gattgacatt tgattctaat aaattggatt tttgtcacac tattatatcg cttgaaatac    1200
   aattgtttaa cataagtacc tgtaggatcg tacaggttta cgcaagaaaa tggtttgtta    1260
   tagtcgatta atcgatttga ttctagattt gttttaacta attaaaggag gaataacata    1320
   tggttaacgc gttggaattc gagctcacta gtgtcgacct gcagggtacc atggaagctt    1380
   actcgaggat ccgcggaaag aagaagaaga agaagaaagc ccgaaaggaa gctgagttgg    1440
   ctgctgccac cgctgagcaa taactagcat aaccccttgg ggcctctaaa cgggtcttga    1500
   ggggtttttt gctgaaagga ggaaccgctc ttcacgctct tcacgc                   1546
<210> 24
   <211> 14
   <212> PRT
   <213> Artificial Sequence
<220>
   <223> TPO-MIMETIC PEPTIDE
<400> 24
   Ile Glu Gly Pro Thr Leu Arg Gln Trp Leu Ala Ala Lys Ala
   1 5 10
<210> 25
   <211> 14
   <212> PRT
   <213> Artificial Sequence
<220>
   <223> TPO-MIMETIC PEPTIDE
<400> 25
   Ile Glu Gly Pro Thr Leu Arg Glu Trp Leu Ala Ala Arg Ala
   1 5 10
<210> 26
   <211> 14
   <212> PRT
   <213> Artificial Sequence
<220>
   <223> TPO-MIMETIC PEPTIDE
<220>
   <221> misc_feature
   <222> (14)..(14)
   <223> At position 14, amino acid linker to an identical sequence
<400> 26
   Ile Glu Gly Pro Thr Leu Arg Gln Trp Leu Ala Ala Arg Ala
   1 5 10
<210> 27
   <211> 14
   <212> PRT
   <213> Artificial Sequence
<220>
   <223> TPO-MIMETIC PEPTIDE
<220>
   <221> misc_feature
   <222> (14)..(14)
   <223> At position 14, amino acid linker to an identical sequence
<400> 27
   Ile Glu Gly Pro Thr Leu Arg Gln Trp Leu Ala Ala Lys Ala
   1 5 10
<210> 28
   <211> 14
   <212> PRT
   <213> Artificial Sequence
<220>
   <223> TPO-MIMETIC PEPTIDE
<220>
   <221> misc_feature
   <222> (9)..(9)
   <223> At position 9 disulfide linkage to position 9 of an identical sequence
<220>
   <221> misc_feature
   <222> (14)..(14)
   <223> At position 14, amino acid linker to an identical sequence
<400> 28
   Ile Glu Gly Pro Thr Leu Arg Gln Cys Leu Ala Ala Arg Ala
   1               5                   10
<210> 29
   <211> 14
   <212> PRT
   <213> Artificial Sequence
<220>
   <223> TPO-MIMETIC PEPTIDE
<220>
   <221> misc_feature
   <222> (14)..(14)
   <223> At position 14, amino acid linker attached N-to-C to Lys and to another linker and an identical sequence
<400> 29
   Ile Glu Gly Pro Thr Leu Arg Gln Trp Leu Ala Ala Arg Ala
   1               5                   10
<210> 30
   <211> 15
   <212> PRT
   <213> Artificial Sequence
<220>
   <223> TPO-MIMETIC PEPTIDE
<220>
   <221> misc_feature
   <222> (14)..(14)
   <223> At position 14, amino acid linker attached N-to-C to Lys and to another linker and an identical sequence;
   polyethylene glycol attached to lys sidechain
<400> 30
<210> 31
   <211> 14
   <212> PRT
   <213> Artificial Sequence
<220>
   <223> TPO-MIMETIC PEPTIDE
<220>
   <221> misc_feature
   <222> (9)..(9)
   <223> Position 9 disulfide bond to residue 9 of a separate identical sequence
<220>
   <221> misc_feature
   <222> (14)..(14)
   <223> At position 14, amino acid linker to an identical sequence
<400> 31
   Ile Glu Gly Pro Thr Leu Arg Gln Cys Leu Ala Ala Arg Ala
   1 5 10
<210> 32
   <211> 14
   <212> PRT
   <213> Artificial Sequence
<220>
   <223> TPO-MIMETIC PEPTIDE
<220>
   <221> misc_feature
   <222> (14)..(14)
   <223> At position 14, amino acid linker attachment site
<400> 32
   Ile Glu Gly Pro Thr Leu Arg Gln Trp Leu Ala Ala Arg Ala
   1 5 10
<210> 33
   <211> 9
   <212> PRT
   <213> Artificial Sequence
<220>
   <223> TPO MIMETIC PEPTIDE
<220>
   <221> misc_feature
   <222> (6, 7 and)..(8)
   <223> Xaa = any amino acid
<400> 33
   Val Arg Asp Gln Ile Xaa Xaa Xaa Leu
   1 5
<210> 34
   <211> 6
   <212> PRT
   <213> Artificial Sequence
<220>
   <223> TPO-MIMETIC PEPTIDE
<400> 34
   Thr Leu Arg Glu Trp Leu
   1 5
<210> 35
   <211> 10
   <212> PRT
   <213> Artificial Sequence
<220>
   <223> TPO-MIMETIC PEPTIDE
<400> 35
   Gly Arg Val Arg Asp Gln Val Ala Gly Trp
   1 5 10
<210> 36
   <211> 10
   <212> PRT
   <213> Artificial Sequence
<220>
   <223> TPO-MIMETIC PEPTIDE
<400> 36
   Gly Arg Val Lys Asp Gln Ile Ala Gln Leu
   1 5 10
<210> 37
   <211> 10
   <212> PRT
   <213> Artificial Sequence
<220>
   <223> TPO-MIMETIC PEPTIDE
<400> 37
   Gly Val Arg Asp Gln Val Ser Trp Ala Leu
   1 5 10
<210> 38
   <211> 10
   <212> PRT
   <213> Artificial Sequence
<220>
   <223> TPO-MIMETIC PEPTIDE
<400> 38
   Glu Ser Val Arg Glu Gln Val Met Lys Tyr
   1               5                   10
<210> 39
   <211> 10
   <212> PRT
   <213> Artificial Sequence
<220>
   <223> TPO-MIMETIC PEPTIDE
<400> 39
   Ser Val Arg Ser Gln Ile Ser Ala Ser Leu
   1               5                   10
<210> 40
   <211> 10
   <212> PRT
   <213> Artificial Sequence
<220>
   <223> TPO-MIMETIC PEPTIDE
<400> 40
   Gly Val Arg Glu Thr Val Tyr Arg His Met
   1               5                   10
<210> 41
   <211> 11
   <212> PRT
   <213> Artificial Sequence
<220>
   <223> TPO-MIMETIC PEPTIDE
<400> 41
   Gly Val Arg Glu Val Ile Val Met His Met Leu
   1               5                   10
<210> 42
   <211> 11
   <212> PRT
   <213> Artificial Sequence
<220>
   <223> TPO-MIMETIC PEPTIDE
<400> 42
   Gly Arg Val Arg Asp Gln Ile Trp Ala Ala Leu
   1 5 10
<210> 43
   <211> 11
   <212> PRT
   <213> Artificial Sequence
<220>
   <223> TPO-MIMETIC PEPTIDE
<400> 43
   Ala Gly Val Arg Asp Gln Ile Leu Ile Trp Leu
   1 5 10
<210> 44
   <211> 11
   <212> PRT
   <213> Artificial Sequence
<220>
   <223> TPO-MIMETIC PEPTIDE
<400> 44
   Gly Arg Val Arg Asp Gln Ile Met Leu Ser Leu
   1 5 10
<210> 45
   <211> 11
   <212> PRT
   <213> Artificial Sequence
<220>
   <223> TPO-MIMETIC PEPTIDE
<220>
   <221> misc_feature
   <222> (8)..(10)
   <223> Xaa = any amino acid
<400> 45
   Gly Arg Val Arg Asp Gln Ile Xaa Xaa Xaa Leu
   1 5 10
<210> 46
   <211> 10
   <212> PRT
   <213> Artificial Sequence
<220>
   <223> TPO-MIMETIC PEPTIDE
<400> 46
   Cys Thr Leu Arg Gln Trp Leu Gln Gly Cys
   1 5 10
<210> 47
   <211> 10
   <212> PRT
   <213> Artificial Sequence
<220>
   <223> TPO-MIMETIC PEPTIDE
<400> 47
   Cys Thr Leu Gln Glu Phe Leu Glu Gly Cys
   1 5 10
<210> 48
   <211> 10
   <212> PRT
   <213> Artificial Sequence
<220>
   <223> TPO-MIMETIC PEPTIDE
<400> 48
   Cys Thr Arg Thr Glu Trp Leu His Gly Cys
   1 5 10
<210> 49
   <211> 12
   <212> PRT
   <213> Artificial Sequence
<220>
   <223> TPO-MIMETIC PEPTIDE
<400> 49
   Cys Thr Leu Arg Glu Trp Leu His Gly Gly Phe Cys
   1 5 10
<210> 50
   <211> 12
   <212> PRT
   <213> Artificial Sequence
<220>
   <223> TPO-MIMETIC PEPTIDE
<400> 50
   Cys Thr Leu Arg Glu Trp Val Phe Ala Gly Leu Cys
   1 5 10
<210> 51
   <211> 13
   <212> PRT
   <213> Artificial Sequence
<220>
   <223> TPO-MIMETIC PEPTIDE
<400> 51
   Cys Thr Leu Arg Gln Trp Leu Ile Leu Leu Gly Met Cys
   1 5 10
<210> 52
   <211> 14
   <212> PRT
   <213> Artificial Sequence
<220>
   <223> TPO-MIMETIC PEPTIDE
<400> 52
   Cys Thr Leu Ala Glu Phe Leu Ala Ser Gly Val Glu Gln Cys
   1 5 10
<210> 53
   <211> 14
   <212> PRT
   <213> Artificial Sequence
<220>
   <223> TPO-MIMETIC PEPTIDE
<400> 53
   Cys Ser Leu Gln Glu Phe Leu Ser His Gly Gly Tyr Val Cys
   1 5 10
<210> 54
   <211> 14
   <212> PRT
   <213> Artificial Sequence
<220>
   <223> TPO-MIMETIC PEPTIDE
<400> 54
   Cys Thr Leu Arg Glu Phe Leu Asp Pro Thr Thr Ala Val Cys
   1 5 10
<210> 55
   <211> 14
   <212> PRT
   <213> Artificial Sequence
<220>
   <223> TPO-MIMETIC PEPTIDE
<400> 55
   Cys Thr Leu Lys Glu Trp Leu Val Ser His Glu Val Trp Cys
   1 5 10
<210> 56
   <211> 10
   <212> PRT
   <213> Artificial Sequence
<220>
   <223> TPO-MIMETIC PEPTIDE
<220>
   <221> misc_feature
   <222> (8).. (9)
   <223> Xaa = any amino acid
<400> 56
   Cys Thr Leu Arg Glu Trp Leu Xaa Xaa Cys
   1 5 10
<210> 57
   <211> 11
   <212> PRT
   <213> Artificial Sequence
<220>
   <223> TPO-MIMETIC PEPTIDE
<220>
   <221> misc_feature
   <222> (8)..(10)
   <223> Xaa = any amino acid
<400> 57
   Cys Thr Leu Arg Glu Trp Leu Xaa Xaa Xaa Cys
   1 5 10
<210> 58
   <211> 12
   <212> PRT
   <213> Artificial Sequence
<220>
   <223> TPO-MIMETIC PEPTIDE
<220>
   <221> misc_feature
   <222> (8)..(11)
   <223> Xaa = any amino acid
<400> 58
   Cys Thr Leu Arg Glu Trp Leu Xaa Xaa Xaa Xaa Cys
   1 5 10
<210> 59
   <211> 13
   <212> PRT
   <213> Artificial Sequence
<220>
   <223> TPO-MIMETIC PEPTIDE
<220>
   <221> misc_feature
   <222> (8)..(12)
   <223> Xaa = any amino acid
<400> 59
   Cys Thr Leu Arg Glu Trp Leu Xaa Xaa Xaa Xaa Xaa Cys
   1 5 10
<210> 60
   <211> 14
   <212> PRT
   <213> Artificial Sequence
<220>
   <223> TPO-MIMETIC PEPTIDE
<220>
   <221> misc_feature
   <222> (8)..(13)
   <223> Xaa = any amino acid
<400> 60
   Cys Thr Leu Arg Glu Trp Leu Xaa Xaa Xaa Xaa Xaa Xaa Cys
   1 5 10
<210> 61
   <211> 10
   <212> PRT
   <213> Artificial Sequence
<220>
   <223> TPO-MIMETIC PEPTIDE
<400> 61
   Arg Glu Gly Pro Thr Leu Arg Gln Trp Met
   1 5 10
<210> 62
   <211> 10
   <212> PRT
   <213> Artificial Sequence
<220>
   <223> TPO-MIMETIC PEPTIDE
<400> 62
   Glu Gly Pro Thr Leu Arg Gln Trp Leu Ala
   1 5 10
<210> 63
   <211> 10
   <212> PRT
   <213> Artificial Sequence
<220>
   <223> TPO-MIMETIC PEPTIDE
<400> 63
   Glu Arg Gly Pro Phe Trp Ala Lys Ala Cys
   1 5 10
<210> 64
   <211> 10
   <212> PRT
   <213> Artificial Sequence
<220>
   <223> TPO-MIMETIC PEPTIDE
<400> 64
   Arg Glu Gly Pro Arg Cys Val Met Trp Met
   1 5 10
<210> 65
   <211> 14
   <212> PRT
   <213> Artificial Sequence

<220>
   <223> TPO-MIMETIC PEPTIDE
<400> 65
   Cys Gly Thr Glu Gly Pro Thr Leu Ser Thr Trp Leu Asp Cys
   1 5 10
<210> 66
   <211> 14
   <212> PRT
   <213> Artificial Sequence
<220>
   <223> TPO-MIMETIC PEPTIDE
<400> 66
   Cys Glu Gln Asp Gly Pro Thr Leu Leu Glu Trp Leu Lys Cys
   1 5 10
<210> 67
   <211> 14
   <212> PRT
   <213> Artificial Sequence
<220>
   <223> TPO-MIMETIC PEPTIDE
<400> 67
   Cys Glu Leu Val Gly Pro Ser Leu Met Ser Trp Leu Thr Cys
   1 5 10
<210> 68
   <211> 14
   <212> PRT
   <213> Artificial Sequence
<220>
   <223> TPO-MIMETIC PEPTIDE
<400> 68
   Cys Leu Thr Gly Pro Phe Val Thr Gln Trp Leu Tyr Glu Cys
   1 5 10
<210> 69
   <211> 14
   <212> PRT
   <213> Artificial Sequence
<220>
   <223> TPO-MIMETIC PEPTIDE
<400> 69
   Cys Arg Ala Gly Pro Thr Leu Leu Glu Trp Leu Thr Leu Cys
   1 5 10
<210> 70
   <211> 14
   <212> PRT
   <213> Artificial Sequence
<220>
   <223> TPO-MIMETIC PEPTIDE
<400> 70
   Cys Ala Asp Gly Pro Thr Leu Arg Glu Trp Ile Ser Phe Cys
   1 5 10
<210> 71
   <211> 13
   <212> PRT
   <213> Artificial Sequence
<220>
   <223> TPO-MIMETIC PEPTIDE
<220>
   <221> misc_feature
   <222> (2)..(12)
   <223> Xaa = any amino acid
<400> 71
   Cys Xaa Glu Gly Pro Thr Leu Arg Glu Trp Leu Xaa Cys
   1 5 10
<210> 72
   <211> 14
   <212> PRT
   <213> Artificial Sequence
<220>
   <223> TPO-MIMETIC PEPTIDE
<220>
   <221> misc_feature
   <222> (2, 3 )..(13)
   <223> Xaa = any amino acid
<400> 72
   Cys Xaa Xaa Glu Gly Pro Thr Leu Arg Glu Trp Leu Xaa Cys
   1 5 10
<210> 73
   <211> 14
   <212> PRT
   <213> Artificial Sequence
<220>
   <223> TPO-MIMETIC PEPTIDE
<220>
   <221> misc_feature
   <222> (2, 12)..(13)
   <223> Xaa = any amino acid
<400> 73
   Cys Xaa Glu Gly Pro Thr Leu Arg Glu Trp Leu Xaa Xaa Cys
   1 5 10
<210> 74
   <211 > 15
   <212> PRT
   <213> Artificial Sequence
<220>
   <223> TPO-MIMETIC PEPTIDE
<220>
   <221> misc_feature
   <222> (2, 3, 13)..(14)
   <223> Xaa = any amino acid
<400> 74
   Cys Xaa Xaa Glu Gly Pro Thr Leu Arg Glu Trp Leu Xaa Xaa Cys
   1 5 10 15
<210> 75
   <211> 16
   <212> PRT
   <213> Artificial Sequence
<220>
   <223> TPO-MIMETIC PEPTIDE
<400> 75
   Gly Gly Cys Thr Leu Arg Glu Trp Leu His Gly Gly Phe Cys Gly Gly
   1 5 10 15
<210> 76
   <211> 18
   <212> PRT
   <213> Artificial Sequence
<220>
   <223> TPO-MIMETIC PEPTIDE
<400> 76
   Gly Gly Cys Ala Asp Gly Pro Thr Leu Arg Glu Trp Ile Ser Phe Cys
   1 5 10 15
   Gly Gly
<210> 77
   <211> 19
   <212> PRT
   <213> Artificial Sequence
<220>
   <223> TPO-MIMETIC PEPTIDE
<400> 77
   Gly Asn Ala Asp Gly Pro Thr Leu Arg Gln Trp Leu Glu Gly Arg Arg
   1 5 10 15
   Pro Lys Asn
<210> 78
   <211> 19
   <212> PRT
   <213> Artificial Sequence
<220>
   <223> TPO-MIMETIC PEPTIDE
<400> 78
   Leu Ala Ile Glu Gly Pro Thr Leu Arg Gln Trp Leu His Gly Asn Gly
   1 5 10 15
   Arg Asp Thr
<210> 79
   <211> 19
   <212> PRT
   <213> Artificial Sequence
<220>
   <223> TPO-MIMETIC PEPTIDE
<400> 79
   His Gly Arg Val Gly Pro Thr Leu Arg Glu Trp Lys Thr Gln Val Ala
   1 5 10 15
   Thr Lys Lys
<210> 80
   <211> 18
   <212> PRT
   <213> Artificial Sequence
<220>
   <223> TPO-MIMETtC PEPTIDE
<400> 80
   Thr Ile Lys Gly Pro Thr Leu Arg Gln Trp Leu Lys Ser Arg Glu His
   1 5 10 15
   Thr Ser
<210> 81
   <211> 18
   <212> PRT
   <213> Artificial Sequence
<220>
   <223> TPO-MIMETIC PEPTIDE
<400> 81
   Ile Ser Asp Gly Pro Thr Leu Lys Glu Trp Leu Ser Val Thr Arg Gly
   1 5 10 15
   Ala Ser
<210> 82
   <211> 18
   <212> PRT
   <213> Artificial Sequence
<220>
   <223> TPO MIMETIC PEPTIDE
<400> 82
   Ser Ile Glu Gly Pro Thr Leu Arg Glu Trp Leu Thr Ser Arg Thr Pro
   1 5 10 15
   His Ser
<210> 83
   <211> 14
   <212> PRT
   <213> Artificial Sequence
<220>
   <223> EPO-MIMETIC PEPTIDE
<220>
   <221> misc_feature
   <222> (2, 4, 5, 8, 11 )..(13)
   <223> Xaa = any amino acid
<400> 83
   Tyr Xaa Cys Xaa Xaa Gly Pro Xaa Thr Trp Xaa Cys Xaa Pro
   1 5 10
<210> 84
   <211> 28
   <212> PRT
   <213> Artificial Sequence
<220>
   <223> EPO-MIMETIC PEPTIDE
<220>
   <221> misc_feature
   <222> (2, 4, 5, 8, 11, 13, 16, 18, 19, 22, 25)..(27)
   <223> Xaa = any amino acid
<400> 84
   Tyr Xaa Cys Xaa Xaa Gly Pro Xaa Thr Trp Xaa Cys Xaa Pro Tyr Xaa
   1 5 10 15
   Cys Xaa Xaa Gly Pro Xaa Thr Trp Xaa Cys Xaa Pro
   20 25
<210> 85
   <211> 14
   <212> PRT
   <213> Artificial Sequence
<220>
   <223> EPO-MIMETIC PEPTIDE
<220>
   <221> misc_feature
   <222> (2, 4, 5, 8, 11)..(13)
   <223> Xaa = any amino acid
<220>
   <221> misc_feature
   <222> (14)..(14)
   <223> At position 14, amino acid linker to an identical sequence
<400> 85
   Tyr Xaa Cys Xaa Xaa Gly Pro Xaa Thr Trp Xaa Cys Xaa Pro
   1               5                   10
<210> 86
   <211> 14
   <212> PRT
   <213> Artificial Sequence
<220>
   <223> EPO-MIMETIC PEPTIDE
<220>
   <221> misc_feature
   <222> (2, 4, 5, 8, 11 )..(13)
   <223> Xaa = any amino acid
<400> 86
   Tyr Xaa Cys Xaa Xaa Gly Pro Xaa Thr Trp Xaa Cys Xaa Pro
   1               5                   10
<210> 87
   <211> 20
   <212> PRT
   <213> Artificial Sequence
<220>
   <223> EPO-MIMETIC PEPTIDE
<400> 87
   Gly Gly Thr Tyr Ser Cys His Phe Gly Pro Leu Thr Trp Val Cys Lys
   1               5                   10                  15
   Pro Gln Gly Gly
               20
<210> 88
   <211> 20
   <212> PRT
   <213> Artificial Sequence
<220>
   <223> EPO-MIMETIC PEPTIDE
<400> 88
   Gly Gly Asp Tyr His Cys Arg Met Gly Pro Leu Thr Trp Val Cys Lys
   1               5                   10                  15
   Pro Leu Gly Gly
               20
<210> 89
   <211> 20
   <212> PRT
   <213> Artificial Sequence
<220>
   <223> EPO-MIMETIC PEPTIDE
<400> 89
   Gly Gly Val Tyr Ala Cys Arg Met Gly Pro Ile Thr Trp Val Cys Ser
   1               5                   10                  15
   Pro Leu Gly Gly
               20
<210> 90
   <211> 20
   <212> PRT
   <213> Artificial Sequence
<220>
   <223> EPO-MIMETIC PEPTIDE
<400> 90
   Val Gly Asn Tyr Met Cys His Phe Gly Pro Ile Thr Trp Val Cys Arg
   1               5                   10                  15
   Pro Gly Gly Gly
               20
<210> 91
   <211> 20
   <212> PRT
   <213> Artificial Sequence
<220>
   <223> EPO-MIMETIC PEPTIDE
<400> 91
   Gly Gly Leu Tyr Leu Cys Arg Phe Gly Pro Val Thr Trp Asp Cys Gly
   1 5 10 15
   Tyr Lys Gly Gly
   20
<210> 92
   <211> 40
   <212> PRT
   <213> Artificial Sequence
<220>
   <223> EPO-MIMETIC PEPTIDE
<400> 92
   Gly Gly Thr Tyr Ser Cys His Phe Gly Pro Leu Thr Trp Val Cys Lys
   1 5 10 15
   Pro Gln Gly Gly Gly Gly Thr Tyr Ser Cys His Phe Gly Pro Leu Thr
   20 25 30
   Trp Val Cys Lys Pro Gln Gly Gly
   35 40
<210> 93
   <211> 20
   <212> PRT
   <213> Artificial Sequence
<220>
   <223> EPO-MIMETIC PEPTIDE
<220>
   <221> misc_feature
   <222> (20)..(20)
   <223> Position 20, amino acid linker to an identical sequence
<400> 93
   Gly Gly Thr Tyr Ser Cys His Phe Gly Pro Leu Thr Trp Val Cys Lys
   1 5 10 15
   Pro Gln Gly Gly
   20
<210> 94
   <211> 23
   <212> PRT
   <213> Artificial Sequence
<220>
   <223> EPO-MIMETIC PEPTIDE
<400> 94
   Gly Gly Thr Tyr Ser Cys His Phe Gly Pro Leu Thr Trp Val Cys Lys
   1               5                   10                  15
   Pro Gln Gly Gly Ser Ser Lys
               20
<210> 95
   <211> 46
   <212> PRT
   <213> Artificial Sequence
<220>
   <223> EPO-MIMETIC PEPTIDE
<400> 95
   Gly Gly Thr Tyr Ser Cys His Phe Gly Pro Leu Thr Trp Val Cys Lys
   1               5                   10                  15
   Pro Gln Gly Gly Ser Ser Lys Gly Gly Thr Tyr Ser Cys His Phe Gly
               20                  25                  30
   Pro Leu Thr Trp Val Cys Lys Pro Gln Gly Gly Ser Ser Lys
           35                  40                  45
<210> 96
   <211> 23
   <212> PRT
   <213> Artificial Sequence
<220>
   <223> EPO-MIMETIC PEPTIDE
<220>
   <221> misc_feature
   <222> (23)..(23)
   <223> Position 23, amino acid linker to an identical sequence
<400> 96
   Gly Gly Thr Tyr Ser Cys His Phe Gly Pro Leu Thr Trp Val Cys Lys
   1               5                   10                  15
   Pro Gln Gly Gly Ser Ser Lys
               20
<210> 97
   <211> 22
   <212> PRT
   <213> Artificial Sequence
<220>
   <223> EPO-MIMETIC PEPTIDE
<220>
   <221> misc_feature
   <222> (22)..(22)
   <223> Position 22 linked through epsilon amine to lysyl, which is linked to a separate identical sequence through
   that sequence's alpha amine
<400> 97
   Gly Gly Thr Tyr Ser Cys His Phe Gly Pro Leu Thr Trp Val Cys Lys
   1 5 10 15
   Pro Gln Gly Gly Ser Ser
   20
<210> 98
   <211> 23
   <212> PRT
   <213> Artificial Sequence
<220>
   <223> EPO-MIMETIC PEPTIDE
<220>
   <221> misc_feature
   <222> (23)..(23)
   <223> At position 23 biotin linked to the sidechain through a linker
<400> 98
   Gly Gly Thr Tyr Ser Cys His Phe Gly Pro Leu Thr Trp Val Cys Lys
   1 5 10 15
   Pro Gln Gly Gly Ser Ser Lys
   20
<210> 99
   <211> 5
   <212> PRT
   <213> Artificial Sequence
<220>
   <223> G-CSF-MIMETIC PEPTIDE
<220>
   <221> misc_feature
   <222> (4)..(4)
   <223> At position 4 disulfide bond to residue 4 of a separate identical sequence
<400> 99
   Glu Glu Asp Cys Lys
   1 5
<210> 100
   <211> 5
   <212> PRT
   <213> Artificial Sequence
<220>
   <223> G-CSF-MIMETIC PEPTIDE
<220>
   <221> misc_feature
   <222> (4)..(4)
   <223> At position 4, Xaa is an isoteric ethylene spacer linked to a separate identical sequence
<400> 100
   Glu Glu Asp Xaa Lys
   1               5
<210> 101
   <211> 6
   <212> PRT
   <213> Artificial Sequence
<220>
   <223> G-CSF-MIMETIC PEPTIDE
<220>
   <221> misc_feature
   <222> (1)..(1)
   <223> Position 1, Xaa is a pyroglutamic acid residue
<220>
   <221> misc_feature
   <222> (5)..(5)
   <223> Position 5, Xaa is an isoteric ethylene spacer linked to a separate identical sequence.
<400> 101
<210> 102
   <211> 5
   <212> PRT
   <213> Artificial Sequence
<220>
   <223> G-CSF-MIMETIC PEPTIDE
<220>
   <221> misc_feature
   <222> (1)..(1)
   <223> Position 1, Xaa is a picolinic acid residue
<220>
   <221> misc_feature
   <222> (4).. (4)
   <223> Position 4, Xaa is an isoteric ethylene spacer linked to a separate identical sequence.
<400> 102
   Xaa Ser Asp Xaa Lys
   1 5
<210> 103
   <211> 5
   <212> PRT
   <213> Artificial Sequence
<220>
   <223> G-CSF-MIMETIC PEPTIDE
<220>
   <221> misc_feature
   <222> (5)..(5)
   <223> At position 5, amino acid linker to an identical sequence
<400> 103
   Glu Glu Asp Cys Lys
   1 5
<210> 104
   <211> 5
   <212> PRT
   <213> Artificial Sequence
<220>
   <223> G-CSF-MIMETIC PEPTIDE
<220>
   <221> misc_feature
   <222> (4).. (4)
   <223> Xaa = any amino acid
<220>
   <221> misc_feature
   <222> (5)..(5)
   <223> At position 5, amino acid linker to an identical sequence
<400> 104
   Glu Glu Asp Xaa Lys
   1 5
<210> 105
   <211> 6
   <212> PRT
   <213> Artificial Sequence
<220>
   <223> ANTIVIRAL (HBV)
<400> 105
   Leu Leu Gly Arg Met Lys
   1 5
<210> 106
   <211> 11
   <212> PRT
   <213> Artificial Sequence
<220>
   <223> TNF ANTAGONIST PEPTIDE
<400> 106
   Tyr Cys Phe Thr Ala Ser Glu Asn His Cys Tyr
   1               5                   10
<210> 107
   <211> 11
   <212> PRT
   <213> Artificial Sequence
<220>
   <223> TNF ANTAGONIST PEPTIDE
<400> 107
   Tyr Cys Phe Thr Asn Ser Glu Asn His Cys Tyr
   1               5                   10
<210> 108
   <211> 11
   <212> PRT
   <213> Artificial Sequence
<220>
   <223> TNF ANTAGONIST PEPTIDE
<400> 108
   Tyr Cys Phe Thr Arg Ser Glu Asn His Cys Tyr
   1               5                   10
<210> 109
   <211> 9
   <212> PRT
   <213> Artificial Sequence
<220>
   <223> TNF ANTAGONIST PEPTIDE
<400> 109
   Phe Cys Ala Ser Glu Asn His Cys Tyr
   1               5
<210> 110
   <211> 9
   <212> PRT
   <213> Artificial Sequence
<220>
   <223> TNF ANTAGONIST PEPTIDE
<400> 110
   Tyr Cys Ala Ser Glu Asn His Cys Tyr
   1 5
<210> 111
   <211> 9
   <212> PRT
   <213> Artificial Sequence
<220>
   <223> TNF ANTAGONIST PEPTIDE
<400> 111
   Phe Cys Asn Ser Glu Asn His Cys Tyr
   1 5
<210> 112
   <211> 9
   <212> PRT
   <213> Artificial Sequence
<220>
   <223> TNF ANTAGONIST PEPTIDE
<400> 112
   Phe Cys Asn Ser Glu Asn Arg Cys Tyr
   1 5
<210> 113
   <211> 10
   <212> PRT
   <213> Artificial Sequence
<220>
   <223> TNF ANTAGONIST PEPTIDE
<400> 113
   Phe Cys Asn Ser Val Glu Asn Arg Cys Tyr
   1 5 10
<210> 114
   <211> 11
   <212> PRT
   <213> Artificial Sequence
<220>
   <223> TNF ANTAGONIST PEPTIDE
<400> 114
   Tyr Cys Ser Gln Ser Val Ser Asn Asp Cys Phe
   1 5 10
<210> 115
   <211> 9
   <212> PRT
   <213> Artificial Sequence
<220>
   <223> TNF ANTAGONIST PEPTIDE
<400> 115
   Phe Cys Val Ser Asn Asp Arg Cys Tyr
   1 5
<210> 116
   <211> 11
   <212> PRT
   <213> Artificial Sequence
<220>
   <223> TNF ANTAGONIST PEPTIDE
<400> 116
   Tyr Cys Arg Lys Glu Leu Gly Gln Val Cys Tyr
   1 5 10
<210> 117
   <211> 9
   <212> PRT
   <213> Artificial Sequence
<220>
   <223> TNF ANTAGONIST PEPTIDE
<400> 117
   Tyr Cys Lys Glu Pro Gly Gln Cys Tyr
   1 5
<210> 118
   <211> 9
   <212> PRT
   <213> Artificial Sequence
<220>
   <223> TNF ANTAGONIST PEPTIDE
<400> 118
   Tyr Cys Arg Lys Glu Met Gly Cys Tyr
   1 5
<210> 119
   <211> 9
   <212> PRT
   <213> Artificial Sequence
<220>
   <223> TNF ANTAGONIST PEPTIDE
<400> 119
   Phe Cys Arg Lys Glu Met Gly Cys Tyr
   1 5
<210> 120
   <211> 9
   <212> PRT
   <213> Artificial Sequence
<220>
   <223> TNF ANTAGONIST PEPTIDE
<400> 120
   Tyr Cys Trp Ser Gln Asn Leu Cys Tyr
   1 5
<210> 121
   <211> 10
   <212> PRT
   <213> Artificial Sequence
<220>
   <223> TNF ANTAGONIST PEPTIDE
<400> 121
   Tyr Cys Glu Leu Ser Gln Tyr Leu Cys Tyr
   1 5 10
<210> 122
   <211> 9
   <212> PRT
   <213> Artificial Sequence
<220>
   <223> TNF ANTAGONIST PEPTIDE
<400> 122
   Tyr Cys Trp Ser Gln Asn Tyr Cys Tyr
   1 5
<210> 123
   <211> 9
   <212> PRT
   <213> Artificial Sequence
<220>
   <223> TNF ANTAGONIST PEPTIDE
<400> 123
   Tyr Cys Trp Ser Gln Tyr Leu Cys Tyr
   1 5
<210> 124
   <211> 10
   <212> PRT
   <213> Artificial Sequence
<220>
   <223> EPO-MIMETIC PEPTIDE
<220>
   <221> misc_feature
   <222> (1)..(1)
   <223> Xaa (Pos1) can be C, A, α-amino-g-bromobutyric acid or Hoc.
<220>
   <221> misc_feature
   <222> (2)..(2)
   <223> Xaa can be R, H, L or W.
<220>
   <221> misc_feature
   <222> (3)..(3)
   <223> Xaa can be M, F or I.
<220>
   <221> misc_feature
   <222> (6)..(6)
   <223> Xaa can be any one of the 20 L-amino acids or the stereoisomeric D-amino acids.
<220>
   <221> misc_feature
   <222> (9)..(9)
   <223> Xaa can be D, E, I, L or V.
<220>
   <221> misc_feature
   <222> (10)..(10)
   <223> Xaa can be α-amino-g-bromobutyric acid or Hoc, provided that either Xaa (Pos1) or Xaa (Pos10) is C or Hoc.
<400> 124
   Xaa Xaa Xaa Gly Pro Xaa Thr Trp Xaa Xaa
   1 5 10
<210> 125
   <211> 15
   <212> PRT
   <213> Artificial Sequence
<220>
   <223> CTLA4-MIMETIC
<400> 125
   Gly Phe Val Cys Ser Gly Ile Phe Ala Val Gly Val Gly Arg Cys
   1 5 10 15
<210> 126
   <211> 15
   <212> PRT
   <213> Artificial Sequence
<220>
   <223> CTLA4-MIMETIC
<400> 126
   Ala Pro Gly Val Arg Leu Gly Cys Ala Val Leu Gly Arg Tyr Cys
   1 5 10 15
<210> 127
   <211> 27
   <212> PRT
   <213> Artificial Sequence
<220>
   <223> C3B ANTAGONIST
<400> 127
   Ile Cys Val Val Gln Asp Trp Gly His His Arg Cys Thr Ala Gly His
   1 5 10 15
   Met Ala Asn Leu Thr Ser His Ala Ser Ala Ile
   20 25
<210> 128
   <211> 13
   <212> PRT
   <213> Artificial Sequence
<220>
   <223> C3B ANTAGONIST
<400> 128
   Ile Cys Val Val Gln Asp Trp Gly His His Arg Cys Thr
   1 5 10

<210> 129
   <211> 11
   <212> PRT
   <213> Artificial Sequence
<220>
   <223> C3B ANTAGONIST
<400> 129
   Cys Val Val Gln Asp Trp Gly His His Ala Cys
   1 5 10
<210> 130
   <211> 6
   <212> PRT
   <213> Artificial Sequence
<220>
   <223> MDM/HDM ANTAGONIST PEPTIDE
<400> 130
   Thr Phe Ser Asp Leu Trp
   1 5
<210> 131
   <211> 12
   <212> PRT
   <213> Artificial Sequence
<220>
   <223> MDM/HDM ANTAGONIST PEPTIDE
<400> 131
   Gln Glu Thr Phe Ser Asp Leu Trp Lys Leu Leu Pro
   1 5 10
<210> 132
   <211> 12
   <212> PRT
   <213> Artificial Sequence
<220>
   <223> MDM/HDM ANTAGONIST PEPTIDE
<400> 132
   Gln Pro Thr Phe Ser Asp Leu Trp Lys Leu Leu Pro
   1 5 10
<210> 133
   <211> 12
   <212> PRT
   <213> Artificial Sequence
<220>
   <223> MDM/HDM ANTAGONIST PEPTIDE
<400> 133
   Gln Glu Thr Phe Ser Asp Tyr Trp Lys Leu Leu Pro
   1 5 10
<210> 134
   <211> 12
   <212> PRT
   <213> Artificial Sequence
<220>
   <223> MDM/HDM ANTAGONIST PEPTIDE
<400> 134
   Gln Pro Thr Phe Ser Asp Tyr Trp Lys Leu Leu Pro
   1               5                   10
<210> 135
   <211> 12
   <212> PRT
   <213> Artificial Sequence
<220>
   <223> MDM/HDM ANTAGONIST PEPTIDE
<400> 135
   Met Pro Arg Phe Met Asp Tyr Trp Glu Gly Leu Asn
   1               5                   10
<210> 136
   <211> 12
   <212> PRT
   <213> Artificial Sequence
<220>
   <223> MDM/HDM ANTAGONIST PEPTIDE
<400> 136
   Val Gln Asn Phe Ile Asp Tyr Trp Thr Gln Gln Phe
   1               5                   10
<210> 137
   <211> 12
   <212> PRT
   <213> Artificial Sequence
<220>
   <223> MDM/HDM ANTAGONIST PEPTIDE
<400> 137
   Thr Gly Pro Ala Phe Thr His Tyr Trp Ala Thr Phe
   1               5                   10
<210> 138
   <211> 15
   <212> PRT
   <213> Artificial Sequence
<220>
   <223> MDM/HDM ANTAGONIST PEPTIDE
<400> 138
   Ile Asp Arg Ala Pro Thr Phe Arg Asp His Trp Phe Ala Leu Val
   1 5 10 15
<210> 139
   <211> 15
   <212> PRT
   <213> Artificial Sequence
<220>
   <223> MDM/HDM ANTAGONIST PEPTIDE
<400> 139
   Pro Arg Pro Ala Leu Val Phe Ala Asp Tyr Trp Glu Thr Leu Tyr
   1 5 10 15
<210> 140
   <211> 15
   <212> PRT
   <213> Artificial Sequence
<220>
   <223> MDM/HDM ANTAGONIST PEPTIDE
<400> 140
   Pro Ala Phe Ser Arg Phe Trp Ser Asp Leu Ser Ala Gly Ala His
   1 5 10 15
<210> 141
   <211> 15
   <212> PRT
   <213> Artificial Sequence
<220>
   <223> MDM/HDM ANTAGONIST PEPTIDE
<400> 141
   Pro Ala Phe Ser Arg Phe Trp Ser Lys Leu Ser Ala Gly Ala His
   1 5 10 15
<210> 142
   <211> 10
   <212> PRT
   <213> Artificial Sequence
<220>
   <223> MDM/HDM ANTAGONIST PEPTIDE
<220>
   <221> misc_feature
   <222> (2, 4, 8 )..(9)
   <223> Xaa = any amino acid
<400> 142
   Pro Xaa Phe Xaa Asp Tyr Trp Xaa Xaa Leu
   1 5 10
<210> 143
   <211> 12
   <212> PRT
   <213> Artificial Sequence
<220>
   <223> MDM/HDM ANTAGONIST PEPTIDE
<400> 143
   Gln Glu Thr Phe Ser Asp Leu Trp Lys Leu Leu Pro
   1 5 10
<210> 144
   <211> 12
   <212> PRT
   <213> Artificial Sequence
<220>
   <223> MDM/HDM ANTAGONIST PEPTIDE
<400> 144
   Gln Pro Thr Phe Ser Asp Leu Trp Lys Leu Leu Pro
   1 5 10
<210> 145
   <211> 12
   <212> PRT
   <213> Artificial Sequence
<220>
   <223> MDM/HDM ANTAGONIST PEPTIDE
<400> 145
   Gln Glu Thr Phe Ser Asp Tyr Trp Lys Leu Leu Pro
   1 5 10
<210> 146
   <211> 12
   <212> PRT
   <213> Artificial Sequence
<220>
   <223> MDM/HDM ANTAGONIST PEPTIDE
<400> 146
   Gln Pro Thr Phe Ser Asp Tyr Trp Lys Leu Leu Pro
   1 5 10
<210> 147
   <211> 12
   <212> PRT
   <213> Artificial Sequence
<220>
   <223> SELECTIN ANTAGONIST PEPTIDE
<400> 147
   Asp Ile Thr Trp Asp Gln Leu Trp Asp Leu Met Lys
   1 5 10
<210> 148
   <211> 12
   <212> PRT
   <213> Artificial Sequence
<220>
   <223> SELECTIN ANTAGONIST PEPTIDE
<400> 148
   Asp Ile Thr Trp Asp Glu Leu Trp Lys Ile Met Asn
   1 5 10
<210> 149
   <211> 12
   <212> PRT
   <213> Artificial Sequence
<220>
   <223> SELECTIN ANTAGONIST PEPTIDE
<400> 149
   Asp Tyr Thr Trp Phe Glu Leu Trp Asp Met Met Gln
   1 5 10
<210> 150
   <211> 12
   <212> PRT
   <213> Artificial Sequence
<220>
   <223> SELECTIN ANTAGONIST PEPTIDE
<400> 150
   Gln Ile Thr Trp Ala Gln Leu Trp Asn Met Met Lys
   1 5 10
<210> 151
   <211> 12
   <212> PRT
   <213> Artificial Sequence
<220>
   <223> SELECTIN ANTAGONIST PEPTIDE
<400> 151
   Asp Met Thr Trp His Asp Leu Trp Thr Leu Met Ser
   1 5 10
<210> 152
   <211> 12
   <212> PRT
   <213> Artificial Sequence
<220>
   <223> SELECTIN ANTAGONIST PEPTIDE
<400> 152
   Asp Tyr Ser Trp His Asp Leu Trp Glu Met Met Ser
   1 5 10
<210> 153
   <211> 12
   <212> PRT
   <213> Artificial Sequence
<220>
   <223> SELECTIN ANTAGONIST PEPTIDE
<400> 153
   Glu Ile Thr Trp Asp Gln Leu Trp Glu Val Met Asn
   1 5 10
<210> 154
   <211> 12
   <212> PRT
   <213> Artificial Sequence
<220>
   <223> SELECTIN ANTAGONIST PEPTIDE
<400> 154
   His Val Ser Trp Glu Gln Leu Trp Asp Ile Met Asn
   1 5 10
<210> 155
   <211> 12
   <212> PRT
   <213> Artificial Sequence
<220>
   <223> SELECTIN ANTAGONIST PEPTIDE
<400> 155
   His Ile Thr Trp Asp Gln Leu Trp Arg Ile Met Thr
   1 5 10
<210> 156
   <211> 13
   <212> PRT
   <213> Artificial Sequence
<220>
   <223> SELECTIN ANTAGONIST PEPTIDE
<400> 156
   Arg Asn Met Ser Trp Leu Glu Leu Trp Glu His Met Lys
   1 5 10
<210> 157
   <211> 18
   <212> PRT
   <213> Artificial Sequence
<220>
   <223> SELECTIN ANTAGONIST PEPTIDE
<400> 157
   Ala Glu Trp Thr Trp Asp Gln Leu Trp His Val Met Asn Pro Ala Glu
   1 5 10 15
   Ser Gln
<210> 158
   <211> 14
   <212> PRT
   <213> Artificial Sequence
<220>
   <223> SELECTIN ANTAGONIST PEPTIDE
<400> 158
   His Arg Ala Glu Trp Leu Ala Leu Trp Glu Gln Met Ser Pro
   1 5 10
<210> 159
   <211> 14
   <212> PRT
   <213> Artificial Sequence
<220>
   <223> SELECTIN ANTAGONIST PEPTIDE
<400> 159
   Lys Lys Glu Asp Trp Leu Ala Leu Trp Arg Ile Met Ser Val
   1 5 10
<210> 160
   <211> 11
   <212> PRT
   <213> Artificial Sequence
<220>
   <223> SELECTIN ANTAGONIST PEPTIDE
<400> 160
   Ile Thr Trp Asp Gln Leu Trp Asp Leu Met Lys
   1 5 10
<210> 161
   <211> 12
   <212> PRT
   <213> Artificial Sequence
<220>
   <223> SELECTIN ANTAGONIST PEPTIDE
<400> 161
   Asp Ile Thr Trp Asp Gln Leu Trp Asp Leu Met Lys
   1 5 10
<210> 162
   <211> 12
   <212> PRT
   <213> Artificial Sequence
<220>
   <223> SELECTIN ANTAGONIST PEPTIDE
<400> 162
   Asp Ile Thr Trp Asp Gln Leu Trp Asp Leu Met Lys
   1 5 10
<210> 163
   <211> 12
   <212> PRT
   <213> Artificial Sequence
<220>
   <223> SELECTIN ANTAGONIST PEPTIDE
<400> 163
   Asp Ile Thr Trp Asp Gln Leu Trp Asp Leu Met Lys
   1 5 10
<210> 164
   <211> 13
   <212> PRT
   <213> Artificial Sequence
<220>
   <223> CALMODULIN ANTAGONIST PEPTIDE
<400> 164
   Ser Cys Val Lys Trp Gly Lys Lys Glu Phe Cys Gly Ser
   1 5 10
<210> 165
   <211> 12
   <212> PRT
   <213> Artificial Sequence
<220>
   <223> CALMODULIN ANTAGONIST PEPTIDE
<400> 165
   Ser Cys Trp Lys Tyr Trp Gly Lys Glu Cys Gly Ser
   1 5 10
<210> 166
   <211> 13
   <212> PRT
   <213> Artificial Sequence
<220>
   <223> CALMODULIN ANTAGONIST PEPTIDE
<400> 166
   Ser Cys Tyr Glu Trp Gly Lys Leu Arg Trp Cys Gly Ser
   1 5 10
<210> 167
   <211> 13
   <212> PRT
   <213> Artificial Sequence
<220>
   <223> CALMODULIN ANTAGONIST PEPTIDE
<400> 167
   Ser Cys Leu Arg Trp Gly Lys Trp Ser Asn Cys Gly Ser
   1 5 10
<210> 168
   <211> 13
   <212> PRT
   <213> Artificial Sequence
<220>
   <223> CALMODULIN ANTAGONIST PEPTIDE
<400> 168
   Ser Cys Trp Arg Trp Gly Lys Tyr Gln Ile Cys Gly Ser
   1 5 10
<210> 169
   <211> 13
   <212> PRT
   <213> Artificial Sequence
<220>
   <223> CALMODULIN ANTAGONIST PEPTIDE
<400> 169
   Ser Cys Val Ser Trp Gly Ala Leu Lys Leu Cys Gly Ser
   1 5 10
<210> 170
   <211> 13
   <212> PRT
   <213> Artificial Sequence
<220>
   <223> CALMODULIN ANTAGONIST PEPTIDE
<400> 170
   Ser Cys Ile Arg Trp Gly Gln Asn Thr Phe Cys Gly Ser
   1 5 10
<210> 171
   <211> 13
   <212> PRT
   <213> Artificial Sequence
<220>
   <223> CALMODULIN ANTAGONIST PEPTIDE
<400> 171
   Ser Cys Trp Gln Trp Gly Asn Leu Lys Ile Cys Gly Ser
   1 5 10
<210> 172
   <211> 13
   <212> PRT
   <213> Artificial Sequence
<220>
   <223> CALMODULIN ANTAGONIST PEPTIDE
<400> 172
   Ser Cys Val Arg Trp Gly Gln Leu Ser Ile Cys Gly Ser
   1 5 10
<210> 173
   <211> 21
   <212> PRT
   <213> Artificial Sequence
<220>
   <223> CALMODULIN ANTAGONIST PEPTIDE
<400> 173
   Leu Lys Lys Phe Asn Ala Arg Arg Lys Leu Lys Gly Ala Ile Leu Thr
   1 5 10 15
   Thr Met Leu Ala Lys
   20
<210> 174
   <211> 18
   <212> PRT
   <213> Artificial Sequence
<220>
   <223> CALMODULIN ANTAGONIST PEPTIDE
<400> 174
   Arg Arg Trp Lys Lys Asn Phe Ile Ala Val Ser Ala Ala Asn Arg Phe
   1 5 10 15
   Lys Lys
<210> 175
   <211> 18
   <212> PRT
   <213> Artificial Sequence
<220>
   <223> CALMODULIN ANTAGONIST PEPTIDE
<400> 175
   Arg Lys Trp Gln Lys Thr Gly His Ala Val Arg Ala Ile Gly Arg Leu
   1 5 10 15
   Ser Ser
<210> 176
   <211> 14
   <212> PRT
   <213> Artificial Sequence
<220>
   <223> CALMODULIN ANTAGONIST PEPTIDE
<400> 176
   Ile Asn Leu Lys Ala Leu Ala Ala Leu Ala Lys Lys Ile Leu
   1 5 10
<210> 177
   <211> 18
   <212> PRT
   <213> Artificial Sequence
<220>
   <223> CALMODULIN ANTAGONIST PEPTIDE
<400> 177
   Lys Ile Trp Ser Ile Leu Ala Pro Leu Gly Thr Thr Leu Val Lys Leu
   1 5 10 15
   Val Ala
<210> 178
   <211> 14
   <212> PRT
   <213> Artificial Sequence
<220>
   <223> CALMODULIN ANTAGONIST PEPTIDE
<400> 178
   Leu Lys Lys Leu Leu Lys Leu Leu Lys Lys Leu Leu Lys Leu
   1 5 10
<210> 179
   <211> 18
   <212> PRT
   <213> Artificial Sequence
<220>
   <223> CALMODULIN ANTAGONIST PEPTIDE
<400> 179
   Leu Lys Trp Lys Lys Leu Leu Lys Leu Leu Lys Lys Leu Leu Lys Lys
   1 5 10 15
   Leu Leu
<210> 180
   <211> 17
   <212> PRT
   <213> Artificial Sequence
<220>
   <223> CALMODULIN ANTAGONIST PEPTIDE
<400> 180
   Ala Glu Trp Pro Ser Leu Thr Glu Ile Lys Thr Leu Ser His Phe Ser
   1 5 10 15
   Val
<210> 181
   <211> 17
   <212> PRT
   <213> Artificial Sequence
<220>
   <223> CALMODULIN ANTAGONIST PEPTIDE
<400> 181
   Ala Glu Trp Pro Ser Pro Thr Arg Val Ile Ser Thr Thr Tyr Phe Gly
   1 5 10 15
   Ser
<210> 182
   <211> 17
   <212> PRT
   <213> Artificial Sequence
<220>
   <223> CALMODULIN ANTAGONIST PEPTIDE
<400> 182
   Ala Glu Leu Ala His Trp Pro Pro Val Lys Thr Val Leu Arg Ser Phe
   1 5 10 15
   Thr
<210> 183
   <211> 17
   <212> PRT
   <213> Artificial Sequence
<220>
   <223> CALMODULIN ANTAGONIST PEPTIDE
<400> 183
   Ala Glu Gly Ser Trp Leu Gln Leu Leu Asn Leu Met Lys Gln Met Asn
   1 5 10 15
   Asn
<210> 184
   <211> 10
   <212> PRT
   <213> Artificial Sequence
<220>
   <223> CALMODULIN ANTAGONIST PEPTIDE
<400> 184
   Ala Glu Trp Pro Ser Leu Thr Glu Ile Lys
   1 5 10
<210> 185
   <211> 27
   <212> PRT
   <213> Artificial Sequence
<220>
   <223> VINCULIN-BINDING
<400> 185
   Ser Thr Gly Gly Phe Asp Asp Val Tyr Asp Trp Ala Arg Gly Val Ser
   1 5 10 15
   Ser Ala Leu Thr Thr Thr Leu Val Ala Thr Arg
   20 25
<210> 186
   <211> 27
   <212> PRT
   <213> Artificial Sequence
<220>
   <223> VINCULIN-BINDING
<400> 186
   Ser Thr Gly Gly Phe Asp Asp Val Tyr Asp Trp Ala Arg Arg Val Ser
   1 5 10 15
   Ser Ala Leu Thr Thr Thr Leu Val Ala Thr Arg
   20 25
<210> 187
   <211> 30
   <212> PRT
   <213> Artificial Sequence
<220>
   <223> VINCULIN-BINDING
<400> 187
   Ser Arg Gly Val Asn Phe Ser Glu Trp Leu Tyr Asp Met Ser Ala Ala
   1 5 10 15
   Met Lys Glu Ala Ser Asn Val Phe Pro Ser Arg Arg Ser Arg
   20 25 30
<210> 188
   <211> 30
   <212> PRT
   <213> Artificial Sequence
<220>
   <223> VINCULIN-BINDING
<400> 188
   Ser Ser Gln Asn Trp Asp Met Glu Ala Gly Val Glu Asp Leu Thr Ala
   1               5                   10                  15
   Ala Met Leu Gly Leu Leu Ser Thr Ile His Ser Ser Ser Arg
               20                  25                  30
<210> 189
   <211> 31
   <212> PRT
   <213> Artificial Sequence
<220>
   <223> VINCULIN-BINDING
<400> 189
   Ser Ser Pro Ser Leu Tyr Thr Gln Phe Leu Val Asn Tyr Glu Ser Ala
   1               5                   10                  15
   Ala Thr Arg Ile Gln Asp Leu Leu Ile Ala Ser Arg Pro Ser Arg
               20                  25                  30
<210> 190
   <211> 31
   <212> PRT
   <213> Artificial Sequence
<220>
   <223> VINCULIN-BINDING
<400> 190
   Ser Ser Thr Gly Trp Val Asp Leu Leu Gly Ala Leu Gln Arg Ala Ala
   1               5                   10                  15
   Asp Ala Thr Arg Thr Ser Ile Pro Pro Ser Leu Gln Asn Ser Arg
               20                  25                  30
<210> 191
   <211> 18
   <212> PRT
   <213> Artificial Sequence
<220>
   <223> VINCULIN-BINDING
<400> 191
   Asp Val Tyr Thr Lys Lys Glu Leu Ile Glu Cys Ala Arg Arg Val Ser
   1 5 10 15
   Glu Lys
<210> 192
   <211> 22
   <212> PRT
   <213> Artificial Sequence
<220>
   <223> C4BP-BINDING
<400> 192
   Glu Lys Gly Ser Tyr Tyr Pro Gly Ser Gly Ile Ala Gln Phe His Ile
   1 5 10 15
   Asp Tyr Asn Asn Val Ser
   20
<210> 193
   <211> 22
   <212> PRT
   <213> Artificial Sequence
<220>
   <223> C4BP-BINDING
<400> 193
   Ser Gly Ile Ala Gln Phe His Ile Asp Tyr Asn Asn Val Ser Ser Ala
   1 5 10 15
   Glu Gly Trp His Val Asn
   20
<210> 194
   <211> 34
   <212> PRT
   <213> Artificial Sequence
<220>
   <223> C4BP-BINDING
<400> 194
   Leu Val Thr Val Glu Lys Gly Ser Tyr Tyr Pro Gly Ser Gly Ile Ala
   1 5 10 15
   Gln Phe His Ile Asp Tyr Asn Asn Val Ser Ser Ala Glu Gly Trp His
   20 25 30
   Val Asn
<210> 195
   <211> 14
   <212> PRT
   <213> Artificial Sequence
<220>
   <223> C4BP-BINDING
<400> 195
   Ser Gly Ile Ala Gln Phe His Ile Asp Tyr Asn Asn Val Ser
   1 5 10
<210> 196
   <211> 17
   <212> PRT
   <213> Artificial Sequence
<220>
   <223> UKR ANTAGONIST PEPTIDE
<400> 196
   Ala Glu Pro Met Pro His Ser Leu Asn Phe Ser Gln Tyr Leu Trp Tyr
   1 5 10 15
   Thr
<210> 197
   <211> 17
   <212> PRT
   <213> Artificial Sequence
<220>
   <223> UKR ANTAGONIST PEPTIDE
<400> 197
   Ala Glu His Thr Tyr Ser Ser Leu Trp Asp Thr Tyr Ser Pro Leu Ala
   1 5 10 15
   Phe
<210> 198
   <211> 17
   <212> PRT
   <213> Artificial Sequence
<220>
   <223> UKR ANTAGONIST PEPTIDE
<400> 198
   Ala Glu Leu Asp Leu Trp Met Arg His Tyr Pro Leu Ser Phe Ser Asn
   1 5 10 15
   Arg
<210> 199
   <211> 17
   <212> PRT
   <213> Artificial Sequence
<220>
   <223> UKR ANTAGONIST PEPTIDE
<400> 199
   Ala Glu Ser Ser Leu Trp Thr Arg Tyr Ala Trp Pro Ser Met Pro Ser
   1 5 10 15
   Tyr
<210> 200
   <211> 17
   <212> PRT
   <213> Artificial Sequence
<220>
   <223> UKR ANTAGONIST PEPTIDE
<400> 200
   Ala Glu Trp His Pro Gly Leu Ser Phe Gly Ser Tyr Leu Trp Ser Lys
   1 5 10 15
   Thr

<210> 201
   <211> 17
   <212> PRT
   <213> Artificial Sequence
<220>
   <223> UKR ANTAGONIST PEPTIDE
<400> 201
   Ala Glu Pro Ala Leu Leu Asn Trp Ser Phe Phe Phe Asn Pro Gly Leu
   1 5 10 15
   His
<210> 202
   <211> 17
   <212> PRT
   <213> Artificial Sequence
<220>
   <223> UKR ANTAGONIST PEPTIDE
<400> 202
   Ala Glu Trp Ser Phe Tyr Asn Leu His Leu Pro Glu Pro Gln Thr Ile
   1 5 10 15
   Phe
<210> 203
   <211> 17
   <212> PRT
   <213> Artificial Sequence
<220>
   <223> UKR ANTAGONIST PEPTIDE
<400> 203
   Ala Glu Pro Leu Asp Leu Trp Ser Leu Tyr Ser Leu Pro Pro Leu Ala
   1 5 10 15
   Met
<210> 204
   <211> 17
   <212> PRT
   <213> Artificial Sequence
<220>
   <223> UKR ANTAGONIST PEPTIDE
<400> 204
   Ala Glu Pro Thr Leu Trp Gln Leu Tyr Gln Phe Pro Leu Arg Leu Ser
   1 5 10 15
   Gly
<210> 205
   <211> 17
   <212> PRT
   <213> Artificial Sequence
<220>
   <223> UKR ANTAGONIST PEPTIDE
<400> 205
   Ala Glu Ile Ser Phe Ser Glu Leu Met Trp Leu Arg Ser Thr Pro Ala
   1 5 10 15
   Phe
<210> 206
   <211> 17
   <212> PRT
   <213> Artificial Sequence
<220>
   <223> UKR ANTAGONIST PEPTIDE
<400> 206
   Ala Glu Leu Ser Glu Ala Asp Leu Trp Thr Thr Trp Phe Gly Met Gly
   1 5 10 15
   Ser
<210> 207
   <211> 17
   <212> PRT
   <213> Artificial Sequence
<220>
   <223> UKR ANTAGONIST PEPTIDE
<400> 207
   Ala Glu Ser Ser Leu Trp Arg Ile Phe Ser Pro Ser Ala Leu Met Met
   1 5 10 15
   Ser
<210> 208
   <211> 17
   <212> PRT
   <213> Artificial Sequence
<220>
   <223> UKR ANTAGONIST PEPTIDE
<400> 208
   Ala Glu Ser Leu Pro Thr Leu Thr Ser Ile Leu Trp Gly Lys Glu Ser
   1 5 10 15
   Val
<210> 209
   <211> 17
   <212> PRT
   <213> Artificial Sequence
<220>
   <223> UKR ANTAGONIST PEPTIDE
<400> 209
   Ala Glu Thr Leu Phe Met Asp Leu Trp His Asp Lys His Ile Leu Leu
   1 5 10 15
   Thr
<210> 210
   <211> 17
   <212> PRT
   <213> Artificial Sequence
<220>
   <223> UKR ANTAGONIST PEPTIDE
<400> 210
   Ala Glu Ile Leu Asn Phe Pro Leu Trp His Glu Pro Leu Trp Ser Thr
   1 5 10 15
   Glu
<210> 211
   <211> 17
   <212> PRT
   <213> Artificial Sequence
<220>
   <223> UKR ANTAGONIST PEPTIDE
<400> 211
   Ala Glu Ser Gln Thr Gly Thr Leu Asn Thr Leu Phe Trp Asn Thr Leu
   1 5 10 15
   Arg
<210> 212
   <211> 9
   <212> PRT
   <213> Artificial Sequence
<220>
   <223> IL-1 ANTAGONIST PEPTIDE
<220>
   <221> mise_feature
   <222> (1)..(1)
   <223> Xaa is V, L, I, E, P, G, Y, M, T or D.
<220>
   <221> misc_feature
   <222> (2)..(2)
   <223> Xaa is Y, W or F.
<220>
   <221> misc_feature
   <222> (3)..(3)
   <223> Xaa is F, W or Y.
<220>
   <221> mise_feature
   <222> (5).. (5)
   <223> Xaa is P or Azetidine.
<220>
   <221> misc_feature
   <222> (7)..(7)
   <223> Xaa is S, A, V or L.
<220>
   <221> misc_feature
   <222> (8)..(8)
   <223> Xaa is V, L, I or E.
<220>
   <221> misc_feature
   <222> (9)..(9)
   <223> Xaa is Q or P.
<400> 212
   Xaa Xaa Xaa Gln Xaa Tyr Xaa Xaa Xaa
   1               5
<210> 213
   <211> 21
   <212> PRT
   <213> Artificial Sequence
<220>
   <223> IL-1 ANTAGONIST PEPTIDE
<400> 213
   Thr Ala Asn Val Ser Ser Phe Glu Trp Thr Pro Tyr Tyr Trp Gln Pro
   1               5                   10                  15
   Tyr Ala Leu Pro Leu
               20
<210> 214
   <211> 18
   <212> PRT
   <213> Artificial Sequence
<220>
   <223> IL-1 ANTAGONIST PEPTIDE
<400> 214
   Ser Trp Thr Asp Tyr Gly Tyr Trp Gln Pro Tyr Ala Leu Pro Ile Ser
   1 5 10 15
   Gly Leu
<210> 215
   <211> 21
   <212> PRT
   <213> Artificial Sequence
<220>
   <223> IL-1 ANTAGONIST PEPTIDE
<400> 215
   Glu Thr Pro Phe Thr Trp Glu Glu Ser Asn Ala Tyr Tyr Trp Gln Pro
   1 5 10 15
   Tyr Ala Leu Pro Leu
   20
<210> 216
   <211> 21
   <212> PRT
   <213> Artificial Sequence
<220>
   <223> IL-1 ANTAGONIST PEPTIDE
<400> 216
   Glu Asn Thr Tyr Ser Pro Asn Trp Ala Asp Ser Met Tyr Trp Gln Pro
   1 5 10 15
   Tyr Ala Leu Pro Leu
   20
<210> 217
   <211> 21
   <212> PRT
   <213> Artificial Sequence
<220>
   <223> IL-1 ANTAGONIST PEPTIDE
<400> 217
   Ser Val Gly Glu Asp His Asn Phe Trp Thr Ser Glu Tyr Trp Gln Pro
   1 5 10 15
   Tyr Ala Leu Pro Leu
   20
<210> 218
   <211> 21
   <212> PRT
   <213> Artificial Sequence
<220>
   <223> IL-1 ANTAGONIST PEPTIDE
<400> 218
   Asp Gly Tyr Asp Arg Trp Arg Gln Ser Gly Glu Arg Tyr Trp Gln Pro
   1 5 10 15
   Tyr Ala Leu Pro Leu
   20
<210> 219
   <211> 11
   <212> PRT
   <213> Artificial Sequence
<220>
   <223> IL-1 ANTAGONIST PEPTIDE
<400> 219
   Phe Glu Trp Thr Pro Gly Tyr Trp Gln Pro Tyr
   1 5 10
<210> 220
   <211> 11
   <212> PRT
   <213> Artificial Sequence
<220>
   <223> IL-1 ANTAGONIST PEPTIDE
<400> 220
   Phe Glu Trp Thr Pro Gly Tyr Trp Gln His Tyr
   1 5 10
<210> 221
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 221
   Phe Glu Trp Thr Pro Gly Trp Tyr Gln Xaa Tyr
   1 5 10
<210> 222
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, optionally acetlated at N terminus
<220>
   <221> mise_feature
   <222> (10)..(10)
   <223> Position 10, Xaa = azetidine
<400> 222
   Phe Glu Trp Thr Pro Gly Trp Tyr Gln Xaa Tyr
   1 5 10
<210> 223
   <211> 12
   <212> PRT
   <213> Artificial Sequence
<220>
   <223> IL-1 ANTAGONIST PEPTIDE
<220>
   <221> misc_feature
   <222> (11)..(11)
   <223> Position 11, Xaa = azetidine
<400> 223
   Phe Glu Trp Thr Pro Gly Trp Pro Tyr Gln Xaa Tyr
   1 5 10
<210> 224
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 224
   Phe Ala Trp Thr Pro Gly Tyr Trp Gln Xaa Tyr
   1 5 10
<210> 225
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 225
   Phe Glu Trp Ala Pro Gly Tyr Trp Gln Xaa Tyr
   1 5 10
<210> 226
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 226
   Phe Glu Trp Val Pro Gly Tyr Trp Gln Xaa Tyr
   1 5 10
<210> 227
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 227
   Phe Glu Trp Thr Pro Gly Tyr Trp Gln Xaa Tyr
   1 5 10
<210> 228
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, optionally acetylated at N terminus
<220>
   <221> misc_feature
   <222> (10)..(10)
   <223> Position 10, Xaa = azetidine
<400> 228
   Phe Glu Trp Thr Pro Gly Tyr Trp Gln Xaa Tyr
   1               5                   10
<210> 229
   <211> 11
   <212> PRT
   <213> Artificial Sequence
<220>
   <223> IL-1 ANTAGONIST PEPTIDE
<220>
   <221> mise_feature
   <222> (6 )..(6)
   <223> Position 6, Xaa products = "MeGly"
<220>
   <221> misc_feature
   <222> (10)..(10)
   <223> Position 10, Xaa = azetidine
<400> 229
   Phe Glu Trp Thr Pro Xaa Trp Tyr Gln Xaa Tyr
   1               5                   10
<210> 230
   <211> 11
   <212> PRT
   <213> Artificial Sequence
<220>
   <223> IL-1 ANTAGONIST PEPTIDE
<220>
   <221> misc_feature
   <222> (6)..(6)
   <223> Position 6, Xaa = MeGly
<220>
   <221> misc_feature
   <222> (10)..(10)
   <223> Position 10, Xaa = azetidine
<400> 230
   Phe Glu Trp Thr Pro Xaa Trp Tyr Gln Xaa Tyr
   1 5 10
<210> 231
   <211> 11
   <212> PRT
   <213> Artificial Sequence
<220>
   <223> IL-1 ANTAGONIST PEPTIDE
<400> 231
   Phe Glu Trp Thr Pro Gly Tyr Tyr Gln Pro Tyr
   1 5 10
<210> 232
   <211> 11
   <212> PRT
   <213> Artificial Sequence
<220>
   <223> IL-1 ANTAGONIST PEPTIDE
<400> 232
   Phe Glu Trp Thr Pro Gly Trp Trp Gln Pro Tyr
   1 5 10
<210> 233
   <211> 11
   <212> PRT
   <213> Artificial Sequence
<220>
   <223> IL-1 ANTAGONIST PEPTIDE
<400> 233
   Phe Glu Trp Thr Pro Asn Tyr Trp Gln Pro Tyr
   1 5 10
<210> 234
   <211> 11
   <212> PRT
   <213> Artificial Sequence
<220>
   <223> IL-1 ANTAGONIST PEPTIDE
<220>
   <221> misc_feature
   <222> (5)..(5)
   <223> Position 5, Xaa = pipecolic acid
<220>
   <221> misc_feature
   <222> (10)..(10)
   <223> Position 10, Xaa = azetidine
<400> 234
   Phe Glu Trp Thr Xaa Val Tyr Trp Gln Xaa Tyr
   1 5 10
<210> 235
   <211> 11
   <212> PRT
   <213> Artificial Sequence
<220>
   <223> IL-1 ANTAGONIST PEPTIDE
<220>
   <221> misc_feature
   <222> (5)..(5)
   <223> Position 5, Xaa = pipecolic acid
<220>
   <221> misc_feature
   <222> (10)..(10)
   <223> Position 10, Xaa = azetidine
<400> 235
   Phe Glu Trp Thr Xaa Gly Tyr Trp Gln Xaa Tyr
   1 5 10
<210> 236
   <211> 11
   <212> PRT
   <213> Artificial Sequence
<220>
   <223> IL-1 ANTAGONIST PEPTIDE
<220>
   <221> misc_feature
   <222> (6 )..(6)
   <223> Position 6, Xaa = Aib
<220>
   <221> misc_feature
   <222> (10)..(10)
   <223> Position 10, Xaa = azetidine
<400> 236
   Phe Glu Trp Thr Pro Xaa Tyr Trp Gln Xaa Tyr
   1 5 10
<210> 237
   <211> 11
   <212> PRT
   <213> Artificial Sequence
<220>
   <223> IL-1 ANTAGONIST PEPTIDE
<220>
   <221> misc_feature
   <222> (5) ..(5)
   <223> Position 5, Xaa = MeGly
<220>
   <221> misc_feature
   <222> (10)..(10)
   <223> Position 10, Xaa = azetidine
<400> 237
   Phe Glu Trp Thr Xaa Gly Tyr Trp Gln Xaa Tyr
   1 5 10
<210> 238
   <211> 11
   <212> PRT
   <213> Artificial Sequence
<220>
   <223> IL-1 ANTAGONIST PEPTIDE
<220>
   <221> misc_feature
   <222> (11)..(11)
   <223> Position 11, amino group added at C terminus
<400> 238
   Phe Glu Trp Thr Pro Gly Tyr Trp Gln Pro Tyr
   1 5 10
<210> 239
   <211> 11
   <212> PRT
   <213> Artificial Sequence
<220>
   <223> IL-1 ANTAGONIST PEPTIDE
<220>
   <221> misc_feature
   <222> (11)..(11)
   <223> Position 11, amino group added at C-terminus
<400> 239
   Phe Glu Trp Thr Pro Gly Tyr Trp Gln His Tyr
   1 5 10
<210> 240
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue Position 11 amino group added at C-terminus
<220>
   <221> misc_feature
   <222> (11)..(11)
   <223> Position 11 amino group added at C-terminus
<400> 240
   Phe Glu Trp Thr Pro Gly Trp Tyr Gln Xaa Tyr
   1 5 10
<210> 241
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1 optionally acetylated at N-terminus
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 241
   Phe Glu Trp Thr Pro Gly Trp Tyr Gln Xaa Tyr
   1 5 10
<210> 242
   <211> 11
   <212> PRT
   <213> Artificial Sequence
<220>
   <223> IL-1 ANTAGONIST PEPTIDE
<220>
   <221> misc_feature
   <222> (8)..(8)
   <223> Position 8, Xaa is a phyosphotyrosyl residue
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 242
   Phe Glu Trp Thr Pro Gly Trp Xaa Gln Xaa Tyr
   1               5                   10
<210> 243
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 243
   Phe Ala Trp Thr Pro Gly Tyr Trp Gln Xaa Tyr
   1               5                   10
<210> 244
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 244
   Phe Glu Trp Ala Pro Gly Tyr Trp Gln Xaa Tyr
   1 5 10
<210> 245
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<220>
   <221> misc_feature
   <222> (11 )..(11)
   <223> Position 11 amino group added at C-terminus
<400> 245
   Phe Glu Trp Val Pro Gly Tyr Trp Gln Xaa Tyr
   1 5 10
<210> 246
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 246
   Phe Glu Trp Thr Pro Gly Tyr Trp Gln Xaa Tyr
   1 5 10
<210> 247
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1 acetylated at N-terminus
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 247
   Phe Glu Trp Thr Pro Gly Tyr Trp Gln Xaa Tyr
   1               5                   10
<210> 248
   <211> 11
   <212> PRT
   <213> Artificial Sequence
<220>
   <223> IL-1 ANTAGONIST PEPTIDE
<220>
   <221> misc_feature
   <222> (6)..(6)
   <223> Position 6, D amino acid residue
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 248
   Phe Glu Trp Thr Pro Ala Trp Tyr Gln Xaa Tyr
   1               5                   10
<210> 249
   <211> 11
   <212> PRT
   <213> Artificial Sequence
<220>
   <223> IL-1 ANTAGONIST PEPTIDE
<220>
   <221> misc_feature
   <222> (6)..(6)
   <223> Position 6, Xaa is a sarcosine residue
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 249
   Phe Glu Trp Thr Pro Xaa Trp Tyr Gln Xaa Tyr
   1 5 10
<210> 250
   <211> 11
   <212> PRT
   <213> Artificial Sequence
<220>
   <223> IL-1 ANTAGONIST PEPTIDE
<220>
   <221> misc_feature
   <222> (11)..(11)
   <223> Position 11 amino group added at C-terminus
<400> 250
   Phe Glu Trp Thr Pro Gly Tyr Tyr Gln Pro Tyr
   1 5 10
<210> 251
   <211> 11
   <212> PRT
   <213> Artificial Sequence
<220>
   <223> IL-1 ANTAGONIST PEPTIDE
<220>
   <221> misc_feature
   <222> (11)..(11)
   <223> Position 11 amino group added at C-terminus
<400> 251
   Phe Glu Trp Thr Pro Gly Trp Trp Gln Pro Tyr
   1 5 10
<210> 252
   <211> 11
   <212> PRT
   <213> Artificial Sequence
<220>
   <223> IL-1 ANTAGONIST PEPTIDE
<220>
   <221> misc_feature
   <222> (11)..(11)
   <223> Position 11 amino group added at C-terminus
<400> 252
   Phe Glu Trp Thr Pro Asn Tyr Trp Gln Pro Tyr
   1 5 10
<210> 253
   <211> 11
   <212> PRT
   <213> Artificial Sequence
<220>
   <223> IL-1 ANTAGONIST PEPTIDE
<220>
   <221> mise_feature
   <222> (6)..(6)
   <223> Position 6, D amino acid residue
<220>
   <221> misc_feature
   <222> (10)..(10)
   <223> Position 10, Xaa is an azetidine residue
<220>
   <221> mise_feature
   <222> (11)..(11)
   <223> Position 11 amino group added at C-terminus
<400> 253
   Phe Glu Trp Thr Pro Val Tyr Trp Gln Xaa Tyr
   1 5 10
<210> 254
   <211> 11
   <212> PRT
   <213> Artificial Sequence
<220>
   <223> IL-1 ANTAGONIST PEPTIDE

<220>
   <221> misc_feature
   <222> (5)..(5)
   <223> Position 5, Xaa is a pipecolic acid residue
<220>
   <221> misc_feature
   <222> (10)..(10)
   <223> Position 10, Xaa is an azetidine residue
<220>
   <221> misc_feature
   <222> (11 )..(11)
   <223> Position 11 amino group added at C-terminus
<400> 254
   Phe Glu Trp Thr Xaa Gly Tyr Trp Gln Xaa Tyr
   1               5                   10
<210> 255
   <211> 11
   <212> PRT
   <213> Artificial Sequence
<220>
   <223> IL-1 ANTAGONIST PEPTIDE
<220>
   <221> mise_feature
   <222> (6)..(6)
   <223> Position 6, Xaa = pipecolic acid
<220>
   <221> misc_feature
   <222> (10)..(10)
   <223> Position 10, Xaa = azetidine
<400> 255
   Phe Glu Trp Thr Pro Xaa Tyr Trp Gln Xaa Tyr
   1               5                   10
<210> 256
   <211> 11
   <212> PRT
   <213> Artificial Sequence
<220>
   <223> IL-1 ANTAGONIST PEPTIDE
<220>
   <221> misc_feature
   <222> (5)..(5)
   <223> Position 5, Xaa = MeGly
<220>
   <221> misc_feature
   <222> (10)..(10)
   <223> Position 10, Xaa = azetidine
<400> 256
   Phe Glu Trp Thr Xaa Gly Tyr Trp Gln Xaa Tyr
   1 5 10
<210> 257
   <211> 15
   <212> PRT
   <213> Artificial Sequence
<220>
   <223> IL-1 ANTAGONIST PEPTIDE
<400> 257
   Phe Glu Trp Thr Pro Gly Tyr Trp Gln Pro Tyr Ala Leu Pro Leu
   1 5 10 15
<210> 258
   <211> 11
   <212> PRT
   <213> Artificial Sequence
<220>
   <223> IL-1 ANTAGONIST PEPTIDE
<220>
   <221> misc_feature
   <222> (1)..(1)
   <223> position 1, Xaa is a 1-naphthylalanine residue
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 258
   Xaa Glu Trp Thr Pro Gly Tyr Tyr Gln Xaa Tyr
   1 5 10
<210> 259
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 259
   Tyr Glu Trp Thr Pro Gly Tyr Tyr Gln Xaa Tyr
   1               5                   10
<210> 260
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<220>
   <221> misc_feature
   <222> (11)..(11)
   <223> Position. 11 amino group added at C-terminus
<400> 260
   Phe Glu Trp Val Pro Gly Tyr Tyr Gln Xaa Tyr
   1               5                   10
<210> 261
   <211> 11
   <212> PRT
   <213> Artificial Sequence
<220>
   <223> IL-1 ANTAGONIST PEPTIDE
<220>
   <221> misc_feature
   <222> (6)..(6)
   <223> Position 6, D amino acid residue
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 261
   Phe Glu Trp Thr Pro Ser Tyr Tyr Gln Xaa Tyr
   1 5 10
<210> 262
   <211> 11
   <212> PRT
   <213> Artificial Sequence
<220>
   <223> IL-1 ANTAGONIST PEPTIDE
<220>
   <221> misc_feature
   <222> (6)..(6)
   <223> Position 6, D amino acid residue
<220>
   <221> misc_feature
   <222> (10)..(10)
   <223> Position 10, Xaa is an azetidine residue
<220>
   <221> misc_feature
   <222> (11)..(11)
   <223> Position 11 amino group added at C-terminus
<400> 262
   Phe Glu Trp Thr Pro Asn Tyr Tyr Gln Xaa Tyr
   1 5 10
<210> 263
   <211> 4
   <212> PRT
   <213> Artificial Sequence
<220>
   <223> IL-1 ANTAGONIST PEPTIDE
<400> 263
   Thr Lys Pro Arg
   1
<210> 264
   <211> 5
   <212> PRT
   <213> Artificial Sequence
<220>
   <223> IL-1 ANTAGONIST PEPTIDE
<400> 264
   Arg Lys Ser Ser Lys
   1 5
<210> 265
   <211> 5
   <212> PRT
   <213> Artificial Sequence
<220>
   <223> IL-1 ANTAGONIST PEPTIDE
<400> 265
   Arg Lys Gln Asp Lys
   1 5
<210> 266
   <211> 6
   <212> PRT
   <213> Artificial Sequence
<220>
   <223> IL-1 ANTAGONIST PEPTIDE
<400> 266
   Asn Arg Lys Gln Asp Lys
   1 5
<210> 267
   <211> 6
   <212> PRT
   <213> Artificial Sequence
<220>
   <223> IL-1 ANTAGONIST PEPTIDE
<400> 267
   Arg Lys Gln Asp Lys Arg
   1 5
<210> 268
   <211> 9
   <212> PRT
   <213> Artificial Sequence
<220>
   <223> IL-1 ANTAGONIST PEPTIDE
<400> 268
   Glu Asn Arg Lys Gln Asp Lys Arg Phe
   1 5
<210> 269
   <211> 6
   <212> PRT
   <213> Artificial Sequence
<220>
   <223> IL-1 ANTAGONIST PEPTIDE
<400> 269
   Val Thr Lys Phe Tyr Phe
   1 5
<210> 270
   <211> 5
   <212> PRT
   <213> Artificial Sequence
<220>
   <223> IL-1 ANTAGONIST PEPTIDE
<400> 270
   Val Thr Lys Phe Tyr
   1 5
<210> 271
   <211> 5
   <212> PRT
   <213> Artificial Sequence
<220>
   <223> IL-1 ANTAGONIST PEPTIDE
<400> 271
   Val Thr Asp Phe Tyr
   1 5
<210> 272
   <211> 17
   <212> PRT
   <213> Artificial Sequence
<220>
   <223> MAST CELL ANTAGONISTS/ PROTEASE .INHIBITOR PEPTIDE
<400> 272
   Ser Gly Ser Gly Val Leu Lys Arg Pro Leu Pro Ile Leu Pro Val Thr
   1 5 10 15
   Arg
<210> 273
   <211> 17
   <212> PRT
   <213> Artificial Sequence
<220>
   <223> MAST CELL ANTAGONISTS/ PROTEASE INHIBITOR PEPTIDE
<400> 273
   Arg Trp Leu Ser Ser Arg Pro Leu Pro Pro Leu Pro Leu Pro Pro Arg
   1               5                   10                  15
   Thr
<210> 274
   <211> 20
   <212> PRT
   <213> Artificial Sequence
<220>
   <223> MAST CELL ANTAGONISTS/ PROTEASE INHIBITOR PEPTIDE
<400> 274
   Gly Ser Gly Ser Tyr Asp Thr Leu Ala Leu Pro Ser Leu Pro Leu His
   1               5                   10                  15
   Pro Met Ser Ser
               20
<210> 275
   <211> 20
   <212> PRT
   <213> Artificial Sequence
<220>
   <223> MAST CELL ANTAGONISTS/ PROTEASE INHIBITOR PEPTIDE
<400> 275
   Gly Ser Gly Ser Tyr Asp Thr Arg Ala Leu Pro Ser Leu Pro Leu His
   1               5                   10                  15
   Pro Met Ser Ser
               20
<210> 276
   <211> 20
   <212> PRT
   <213> Artificial Sequence
<220>
   <223> MAST CELL ANTAGONISTS/ PROTEASE INHIBITOR PEPTIDE
<400> 276
   Gly Ser Gly Ser Ser Gly Val Thr Met Tyr Pro Lys Leu Pro Pro His
   1               5                   10                  15
   Trp Ser Met Ala
               20
<210> 277
   <211> 20
   <212> PRT
   <213> Artificial Sequence
<220>
   <223> MAST CELL ANTAGONISTS/ PROTEASE INHIBITOR PEPTIDE
<400> 277
   Gly Ser Gly Ser Ser Gly Val Arg Met Tyr Pro Lys Leu Pro Pro His
   1 5 10 15
   Trp Ser Met Ala
   20
<210> 278
   <211> 20
   <212> PRT
   <213> Artificial Sequence
<220>
   <223> MAST CELL ANTAGONISTS/ PROTEASE INHIBITOR PEPTIDE
<400> 278
   Gly Ser Gly Ser Ser Ser Met Arg Met Val Pro Thr Ile Pro Gly Ser
   1 5 10 15
   Ala Lys His Gly
   20
<210> 279
   <211> 6
   <212> PRT
   <213> Artificial Sequence
<220>
   <223> ANTI-HBV
<400> 279
   Leu Leu Gly Arg Met Lys
   1 5
<210> 280
   <211> 8
   <212> PRT
   <213> Artificial Sequence
<220>
   <223> ANTI-HBV
<400> 280
   Ala Leu Leu Gly Arg Met Lys Gly
   1 5
<210> 281
   <211> 6
   <212> PRT
   <213> Artificial Sequence
<220>
   <223> ANTI-HBV
<400> 281
   Leu Asp Pro Ala Phe Arg
   1 5
<210> 282
   <211> 7
   <212> PRT
   <213> Artificial Sequence
<220>
   <223> SH3 ANTAGONIST PEPTIDE
<400> 282
   Arg Pro Leu Pro Pro Leu Pro
   1 5
<210> 283
   <211> 7
   <212> PRT
   <213> Artificial Sequence
<220>
   <223> SH3 ANTAGONIST PEPTIDE
<400> 283
   Arg Glu Leu Pro Pro Leu Pro
   1 5
<210> 284
   <211> 7
   <212> PRT
   <213> Artificial Sequence
<220>
   <223> SH3 ANTAGONIST PEPTIDE
<400> 284
   Ser Pro Leu Pro Pro Leu Pro
   1 5
<210> 285
   <211> 7
   <212> PRT
   <213> Artificial Sequence
<220>
   <223> SH3 ANTAGONIST PEPTIDE
<400> 285
   Gly Pro Leu Pro Pro Leu Pro
   1 5
<210> 286
   <211> 7
   <212> PRT
   <213> Artificial Sequence
<220>
   <223> SH3 ANTAGONIST PEPTIDE
<400> 286
   Arg Pro Leu Pro Ile Pro Pro
   1 5
<210> 287
   <211> 7
   <212> PRT
   <213> Artificial Sequence
<220>
   <223> SH3 ANTAGONIST PEPTIDE
<400> 287
   Arg Pro Leu Pro Ile Pro Pro
   1 5
<210> 288
   <211> 7
   <212> PRT
   <213> Artificial Sequence
<220>
   <223> SH3 ANTAGONIST PEPTIDE
<400> 288
   Arg Arg Leu Pro Pro Thr Pro
   1 5
<210> 289
   <211> 7
   <212> PRT
   <213> Artificial Sequence
<220>
   <223> SH3 ANTAGONIST PEPTIDE
<400> 289
   Arg Gln Leu Pro Pro Thr Pro
   1 5
<210> 290
   <211> 7
   <212> PRT
   <213> Artificial Sequence
<220>
   <223> SH3 ANTAGONIST PEPTIDE
<400> 290
   Arg Pro Leu Pro Ser Arg Pro
   1 5
<210> 291
   <211> 7
   <212> PRT
   <213> Artificial Sequence
<220>
   <223> SH3 ANTAGONIST PEPTIDE
<400> 291
   Arg Pro Leu Pro Thr Arg Pro
   1 5
<210> 292
   <211> 7
   <212> PRT
   <213> Artificial Sequence
<220>
   <223> SH3 ANTAGONIST PEPTIDE
<400> 292
   Ser Arg Leu Pro Pro Leu Pro
   1 5
<210> 293
   <211> 7
   <212> PRT
   <213> Artificial Sequence
<220>
   <223> SH3 ANTAGONIST PEPTIDE
<400> 293
   Arg Ala Leu Pro Ser Pro Pro
   1 5
<210> 294
   <211> 7
   <212> PRT
   <213> Artificial Sequence
<220>
   <223> SH3 ANTAGONIST PEPTIDE
<400> 294
   Arg Arg Leu Pro Arg Thr Pro
   1 5
<210> 295
   <211> 7
   <212> PRT
   <213> Artificial Sequence
<220>
   <223> SH3 ANTAGONIST PEPTIDE
<400> 295
   Arg Pro Val Pro Pro Ile Thr
   1 5
<210> 296
   <211> 7
   <212> PRT
   <213> Artificial Sequence
<220>
   <223> SH3 ANTAGONIST PEPTIDE
<400> 296
   Ile Leu Ala Pro Pro Val Pro
   1 5
<210> 297
   <211> 7
   <212> PRT
   <213> Artificial Sequence
<220>
   <223> SH3 ANTAGONIST PEPTIDE
<400> 297
   Arg Pro Leu Pro Met Leu Pro
   1 5
<210> 298
   <211> 7
   <212> PRT
   <213> Artificial Sequence
<220>
   <223> SH3 ANTAGONIST PEPTIDE
<400> 298
   Arg Pro Leu Pro Ile Leu Pro
   1 5
<210> 299
   <211> 7
   <212> PRT
   <213> Artificial Sequence
<220>
   <223> SH3 ANTAGONIST PEPTIDE
<400> 299
   Arg Pro Leu Pro Ser Leu Pro
   1 5
<210> 300
   <211> 7
   <212> PRT
   <213> Artificial Sequence
<220>
   <223> SH3 ANTAGONIST PEPTIDE
<400> 300
   Arg Pro Leu Pro Ser Leu Pro
   1 5
<210> 301
   <211> 7
   <212> PRT
   <213> Artificial Sequence
<220>
   <223> SH3 ANTAGONIST PEPTIDE
<400> 301
   Arg Pro Leu Pro Met Ile Pro
   1 5
<210> 302
   <211> 7
   <212> PRT
   <213> Artificial Sequence
<220>
   <223> SH3 ANTAGONIST PEPTIDE
<400> 302
   Arg Pro Leu Pro Leu Ile Pro
   1 5
<210> 303
   <211> 7
   <212> PRT
   <213> Artificial Sequence
<220>
   <223> SH3 ANTAGONIST PEPTIDE
<400> 303
   Arg Pro Leu Pro Pro Thr Pro
   1 5
<210> 304
   <211> 7
   <212> PRT
   <213> Artificial Sequence
<220>
   <223> SH3 ANTAGONIST PEPTIDE
<400> 304
   Arg Ser Leu Pro Pro Leu Pro
   1 5
<210> 305
   <211> 7
   <212> PRT
   <213> Artificial Sequence
<220>
   <223> SH3 ANTAGONIST PEPTIDE
<400> 305
   Arg Pro Gln Pro Pro Pro Pro
   1 5
<210> 306
   <211> 7
   <212> PRT
   <213> Artificial Sequence
<220>
   <223> SH3 ANTAGONIST PEPTIDE
<400> 306
   Arg Gln Leu Pro Ile Pro Pro
   1 5
<210> 307
   <211> 12
   <212> PRT
   <213> Artificial Sequence
<220>
   <223> SH3 ANTAGONIST PEPTIDE
<220>
   <221> misc_feature
   <222> (1, 2, 3)..(11)
   <223> Xaa = any amino acid
<400> 307
   Xaa Xaa Xaa Arg Pro Leu Pro Pro Leu Pro Xaa Pro
   1 5 10
<210> 308
   <211> 12
   <212> PRT
   <213> Artificial Sequence
<220>
   <223> SH3 ANTAGONIST PEPTIDE
<220>
   <221> misc_feature
   <222> (1, 2, 3, 11)..(12)
   <223> Xaa = any amino acid
<400> 308
   Xaa Xaa Xaa Arg Pro Leu Pro Pro Ile Pro Xaa Xaa
   1 5 10
<210> 309
   <211> 12
   <212> PRT
   <213> Artificial Sequence
<220>
   <223> SH3 ANTAGONIST PEPTIDE
<220>
   <221> misc_feature
   <222> (1, 2, 3, 11,)..(12)
   <223> Xaa = any amino acid
<400> 309
   Xaa Xaa Xaa Arg Pro Leu Pro Pro Leu Pro Xaa Xaa
   1 5 10
<210> 310
   <211> 12
   <212> PRT
   <213> Artificial Sequence
<220>
   <223> SH3 ANTAGONIST PEPTIDE
<220>
   <221> misc_feature
   <222> (2, 3, 10)..(11)
   <223> Xaa = any amino acid
<400> 310
   Arg Xaa Xaa Arg Pro Leu Pro Pro Leu Pro Xaa Pro
   1 5 10
<210> 311
   <211> 12
   <212> PRT
   <213> Artificial Sequence
<220>
   <223> SH3 ANTAGONIST PEPTIDE
<220>
   <221> misc_feature
   <222> (2)..(3)
   <223> Xaa = any amino acid
<400> 311
   Arg Xaa Xaa Arg Pro Leu Pro Pro Leu Pro Pro Pro
   1 5 10
<210> 312
   <211> 12
   <212> PRT
   <213> Artificial Sequence
<220>
   <223> SH3 ANTAGONIST PEPTIDE
<220>
   <221> misc_feature
   <222> (11)..(12)
   <223> Xaa = any amino acid
<400> 312
   Pro Pro Pro Tyr Pro Pro Pro Pro Ile Pro Xaa Xaa
   1 5 10
<210> 313
   <211> 12
   <212> PRT
   <213> Artificial Sequence
<220>
   <223> SH3 ANTAGONIST PEPTIDE
<220>
   <221> misc_teature
   <222> (11)..(12)
   <223> Xaa = any amino acid
<400> 313
   Pro Pro Pro Tyr Pro Pro Pro Pro Val Pro Xaa Xaa
   1 5 10
<210> 314
   <211> 10
   <212> PRT
   <213> Artificial Sequence
<220>
   <223> SH3 ANTAGONIST PEPTIDE
<220>
   <221> misc_feature
   <222> (2, 3)..(8)
   <223> Xaa (Pos2, 3, 8) is any amino acid
<220>
   <221> misc_feature
   <222> (9)..(9)
   <223> Xaa (Pos 9) represents an aliphatic amino acid residue
<400> 314
   Leu Xaa Xaa Arg Pro Leu Pro Xaa Xaa Pro
   1               5                   10
<210> 315
   <211> 10
   <212> PRT
   <213> Artificial Sequence
<220>
   <223> SH3 ANTAGONIST PEPTIDE
<220>
   <221> misc_feature
   <222> (1)..(1)
   <223> Position 1, Xaa is an aliphatic amino acid residue
<220>
   <221> misc_feature
   <222> (2, 3)..(8)
   <223> Positions 2, 3 & 8, Xaa is any amino acid
<400> 315
   Xaa Xaa Xaa Arg Pro Leu Pro Xaa Leu Pro
   1               5                   10
<210> 316
   <211> 10
   <212> PRT
   <213> Artificial Sequence
<220>
   <223> SH3 ANTAGONIST PEPTIDE
<220>
   <221> misc_feature
   <222> (3)..(3)
   <223> Position 3, Xaa is any amino acid residue
<220>
   <221> misc_feature
   <222> (4)..(4)
   <223> Position 4, Xaa is an aromatic amino acid residue
<220>
   <221> misc_feature
   <222> (9)..(9)
   <223> Position 9, Xaa is an aliphatic amino acid residue
<400> 316
   Pro Pro Xaa Xaa Tyr Pro Pro Pro Xaa Pro
   1 5 10
<210> 317
   <211> 11
   <212> PRT
   <213> Artificial Sequence
<220>
   <223> SH3 ANTAGONIST PEPTIDE
<220>
   <221> misc_feature
   <222> (1)..(1)
   <223> Position 1, Xaa is a basic amino acid residue
<220>
   <221> misc_feature
   <222> (4)..(4) residue
   <223> Position 4, Xaa is an aliphatic amino acid residue
<220>
   <221> mise_feature
   <222> (6)..(9)
   <223> Positions 6 & 9, Xaa is any amino acid residue
<400> 317
   Xaa Pro Pro Xaa Pro Xaa Lys Pro Xaa Trp Leu
   1 5 10
<210> 318
   <211> 11
   <212> PRT
   <213> Artificial Sequence
<220>
   <223> SH3 ANTAGONIST PEPTIDE
<220>
   <221> misc_feature
   <222> (3, 4)..(6)
   <223> Positions 3, 4 & 6, Xaa is an aliphatic amino acid residue
<220>
   <221> misc_feature
   <222> (8)..(8)
   <223> Position 8, Xaa is a basic amino acid residue
<220>
   <221> misc_feature
   <222> (10)..(10)
   <223> Position 10, Xaa is any amino acid residue
<400> 318
   Arg Pro Xaa Xaa Pro Xaa Arg Xaa Ser Xaa Pro
   1 5 10
<210> 319
   <211> 11
   <212> PRT
   <213> Artificial Sequence

<220>
   <223> SH3 ANTAGONIST PEPTIDE
<220>
   <221> misc_feature
   <222> (8)..(9)
   <223> Xaa = any amino acid
<400> 319
   Pro Pro Val Pro Pro Arg Pro Xaa Xaa Thr Leu
   1 5 10
<210> 320
   <211> 7
   <212> PRT
   <213> Artificial Sequence
<220>
   <223> SH3 ANTAGONIST PEPTIDE
<220>
   <221> misc_feature
   <222> (1, 3)..(6)
   <223> Positions 1, 3 and 6, Xaa is an aliphatic amino acid residue
<400> 320
   Xaa Pro Xaa Leu Pro Xaa Lys
   1 5
<210> 321
   <211> 10
   <212> PRT
   <213> Artificial Sequence
<220>
   <223> SH3 ANTAGONIST PEPTIDE
<220>
   <221> misc_feature
   <222> (1)..(1)
   <223> Position 1, Xaa is a basic amino acid residue
<220>
   <221> misc_feature
   <222> (2)..(2)
   <223> Position 2, Xaa is an aromatic amino acid residue
<220>
   <221> misc_feature
   <222> (4).. (8)
   <223> Positions 4 & 8, Xaa is any amino acid residue
<400> 321
   Xaa Xaa Asp Xaa Pro Leu Pro Xaa Leu Pro
   1 5 10
<210> 322
   <211> 7
   <212> PRT
   <213> Artificial Sequence
<220>
   <223> INHIBITION OF PLATELET AGGREGATION
<220>
   <221> misc_feature
   <222> (2)..(3)
   <223> Xaa = any amino acid
<400> 322
   Cys Xaa Xaa Arg Gly Asp Cys
   1 5
<210> 323
   <211> 7
   <212> PRT
   <213> Artificial Sequence
<220>
   <223> SRC ANTAGONIST
<400> 323
   Arg Pro Leu Pro Pro Leu Pro
   1 5
<210> 324
   <211> 6
   <212> PRT
   <213> Artificial Sequence
<220>
   <223> SRC ANTAGONIST
<400> 324
   Pro Pro Val Pro Pro Arg
   1 5
<210> 325
   <211> 11
   <212> PRT
   <213> Artificial Sequence
<220>
   <223> ANTI-CANCER
<220>
   <221> mise_feature
   <222> (1, 3, 5, 7, 8, 10)..(11)
   <223> Xaa = any amino acid
<400> 325
   Xaa Phe Xaa Asp Xaa Trp Xaa Xaa Leu Xaa Xaa
   1 5 10
<210> 326
   <211> 20
   <212> PRT
   <213> Artificial Sequence
<220>
   <223> P16-MIMETIC
<400> 326
   Lys Ala Cys Arg Arg Leu Phe Gly Pro Val Asp Ser Glu Gln Leu Ser
   1 5 10 15
   Arg Asp Cys Asp
   20
<210> 327
   <211> 20
   <212> PRT
   <213> Artificial Sequence
<220>
   <223> P16-MIMETIC
<400> 327
   Arg Glu Arg Trp Asn Phe Asp Phe Val Thr Glu Thr Pro Leu Glu Gly
   1 5 10 15
   Asp Phe Ala Trp
   20
<210> 328
   <211> 20
   <212> PRT
   <213> Artificial Sequence
<220>
   <223> P16-MIMETIC
<400> 328
   Lys Arg Arg Gln Thr Ser Met Thr Asp Phe Tyr His Ser Lys Arg Arg
   1 5 10 15
   Leu Ile Phe Ser
   20
<210> 329
   <211> 20
   <212> PRT
   <213> Artificial Sequence
<220>
   <223> P16-MIMETIC
<400> 329
   Thr Ser Met Thr Asp Phe Tyr His Ser Lys Arg Arg Leu Ile Phe Ser
   1 5 10 15
   Lys Arg Lys Pro
   20
<210> 330
   <211> 5
   <212> PRT
   <213> Artificial Sequence
<220>
   <223> P16-MIMETIC
<400> 330
   Arg Arg Leu Ile Phe
   1 5
<210> 331
   <211> 36
   <212> PRT
   <213> Artificial Sequence
<220>
   <223> P16-MIMETIC
<400> 331
<210> 332
   <211> 24
   <212> PRT
   <213> Artificial Sequence
<220>
   <223> P16-MIMETIC
<400> 332
   Lys Arg Arg Leu Ile Phe Ser Lys Arg Gln Ile Lys Ile Trp Phe Gln
   1 5 10 15
   Asn Arg Arg Met Lys Trp Lys Lys
   20
<210> 333
   <211> 8
   <212> PRT
   <213> Artificial Sequence
<220>
   <223> PREFERRED LINKER
<400> 333
   Gly Gly Gly Lys Gly Gly Gly Gly
   1 5
<210> 334
   <211> 8
   <212> PRT
   <213> Artificial Sequence
<220>
   <223> PREFERRED LINKER
<400> 334
   Gly Gly Gly Asn Gly Ser Gly Gly
   1 5
<210> 335
   <211> 8
   <212> PRT
   <213> Artificial Sequence
<220>
   <223> PREFERRED LINKER
<400> 335
   Gly Gly Gly Cys Gly Gly Gly Gly
   1 5
<210> 336
   <211> 5
   <212> PRT
   <213> Artificial Sequence
<220>
   <223> PREFERRED LINKER
<400> 336
   Gly Pro Asn Gly Gly
   1 5
<210> 337
   <211> 41
   <212> PRT
   <213> Artificial Sequence
<220>
   <223> TPO-MIMETIC
<220>
   <221> misc_feature
   <222> (1)..(1)
   <223> Fc domain attached at Position 1 of the N-terminus
<400> 337
   Gly Gly Gly Gly Gly Ile Glu Gly Pro Thr Leu Arg Gln Trp Leu Ala
   1 5 10 15
   Ala Arg Ala Gly Gly Gly Gly Gly Gly Gly Gly Ile Glu Gly Pro Thr
   20 25 30
   Leu Arg Gln Trp Leu Ala Ala Arg Ala
   35 40
<210> 338
   <211> 41
   <212> PRT
   <213> Artificial Sequence
<220>
   <223> TPO-MIMETIC
<220>
   <221> misc_feature
   <222> (41)..(41)
   <223> Fc domain attached at Position 41 of the C-terminus
<400> 338
   Ile Glu Gly Pro Thr Leu Arg Gln Trp Leu Ala Ala Arg Ala Gly Gly
   1               5                   10                  15
   Gly Gly Gly Gly Gly Gly Ile Glu Gly Pro Thr Leu Arg Gln Trp Leu
               20                  25                  30
   Ala Ala Arg Ala Gly Gly Gly Gly Gly
           35                  40
<210> 339
   <211> 49
   <212> PRT
   <213> Artificial Sequence
<220>
   <223> EPO-MIMETIC
<220>
   <221> misc_feature
   <222> (1).. (1)
   <223> Fc domain attached at Position 1 of the N-terminus
<400> 339
   Gly Gly Gly Gly Gly Gly Gly Thr Tyr Ser Cys His Phe Gly Pro Leu
   1               5                   10                  15
   Thr Trp Val Cys Lys Pro Gln Gly Gly Gly Gly Gly Gly Gly Gly Thr
               20                  25                  30
   Tyr Ser Cys His Phe Gly Pro Leu Thr Trp Val Cys Lys Pro Gln Gly
           35                  40                  45
   Gly
<210> 340
   <211> 49
   <212> PRT
   <213> Artificial Sequence
<220>
   <223> EPO-MIMETIC
<220>
   <221> misc_feature
   <222> (49)..(49)
   <223> Fc domain attached at Position 49 of the C-terminus
<400> 340
   Gly Gly Thr Tyr Ser Cys His Phe Gly Pro Leu Thr Trp Val Cys Lys
   1 5 10 15
   Pro Gln Gly Gly Gly Gly Gly Gly Gly Gly Thr Tyr Ser Cys His Phe
   20 25 30
   Gly Pro Leu Thr Trp Val Cys Lys Pro Gln Gly Gly Gly Gly Gly Gly
   35 40 45
   Gly
<210> 341
   <211> 28
   <212> PRT
   <213> Artificial Sequence
<220>
   <223> TPO-MIMETIC PEPTIDES
<400> 341
   Ile Glu Gly Pro Thr Leu Arg Gln Trp Leu Ala Ala Arg Ala Ile Glu
   1 5 10 15
   Gly Pro Thr Leu Arg Gln Trp Leu Ala Ala Arg Ala
   20 25
<210> 342
   <211> 29
   <212> PRT
   <213> Artificial Sequence
<220>
   <223> TPO-MIMETIC PEPTIDES
<400> 342
   Ile Glu Gly Pro Thr Leu Arg Gln Trp Leu Ala Ala Arg Ala Gly Ile
   1 5 10 15
   Glu Gly Pro Thr Leu Arg Gln Trp Leu Ala Ala Arg Ala
   20 25
<210> 343
   <211> 30
   <212> PRT
   <213> Artificial Sequence
<220>
   <223> TPO-MIMETIC PEPTIDES
<400> 343
   Ile Glu Gly Pro Thr Leu Arg Gln Trp Leu Ala Ala Arg Ala Gly Gly
   1 5 10 15
   Ile Glu Gly Pro Thr Leu Arg Gln Trp Leu Ala Ala Arg Ala
   20 25 30
<210> 344
   <211> 31
   <212> PRT
   <213> Artificial Sequence
<220>
   <223> TPO-MIMETIC PEPTIDES
<400> 344
   Ile Glu Gly Pro Thr Leu Arg Gln Trp Leu Ala Ala Arg Ala Gly Gly
   1 5 10 15
   Gly Ile Glu Gly Pro Thr Leu Arg Gln Trp Leu Ala Ala Arg Ala
   20 25 30
<210> 345
   <211> 32
   <212> PRT
   <213> Artificial Sequence
<220>
   <223> TPO-MTMETIC PEPTIDES
<400> 345
   Ile Glu Gly Pro Thr Leu Arg Gln Trp Leu Ala Ala Arg Ala Gly Gly
   1 5 10 15
   Gly Gly Ile Glu Gly Pro Thr Leu Arg Gln Trp Leu Ala Ala Arg Ala
   20 25 30
<210> 346
   <211> 33
   <212> PRT
   <213> Artificial Sequence
<220>
   <223> TPO-MIMETIC PEPTIDES
<400> 346
   Ile Glu Gly Pro Thr Leu Arg Gln Trp Leu Ala Ala Arg Ala Gly Gly
   1 5 10 15
   Gly Gly Gly Ile Glu Gly Pro Thr Leu Arg Gln Trp Leu Ala Ala Arg
   20 25 30
   Ala
<210> 347
   <211> 34
   <212> PRT
   <213> Artificial Sequence
<220>
   <223> TPO-MIMETIC PEPTIDES
<400> 347
   Ile Glu Gly Pro Thr Leu Arg Gln Trp Leu Ala Ala Arg Ala Gly Gly
   1 5 10 15
   Gly Gly Gly Gly Ile Glu Gly Pro Thr Leu Arg Gln Trp Leu Ala Ala
   20 25 30
   Arg Ala
<210> 348
   <211> 35
   <212> PRT
   <213> Artificial Sequence
<220>
   <223> TPO-MIMETIC PEPTIDES
<400> 348
   Ile Glu Gly Pro Thr Leu Arg Gln Trp Leu Ala Ala Arg Ala Gly Gly
   1 5 10 15
   Gly Gly Gly Gly Gly Ile Glu Gly Pro Thr Leu Arg Gln Trp Leu Ala
   20 25 30
   Ala Arg Ala
   35
<210> 349
   <211> 36
   <212> PRT
   <213> Artificial Sequence
<220>
   <223> TPO-MIMETIC PEPTIDES
<400> 349
   Ile Glu Gly Pro Thr Leu Arg Gln Trp Leu Ala Ala Arg Ala Gly Gly
   1 5 10 15
   Gly Gly Gly Gly Gly Gly Ile Glu Gly Pro Thr Leu Arg Gln Trp Leu
   20 25 30
   Ala Ala Arg Ala
   35
<210> 350
   <211> 37
   <212> PRT
   <213> Artificial Sequence
<220>
   <223> TPO-MIMETIC PEPTIDES
<400> 350
   Ile Glu Gly Pro Thr Leu Arg Gln Trp Leu Ala Ala Arg Ala Gly Gly
   1 5 10 15
   Gly Gly Gly Gly Gly Gly Gly Ile Glu Gly Pro Thr Leu Arg Gln Trp
   20 25 30
   Leu Ala Ala Arg Ala
   35
<210> 351
   <211> 38
   <212> PRT
   <213> Artificial Sequence
<220>
   <223> TPO-MIMETIG PEPTIDES
<400> 351
   Ile Glu Gly Pro Thr Leu Arg Gln Trp Leu Ala Ala Arg Ala Gly Gly
   1 5 10 15
   Gly Gly Gly Gly Gly Gly Gly Gly Ile Glu Gly Pro Thr Leu Arg Gln
   20 25 30
   Trp Leu Ala Ala Arg Ala
   35
<210> 352
   <211> 42
   <212> PRT
   <213> Artificial Sequence
<220>
   <223> TPO-MIMETIC PEPTIDES
<400> 352
   Ile Glu Gly Pro Thr Leu Arg Gln Trp Leu Ala Ala Arg Ala Gly Gly
   1               5                   10                  15
   Gly Gly Gly Gly Gly Gly Gly Gly Gly Gly Gly Gly Ile Glu Gly Pro
               20                  25                  30
   Thr Leu Arg Gln Trp Leu Ala Ala Arg Ala
           35                  40
<210> 353
   <211> 32
   <212> PRT
   <213> Artificial Sequence
<220>
   <223> TPO-MIMETIC PEPTIDES
<400> 353
   Ile Glu Gly Pro Thr Leu Arg Gln Trp Leu Ala Ala Arg Ala Gly Pro
   1               5                   10                  15
   Asn Gly Ile Glu Gly Pro Thr Leu Arg Gln Trp Leu Ala Ala Arg Ala
               20                  25                  30
<210> 354
   <211> 36
   <212> PRT
   <213> Artificial Sequence
<220>
   <223> TPO-MIMETIC PEPTIDES
<400> 354
   Ile Glu Gly Pro Thr Leu Arg Gln Trp Leu Ala Ala Arg Ala Gly Gly
   1               5                   10                  15
   Gly Gly Gly Gly Gly Gly Ile Glu Gly Pro Thr Leu Arg Gln Trp Leu
               20                  25                  30
   Ala Ala Arg Ala
           35
<210> 355
   <211> 36
   <212> PRT
   <213> Artificial Sequence
<220>
   <223> TPO-MIMETIC PEPTIDES
<400> 355
   Ile Glu Gly Pro Thr Leu Arg Gln Trp Leu Ala Ala Arg Ala Gly Gly
   1 5 10 15
   Gly Gly Gly Gly Gly Gly Ile Glu Gly Pro Thr Leu Arg Gln Trp Leu
   20 25 30
   Ala Ala Arg Ala
   35
<210> 356
   <211> 36
   <212> PRT
   <213> Artificial Sequence
<220>
   <223> TPO-MIMETIC PEPTIDES
<400> 356
   Ile Glu Gly Pro Thr Leu Arg Gln Trp Leu Ala Ala Arg Ala Gly Gly
   1 5 10 15
   Gly Gly Gly Gly Gly Gly Ile Glu Gly Pro Thr Leu Arg Gln Trp Leu
   20 25 30
   Ala Ala Arg Ala
   35
<210> 357
   <211> 36
   <212> PRT
   <213> Artificial Sequence
<220>
   <223> TPO-MIMETIC PEPTIDES
<400> 357
   Ile Glu Gly Pro Thr Leu Arg Gln Trp Leu Ala Ala Arg Ala Gly Gly
   1 5 10 15
   Gly Lys Gly Gly Gly Gly Ile Glu Gly Pro Thr Leu Arg Gln Trp Leu
   20 25 30
   Ala Ala Arg Ala
   35
<210> 358
   <211> 37
   <212> PRT
   <213> Artificial Sequence
<220>
   <223> TPO-MIMETIC PEPTIDES
<220>
   <221> misc_feature
   <222> (18)..(18)
   <223> Position 18, bromoacetyl attached
<400> 358
<210> 359
   <211> 36
   <212> PRT
   <213> Artificial Sequence
<220>
   <223> TPO-MIMETIC PEPTIDES
<400> 359
   Ile Glu Gly Pro Thr Leu Arg Gln Trp Leu Ala Ala Arg Ala Gly Gly
   1               5                   10                  15
   Gly Cys Gly Gly Gly Gly Ile Glu Gly Pro Thr Leu Arg Gln Trp Leu
               20                  25                  30
   Ala Ala Arg Ala
           35
<210> 360
   <211> 37
   <212> PRT
   <213> Artificial Sequence
<220>
   <223> TPO-MIMETIC PEPTIDES
<220>
   <221> misc_feature
   <222> (18)..(18)
   <223> Position 18, Poly(ethylene glycol) attached
<400> 360
<210> 361
   <211> 37
   <212> PRT
   <213> Artificial Sequence
<220>
   <223> TPO-MIMETIC PEPTIDES
<220>
   <221> misc_feature
   <222> (18)..(18)
   <223> Position 18, Poly(ethylene glycol) attached
<400> 361
<210> 362
   <211> 36
   <212> PRT
   <213> Artificial Sequence
<220>
   <223> TPO-MIMETIC PEPTIDES
<400> 362
   Ile Glu Gly Pro Thr Leu Arg Gln Trp Leu Ala Ala Arg Ala Gly Gly
   1 5 10 15
   Gly Asn Gly Ser Gly Gly Ile Glu Gly Pro Thr Leu Arg Gln Trp Leu
   20 25 30
   Ala Ala Arg Ala
   35
<210> 363
   <211> 36
   <212> PRT
   <213> Artificial Sequence
<220>
   <223> TPO-MIMETIC PEPTIDES
<400> 363
   Ile Glu Gly Pro Thr Leu Arg Gln Trp Leu Ala Ala Arg Ala Gly Gly
   1 5 10 15
   Gly Cys Gly Gly Gly Gly Ile Glu Gly Pro Thr Leu Arg Gln Trp Leu
   20 25 30
   Ala Ala Arg Ala
   35
<210> 364
   <211> 57
   <212> DNA
   <213> Artificial Sequence
<220>
   <223> OLIGONUCLEOTIDE USED TO CONSTRUCT TMP
<400> 364
   aaaaaaggat cctcgagatt aagcacgagc agccagccac tgacgcagag tcggacc 57
<210> 365
   <211> 39
   <212> DNA
   <213> Artificial Sequence
<220>
   <223> OLIGONUCLEOTIDE USED TO CONSTRUCT TMP
<400> 365
   aaaggtggag gtggtggtat cgaaggtccg actctgcgt 39
<210> 366
   <211> 42
   <212> DNA
   <213> Artificial Sequence
<220>
   <223> OLIGONUCLEOTIDE USED TO CONSTRUCT TMP
<400> 366
   cagtggctgg ctgctcgtgc ttaatctcga ggatcctttt tt 42
<210> 367
   <211> 81
   <212> DNA
   <213> Artificial Sequence
<220>
   <223> TMP CONSTRUCT
<220>
   <221> CDS
   <222> (1)..(60)
<400> 367
   aaa ggt gga ggt ggt ggt atc gaa ggt ccg act ctg cgt cag tgg ctg 48
   Lys Gly Gly Gly Gly Gly Ile Glu Gly Pro Thr Leu Arg Gln Trp Leu
   1 5 10 15
   gct gct cgt gct taatctcgag gatccttttt t 81
   Ala Ala Arg Ala
   20
<210> 368
   <211> 20
   <212> PRT
   <213> Artificial Sequence
<220>
   <223> Synthetic Construct
<400> 368
   Lys Gly Gly Gly Gly Gly Ile Glu Gly Pro Thr Leu Arg Gln Trp Leu
   1 5 10 15
   Ala Ala Arg Ala
   20
<210> 369
   <211> 22
   <212> DNA
   <213> Artificial Sequence
<220>
   <223> PCR PRIMER FOR Fc CONSTRUCT
<400> 369
   aacataagta cctgtaggat cg 22
<210> 370
   <211> 52
   <212> DNA
   <213> Artificial Sequence
<220>
   <223> PCR PRIMER FOR Fc CONSTRUCT
<400> 370
   ttcgatacca ccacctccac ctttacccgg agacagggag aggctcttct gc 52
<210> 371
   <211> 60
   <212> DNA
   <213> Artificial Sequence
<220>
   <223> OLIGONUCLEOTIDE USED TO CONSTRUCT TMP-TMP SEQUENCE
<400> 371
   aaaggtggag gtggtggtat cgaaggtccg actctgcgtc agtggctggc tgctcgtgct 60
<210> 372
   <211> 48
   <212> DNA
   <213> Artificial Sequence
<220>
   <223> OLIGONUCLEOTIDE USED TO CONSTRUCT TMP-TMP SEQUENCE
<400> 372
   acctccacca ccagcacgag cagccagcca ctgacgcaga gtcggacc 48
<210> 373
   <211> 66
   <212> DNA
   <213> Artificial Sequence
<220>
   <223> OLIGONUCLEOTIDE USED TO CONSTRUCT TMP-TMP SEQUENCE
<400> 373
   ggtggtggag gtggcggcgg aggtattgag ggcccaaccc ttcgccaatg gcttgcagca 60
   cgcgca 66
<210> 374
   <211> 76
   <212> PRT
   <213> Artificial Sequence
<220>
   <223> OLIGONUCLEOTIDE USED TO CONSTRUCT TMP-TMP SEQUENCE
<400> 374
   Ala Ala Ala Ala Ala Ala Ala Gly Gly Ala Thr Cys Cys Thr Cys Gly
   1 5 10 15
   Ala Gly Ala Thr Thr Ala Thr Gly Cys Gly Cys Gly Thr Gly Cys Thr
   20 25 30
   Gly Cys Ala Ala Gly Cys Cys Ala Thr Thr Gly Gly Cys Gly Ala Ala
   35 40 45
   Gly Gly Gly Thr Thr Gly Gly Gly Cys Cys Cys Thr Cys Ala Ala Thr
   50 55 60
   Ala Cys Cys Thr Cys Cys Gly Cys Cys Gly Cys Cys
   65 70 75
<210> 375
   <211> 126
   <212> DNA
   <213> Artificial Sequence
<220>
   <223> TMP-TMP CONSTRUCT
<220>
   <221> CDS
   <222> (1)..(126)
<400> 375
   aaa ggt gga ggt ggt ggt atc gaa ggt ccg act ctg cgt cag tgg ctg      48
   Lys Gly Gly Gly Gly Gly Ile Glu Gly Pro Thr Leu Arg Gln Trp Leu
   1               5                   10                  15
   gct gct cgt gct ggt ggt gga ggt ggc ggc gga ggt att gag ggc cca      96
   Ala Ala Arg Ala Gly Gly Gly Gly Gly Gly Gly Gly Ile Glu Gly Pro
               20                  25                  30
   acc ctt cgc caa tgg ctt gca gca cgc gca                              126
   Thr Leu Arg Gln Trp Leu Ala Ala Arg Ala
           35                  40
<210> 376
   <211> 42
   <212> PRT
   <213> Artificial Sequence
<220>
   <223> Synthetic Construct
<400> 376
   Lys Gly Gly Gly Gly Gly Ile Glu Gly Pro Thr Leu Arg Gln Trp Leu
   1               5                   10                  15
   Ala Ala Arg Ala Gly Gly Gly Gly Gly Gly Gly Gly Ile Glu Gly Pro
               20                  25                  30
   Thr Leu Arg Gln Trp Leu Ala Ala Arg Ala
           35                  40
<210> 377
   <211> 39
   <212> DNA
   <213> Artificial Sequence
<220>
   <223> OLIGONUCLEOTIDE USED IN CONSTRUCTION OF TMP-TMP CONSTRUCT

<400> 377
   ttttttcata tgatcgaagg tccgactctg cgtcagtgg 39
<210> 378
   <211> 48
   <212> DNA
   <213> Artificial Sequence
<220>
   <223> OLIGONUCLEOTIDE USED IN CONSTRUCTION OF TMP-TMP CONSTRUCT
<400> 378
   agcacgagca gccagccact gacgcagagt cggaccttcg atcatatg 48
<210> 379
   <211> 45
   <212> DNA
   <213> Artificial Sequence
<220>
   <223> OLIGONUCLEOTIDE USED IN CONSTRUCTION OF TMP-TMP CONSTRUCT
<400> 379
   ctggctgctc gtgctggtgg aggcggtggg gacaaaactc acaca 45
<210> 380
   <211> 51
   <212> DNA
   <213> Artificial Sequence
<220>
   <223> OLIGONUCLEOTIDE USED IN CONSTRUCTION OF TMP-TMP CONSTRUCT
<400> 380
   ctggctgctc gtgctggcgg tggtggcgga gggggtggca ttgagggccc a 51
<210> 381
   <211> 54
   <212> DNA
   <213> Artificial Sequence
<220>
   <223> OLIGONUCLEOTIDE USED IN CONSTRUCTION OF TMP-TMP CONSTRUCT
<400> 381
   aagccattgg cgaagggttg ggccctcaat gccaccccct ccgccaccac cgcc 54
<210> 382
   <211> 54
   <212> DNA
   <213> Artificial Sequence
<220>
   <223> OLIGONUCLEOTIDE USED IN CONSTRUCTION OF TMP-TMP CONSTRUCT
<400> 382
   acccttcgcc aatggcttgc agcacgcgca gggggaggcg gtggggacaa aact 54
<210> 383
   <211> 27
   <212> DNA
   <213> Artificial Sequence
<220>
   <223> OLIGONUCLEOTIDE USED IN CONSTRUCTION OF TMP-TMP CONSTRUCT
<400> 383
   cccaccgcct ccccctgcgc gtgctgc 27
<210> 384
   <211> 189
   <212> DNA
   <213> Artificial Sequence
<220>
   <223> TMP-TMP CONSTRUCT
<220>
   <221> CDS
   <222> (10)..(180)
<400> 384
   ttttttcat atg atc gaa ggt ccg act ctg cgt cag tgg ctg gct gct cgt      51
             Met Ile Glu Gly Pro Thr Leu Arg Gln Trp Leu Ala Ala Arg
             1               5                   10
   gct ggc ggt ggt ggc gga ggg ggt ggc att gag ggc cca acc ctt cgc       99
   Ala Gly Gly Gly Gly Gly Gly Gly Gly Ile Glu Gly Pro Thr Leu Arg
   15                  20                  25                  30
   caa tgg ctg gct gct cgt gct ggt gga ggc ggt ggg gac aaa act ctg      147
   Gln Trp Leu Ala Ala Arg Ala Gly Gly Gly Gly Gly Asp Lys Thr Leu
                   35                  40                  45
   gct gct cgt gct ggt gga ggc ggt ggg gac aaa actcacaca                189
   Ala Ala Arg Ala Gly Gly Gly Gly Gly Asp Lys
               50                  55
<210> 385
   <211> 57
   <212> PRT
   <213> Artificial Sequence
<220>
   <223> Synthetic Construct
<400> 385
   Met Ile Glu Gly Pro Thr Leu Arg Gln Trp Leu Ala Ala Arg Ala Gly
   1               5                   10                  15
   Gly Gly Gly Gly Gly Gly Gly Ile Glu Gly Pro Thr Leu Arg Gln Trp
               20                  25                  30
   Leu Ala Ala Arg Ala Gly Gly Gly Gly Gly Asp Lys Thr Leu Ala Ala
           35                  40                  45
   Arg Ala Gly Gly Gly Gly Gly Asp Lys
       50                  55
<210> 386
   <211> 141
   <212> DNA
   <213> Artificial Sequence
<220>
   <223> SEQUENCE COMPRISING PL PROMOTER USED TO CONSTRUCTpAMG21
<400> 386
   ctaattccgc tctcacctac caaacaatgc ccccctgcaa aaaataaatt catataaaaa 60
   acatacagat aaccatctgc ggtgataaat tatctctggc ggtgttgaca taaataccac 120
   tggcggtgat actgagcaca t 141
<210> 387
   <211> 55
   <212> DNA
   <213> Artificial Sequence
<220>
   <223> SEQUENCE COMPRISING PL PROMOTER USED TO CONSTRUCTpAMG21
<400> 387
   cgatttgatt ctagaaggag gaataacata tggttaacgc gttggaattc ggtac 55
<210> 388
   <211> 872
   <212> DNA
   <213> Artificial Sequence
<220>
   <223> SEQUENCE COMPRISING PL PROMOTER USED TO CONSTRUCTGM221
<400> 388
   ttattttcgt gcggccgcac cattatcacc gccagaggta aactagtcaa cacgcacggt 60
   gttagatatt tatcccttgc ggtgatagat tgagcacatc gatttgattc tagaaggagg 120
   gataatatat gagcacaaaa aagaaaccat taacacaaga gcagcttgag gacgcacgtc 180
   gccttaaagc aatttatgaa aaaaagaaaa atgaacttgg cttatcccag gaatctgtcg 240
   cagacaagat ggggatgggg cagtcaggcg ttggtgcttt atttaatggc atcaatgcat 300
   taaatgctta taacgccgca ttgcttacaa aaattctcaa agttagcgtt gaagaattta 360
   gcccttcaat cgccagagaa tctacgagat gtatgaagcg gttagtatgc agccgtcact 420
   tagaagtgag tatgagtacc ctgttttttc tcatgttcag gcagggatgt tctcacctaa 480
   gcttagaacc tttaccaaag gtgatgcgga gagatgggta agcacaacca aaaaagccag 540
   tgattctgca ttctggcttg aggttgaagg taattccatg accgcaccaa caggctccaa 600
   gccaagcttt cctgacggaa tgttaattct cgttgaccct gagcaggctg ttgagccagg 660
   tgatttctgc atagccagac ttgggggtga tgagtttacc ttcaagaaac tgatcaggga 720
   tagcggtcag gtgtttttac aaccactaaa cccacagtac ccaatgatcc catgcaatga 780
   gagttgttcc gttgtgggga aagttatcgc tagtcagtgg cctgaagaga cgtttggctg 840
   atagactagt ggatccacta gtgtttctgc cc 872
<210> 389
   <211> 1197
   <212> DNA
   <213> Artificial Sequence
<220>
   <223> SEQUENCE COMPRISING PL PROMOTER USED TO CONSTRUCTGM221
<400> 389
   ggcggaaacc gacgtccatc gaatggtgca aaacctttcg cggtatggca tgatagcgcc 60
   cggaagagag tcaattcagg gtggtgaatg tgaaaccagt aacgttatac gatgtcgcag 120
   agtatgccgg tgtctcttat cagaccgttt cccgcgtggt gaaccaggcc agccacgttt 180
   ctgcgaaaac gcgggaaaaa gtcgaagcgg cgatggcgga gctgaattac attcccaacc 240
   gcgtggcaca acaactggcg ggcaaacagt cgctcctgat tggcgttgcc acctccagtc 300
   tggccctgca cgcgccgtcg caaattgtcg cggcgattaa atctcgcgcc gatcaactgg 360
   gtgccagcgt ggtggtgtcg atggtagaac gaagcggcgt cgaagcctgt aaagcggcgg 420
   tgcacaatct tctcgcgcaa cgcgtcagtg ggctgatcat taactatccg ctggatgacc 480
   aggatgccat tgctgtggaa gctgcctgca ctaatgttcc ggcgttattt cttgatgtct 540
   ctgaccagac acccatcaac agtattattt tctcccatga agacggtacg cgactgggcg 600
   tggagcatct ggtcgcattg ggtcaccagc aaatcgcgct gttagcgggc ccattaagtt 660
   ctgtctcggc gcgtctgcgt ctggctggct ggcataaata tctcactcgc aatcaaattc 720
   agccgatagc ggaacgggaa ggcgactgga gtgccatgtc cggttttcaa caaaccatgc 780
   aaatgctgaa tgagggcatc gttcccactg cgatgctggt tgccaacgat cagatggcgc 840
   tgggcgcaat gcgcgccatt accgagtccg ggctgcgcgt tggtgcggat atctcggtag 900
   tgggatacga cgataccgaa gacagctcat gttatatccc gccgttaacc accatcaaac 960
   aggattttcg cctgctgggg caaaccagcg tggaccgctt gctgcaactc tctcagggcc 1020
   aggcggtgaa gggcaatcag ctgttgcccg tctcactggt gaaaagaaaa accaccctgg 1080
   cgcccaatac gcaaaccgcc tctccccgcg cgttggccga ttcattaatg cagctggcac 1140
   gacaggtttc ccgactggaa agcggacagt aaggtaccat aggatccagg cacagga 1197
<210> 390
   <211> 61
   <212> DNA
   <213> Artificial Sequence
<220>
   <223> SEQUENCE COMPRISING PL PROMOTER USED TO CONSTRUCT EMP
<400> 390
   tatgaaaggt ggaggtggtg gtggaggtac ttactcttgc cacttcggcc cgctgacttg 60
   g 61
<210> 391
   <211> 72
   <212> DNA
   <213> Artificial Sequence
<220>
   <223> SEQUENCE COMPRISING PL PROMOTER USED TO CONSTRUCT EMP
<400> 391
   cggtttgcaa acccaagtca gcgggccgaa gtggcaagag taagtacctc caccaccacc 60
   tccacctttc at 72
<210> 392
   <211> 57
   <212> DNA
   <213> Artificial Sequence
<220>
   <223> SEQUENCE COMPRISING PL PROMOTER USED TO CONSTRUCT EMP
<400> 392
   gtttgcaaac cgcagggtgg cggcggcggc ggcggtggta cctattcctg tcatttt 57
<210> 393
   <211> 60
   <212> DNA
   <213> Artificial Sequence
<220>
   <223> SEQUENCE COMPRISING PL PROMOTER USED TO CONSTRUCT EMP
<400> 393
   ccaggtcagc gggccaaaat gacaggaata ggtaccaccg ccgccgccgc cgccaccctg 60
<210> 394
   <211> 118
   <212> DNA
   <213> Artificial Sequence
<220>
   <223> SEQUENCE COMPRISING PL PROMOTER USED TO CONSTRUCT EMP
<220>
   <221> CDS
   <222> (2)..(118)
<400> 394
   t atg aaa ggt gga ggt ggt ggt gga ggt act tac tct tgc cac ttc ggc      49
     Met Lys Gly Gly Gly Gly Gly Gly Gly Thr Tyr Ser Cys His Phe Gly
     1               5                   10                  15
   ccg ctg act tgg gtt tgc aaa ccg cag ggt ggc ggc ggc ggc ggc ggt        97
   Pro Leu Thr Trp Val Cys Lys Pro Gln Gly Gly Gly Gly Gly Gly Gly
               20                  25                  30
   ggt acc tat tcc tgt cat ttt                                           118
   Gly Thr Tyr Ser Cys His Phe
           35
<210> 395
   <211> 39
   <212> PRT
   <213> Artificial Sequence
<220>
   <223> Synthetic Construct
<400> 395
   Met Lys Gly Gly Gly Gly Gly Gly Gly Thr Tyr Ser Cys His Phe Gly
   1 5 10 15
   Pro Leu Thr Trp Val Cys Lys Pro Gln Gly Gly Gly Gly Gly Gly Gly
   20 25 30
   Gly Thr Tyr Ser Cys His Phe
   35
<210> 396
   <211> 61
   <212> DNA
   <213> Artificial Sequence
<220>
   <223> SENSE PCR PRIMER TO AMPLIFY EMP CONSTRUCT
<400> 396
   gcagaagagc ctctccctgt ctccgggtaa aggtggaggt ggtggtggag gtacttactc 60
   t 61
<210> 397
   <211> 40
   <212> DNA
   <213> Artificial Sequence
<220>
   <223> ANTISENSE PCR PRIMER TO AMPLIFY EMP CONSTRUCT
<400> 397
   ctaattggat ccacgagatt aaccaccctg cggtttgcaa 40
<210> 398
   <211> 18
   <212> DNA
   <213> Artificial Sequence
<220>
   <223> Fc PRIMER
<400> 398
<210> 399
   <211> 61
   <212> DNA
   <213> Artificial Sequence
<220>
   <223> PCR PRIMER FOR Fc-LINKER SEQUENCE
<400> 399
   agagtaagta cctccaccac cacctccacc tttacccgga gacagggaga ggctcttctg 60
   c 61
<210> 400
   <211> 61
   <212> DNA
   <213> Artificial Sequence
<220>
   <223> OLIGONUCLEOTIDE USED TO CONSTRUCT EMP
<400> 400
   ggcccgctga cctgggtatg taagccacaa gggggtgggg gaggcggggg gtaatctcga 60
   g 61
<210> 401
   <211> 50
   <212> DNA
   <213> Artificial Sequence
<220>
   <223> OLIGONUCLEOTIDE USED TO CONSTRUCT EMP
<400> 401
   gatcctcgag attacccccc gcctccccca cccccttgtg gcttacatac 50
<210> 402
   <211> 118
   <212> DNA
   <213> Artificial Sequence
<220>
   <223> EMP CONSTRUCT
<220>
   <221> CDS
   <222> (1)..(108)
<400> 402
   gtt tgc aaa ccg cag ggt ggc ggc ggc ggc ggc ggt ggt acc tat tcc      48
   Val Cys Lys Pro Gln Gly Gly Gly Gly Gly Gly Gly Gly Thr Tyr Ser
   1               5                   10                  15
   tgt cat ttt ggc ccg ctg acc tgg gta tgt aag cca caa ggg ggt ggg      96
   Cys His Phe Gly Pro Leu Thr Trp Val Cys Lys Pro Gln Gly Gly Gly
               20                  25                  30
   gga ggc ggg ggg taatctcgag                                           118
   Gly Gly Gly Gly
           35
<210> 403
   <211> 36
   <212> PRT
   <213> Artificial Sequence
<220>
   <223> Synthetic Construct
<400> 403
   Val Cys Lys Pro Gln Gly Gly Gly Gly Gly Gly Gly Gly Thr Tyr Ser
   1 5 10 15
   Cys His Phe Gly Pro Leu Thr Trp Val Cys Lys Pro Gln Gly Gly Gly
   20 25 30
   Gly Gly Gly Gly
   35
<210> 404
   <211> 39
   <212> DNA
   <213> Artificial Sequence
<220>
   <223> SENSE PCR PRIMER FOR EMP CONSTRUCT
<400> 404
   ttatttcata tgaaaggtgg taactattcc tgtcatttt 39
<210> 405
   <211> 43
   <212> DNA
   <213> Artificial Sequence
<220>
   <223> ANTISENSE PCR PRIMER FOR EMP CONSTRUCT
<400> 405
   tggacatgtg tgagttttgt cccccccgcc tcccccaccc cct 43
<210> 406
   <211> 43
   <212> DNA
   <213> Artificial Sequence
<220>
   <223> PCR PRIMER FOR Fc CONSTRUCT
<400> 406
   agggggtggg ggaggcgggg gggacaaaac tcacacatgt cca 43
<210> 407
   <211> 20
   <212> DNA
   <213> Artificial Sequence
<220>
   <223> PCR PRIMER FOR Fc CONSTRUCT
<400> 407
   gttattgctc agcggtggca 20
<210> 408
   <211> 60
   <212> DNA
   <213> Artificial Sequence
<220>
   <223> OLIGONUCLEOTIDE USED TO CONSTRUCT EMP-EMP-Fc
<400> 408
   ttttttatcg atttgattct agatttgagt tttaactttt agaaggagga ataaaatatg 60
<210> 409
   <211> 41
   <212> DNA
   <213> Artificial Sequence
<220>
   <223> OLIGONUCLEOTIDE USED TO CONSTRUCT EMP-EMP-Fc
<400> 409
   taaaagttaa aactcaaatc tagaatcaaa tcgataaaaa a 41
<210> 410
   <211> 51
   <212> DNA
   <213> Artificial Sequence
<220>
   <223> OLIGONUCLEOTIDE USED TO CONSTRUCT EMP-EMP-Fc
<400> 410
   ggaggtactt actcttgcca cttcggcccg ctgacttggg tttgcaaacc g 51
<210> 411
   <211> 55
   <212> DNA
   <213> Artificial Sequence
<220>
   <223> OLIGONUCLEOTIDE USED TO CONSTRUCT EMP-EMP-Fc
<400> 411
   agtcagcggg ccgaagtggc aagagtaagt acctcccata ttttattcct ccttc 55
<210> 412
   <211> 60
   <212> DNA
   <213> Artificial Sequence
<220>
   <223> OLIGONUCLEOTIDE USED TO CONSTRUCT EMP-EMP-Fc
<400> 412
   cagggtggcg gcggcggcgg cggtggtacc tattcctgtc attttggccc gctgacctgg 60
<210> 413
   <211 > 60
   <212> DNA
   <213> Artificial Sequence
<220>
   <223> OLIGONUCLEOTIDE USED TO CONSTRUCT EMP-EMP-Fc
<400> 413
   aaaatgacag gaataggtac caccgccgcc gccgccgcca ccctgcggtt tgcaaaccca 60
<210> 414
   <211> 57
   <212> DNA
   <213> Artificial Sequence
<220>
   <223> OLIGONUCLEOTIDE USED TO CONSTRUCT EMP-EMP-Fc
<400> 414
   gtatgtaagc cacaaggggg tgggggaggc gggggggaca aaactcacac atgtcca 57
<210> 415
   <211 > 60
   <212> DNA
   <213> Artificial Sequence
<220>
   <223> OLIGONUCLEOTIDE USED TO CONSTRUCT EMP-EMP-Fc
<400> 415
   agttttgtcc cccccgcctc ccccaccccc ttgtggctta catacccagg tcagcgggcc 60
<210> 416
   <211> 228
   <212> DNA
   <213> Artificial Sequence
<220>
   <223> EMP-EMP CONSTRUCT
<220>
   <221> CDS
   <222> (58).. (228)
<400> 416
   ttttttatcg atttgattct agatttgagt tttaactttt agaaggagga ataaaat          57
   atg gga ggt act tac tct tgc cac ttc ggc ccg ctg act tgg gtt tgc       105
   Met Gly Gly Thr Tyr Ser Cys His Phe Gly Pro Leu Thr Trp Val Cys
   1               5                   10                  15
   aaa ccg cag ggt ggc ggc ggc ggc ggc ggt ggt acc tat tcc tgt cat       153
   Lys Pro Gln Gly Gly Gly Gly Gly Gly Gly Gly Thr Tyr Ser Cys His
               20                  25                  30
   ttt ggc ccg ctg acc tgg gta tgt aag cca caa ggg ggt ggg gga ggc       201
   Phe Gly Pro Leu Thr Trp Val Cys Lys Pro Gln Gly Gly Gly Gly Gly
           35                  40                  45
   ggg ggg gac aaa act cac aca tgt cca                                   228
   Gly Gly Asp Lys Thr His Thr Cys Pro
       50                  55
<210> 417
   <211> 57
   <212> PRT
   <213> Artificial Sequence
<220>
   <223> Synthetic Construct
<400< 417
<210> 418
   <211 > 40
   <212> DNA
   <213> Artificial Sequence
<220>
   <223> PCR PRIMER FOR EMP-EMP CONSTRUCT
<400> 418
   ctaattggat cctcgagatt aacccccttg tggcttacat 40
<210> 419
   <211> 16
   <212> PRT
   <213> Artificial Sequence
<220>
   <223> EPO-MIMETIC PEPTIDE
<220>
   <221> misc_feature
   <222> (1)..(1)
   <223> Xaa (Positions 1) can be any one of the 20 L-amino acids
<220>
   <221> misc_feature
   <222> (3)..(4)
   <223> Xaa (Positions 3, 4) can be any one of the 20 L-amino acids
<220>
   <221> misc_feature
   <222> (5)..(5)
   <223> Xaa can be R, H, L or W
<220>
   <221> misc_feature
   <222> (6).. (6)
   <223> Xaa can be M, F or I
<220>
   <221> misc_feature
   <222> (9)..(9)
   <223> Xaa (Position 9) can be any one of the 20 L-amino acids
<220>
   <221> misc_feature
   <222> (12)..(12)
   <223> Xaa can be D, E, I, L or V
<220>
   <221> misc_feature
   <222> (13)..(13)
   <223> Xaa can be C, A, α-amino-γ-bromobutyric acid or Hoc
<220>
   <221> misc_feature
   <222> (14)..(16)
   <223> Xaa (Positions 14, 15, 16) can be any one of the 20 L-amino acids
<400> 419
   Xaa Tyr Xaa Xaa Xaa Xaa Gly Pro Xaa Thr Trp Xaa Xaa Xaa Xaa Xaa
   1               5                   10                  15
<210> 420
   <211> 16
   <212> PRT
   <213> Artificial Sequence
<220>
   <223> EPO-MIMETIC PEPTIDE
<220>
   <221> misc_feature
   <222> (1, 3, 5, 6, 9, 12, 14, 15)..(16)
   <223> Xaa = any amino acid residue
<400> 420
   Xaa Tyr Xaa Cys Xaa Xaa Gly Pro Xaa Thr Trp Xaa Cys Xaa Xaa Xaa
   1 5 10 15
<210> 421
   <211> 10
   <212> PRT
   <213> Artificial Sequence
<220>
   <223> EPO-MIMETIC PEPTIDE
<220>
   <221> misc_feature
   <222> (2)..(2)
   <223> Xaa can be R, H, L, or W
<220>
   <221> misc_feature
   <222> (3)..(3)
   <223> Xaa can be M, F, or I
<220>
   <221> misc_feature
   <222> (6)..(6)
   <223> Xaa is independently selected from any one of the 20 genetically coded L-amino acids or the steroisomeric
   D-amino acids
<220>
   <221> misc_feature
   <222> (9)..(9)
   <223> Xaa can be D, E, I, L, or V.
<400> 421
   Cys Xaa Xaa Gly Pro Xaa Thr Trp Xaa Cys
   1 5 10
<210> 422
   <211> 19
   <212> PRT
   <213> Artificial Sequence
<220>
   <223> EPO-MIMETIC PEPTIDE
<400> 422
   Gly Gly Thr Tyr Ser Cys His Gly Pro Leu Thr Trp Val Cys Lys Pro
   1 5 10 15
   Gln Gly Gly
<210> 423
   <211> 19
   <212> PRT
   <213> Artificial Sequence
<220>
   <223> EPO-MIMETIC PEPTIDE
<400> 423
   Val Gly Asn Tyr Met Ala His Met Gly Pro Ile Thr Trp Val Cys Arg
   1 5 10 15
   Pro Gly Gly
<210> 424
   <211> 18
   <212> PRT
   <213> Artificial Sequence
<220>
   <223> EPO-MIMETIC PEPTIDE
<400> 424
   Gly Gly Pro His His Val Tyr Ala Cys Arg Met Gly Pro Leu Thr Trp
   1 5 10 15
   Ile Cys
<210> 425
   <211> 18
   <212> PRT
   <213> Artificial Sequence
<220>
   <223> EPO-MIMETIC PEPTIDE
<400> 425
   Gly Gly Thr Tyr Ser Cys His Phe Gly Pro Leu Thr Trp Val Cys Lys
   1 5 10 15
   Pro Gln
<210> 426
   <211> 20
   <212> PRT
   <213> Artificial Sequence
<220>
   <223> EPO-MIMETIC PEPTIDE
<400> 426
   Gly Gly Leu Tyr Ala Cys His Met Gly Pro Met Thr Trp Val Cys Gln
   1 5 10 15
   Pro Leu Arg Gly
   20
<210> 427
   <211> 22
   <212> PRT
   <213> Artificial Sequence
<220>
   <223> EPO-MIMETIC PEPTIDE
<400> 427
   Thr Ile Ala Gln Tyr Ile Cys Tyr Met Gly Pro Glu Thr Trp Glu Cys
   1 5 10 15
   Arg Pro Ser Pro Lys Ala
   20
<210> 428
   <211> 13
   <212> PRT
   <213> Artificial Sequence
<220>
   <223> EPO-MIMETIC PEPTIDE
<400> 428
   Tyr Ser Cys His Phe Gly Pro Leu Thr Trp Val Cys Lys
   1 5 10
<210> 429
   <211> 11
   <212> PRT
   <213> Artificial Sequence
<220>
   <223> EPO-MIMETIC PEPTIDE
<400> 429
   Tyr Cys His Phe Gly Pro Leu Thr Trp Val Cys
   1 5 10
<210> 430
   <211> 17
   <212> PRT
   <213> Artificial Sequence
<220>
   <223> UKR ANTAGONIST PEPTIDE
<400> 430
   Ala Glu Pro Val Tyr Gln Tyr Glu Leu Asp Ser Tyr Leu Arg Ser Tyr
   1 5 10 15
   Tyr
<210> 431
   <211> 17
   <212> PRT
   <213> Artificial Sequence
<220>
   <223> UKR ANTAGONIST PEPTIDE
<400> 431
   Ala Glu Leu Asp Leu Ser Thr Phe Tyr Asp Ile Gln Tyr Leu Leu Arg
   1 5 10 15
   Thr
<210> 432
   <211> 17
   <212> PRT
   <213> Artificial Sequence
<220>
   <223> UKR ANTAGONIST PEPTIDE
<400> 432
   Ala Glu Phe Phe Lys Leu Gly Pro Asn Gly Tyr Val Tyr Leu His Ser
   1 5 10 15
   Ala
<210> 433
   <211> 11
   <212> PRT
   <213> Artificial Sequence
<220>
   <223> UKR ANTAGONIST PEPTIDE
<220>
   <221> misc_feature
   <222> (4, 5)..(6)
   <223> Xaa = any amino acid
<400> 433
   Phe Lys Leu Xaa Xaa Xaa Gly Tyr Val Tyr Leu
   1 5 10
<210> 434
   <211> 17
   <212> PRT
   <213> Artificial Sequence
<220>
   <223> UKR ANTAGONIST PEPTIDE
<400> 434
   Ala Glu Ser Thr Tyr His His Leu Ser Leu Gly Tyr Met Tyr Thr Leu
   1 5 10 15
   Asn
<210> 435
   <211> 11
   <212> PRT
   <213> Artificial Sequence
<220>
   <223> UKR ANTAGONIST PEPTIDE
<220>
   <221> misc_feature
   <222> (3, 5)..(6)
   <223> Xaa = any amino acid
<400> 435
   Tyr His Xaa Leu Xaa Xaa Gly Tyr Met Tyr Thr
   1 5 10
<210> 436
   <211> 6
   <212> PRT
   <213> Artificial Sequence
<220>
   <223> MAST CELL ANTAGONISTS/PROTEASE INHIBITOR PEPTIDE
<400> 436
   Arg Asn Arg Gln Lys Thr
   1 5
<210> 437
   <211> 4
   <212> PRT
   <213> Artificial Sequence
<220>
   <223> MAST CELL ANTAGONISTS/PROTEASE INHIBITOR PEPTIDE
<400> 437
   Arg Asn Arg Gln
   1
<210> 438
   <211> 5
   <212> PRT
   <213> Artificial Sequence
<220>
   <223> MAST CELL ANTAGONISTS/PROTEASE INHIBITOR PEPTIDE
<400> 438
   Arg Asn Arg Gln Lys
   1 5
<210> 439
   <211> 5
   <212> PRT
   <213> Artificial Sequence
<220>
   <223> MAST CELL ANTAGONISTS/PROTEASE INHIBITOR PEPTIDE
<400> 439
   Asn Arg Gln Lys Thr
   1 5
<210> 440
   <211> 4
   <212> PRT
   <213> Artificial Sequence
<220>
   <223> MAST CELL ANTAGONISTS/PROTEASE INHIBITOR PEPTIDE
<400> 440
   Arg Gln Lys Thr
   1
<210> 441
   <211> 7
   <212> PRT
   <213> Artificial Sequence
<220>
   <223> INTEGRIN-BINDING PEPTIDE
<220>
   <221> misc_feature
   <222> (2, 5)..(7)
   <223> Xaa = any amino acid
<400> 441
   Arg Xaa Glu Thr Xaa Trp Xaa
   1 5
<210> 442
   <211> 7
   <212> PRT
   <213> Artificial Sequence
<220>
   <223> INTEGRIN-BINDING PEPTIDE
<220>
   <221> misc_feature
   <222> (2, 5)..(7)
   <223> Xaa = any amino acid
<400> 442
   Arg Xaa Glu Thr Xaa Trp Xaa
   1 5

<210> 443
   <211> 5
   <212> PRT
   <213> Artificial Sequence
<220>
   <223> INTEGRIN-BINDING PEPTIDE
<220>
   <221> misc_feature
   <222> (5)..(5)
   <223> Xaa = any amino acid
<400> 443
   Arg Gly Asp Gly Xaa
   1 5
<210> 444
   <211> 7
   <212> PRT
   <213> Artificial Sequence
<220>
   <223> INTEGRIN-BINDING PEPTIDE
<220>
   <221> misc_feature
   <222> (6)..(6)
   <223> Xaa = any amino acid
<400> 444
   Cys Arg Gly Asp Gly Xaa Cys
   1 5
<210> 445
   <211> 15
   <212> PRT
   <213> Artificial Sequence
<220>
   <223> INTEGRIN-BINDING PEPTIDE
<220>
   <221> misc_feature
   <222> (2, 3, 4, 8, 9, 10, 11, 12, 13)..(14)
   <223> Xaa = any amino acid
<400> 445
   Cys Xaa Xaa Xaa Arg Leu Asp Xaa Xaa Xaa Xaa Xaa Xaa Xaa Cys
   1               5                   10                  15
<210> 446
   <211> 9
   <212> PRT
   <213> Artificial Sequence
<220>
   <223> INTEGRIN-BINDING PEPTIDE
<400> 446
   Cys Ala Arg Arg Leu Asp Ala Pro Cys
   1               5
<210> 447
   <211> 9
   <212> PRT
   <213> Artificial Sequence
<220>
   <223> INTEGRIN-BINDING PEPTIDE
<400> 447
   Cys Pro Ser Arg Leu Asp Ser Pro Cys
   1               5
<210> 448
   <211> 9
   <212> PRT
   <213> Artificial Sequence
<220>
   <223> INTEGRIN-BINDING PEPTIDE
<220>
   <221> misc_feature
   <222> (1, 2, 3, 7, 8)..(9)
   <223> Xaa are capable of forming a cyclizing bond
<220>
   <221> misc_feature
   <222> (2)..(5)
   <223> Feature at 1, 5 is an amino acid capable of forming a cyclying bond and attached to 1-5 amino acid linker
<400> 448
   Xaa Xaa Xaa Arg Gly Asp Xaa Xaa Xaa
   1 5
<210> 449
   <211> 9
   <212> PRT
   <213> Artificial Sequence
<220>
   <223> INTEGRIN-BINDING PEPTIDE
<220>
   <221> misc_feature
   <222> (2)..(8)
   <223> Xaa = any amino acid
<400> 449
   Cys Xaa Cys Arg Gly Asp Cys Xaa Cys
   1 5
<210> 450
   <211> 9
   <212> PRT
   <213> Artificial Sequence
<220>
   <223> INTEGRIN-BINDING PEPTIDE
<400> 450
   Cys Asp Cys Arg Gly Asp Cys Phe Cys
   1 5
<210> 451
   <211> 9
   <212> PRT
   <213> Artificial Sequence
<220>
   <223> INTEGRIN-BINDING PEPTIDE
<400> 451
   Cys Asp Cys Arg Gly Asp Cys Leu Cys
   1 5
<210> 452
   <211> 9
   <212> PRT
   <213> Artificial Sequence
<220>
   <223> INTEGRIN-BINDING PEPTIDE
<400> 452
   Cys Leu Cys Arg Gly Asp Cys Ile Cys
   1 5
<210> 453
   <211> 8
   <212> PRT
   <213> Artificial Sequence
<220>
   <223> INTEGRIN-BINDING PEPTIDE
<220>
   <221> misc_feature
   <222> (1, 2, 5, 6, 7)..(8)
   <223> Xaa = any amino acid
<400> 453
   Xaa Xaa Asp Asp Xaa Xaa Xaa Xaa
   1 5
<210> 454
   <211> 10
   <212> PRT
   <213> Artificial Sequence
<220>
   <223> INTEGRIN-BINDING PEPTIDE
<220>
   <221> misc_feature
   <222> (1, 2, 3, 6, 7, 8, 9)..(10)
   <223> Xaa = any amino acid
<400> 454
   Xaa Xaa Xaa Asp Asp Xaa Xaa Xaa Xaa Xaa
   1 5 10
<210> 455
   <211> 8
   <212> PRT
   <213> Artificial Sequence
<220>
   <223> INTEGRIN-BINDING PEPTIDE
<400> 455
   Cys Trp Asp Asp Gly Trp Leu Cys
   1 5
<210> 456
   <211> 9
   <212> PRT
   <213> Artificial Sequence
<220>
   <223> INTEGRIN-BINDING PEPTIDE
<400> 456
   Cys Trp Asp Asp Leu Trp Trp Leu Cys
   1 5
<210> 457
   <211> 8
   <212> PRT
   <213> Artificial Sequence
<220>
   <223> INTEGRIN-BINDING PEPTIDE
<400> 457
   Cys Trp Asp Asp Gly Leu Met Cys
   1 5
<210> 458
   <211> 8
   <212> PRT
   <213> Artificial Sequence
<220>
   <223> INTEGRIN-BINDING PEPTIDE
<400> 458
   Cys Trp Asp Asp Gly Trp Met Cys
   1 5
<210> 459
   <211> 9
   <212> PRT
   <213> Artificial Sequence
<220>
   <223> INTEGRIN-BINDING PEPTIDE
<400> 459
   Cys Ser Trp Asp Asp Gly Trp Leu Cys
   1 5
<210> 460
   <211> 9
   <212> PRT
   <213> Artificial Sequence
<220>
   <223> INTEGRIN-BINDING PEPTIDE
<400> 460
   Cys Pro Asp Asp Leu Trp Trp Leu Cys
   1 5
<210> 461
   <211> 12
   <212> PRT
   <213> Artificial Sequence
<220>
   <223> EPO-MIMETIC PEPTIDE
<220>
   <221> misc_feature
   <222> (2)..(8)
   <223> Xaa can be any of the 20 L-amino acids
<220>
   <221> misc_feature
   <222> (3)..(3)
   <223> Xaa can be C, A, a-amino-y-bromobutyric acid or Hoc
<220>
   <221> misc_feature
   <222> (4)..(4)
   <223> Xaa can be R, H, L or W
<220>
   <221> misc_feature
   <222> (5).. (5)
   <223> Xaa can be M, F or I; Xaa
<220>
   <221> misc_feature
   <222> (11)..(11)
   <223> Xaa can be D, E, I, L or V
<220>
   <221> misc_feature
   <222> (12)..(12)
   <223> Xaa can be C, A, a-amino-y-bromobutyric acid or Hoc; provided that Xaa (Pos3 or 12) is C or Hoc.
<400> 461
   Tyr Xaa Xaa Xaa Xaa Gly Pro Xaa Thr Trp Xaa Xaa
   1 5 10
<210> 462
   <211> 16
   <212> PRT
   <213> Artificial Sequence
<220>
   <223> SELECTIN ANTAGONIST PEPTIDE
<400> 462
   Cys Gln Asn Arg Tyr Thr Asp Leu Val Ala Ile Gln Asn Lys Asn Glu
   1 5 10 15
<210> 463
   <211> 17
   <212> PRT
   <213> Artificial Sequence
<220>
   <223> SELECTIN ANTAGONIST PEPTIDE
<400> 463
   Ala Glu Asn Trp Ala Asp Asn Glu Pro Asn Asn Lys Arg Asn Asn Glu
   1 5 10 15
   Asp
<210> 464
   <211> 19
   <212> PRT
   <213> Artificial Sequence
<220>
   <223> SELECTIN ANTAGONIST PEPTIDE
<400> 464
   Arg Lys Asn Asn Lys Thr Trp Thr Trp Val Gly Thr Lys Lys Ala Leu
   1 5 10 15
   Thr Asn Glu
<210> 465
   <211> 13
   <212> PRT
   <213> Artificial Sequence
<220>
   <223> SELECTIN ANTAGONIST PEPTIDE
<400> 465
   Lys Lys Ala Leu Thr Asn Glu Ala Glu Asn Trp Ala Asp
   1 5 10
<210> 466
   <211> 16
   <212> PRT
   <213> Artificial Sequence
<220>
   <223> SELECTIN ANTAGONIST PEPTIDE
<220>
   <221> misc_feature
   <222> (3)..(15)
   <223> Xaa = any amino acid
<400> 466
   Cys Gln Xaa Arg Tyr Thr Asp Leu Val Ala Ile Gln Asn Lys Xaa Glu
   1 5 10 15
<210> 467
   <211> 19
   <212> PRT
   <213> Artificial Sequence
<220>
   <223> SELECTIN ANTAGONIST PEPTIDE
<220>
   <221> misc_feature
   <222> (3, 5, 6, 13)..(15)
   <223> Xaa = any amino acid
<400> 467
   Arg Lys Xaa Asn Xaa Xaa Trp Thr Trp Val Gly Thr Xaa Lys Xaa Leu
   1 5 10 15
   Thr Glu Glu
<210> 468
   <211> 17
   <212> PRT
   <213> Artificial Sequence
<220>
   <223> SELECTIN ANTAGONIST PEPTIDE
<220>
   <221> misc_feature
   <222> (13)..(15)
   <223> Xaa = any amino acid
<400> 468
   Ala Glu Asn Trp Ala Asp Gly Glu Pro Asn Asn Lys Xaa Asn Xaa Glu
   1 5 10 15
   Asp
<210> 469
   <211> 16
   <212> PRT
   <213> Artificial Sequence
<220>
   <223> SELECT1N ANTAGONIST PEPTIDE
<220>
   <221> misc_feature
   <222> (2, 3, 4, 7)..(15)
   <223> Xaa = any amino acid
<400> 469
   Cys Xaa Xaa Xaa Tyr Thr Xaa Leu Val Ala Ile Gln Asn Lys Xaa Glu
   1 5 10 15
<210> 470
   <211> 19
   <212> PRT
   <213> Artificial Sequence
<220>
   <223> SELECTIN ANTAGONIST PEPTIDE
<220>
   <221> misc_feature
   <222> (3, 4, 5, 6, 8, 13, 15)..(18)
   <223> Xaa = any amino acid
<400> 470
   Arg Lys Xaa Xaa Xaa Xaa Trp Xaa Trp Val Gly Thr Xaa Lys Xaa Leu
   1 5 10 15
   Thr Xaa Glu
<210> 471
   <211> 16
   <212> PRT
   <213> Artificial Sequence
<220>
   <223> SELECTIN ANTAGONIST PEPTIDE
<220>
   <221> misc_feature
   <222> (2, 5, 6, 7, 12, 13)..(14)
   <223> Xaa = any amino acid
<400> 471
   Ala Xaa Asn Trp Xaa Xaa Xaa Glu Pro Asn Asn Xaa Xaa Xaa Glu Asp
   1 5 10 15
<210> 472
   <211> 13
   <212> PRT
   <213> Artificial Sequence
<220>
   <223> SELECTIN ANTAGONIST PEPTIDE
<220>
   <221> misc_feature
   <222> (1, 3, 6, 9, 12)..(13)
   <223> Xaa = any amino acid
<400> 472
   Xaa Lys Xaa Lys Thr Xaa Glu Ala Xaa Asn Trp Xaa Xaa
   1 5 10
<210> 473
   <211> 12
   <212> PRT
   <213> Artificial Sequence
<220>
   <223> SOMATOSTATIN OR CORTISTATIN MIMETIC PEPTIDE
<220>
   <221> misc_feature
   <222> (2)..(2)
   <223> Xaa is Arg or Lys
<220>
   <221> misc_feature
   <222> (10)..(10)
   <223> Xaa is Ser or Thr
<400> 473
   Cys Xaa Asn Phe Phe Trp Lys Thr Phe Xaa Ser Cys
   1 5 10
<210> 474
   <211> 17
   <212> PRT
   <213> Artificial Sequence
<220>
   <223> SOMATOSTATIN OR CORTISTATIN MIMETIC PEPTIDE
<400> 474
   Asp Arg Met Pro Cys Arg Asn Phe Phe Trp Lys Thr Phe Ser Ser Cys
   1 5 10 15
   Lys
<210> 475
   <211 > 15
   <212> PRT
   <213> Artificial Sequence
<220>
   <223> SOMATOSTATIN OR CORTISTATIN MIMETIC PEPTIDE
<400> 475
   Met Pro Cys Arg Asn Phe Phe Trp Lys Thr Phe Ser Ser Cys Lys
   1 5 10 15
<210> 476
   <211> 13
   <212> PRT
   <213> Artificial Sequence
<220>
   <223> SOMATOSTATIN OR CORTISTATIN MIMETIC PEPTIDE
<400> 476
   Cys Arg Asn Phe Phe Trp Lys Thr Phe Ser Ser Cys Lys
   1 5 10
<210> 477
   <211> 16
   <212> PRT
   <213> Artificial Sequence
<220>
   <223> SOMATOSTATIN OR CORTISTATIN MIMETIC PEPTIDE
<400> 477
   Asp Arg Met Pro Cys Arg Asn Phe Phe Trp Lys Thr Phe Ser Ser Cys
   1 5 10 15
<210> 478
   <211> 14
   <212> PRT
   <213> Artificial Sequence
<220>
   <223> SOMATOSTATIN OR CORTISTATIN MIMETIC PEPTIDE
<400> 478
   Met Pro Cys Arg Asn Phe Phe Trp Lys Thr Phe Ser Ser Cys
   1 5 10
<210> 479
   <211> 12
   <212> PRT
   <213> Artificial Sequence
<220>
   <223> SOMATOSTATIN OR CORTISTATIN MIMETIC PEPTIDE
<400> 479
   Cys Arg Asn Phe Phe Trp Lys Thr Phe Ser Ser Cys
   1 5 10
<210> 480
   <211> 16
   <212> PRT
   <213> Artificial Sequence
<220>
   <223> SOMATOSTATIN OR CORTISTATIN MIMETIC PEPTIDE
<400> 480
   Asp Arg Met Pro Cys Lys Asn Phe Phe Trp Lys Thr Phe Ser Ser Cys
   1 5 10 15
<210> 481
   <211> 15
   <212> PRT
   <213> Artificial Sequence
<220>
   <223> SOMATOSTATIN OR CORTISTATIN MIMETIC PEPTIDE
<400> 481
   Met Pro Cys Lys Asn Phe Phe Trp Lys Thr Phe Ser Ser Cys Lys
   1 5 10 15
<210> 482
   <211 > 13
   <212> PRT
   <213> Artificial Sequence
<220>
   <223> SOMATOSTATIN OR CORTISTATIN MIMETIC PEPTIDE
<400> 482
   Cys Lys Asn Phe Phe Trp Lys Thr Phe Ser Ser Cys Lys
   1 5 10
<210> 483
   <211> 16
   <212> PRT
   <213> Artificial Sequence
<220>
   <223> SOMATOSTATIN OR CORTISTATIN MIMETIC PEPTIDE
<400> 483
   Asp Arg Met Pro Cys Lys Asn Phe Phe Trp Lys Thr Phe Ser Ser Cys
   1 5 10 15
<210> 484
   <211> 14
   <212> PRT
   <213> Artificial Sequence
<220>
   <223> SOMATOSTATIN OR CORTISTATIN MIMETIC PEPTIDE
<400> 484
   Met Pro Cys Lys Asn Phe Phe Trp Lys Thr Phe Ser Ser Cys
   1 5 10
<210> 485
   <211> 12
   <212> PRT
   <213> Artificial Sequence
<220>
   <223> SOMATOSTATIN OR CORTISTATIN MIMETIC PEPTIDE
<400> 485
   Cys Lys Asn Phe Phe Trp Lys Thr Phe Ser Ser Cys
   1 5 10
<210> 486
   <211> 17
   <212> PRT
   <213> Artificial Sequence
<220>
   <223> SOMATOSTATIN OR CORTISTATIN MIMETIC PEPTIDE
<400> 486
   Asp Arg Met Pro Cys Arg Asn Phe Phe Trp Lys Thr Phe Thr Ser Cys
   1 5 10 15
   Lys
<210> 487
   <211> 15
   <212> PRT
   <213> Artificial Sequence
<220>
   <223> SOMATOSTATIN OR CORTISTATIN MIMETIC PEPTIDE
<400> 487
   Met Pro Cys Arg Asn Phe Phe Trp Lys Thr Phe Thr Ser Cys Lys
   1 5 10 15
<210> 488
   <211 13
   <212> PRT
   <213> Artificial Sequence
<220>
   <223> SOMATOSTATIN OR CORTISTATIN MIMETIC PEPTIDE
<400> 488
<210> 489
   <211> 16
   <212> PRT
   <213> Artificial Sequence
<220>
   <223> SOMATOSTATIN OR CORTISTATIN MIMETIC PEPTIDE
<400> 489
   Asp Arg Met Pro Cys Arg Asn Phe Phe Trp Lys Thr Phe Thr Ser Cys
   1 5 10 15
<210> 490
   <211> 14
   <212> PRT
   <213> Artificial Sequence
<220>
   <223> SOMATOSTATIN OR CORTISTATIN MIMETIC PEPTIDE
<400> 490
   Met Pro Cys Arg Asn Phe Phe Trp Lys Thr Phe Thr Ser Cys
   1 5 10
<210> 491
   <211> 12
   <212> PRT
   <213> Artificial Sequence
<220>
   <223> SOMATOSTATIN OR CORTISTATIN MIMETIC PEPTIDE
<400> 491
   Cys Arg Asn Phe Phe Trp Lys Thr Phe Thr Ser Cys
   1 5 10
<210> 492
   <211> 17
   <212> PRT
   <213> Artificial Sequence
<220>
   <223> SOMATOSTATIN OR CORTISTATIN MIMETIC PEPTIDE
<400> 492
   Asp Arg Met Pro Cys Lys Asn Phe Phe Trp Lys Thr Phe Thr Ser Cys
   1 5 10 15
   Lys
<210> 493
   <211> 15
   <212> PRT
   <213> Artificial Sequence
<220>
   <223> SOMATOSTATIN OR CORTISTATIN MIMETIC PEPTIDE
<400> 493
   Met Pro Cys Lys Asn Phe Phe Trp Lys Thr Phe Thr Ser Cys Lys
   1 5 10 15
<210> 494
   <211> 13
   <212> PRT
   <213> Artificial Sequence
<220>
   <223> SOMATOSTATIN OR CORTISTATIN MIMETIC PEPTIDE
<400> 494
   Cys Lys Asn Phe Phe Trp Lys Thr Phe Thr Ser Cys Lys
   1 5 10
<210> 495
   <211> 16
   <212> PRT
   <213> Artificial Sequence
<220>
   <223> SOMATOSTATIN OR CORTISTATIN MIMETIC PEPTIDE
<400> 495
   Asp Arg Met Pro Cys Lys Asn Phe Phe Trp Lys Thr Phe Thr Ser Cys
   1 5 10 15
<210> 496
   <211> 14
   <212> PRT
   <213> Artificial Sequence
<220>
   <223> SOMATOSTATIN OR CORTISTATIN MIMETIC PEPTIDE
<400> 496
   Met Pro Cys Lys Asn Phe Phe Trp Lys Thr Phe Thr Ser Cys
   1 5 10
<210> 497
   <211 > 12
   <212> PRT
   <213> Artificial Sequence
<220>
   <223> SOMATOSTATIN OR CORTISTATIN MIMETIC PEPTIDE
<400> 497
   Cys Lys Asn Phe Phe Trp Lys Thr Phe Thr Ser Cys
   1               5                   10
<210> 498
   <211> 25
   <212> PRT
   <213> Artificial Sequence
<220>
   <223> CAP37 MIMETIC/LPS BINDING PEPTIDE
<400> 498
   Asn Gln Gly Arg His Phe Cys Gly Gly Ala Leu Ile His Ala Arg Phe
   1               5                   10                  15
   Val Met Thr Ala Ala Ser Cys Phe Gln
               20                  25
<210> 499
   <211> 20
   <212> PRT
   <213> Artificial Sequence
<220>
   <223> CAP37 MIMETIC/LPS BINDING PEPTIDE
<400> 499
   Arg His Phe Cys Gly Gly Ala Leu Ile His Ala Arg Phe Val Met Thr
   1               5                   10                  15
   Ala Ala Ser Cys
               20
<210> 500
   <211> 27
   <212> PRT
   <213> Artificial Sequence
<220>
   <223> CAP37 MIMETIC/LPS BINDING PEPTIDE
<400> 500
   Gly Thr Arg Cys Gln Val Ala Gly Trp Gly Ser Gln Arg Ser Gly Gly
   1               5                   10                  15
   Arg Leu Ser Arg Phe Pro Arg Phe Val Asn Val
               20                  25
<210> 501
   <211> 18
   <212> PRT
   <213> Artificial Sequence
<220>
   <223> VEGF- ANTAGONIST PEPTIDE
<400> 501
   Gly Glu Arg Trp Cys Phe Asp Gly Pro Arg Ala Trp Val Cys Gly Trp
   1 5 10 15
   Glu Ile
<210> 502
   <211> 18
   <212> PRT
   <213> Artificial Sequence
<220>
   <223> VEGF- ANTAGONIST PEPTIDE
<400> 502
   Glu Glu Leu Trp Cys Phe Asp Gly Pro Arg Ala Trp Val Cys Gly Tyr
   1 5 10 15
   Val Lys
<210> 503
   <211> 33
   <212> PRT
   <213> Artificial Sequence
<220>
   <223> ANTIPATHOGENIC PEPTIDE
<400> 503
   Gly Phe Phe Ala Leu Ile Pro Lys Ile Ile Ser Ser Pro Leu Phe Lys
   1 5 10 15
   Thr Leu Leu Ser Ala Val Gly Ser Ala Leu Ser Ser Ser Gly Gly Gln
   20 25 30
   Gln
<210> 504
   <211> 33
   <212> PRT
   <213> Artificial Sequence
<220>
   <223> ANTIPATHOGENIC PEPTIDE
<220>
   <221> misc_feature
   <222> (7, 18,)..(19)
   <223> Positions 7, 18, and 19, D amino acid residue
<400> 504
   Gly Phe Phe Ala Leu Ile Pro Lys Ile Ile Ser Ser Pro Leu Phe Lys
   1 5 10 15
   Thr Leu Leu Ser Ala Val Gly Ser Ala Leu Ser Ser Ser Gly Gly Gln
   20 25 30
   Glu
<210> 505
   <211> 22
   <212> PRT
   <213> Artificial Sequence
<220>
   <223> ANTIPATHOGENIC PEPTIDE
<220>
   <221> misc_feature
   <222> (18)..(19)
   <223> Positions 18 and 19, D amino acid residues
<400> 505
   Gly Phe Phe Ala Leu Ile Pro Lys Ile Ile Ser Ser Pro Leu Phe Lys
   1 5 10 15
   Thr Leu Leu Ser Ala Val
   20
<210> 506
   <211> 22
   <212> PRT
   <213> Artificial Sequence
<220>
   <223> ANTIPATHOGENIC PEPTIDE
<220>
   <221> misc_feature
   <222> (7,18)..(19)
   <223> Positions 7, 18 and 19, D amino acid residues
<400> 506
   Gly Phe Phe Ala Leu Ile Pro Lys Ile Ile Ser Ser Pro Leu Phe Lys
   1 5 10 15
   Thr Leu Leu Ser Ala Val
   20
<210> 507
   <211> 23
   <212> PRT
   <213> Artificial Sequence
<220>
   <223> ANTIPATHOGENIC PEPTIDE
<220>
   <221> misc_feature
   <222> (8, 19)..(20)
   <223> Positions 8, 19 and 20, D amino acid residues
<400> 507
   Lys Gly Phe Phe Ala Leu Ile Pro Lys Ile Ile Ser Ser Pro Leu Phe
   1 5 10 15
   Lys Thr Leu Leu Ser Ala Val
   20

<210> 508
   <211> 24
   <212> PRT
   <213> Artificial Sequence
<220>
   <223> ANTIPATHOGENIC PEPTIDE
<220>
   <221> misc_feature
   <222> (9, 20)..(21)
   <223> Positions 9, 20 and 21, D amino acid residues
<400> 508
   Lys Lys Gly Phe Phe Ala Leu Ile Pro Lys Ile Ile Ser Ser Pro Leu
   1 5 10 15
   Phe Lys Thr Leu Leu Ser Ala Val
   20
<210> 509
   <211> 24
   <212> PRT
   <213> Artificial Sequence
<220>
   <223> ANTIPATHOGENIC PEPTIDE
<220>
   <221> misc_feature
   <222> (9, 20)..(21)
   <223> Positions 9, 20 and 21, D amino acid residues
<400> 509
   Lys Lys Gly Phe Phe Ala Leu Ile Pro Lys Ile Ile Ser Ser Pro Leu
   1 5 10 15
   Phe Lys Thr Leu Leu Ser Ala Val
   20
<210> 510
   <211> 11
   <212> PRT
   <213> Artificial Sequence
<220>
   <223> ANTIPATHOGENIC PEPTIDE
<220>
   <221> misc_feature
   <222> (7)..(7)
   <223> Position 7, D amino acid residue
<400> 510
   Gly Phe Phe Ala Leu Ile Pro Lys Ile Ile Ser
   1 5 10
<210> 511
   <211> 26
   <212> PRT
   <213> Artificial Sequence
<220>
   <223> ANTIPATHOGENIC PEPTIDE
<400> 511
   Gly Ile Gly Ala Val Leu Lys Val Leu Thr Thr Gly Leu Pro Ala Leu
   1 5 10 15
   Ile Ser Trp Ile Lys Arg Lys Arg Gln Gln
   20 25
<210> 512
   <211> 26
   <212> PRT
   <213> Artificial Sequence
<220>
   <223> ANTIPATHOGENIC PEPTIDE
<220>
   <221> misc_feature
   <222> (5, 8, 17)..(23)
   <223> Positions 5, 8, 17 and 23, D amino acid residues
<400> 512
   Gly Ile Gly Ala Val Leu Lys Val Leu Thr Thr Gly Leu Pro Ala Leu
   1               5                   10                  15
   Ile Ser Trp Ile Lys Arg Lys Arg Gln Gln
               20                  25
<210> 513
   <211> 26
   <212> PRT
   <213> Artificial Sequence
<220>
   <223> ANTIPATHOGENIC PEPTIDE
<220>
   <221> misc_feature
   <222> (5, 18, 17)..(23)
   <223> Positions 5, 18, 17 and 23, D amino acid residues
<400> 513
   Gly Ile Gly Ala Val Leu Lys Val Leu Thr Thr Gly Leu Pro Ala Leu
   1               5                   10                  15
   Ile Ser Trp Ile Lys Arg Lys Arg Gln Gln
               20                  25
<210> 514
   <211> 22
   <212> PRT
   <213> Artificial Sequence
<220>
   <223> ANTIPATHOGENIC PEPTIDE
<220>
   <221> misc_feature
   <222> (5, 8, 17)..(21)
   <223> Positions 5, 8, 17 and 21, D amino acid residues
<400> 514
   Gly Ile Gly Ala Val Leu Lys Val Leu Thr Thr Gly Leu Pro Ala Leu
   1               5                   10                  15
   Ile Ser Trp Ile Lys Arg
               20
<210> 515
   <211> 19
   <212> PRT
   <213> Artificial Sequence
<220>
   <223> ANTIPATHOGENIC PEPTIDE
<220>
   <221> misc_feature
   <222> (2, 5, 14)..(18)
   <223> Positions 2, 5, 14 and 18, D amino acid residues
<400> 515
   Ala Val Leu Lys Val Leu Thr Thr Gly Leu Pro Ala Leu Ile Ser Trp
   1 5 10 15
   Ile Lys Arg
<210> 516
   <211> 12
   <212> PRT
   <213> Artificial Sequence
<220>
   <223> ANTIPATHOGENIC PEPTIDE
<220>
   <221> misc_feature
   <222> (3, 4, 8)..(10)
   <223> Positions 3, 4, 8 and 10, D amino acid residues
<400> 516
   Lys Leu Leu Leu Leu Leu Lys Leu Leu Leu Leu Lys
   1 5 10
<210> 517
   <211> 12
   <212> PRT
   <213> Artificial Sequence
<220>
   <223> ANTIPATHOGENIC PEPTIDE
<220>
   <221> misc_feature
   <222> (3, 4, 8)..(10)
   <223> Positions 3, 4, 8 and 10, D amino acid residues
<400> 517
   Lys Leu Leu Leu Lys Leu Leu Leu Lys Leu Leu Lys
   1 5 10
<210> 518
   <211> 12
   <212> PRT
   <213> Artificial Sequence
<220>
   <223> ANTIPATHOGENIC PEPTIDE
<220>
   <221> misc_feature
   <222> (3, 4, 8)..(10)
   <223> Positions 3, 4, 8 and 10, D amino acid residues
<400> 518
   Lys Leu Leu Leu Lys Leu Lys Leu Lys Leu Leu Lys
   1               5                   10
<210> 519
   <211> 12
   <212> PRT
   <213> Artificial Sequence
<220>
   <223> ANTIPATHOGENIC PEPTIDE
<400> 519
   Lys Lys Leu Leu Lys Leu Lys Leu Lys Leu Lys Lys
   1               5                   10
<210> 520
   <211> 12
   <212> PRT
   <213> Artificial Sequence
<220>
   <223> ANTIPATHOGENIC PEPTIDE
<400> 520
   Lys Leu Leu Leu Lys Leu Leu Leu Lys Leu Leu Lys
   1               5                   10
<210> 521
   <211> 12
   <212> PRT
   <213> Artificial Sequence
<220>
   <223> ANTIPATHOGENIC PEPTIDE
<400> 521
   Lys Leu Leu Leu Lys Leu Lys Leu Lys Leu Leu Lys
   1               5                   10
<210> 522
   <211> 6
   <212> PRT
   <213> Artificial Sequence
<220>
   <223> ANTIPATHOGENIC PEPTIDE
<400> 522
   Lys Leu Leu Leu Leu Lys
   1 5
<210> 523
   <211> 8
   <212> PRT
   <213> Artificial Sequence
<220>
   <223> ANTIPATHOGENIC PEPTIDE
<400> 523
   Lys Leu Leu Leu Lys Leu Leu Lys
   1 5
<210> 524
   <211> 12
   <212> PRT
   <213> Artificial Sequence
<220>
   <223> ANTIPATHOGENIC PEPTIDE
<400> 524
   Lys Leu Leu Leu Lys Leu Lys Leu Lys Leu Leu Lys
   1 5 10
<210> 525
   <211> 12
   <212> PRT
   <213> Artificial Sequence
<220>
   <223> ANTIPATHOGENIC PEPTIDE
<400> 525
   Lys Leu Leu Leu Lys Leu Lys Leu Lys Leu Leu Lys
   1 5 10
<210> 526
   <211> 12
   <212> PRT
   <213> Artificial Sequence
<220>
   <223> ANTIPATHOGENIC PEPTIDE
<400> 526
   Lys Leu Leu Leu Lys Leu Lys Leu Lys Leu Leu Lys
   1 5 10
<210> 527
   <211> 12
   <212> PRT
   <213> Artificial Sequence
<220>
   <223> ANTIPATHOGENIC PEPTIDE
<400> 527
   Lys Ala Ala Ala Lys Ala Ala Ala Lys Ala Ala Lys
   1 5 10
<210> 528
   <211> 12
   <212> PRT
   <213> Artificial Sequence
<220>
   <223> ANTIPATHOGENIC PEPTIDE
<400> 528
   Lys Val Val Val Lys Val Val Val Lys Val Val Lys
   1 5 10
<210> 529
   <211> 12
   <212> PRT
   <213> Artificial Sequence
<220>
   <223> ANTIPATHOGENIC PEPTIDE
<400> 529
   Lys Val Val Val Lys Val Lys Val Lys Val Val Lys
   1 5 10
<210> 530
   <211> 11
   <212> PRT
   <213> Artificial Sequence
<220>
   <223> ANTIPATHOGENIC PEPTIDE
<400> 530
   Lys Val Val Val Lys Val Lys Val Lys Val Lys
   1 5 10
<210> 531
   <211> 12
   <212> PRT
   <213> Artificial Sequence
<220>
   <223> ANTIPATHOGENIC PEPTIDE
<400> 531
   Lys Val Val Val Lys Val Lys Val Lys Val Val Lys
   1 5 10
<210> 532
   <211> 6
   <212> PRT
   <213> Artificial Sequence
<220>
   <223> ANTIPATHOGENIC PEPTIDE
<400> 532
   Lys Leu Ile Leu Lys Leu
   1 5
<210> 533
   <211> 6
   <212> PRT
   <213> Artificial Sequence
<220>
   <223> ANTIPATHOGENIC PEPTIDE
<400> 533
   Lys Val Leu His Leu Leu
   1 5
<210> 534
   <211> 6
   <212> PRT
   <213> Artificial Sequence
<220>
   <223> ANTIPATHOGENIC PEPTIDE
<400> 534
   Leu Lys Leu Arg Leu Leu
   1 5
<210> 535
   <211> 6
   <212> PRT
   <213> Artificial Sequence
<220>
   <223> ANTIPATHOGENIC PEPTIDE
<400> 535
   Lys Pro Leu His Leu Leu
   1 5
<210> 536
   <211> 8
   <212> PRT
   <213> Artificial Sequence
<220>
   <223> ANTIPATHOGENIC PEPTIDE
<400> 536
   Lys Leu Ile Leu Lys Leu Val Arg
   1 5
<210> 537
   <211> 8
   <212> PRT
   <213> Artificial Sequence
<220>
   <223> ANTIPATHOGENIC PEPTIDE
<400> 537
   Lys Val Phe His Leu Leu His Leu
   1 5
<210> 538
   <211> 8
   <212> PRT
   <213> Artificial Sequence
<220>
   <223> ANTIPATHOGENIC PEPTIDE
<400> 538
   His Lys Phe Arg Ile Leu Lys Leu
   1 5
<210> 539
   <211> 8
   <212> PRT
   <213> Artificial Sequence
<220>
   <223> ANTIPATHOGENIC PEPTIDE
<400> 539
   Lys Pro Phe His Ile Leu His Leu
   1 5
<210> 540
   <211> 12
   <212> PRT
   <213> Artificial Sequence
<220>
   <223> ANTIPATHOGENIC PEPTIDE
<400> 540
   Lys Ile Ile Ile Lys Ile Lys Ile Lys Ile Ile Lys
   1 5 10
<210> 541
   <211> 12
   <212> PRT
   <213> Artificial Sequence
<220>
   <223> ANTIPATHOGENIC PEPTIDE
<400> 541
   Lys Ile Ile Ile Lys Ile Lys Ile Lys Ile Ile Lys
   1 5 10
<210> 542
   <211> 12
   <212> PRT
   <213> Artificial Sequence
<220>
   <223> ANTIPATHOGENIC PEPTIDE
<400> 542
   Lys Ile Ile Ile Lys Ile Lys Ile Lys Ile Ile Lys
   1 5 10
<210> 543
   <211> 12
   <212> PRT
   <213> Artificial Sequence
<220>
   <223> ANTIPATHOGENIC PEPTIDE
<400> 543
   Lys Ile Pro Ile Lys Ile Lys Ile Lys Ile Pro Lys
   1 5 10
<210> 544
   <211> 12
   <212> PRT
   <213> Artificial Sequence
<220>
   <223> ANTIPATHOGENIC PEPTIDE
<400> 544
   Lys Ile Pro Ile Lys Ile Lys Ile Lys Ile Val Lys
   1 5 10
<210> 545
   <211> 12
   <212> PRT
   <213> Artificial Sequence
<220>
   <223> ANTIPATHOGENIC PEPTIDE
<400> 545
   Arg Ile Ile Ile Arg Ile Arg Ile Arg Ile Ile Arg
   1 5 10
<210> 546
   <211> 12
   <212> PRT
   <213> Artificial Sequence
<220>
   <223> ANTIPATHOGENIC PEPTIDE
<400> 546
   Arg Ile Ile Ile Arg Ile Arg Ile Arg Ile Ile Arg
   1 5 10
<210> 547
   <211> 12
   <212> PRT
   <213> Artificial Sequence
<220>
   <223> ANTIPATHOGENIC PEPTIDE
<400> 547
   Arg Ile Ile Ile Arg Ile Arg Ile Arg Ile Ile Arg
   1 5 10
<210> 548
   <211> 12
   <212> PRT
   <213> Artificial Sequence
<220>
   <223> ANTIPATHOGENIC PEPTIDE
<400> 548
   Arg Ile Val Ile Arg Ile Arg Ile Arg Leu Ile Arg
   1 5 10
<210> 549
   <211> 12
   <212> PRT
   <213> Artificial Sequence
<220>
   <223> ANTIPATHOGENIC PEPTIDE
<400> 549
   Arg Ile Ile Val Arg Ile Arg Leu Arg Ile Ile Arg
   1 5 10
<210> 550
   <211> 12
   <212> PRT
   <213> Artificial Sequence
<220>
   <223> ANTIPATHOGENIC PEPTIDE
<400> 550
   Arg Ile Gly Ile Arg Leu Arg Val Arg Ile Ile Arg
   1 5 10
<210> 551
   <211> 12
   <212> PRT
   <213> Artificial Sequence
<220>
   <223> ANTIPATHOGENIC PEPTIDE
<400> 551
   Lys Ile Val Ile Arg Ile Arg Ile Arg Leu Ile Arg
   1 5 10
<210> 552
   <211> 12
   <212> PRT
   <213> Artificial Sequence
<220>
   <223> ANTIPATHOGENIC PEPTIDE
<400> 552
   Arg Ile Ala Val Lys Trp Arg Leu Arg Phe Ile Lys
   1 5 10
<210> 553
   <211> 12
   <212> PRT
   <213> Artificial Sequence
<220>
   <223> ANTIPATHOGENIC PEPTIDE
<400> 553
   Lys Ile Gly Trp Lys Leu Arg Val Arg Ile Ile Arg
   1 5 10
<210> 554
   <211> 12
   <212> PRT
   <213> Artificial Sequence
<220>
   <223> ANTIPATHOGENIC PEPTIDE
<400> 554
   Lys Lys Ile Gly Trp Leu Ile Ile Arg Val Arg Arg
   1 5 10
<210> 555
   <211> 14
   <212> PRT
   <213> Artificial Sequence
<220>
   <223> ANTIPATHOGENIC PEPTIDE
<400> 555
   Arg Ile Val Ile Arg Ile Arg Ile Arg Leu Ile Arg Ile Arg
   1 5 10
<210> 556
   <211> 14
   <212> PRT
   <213> Artificial Sequence
<220>
   <223> ANTIPATHOGENIC PEPTIDE
<400> 556
   Arg Ile Ile Val Arg Ile Arg Leu Arg Ile Ile Arg Val Arg
   1 5 10
<210> 557
   <211> 14
   <212> PRT
   <213> Artificial Sequence
<220>
   <223> ANTIPATHOGENIC PEPTIDE
<400> 557
   Arg Ile Gly Ile Arg Leu Arg Val Arg Ile Ile Arg Arg Val
   1 5 10
<210> 558
   <211> 16
   <212> PRT
   <213> Artificial Sequence
<220>
   <223> ANTIPATHOGENIC PEPTIDE
<400> 558
   Lys Ile Val Ile Arg Ile Arg Ala Arg Leu Ile Arg Ile Arg Ile Arg
   1 5 10 15
<210> 559
   <211> 16
   <212> PRT
   <213> Artificial Sequence
<220>
   <223> ANTIPATHOGENIC PEPTIDE
<400> 559
   Arg Ile Ile Val Lys Ile Arg Leu Arg Ile Ile Lys Lys Ile Arg Leu
   1 5 10 15
<210> 560
   <211> 16
   <212> PRT
   <213> Artificial Sequence
<220>
   <223> ANTIPATHOGENIC PEPTIDE
<400> 560
   Lys Ile Gly Ile Lys Ala Arg Val Arg Ile Ile Arg Val Lys Ile Ile
   1 5 10 15
<210> 561
   <211> 16
   <212> PRT
   <213> Artificial Sequence
<220>
   <223> ANTIPATHOGENIC PEPTIDE
<400> 561
   Arg Ile Ile Val His Ile Arg Leu Arg Ile Ile His His Ile Arg Leu
   1 5 10 15
<210> 562
   <211> 16
   <212> PRT
   <213> Artificial Sequence
<220>
   <223> ANTIPATHOGENIC PEPTIDE
<400> 562
   His Ile Gly Ile Lys Ala His Val Arg Ile Ile Arg Val His Ile Ile
   1 5 10 15
<210> 563
   <211> 16
   <212> PRT
   <213> Artificial Sequence
<220>
   <223> ANTIPATHOGENIC PEPTIDE
<400> 563
   Arg Ile Tyr Val Lys Ile His Leu Arg Tyr Ile Lys Lys Ile Arg Leu
   1 5 10 15
<210> 564
   <211> 16
   <212> PRT
   <213> Artificial Sequence
<220>
   <223> ANTIPATHOGENIC PEPTIDE
<400> 564
   Lys Ile Gly His Lys Ala Arg Val His Ile Ile Arg Tyr Lys Ile Ile
   1 5 10 15
<210> 565
   <211> 16
   <212> PRT
   <213> Artificial Sequence
<220>
   <223> ANTIPATHOGENIC PEPTIDE
<400> 565
   Arg Ile Tyr Val Lys Pro His Pro Arg Tyr Ile Lys Lys Ile Arg Leu
   1 5 10 15
<210> 566
   <211> 16
   <212> PRT
   <213> Artificial Sequence
<220>
   <223> ANTIPATHOGENIC PEPTIDE
<400> 566
   Lys Pro Gly His Lys Ala Arg Pro His Ile Ile Arg Tyr Lys Ile Ile
   1 5 10 15
<210> 567
   <211> 19
   <212> PRT
   <213> Artificial Sequence
<220>
   <223> ANTIPATHOGENIC PEPTIDE
<400> 567
   Lys Ile Val Ile Arg Ile Arg Ile Arg Leu Ile Arg Ile Arg Ile Arg
   1               5                   10                  15
   Lys Ile Val
<210> 568
   <211> 19
   <212> PRT
   <213> Artificial Sequence
<220>
   <223> ANTIPATHOGENIC PEPTIDE
<400> 568
   Arg Ile Ile Val Lys Ile Arg Leu Arg Ile Ile Lys Lys Ile Arg Leu
   1               5                   10                  15
   Ile Lys Lys
<210> 569
   <211> 19
   <212> PRT
   <213> Artificial Sequence
<220>
   <223> ANTIPATHOGENIC PEPTIDE
<400> 569
   Lys Ile Gly Trp Lys Leu Arg Val Arg Ile Ile Arg Val Lys Ile Gly
   1               5                   10                  15
   Arg Leu Arg
<210> 570
   <211> 25
   <212> PRT
   <213> Artificial Sequence
<220>
   <223> ANTIPATHOGENIC PEPTIDE
<400> 570
   Lys Ile Val Ile Arg Ile Arg Ile Arg Leu Ile Arg Ile Arg Ile Arg
   1 5 10 15
   Lys Ile Val Lys Val Lys Arg Ile Arg
   20 25
<210> 571
   <211> 26
   <212> PRT
   <213> Artificial Sequence
<220>
   <223> ANTIPATHOGENIC PEPTIDE
<400> 571
   Arg Phe Ala Val Lys Ile Arg Leu Arg Ile Ile Lys Lys Ile Arg Leu
   1 5 10 15
   Ile Lys Lys Ile Arg Lys Arg Val Ile Lys
   20 25
<210> 572
   <211> 30
   <212> PRT
   <213> Artificial Sequence
<220>
   <223> ANTIPATHOGENIC PEPTIDE
<400> 572
   Lys Ala Gly Trp Lys Leu Arg Val Arg Ile Ile Arg Val Lys Ile Gly
   1 5 10 15
   Arg Leu Arg Lys Ile Gly Trp Lys Lys Arg Val Arg Ile Lys
   20 25 30
<210> 573
   <211> 16
   <212> PRT
   <213> Artificial Sequence
<220>
   <223> ANTIPATHOGENIC PEPTIDE
<400> 573
   Arg Ile Tyr Val Lys Pro His Pro Arg Tyr Ile Lys Lys Ile Arg Leu
   1 5 10 15
<210> 574
   <211> 16
   <212> PRT
   <213> Artificial Sequence
<220>
   <223> ANTIPATHOGENIC PEPTIDE
<400> 574
   Lys Pro Gly His Lys Ala Arg Pro His Ile Ile Arg Tyr Lys Ile Ile
   1 5 10 15
<210> 575
   <211> 19
   <212> PRT
   <213> Artificial Sequence
<220>
   <223> ANTIPATHOGENIC PEPTIDE
<400> 575
   Lys Ile Val Ile Arg Ile Arg Ile Arg Leu Ile Arg Ile Arg Ile Arg
   1 5 10 15
   Lys Ile Val
<210> 576
   <211> 19
   <212> PRT
   <213> Artificial Sequence
<220>
   <223> ANTIPATHOGENIC PEPTIDE
<400> 576
   Arg Ile Ile Val Lys Ile Arg Leu Arg Ile Ile Lys Lys Ile Arg Leu
   1 5 10 15
   Ile Lys Lys
<210> 577
   <211> 16
   <212> PRT
   <213> Artificial Sequence
<220>
   <223> ANTIPATHOGENIC PEPTIDE
<400> 577
   Arg Ile Tyr Val Ser Lys Ile Ser Ile Tyr Ile Lys Lys Ile Arg Leu
   1 5 10 15
<210> 578
   <211> 19
   <212> PRT
   <213> Artificial Sequence
<220>
   <223> ANTIPATHOGENIC PEPTIDE
<400> 578
   Lys Ile Val Ile Phe Thr Arg Ile Arg Leu Thr Ser Ile Arg Ile Arg
   1 5 10 15
   Ser Ile Val
<210> 579
   <211> 16
   <212> PRT
   <213> Artificial Sequence
<220>
   <223> ANTIPATHOGENIC PEPTIDE
<400> 579
   Lys Pro Ile His Lys Ala Arg Pro Thr Ile Ile Arg Tyr Lys Met Ile
   1 5 10 15

<210> 580
   <211> 25
   <212> PRT
   <213> Artificial Sequence
<220>
   <223> ANTIPATHOGENIC PEPTIDE
<220>
   <221> misc_feature
   <222> (1)..(1)
   <223> Disulfide bond to position 25
<220>
   <221> misc_feature
   <222> (25)..(25)
   <223> Disulfide bond to position 1
<400> 580
   Cys Lys Gly Phe Phe Ala Leu Ile Pro Lys Ile Ile Ser Ser Pro Leu
   1 5 10 15
   Phe Lys Thr Leu Leu Ser Ala Val Cys
   20 25
<210> 581
   <211> 26
   <212> PRT
   <213> Artificial Sequence
<220>
   <223> ANTIPATHOGENIC PEPTIDE
<400> 581
   Cys Lys Lys Gly Phe Phe Ala Leu Ile Pro Lys Ile Ile Ser Ser Pro
   1 5 10 15
   Leu Phe Lys Thr Leu Leu Ser Ala Val Cys
   20 25
<210> 582
   <211> 27
   <212> PRT
   <213> Artificial Sequence
<220>
   <223> ANTIPATHOGENIC PEPTIDE
<400> 582
   Cys Lys Lys Lys Gly Phe Phe Ala Leu Ile Pro Lys Ile Ile Ser Ser
   1 5 10 15
   Pro Leu Phe Lys Thr Leu Leu Ser Ala Val Cys
   20 25
<210> 583
   <211> 16
   <212> PRT
   <213> Artificial Sequence
<220>
   <223> ANTIPATHOGENIC PEPTIDE
<220>
   <221> misc_feature
   <222> (1)..(1)
   <223> Disulfide bond to position 16
<220>
   <221> misc_feature
   <222> (16)..(16)
   <223> Disulfide bond to position 1
<400> 583
   Cys Arg Ile Val Ile Arg Ile Arg Ile Arg Leu Ile Arg Ile Arg Cys
   1 5 10 15
<210> 584
   <211> 18
   <212> PRT
   <213> Artificial Sequence
<220>
   <223> ANTIPATHOGENIC PEPTIDE
<220>
   <221> misc_feature
   <222> (1)..(1)
   <223> Disulfide bond to position 18
<220>
   <221> misc_feature
   <222> (18)..(18)
   <223> Disulfide bond to position 1
<400> 584
   Cys Lys Pro Gly His Lys Ala Arg Pro His Ile Ile Arg Tyr Lys Ile
   1               5                   10                  15
   Ile Cys
<210> 585
   <211> 28
   <212> PRT
   <213> Artificial Sequence
<220>
   <223> ANTIPATHOGENIC PEPTIDE
<220>
   <221> misc_feature
   <222> (1)..(1)
   <223> Disulfide bond to position 28
<220>
   <221> misc_feature
   <222> (28)..(28)
   <223> Disulfide bond to position 1
<400> 585
   Cys Arg Phe Ala Val Lys Ile Arg Leu Arg Ile Ile Lys Lys Ile Arg
   1               5                   10                  15
   Leu Ile Lys Lys Ile Arg Lys Arg Val Ile Lys Cys
               20                  25
<210> 586
   <211> 13
   <212> PRT
   <213> Artificial Sequence
<220>
   <223> ANTIPATHOGENIC PEPTIDE
<400> 586
   Lys Leu Leu Leu Lys Leu Leu Leu Lys Leu Leu Lys Cys
   1               5                   10
<210> 587
   <211> 12
   <212> PRT
   <213> Artificial Sequence
<220>
   <223> ANTIPATHOGENIC PEPTIDE
<400> 587
   Lys Leu Leu Leu Lys Leu Leu Leu Lys Leu Leu Lys
   1               5                   10
<210> 588
   <211> 13
   <212> PRT
   <213> Artificial Sequence
<220>
   <223> ANTI PATHOGENIC PEPTIDE
<400> 588
   Lys Leu Leu Leu Lys Leu Lys Leu Lys Leu Leu Lys Cys
   1               5                   10
<210> 589
   <211 > 12
   <212> PRT
   <213> Artificial Sequence
<220>
   <223> ANTIPATHOGENIC PEPTIDE
<400> 589
   Lys Leu Leu Leu Lys Leu Leu Leu Lys Leu Leu Lys
   1               5                   10
<210> 590
   <211> 28
   <212> PRT
   <213> Artificial Sequence
<220>
   <223> VIP-MIMETIC PEPTIDE
<400> 590
   His Ser Asp Ala Val Phe Tyr Asp Asn Tyr Thr Arg Leu Arg Lys Gln
   1               5                   10                  15
   Met Ala Val Lys Lys Tyr Leu Asn Ser Ile Leu Asn
               20                  25
<210> 591
   <211> 28
   <212> PRT
   <213> Artificial Sequence
<220>
   <223> VIP-MIMETIC PEPTIDE
<400> 591
   His Ser Asp Ala Val Phe Tyr Asp Asn Tyr Thr Arg Leu Arg Lys Gln
   1 5 10 15
   Met Ala Val Lys Lys Tyr Leu Asn Ser Ile Leu Asn
   20 25
<210> 592
   <211> 3
   <212> PRT
   <213> Artificial Sequence
<220>
   <223> VIP-MIMETIC PEPTIDE
<220>
   <221> misc_feature
   <222> (1)..(1)
   <223> Position 1, Xaa is L-Lys, D-Lys or an ornithinyl residue
<220>
   <221> misc_feature
   <222> (2)..(2)
   <223> Position 2, Xaa is L-Tyr, D-Tyr, Phe, Trp or a p-aminophenylalanyl residue
<220>
   <221> misc_feature
   <222> (3)..(3)
   <223> Position 3 is a hydrophobic aliphatic amino acid residue, Position 3, optional attachment to Leu, norleucyl,
   D-Ala, Asn-Ser, Asn-Ser-Ile-, Asn-Ser-Tyr, Ser-Ile-Leu, Asn-Ser-Tyr-Leu or Asn-Ser-Tyr-Leu-Asn
<400> 592
   Xaa Xaa Xaa
   1
<210> 593
   <211> 5
   <212> PRT
   <213> Artificial Sequence
<220>
   <223> VIP-MIMETIC PEPTIDE
<220>
   <221> misc_feature
   <222> (1)..(1)
   <223> Xaa is a hydrophobic aliphatic amino acid residue
<220>
   <221> misc_feature
   <222> (3)..(3)
   <223> Xaa is a hydrophobic aliphatic amino acid residue
<400> 593
   Xaa Ser Xaa Leu Asn
   1               5
<210> 594
   <211> 6
   <212> PRT
   <213> Artificial Sequence
<220>
   <223> VIP-MIMETIC PEPTIDE
<220>
   <221> misc_feature
   <222> (1)..(1)
   <223> Xaa at position 1 is a cross-linked amino acid residue
<220>
   <221> misc_feature
   <222> (5).. (5)
   <223> Position 5, Xaa is a hydrophobic aliphatic aminod acid residue
<220>
   <221> misc_feature
   <222> (6)..(6)
   <223> Xaa at position 6 is a cross-linked amino acid residue
<400> 594
   Xaa Lys Lys Tyr Xaa Xaa
   1               5
<210> 595
   <211> 4
   <212> PRT
   <213> Artificial Sequence
<220>
   <223> VIP-MIMETIC PEPTIDE
<400> 595
   Lys Lys Tyr Leu
   1
<210> 596
   <211> 5
   <212> PRT
   <213> Artificial Sequence
<220>
   <223> VIP-MIMETIC PEPTIDE
<400> 596
   Asn Ser Ile Leu Asn
   1 5
<210> 597
   <211> 4
   <212> PRT
   <213> Artificial Sequence
<220>
   <223> VIP-MIMETIC PEPTIDE
<400> 597
   Lys Lys Tyr Leu
   1
<210> 598
   <211> 4.
   <212> PRT
   <213> Artificial Sequence
<220>
   <223> VIP-MIMETIC PEPTIDE
<400> 598
   Lys Lys Tyr Ala
   1
<210> 599
   <211> 6
   <212> PRT
   <213> Artificial Sequence
<220>
   <223> VIP-MIMETIC PEPTIDE
<400> 599
   Ala Val Lys Lys Tyr Leu
   1 5
<210> 600
   <211> 4
   <212> PRT.
   <213> Artificial Sequence
<220>
   <223> VIP-MIMETIC PEPTIDE
<400> 600
   Ser Ile Leu Asn
   1
<210> 601
   <211> 4
   <212> PRT
   <213> Artificial Sequence
<220>
   <223> VIP-MIMETIC PEPTIDE
<400> 601
   Lys Lys Tyr Val
   1
<210> 602
   <211> 4
   <212> PRT
   <213> Artificial Sequence
<220>
   <223> VIP-MIMETIC PEPTIDE
<220>
   <221> misc_feature
   <222> (3).. (3)
   <223> Position 3, Xaa is a lauric acid residue
<400> 602
   Ser Ile Xaa Asn
   1
<210> 603
   <211> 5
   <212> PRT
   <213> Artificial Sequence
<220>
   <223> VIP-MIMETIC PEPTIDE
<220>
   <221> misc_feature
   <222> (5)..(5)
   <223> Position 5, Xaa is a norleucyl residue
<400> 603
   Lys Lys Tyr Leu Xaa
   1 5
<210> 604
   <211> 5
   <212> PRT
   <213> Artificial Sequence
<220>
   <223> VIP-MIMETIC PEPTIDE
<400> 604
   Asn Ser Tyr Leu Asn
   1 5
<210> 605
   <211> 5
   <212> PRT
   <213> Artificial Sequence
<220>
   <223> VIP-MIMET1C PEPTIDE
<400> 605
   Asn Ser Ile Tyr Asn
   1 5
<210> 606
   <211> 11
   <212> PRT
   <213> Artificial Sequence
<220>
   <223> VIP-MIMETIC PEPTIDE
<400> 606
   Lys Lys Tyr Leu Pro Pro Asn Ser Ile Leu Asn
   1 5 10
<210> 607
   <211> 5
   <212> PRT
   <213> Artificial Sequence
<220>
   <223> VIP-MIMETIC PEPTIDE
<220>
   <221> misc_feature
   <222> (1)..(1)
   <223> Position 1, Xaa is a lauric acid residue
<400> 607
   Xaa Lys Lys Tyr Leu
   1 5
<210> 608
   <211> 5
   <212> PRT
   <213> Artificial Sequence
<220>
   <223> VIP-MIMETIC PEPTIDE
<220>
   <221> misc_feature
   <222> (1)..(1)
   <223> Position 1, Xaa is a caproic acid residue
<400> 608
   Xaa Lys Lys Tyr Leu
   1 5
<210> 609
   <211> 4
   <212> PRT
   <213> Artificial Sequence
<220>
   <223> VIP-MIMETIC PEPTIDE
<220>
   <221> misc_feature
   <222> (4)..(4)
   <223> Position 4, Xaa is a norleucyl residue
<400> 609
   Lys Lys Tyr Xaa
   1
<210> 610
   <211> 5
   <212> PRT
   <213> Artificial Sequence

<220>
   <223> VIP-MIMETIC PEPTIDE
<400> 610
   Val Lys Lys Tyr Leu
   1 5
<210> 611
   <211> 6
   <212> PRT
   <213> Artificial Sequence
<220>
   <223> VIP-MIMETIC PEPTIDE
<400>611
   Leu Asn Ser Ile Leu Asn
   1 5
<210> 612
   <211> 7
   <212> PRT
   <213> Artificial Sequence
<220>
   <223> VIP-MIMETIC PEPTIDE
<400> 612
   Tyr Leu Asn Ser Ile Leu Asn
   1 5
<210> 613
   <211> 5
   <212> PRT
   <213> Artificial Sequence
<220>
   <223> VIP-MIMETIC PEPTIDE
<400> 613
   Lys Lys Tyr Leu Asn
   1 5
<210> 614
   <211> 6
   <212> PRT
   <213> Artificial Sequence
<220>
   <223> VIP-MIMETIC PEPTIDE
<400> 614
   Lys Lys Tyr Leu Asn Ser
   1 5
<210> 615
   <211> 7
   <212> PRT
   <213> Artificial Sequence
<220>
   <223> VIP-MIMETIC PEPTIDE
<400> 615
   Lys Lys Tyr Leu Asn Ser Ile
   1 5
<210> 616
   <211> 8
   <212> PRT
   <213> Artificial Sequence
<220>
   <223> VIP-MIMETIC PEPTIDE
<400> 616
   Lys Lys Tyr Leu Asn Ser Ile Leu
   1 5
<210> 617
   <211> 4
   <212> PRT
   <213> Artificial Sequence
<220>
   <223> VIP-MIMETIC PEPTIDE
<400> 617
   Lys Lys Tyr Leu
   1
<210> 618
   <211> 5
   <212> PRT
   <213> Artificial Sequence
<220>
   <223> VIP-MIMETIC PEPTIDE
<400> 618
   Lys Lys Tyr Asp Ala
   1 5
<210> 619
   <211> 6
   <212> PRT
   <213> Artificial Sequence
<220>
   <223> VIP-MIMETIC PEPTIDE
<400> 619
   Ala Val Lys Lys Tyr Leu
   1 5
<210> 620
   <211> 5
   <212> PRT
   <213> Artificial Sequence
<220>
   <223> VIP-MIMETIC PEPTIDE
<400> 620
   Asn Ser Ile Leu Asn
   1 5
<210> 621
   <211> 4
   <212> PRT
   <213> Artificial Sequence
<220>
   <223> VIP-MIMETIC PEPTIDE
<400> 621
   Lys Lys Tyr Val
   1
<210> 622
   <211> 4
   <212> PRT
   <213> Artificial Sequence
<220>
   <223> VIP-MIMETIC PEPTIDE
<220>
   <221> misc_feature
   <222> (1)..(3)
   <223> Position 3, Xaa is a lauric acid residue
<400> 622
   Xaa Ile Xaa Asn
   1
<210> 623
   <211> 5
   <212> PRT
   <213> Artificial Sequence
<220>
   <223> VIP-MIMETIC PEPTIDE
<400> 623
   Asn Ser Tyr Leu Asn
   1 5
<210> 624
   <211> 5
   <212> PRT
   <213> Artificial Sequence
<220>
   <223> VIP-MIMETIC PEPTIDE
<400> 624
   Asn Ser Ile Tyr Asn
   1 5
<210> 625
   <211> 5
   <212> PRT
   <213> Artificial Sequence
<220>
   <223> VIP-MIMETIC PEPTIDE
<220>
   <221> misc_feature
   <222> (5)..(5)
   <223> Position 5, Xaa is a norleucyl residue
<400> 625
   Lys Lys Tyr Leu Xaa
   1 5
<210> 626
   <211> 11
   <212> PRT
   <213> Artificial Sequence
<220>
   <223> VIP-MIMETIC PEPTIDE
<400> 626
   Lys Lys Tyr Leu Pro Pro Asn Ser Ile Leu Asn
   1 5 10
<210> 627
   <211> 4
   <212> PRT
   <213> Artificial Sequence
<220>
   <223> VIP-MIMETIC PEPTIDE
<400> 627
   Lys Lys Tyr Leu
   1
<210> 628
   <211> 5
   <212> PRT
   <213> Artificial Sequence
<220>
   <223> VIP-MIMETIC PEPTIDE
<400> 628
   Lys Lys Tyr Asp Ala
   1 5
<210> 629
   <211> 6
   <212> PRT
   <213> Artificial Sequence
<220>
   <223> VIP-MIMETIC PEPTIDE
<400> 629
   Ala Val Lys Lys Tyr Leu
   1 5
<210> 630
   <211> 5
   <212> PRT
   <213> Artificial Sequence
<220>
   <223> VIP-MIMETIC PEPTIDE
<400> 630
   Asn Ser Ile Leu Asn
   1 5
<210> 631
   <211> 4
   <212> PRT
   <213> Artificial Sequence
<220>
   <223> VIP-MIMETIC PEPTIDE
<400> 631
   Lys Lys Tyr Val
   1
<210> 632
   <211> 4
   <212> PRT
   <213> Artificial Sequence
<220>
   <223> VIP-MIMETIC PEPTIDE
<220>
   <221> misc_feature
   <222> (1)..(3)
   <223> Position 3, Xaa is a lauric acid residue
<400> 632
   Xaa Ile Xaa Asn
   1
<210> 633
   <211> 5
   <212> PRT
   <213> Artificial Sequence
<220>
   <223> VIP-MIMETIC PEPTIDE
<220>
   <221> misc_feature
   <222> (1)..(1)
   <223> Position 1, Xaa is a lauric acid residue
<400> 633
   Xaa Lys Lys Tyr Leu
   1 5
<210> 634
   <211> 5
   <212> PRT
   <213> Artificial Sequence
<220>
   <223> VIP-MIMETIC PEPTIDE
<220>
   <221> misc_feature
   <222> (1)..(1)
   <223> Position 1, Xaa is a caproic acid residue
<400> 634
   Xaa Lys Lys Tyr Leu
   1 5
<210> 635
   <211> 4
   <212> PRT
   <213> Artificial Sequence
<220>
   <223> VIP-MIMETIC PEPTIDE
<220>
   <221> misc_feature
   <222> (4)..(4)
   <223> Position 4, Xaa is a norleucyl residue
<400> 635
   Lys Lys Tyr Xaa
   1
<210> 636
   <211> 5
   <212> PRT
   <213> Artificial Sequence
<220>
   <223> VIP-MIMETIC PEPTIDE
<400> 636
   Val Lys Lys Tyr Leu
   1 5
<210> 637
   <211> 6
   <212> PRT
   <213> Artificial Sequence
<220>
   <223> VIP-MIMETIC PEPTIDE
<400> 637
   Leu Asn Ser Ile Leu Asn
   1 5
<210> 638
   <211> 7
   <212> PRT
   <213> Artificial Sequence
<220>
   <223> VIP-MIMETIC PEPTIDE
<400> 638
   Tyr Leu Asn Ser Ile Leu Asn
   1 5
<210> 639
   <211> 5
   <212> PRT
   <213> Artificial Sequence
<220>
   <223> VIP-MIMETIC PEPTIDE
<220>
   <221> misc_feature
   <222> (5)..(5)
   <223> Position 5, Xaa is a norleucyl residue
<400> 639
   Lys Lys Tyr Leu Xaa
   1 5
<210> 640
   <211> 5
   <212> PRT
   <213> Artificial Sequence
<220>
   <223> VIP-MIMETIC PEPTIDE
<400> 640
   Lys Lys Tyr Leu Asn
   1 5
<210> 641
   <211> 6
   <212> PRT
   <213> Artificial Sequence
<220>
   <223> VIP-MIMETIC PEPTIDE
<400> 641
   Lys Lys Tyr Leu Asn Ser
   1 5
<210> 642
   <211> 7
   <212> PRT
   <213> Artificial Sequence
<220>
   <223> VIP-MIMETIC PEPTIDE
<400> 642
   Lys Lys Tyr Leu Asn Ser Ile
   1 5
<210> 643
   <211> 8
   <212> PRT
   <213> Artificial Sequence
<220>
   <223> VIP-MIMETIC PEPTIDE
<400> 643
   Lys Lys Tyr Leu Asn Ser Ile Leu
   1 5
<210> 644
   <211> 6
   <212> PRT
   <213> Artificial Sequence
<220>
   <223> VIP-MIMETIC PEPTIDE
<400> 644
   Lys Lys Lys Tyr Leu Asp
   1 5
<210> 645
   <211> 6
   <212> PRT
   <213> Artificial Sequence
<220>
   <223> VIP-MIMETIC PEPTIDE
<220>
   <221> misc_feature
   <222> (1)..(1)
   <223> Position 1 disulfide cross-linked to amino acid at position 6
<220>
   <221> misc_feature
   <222> (6)..(6)
   <223> Position 6 disulfide cross-linked to amino acid at position 1
<400> 645
   Cys Lys Lys Tyr Leu Cys
   1 5
<210> 646
   <211> 6
   <212> PRT
   <213> Artificial Sequence
<220>
   <223> VIP-MIMETIC PEPTIDE

<220>
   <221> misc_feature
   <222> (1)..(1)
   <223> Position 1 cross-linked by S-CH2-CO to amino acid at position 6
<220>
   <221> misc_feature
   <222> (6)..(6)
   <223> Position 6 cross-linked by S-CH2-CO to amino acid at position 1
<400> 646
   Cys Lys Lys Tyr Leu Lys
   1 5
<210> 647
   <211> 4
   <212> PRT
   <213> Artificial Sequence
<220>
   <223> VIP-MIMETIC PEPTIDE
<220>
   <221> misc_feature
   <223> Position 4, D amino acid residue
<400> 647
   Lys Lys Tyr Ala
   1
<210> 648
   <211> 8
   <212> PRT
   <213> Artificial Sequence
<220>
   <223> VIP-MIMETIC PEPTIDE
<400> 648
   Trp Trp Thr Asp Thr Gly Leu Trp
   1               5
<210> 649
   <211> 8
   <212> PRT
   <213> Artificial Sequence
<220>
   <223> VIP-MIMETIC PEPTIDE
<400> 649
   Trp Trp Thr Asp Asp Gly Leu Trp
   1               5
<210> 650
   <211> 12
   <212> PRT
   <213> Artificial Sequence
<220>
   <223> VIP-MIMETIC PEPTIDE
<400> 650
   Trp Trp Asp Thr Arg Gly Leu Trp Val Trp Thr Ile
   1               5                   10
<210> 651
   <211> 12
   <212> PRT
   <213> Artificial Sequence
<220>
   <223> VIP-MIMETIC PEPTIDE
<400> 651
   Phe Trp Gly Asn Asp Gly Ile Trp Leu Glu Ser Gly
   1 5 10
<210> 652
   <211> 12
   <212> PRT
   <213> Artificial Sequence
<220>
   <223> VIP-MIMETIC PEPTIDE
<400> 652
   Asp Trp Asp Gln Phe Gly Leu Trp Arg Gly Ala Ala
   1 5 10
<210> 653
   <211> 12
   <212> PRT
   <213> Artificial Sequence
<220>
   <223> VIP-MIMETIC PEPTIDE
<400> 653
   Arg Trp Asp Asp Asn Gly Leu Trp Val Val Val Leu
   1 5 10
<210> 654
   <211> 12
   <212> PRT
   <213> Artificial Sequence
<220>
   <223> VIP-MIMETIC PEPTIDE
<400> 654
   Ser Gly Met Trp Ser His Tyr Gly Ile Trp Met Gly
   1 5 10
<210> 655
   <211>12
   <212> PRT
   <213> Artificial Sequence
<220>
   <223> VIP-MIMETIC PEPTIDE
<400> 655
   Gly Gly Arg Trp Asp Gln Ala Gly Leu Trp Val Ala
   1 5 10
<210> 656
   <211> 12
   <212> PRT
   <213> Artificial Sequence
<220>
   <223> VIP-MIMETIC PEPTIDE
<400> 656
   Lys Leu Trp Ser Glu Gln Gly Ile Trp Met Gly Glu
   1 5 10
<210> 657
   <211> 10
   <212> PRT
   <213> Artificial Sequence
<220>
   <223> VIP-MIMETIC PEPTIDE
<400> 657
   Cys Trp Ser Met His Gly Leu Trp Leu Cys
   1 5 10
<210> 658
   <211> 12
   <212> PRT
   <213> Artificial Sequence
<220>
   <223> VIP-MIMETIC PEPTIDE
<400> 658
   Gly Cys Trp Asp Asn Thr Gly Ile Trp Val Pro Cys
   1 5 10
<210> 659
   <211> 10
   <212> PRT
   <213> Artificial Sequence
<220>
   <223> VIP-MIMETIC PEPTIDE
<400> 659
   Asp Trp Asp Thr Arg Gly Leu Trp Val Tyr
   1 5 10
<210> 660
   <211> 10
   <212> PRT
   <213> Artificial Sequence
<220>
   <223> VIP-MIMETIC PEPTIDE
<400> 660
   Ser Leu Trp Asp Glu Asn Gly Ala Trp Ile
   1 5 10
<210> 661
   <211> 10
   <212> PRT
   <213> Artificial Sequence
<220>
   <223> VIP-MIMETIC PEPTIDE
<400> 661
   Lys Trp Asp Asp Arg Gly Leu Trp Met His
   1 5 10
<210> 662
   <211> 10
   <212> PRT
   <213> Artificial Sequence
<220>
   <223> VIP-MIMETIC PEPTIDE
<400> 662
   Gln Ala Trp Asn Glu Arg Gly Leu Trp Thr
   1 5 10
<210> 663
   <211> 10
   <212> PRT
   <213> Artificial Sequence
<220>
   <223> VIP-MIMETIC PEPTIDE
<400> 663
   Gln Trp Asp Thr Arg Gly Leu Trp Val Ala
   1 5 10
<210> 664
   <211> 9
   <212> PRT
   <213> Artificial Sequence
<220>
   <223> VIP-MIMETIC PEPTIDE
<400> 664
   Trp Asn Val His Gly Ile Trp Gln Glu
   1 5
<210> 665
   <211> 10
   <212> PRT
   <213> Artificial Sequence
<220>
   <223> VIP-MIMETIC PEPTIDE
<400> 665
   Ser Trp Asp Thr Arg Gly Leu Trp Val Glu
   1 5 10
<210> 666
   <211> 10
   <212> PRT
   <213> Artificial Sequence
<220>
   <223> VIP-MIMETIC PEPTIDE
<400> 666
   Asp Trp Asp Thr Arg Gly Leu Trp Val Ala
   1 5 10
<210> 667
   <211> 10
   <212> PRT
   <213> Artificial Sequence
<220>
   <223> VIP-MIMETIC PEPTIDE
<400> 667
   Ser Trp Gly Arg Asp Gly Leu Trp Ile Glu
   1 5 10
<210> 668
   <211> 10
   <212> PRT
   <213> Artificial Sequence
<220>
   <223> VIP-MIMETIC PEPTIDE
<400> 668
   Glu Trp Thr Asp Asn Gly Leu Trp Ala Leu
   1 5 10
<210> 669
   <211> 10
   <212> PRT
   <213> Artificial Sequence
<220>
   <223> VIP-MIMETIC PEPTIDE
<400> 669
   Ser Trp Asp Glu Lys Gly Leu Trp Ser Ala
   1 5 10
<210> 670
   <211> 10
   <212> PRT
   <213> Artificial Sequence
<220>
   <223> VIP-MIMETIC PEPTIDE
<400> 670
   Ser Trp Asp Ser Ser Gly Leu Trp Met Asp
   1 5 10
<210> 671
   <211> 11
   <212> PRT
   <213> Artificial Sequence
<220>
   <223> IL-1 ANTAGONIST PEPTIDE
<400> 671
   Ser His Leu Tyr Trp Gln Pro Tyr Ser Val Gln
   1 5 10
<210> 672
   <211> 12
   <212> PRT
   <213> Artificial Sequence
<220>
   <223> IL-1 ANTAGONIST PEPTIDE
<400> 672
   Thr Leu Val Tyr Trp Gln Pro Tyr Ser Leu Gln Thr
   1 5 10
<210> 673
   <211> 12
   <212> PRT
   <213> Artificial Sequence
<220>
   <223> IL-1 ANTAGONIST PEPTIDE
<400> 673
   Arg Gly Asp Tyr Trp Gln Pro Tyr Ser Val Gln Ser
   1 5 10
<210> 674
   <211> 12
   <212> PRT
   <213> Artificial Sequence
<220>
   <223> IL-1 ANTAGONIST PEPTIDE
<400> 674
   Val His Val Tyr Trp Gln Pro Tyr Ser Val Gln Thr
   1 5 10
<210> 675
   <211> 12
   <212> PRT
   <213> Artificial Sequence
<220>
   <223> IL-1 ANTAGONIST PEPTIDE
<400> 675
   Arg Leu Val Tyr Trp Gln Pro Tyr Ser Val Gln Thr
   1 5 10
<210> 676
   <211> 12
   <212> PRT
   <213> Artificial Sequence
<220>
   <223> IL-1 ANTAGONIST PEPTIDE
<400> 676
   Ser Arg Val Trp Phe Gln Pro Tyr Ser Leu Gln Ser
   1 5 10
<210> 677
   <211> 12
   <212> PRT
   <213> Artificial Sequence
<220>
   <223> IL-1 ANTAGONIST PEPTIDE
<400> 677
   Asn Met Val Tyr Trp Gln Pro Tyr Ser Ile Gln Thr
   1 5 10
<210> 678
   <211> 12
   <212> PRT
   <213> Artificial Sequence
<220>
   <223> IL-1 ANTAGONIST PEPTIDE
<400> 678
   Ser Val Val Phe Trp Gln Pro Tyr Ser Val Gln Thr
   1 5 10
<210> 679
   <211> 12
   <212> PRT
   <213> Artificial Sequence
<220>
   <223> IL-1 ANTAGONIST PEPTIDE
<400> 679
   Thr Phe Val Tyr Trp Gln Pro Tyr Ala Leu Pro Leu
   1 5 10
<210> 680
   <211> 12
   <212> PRT
   <213> Artificial Sequence
<220>
   <223> IL-1 ANTAGONIST PEPTIDE
<400> 680
   Thr Leu Val Tyr Trp Gln Pro Tyr Ser Ile Gln Arg
   1 5 10
<210> 681
   <211> 12
   <212> PRT
   <213> Artificial Sequence
<220>
   <223> IL-1 ANTAGONIST PEPTIDE
<400> 681
   Arg Leu Val Tyr Trp Gln Pro Tyr Ser Val Gln Arg
   1 5 10
<210> 682
   <211> 12
   <212> PRT
   <213> Artificial Sequence
<220>
   <223> IL-1 ANTAGONIST PEPTIDE
<400> 682
   Ser Pro Val Phe Trp Gln Pro Tyr Ser Ile Gln Ile
   1 5 10
<210> 683
   <211> 12
   <212> PRT
   <213> Artificial Sequence
<220>
   <223> IL-1 ANTAGONIST PEPTIDE
<400> 683
   Trp Ile Glu Trp Trp Gln Pro Tyr Ser Val Gln Ser
   1 5 10
<210> 684
   <211> 12
   <212> PRT
   <213> Artificial Sequence
<220>
   <223> IL-1 ANTAGONIST PEPTIDE
<400> 684
   Ser Leu Ile Tyr Trp Gln Pro Tyr Ser Leu Gln Met
   1 5 10
<210> 685
   <211> 12
   <212> PRT
   <213> Artificial Sequence
<220>
   <223> IL-1 ANTAGONIST PEPTIDE
<400> 685
   Thr Arg Leu Tyr Trp Gln Pro Tyr Ser Val Gln Arg
   1 5 10

<210> 686
   <211> 12
   <212> PRT
   <213> Artificial Sequence
<220>
   <223> IL-1 ANTAGONIST PEPTIDE
<400> 686
   Arg Cys Asp Tyr Trp Gln Pro Tyr Ser Val Gln Thr
   1 5 10
<210> 687
   <211> 12
   <212> PRT
   <213> Artificial Sequence
<220>
   <223> IL-1 ANTAGONIST PEPTIDE
<400> 687
   Met Arg Val Phe Trp Gln Pro Tyr Ser Val Gln Asn
   1 5 10
<210> 688
   <211> 12
   <212> PRT
   <213> Artificial Sequence
<220>
   <223> IL-1 ANTAGONIST PEPTIDE
<400> 688
   Lys Ile Val Tyr Trp Gln Pro Tyr Ser Val Gln Thr
   1 5 10
<210> 689
   <211> 12
   <212> PRT
   <213> Artificial Sequence
<220>
   <223> IL-1 ANTAGONIST PEPTIDE
<400> 689
   Arg His Leu Tyr Trp Gln Pro Tyr Ser Val Gln Arg
   1 5 10
<210> 690
   <211> 12
   <212> PRT
   <213> Artificial Sequence
<220>
   <223> IL-1 ANTAGONIST PEPTIDE
<400> 690
   Ala Leu Val Trp Trp Gln Pro Tyr Ser Glu Gln Ile
   1 5 10
<210> 691
   <211> 12
   <212> PRT
   <213> Artificial Sequence
<220>
   <223> IL-1 ANTAGONIST PEPTIDE
<400> 691
   Ser Arg Val Trp Phe Gln Pro Tyr Ser Leu Gln Ser
   1 5 10
<210> 692
   <211> 10
   <212> PRT
   <213> Artificial Sequence
<220>
   <223> IL-1 ANTAGONIST PEPTIDE
<400> 692
   Trp Glu Gln Pro Tyr Ala Leu Pro Leu Glu
   1 5 10
<210> 693
   <211> 12
   <212> PRT
   <213> Artificial Sequence
<220>
   <223> IL-1 ANTAGONIST PEPTIDE
<400> 693
   Gln Leu Val Trp Trp Gln Pro Tyr Ser Val Gln Arg
   1 5 10
<210> 694
   <211> 12
   <212> PRT
   <213> Artificial Sequence
<220>
   <223> IL-1 ANTAGONIST PEPTIDE
<400> 694
   Asp Leu Arg Tyr Trp Gln Pro Tyr Ser Val Gln Val
   1 5 10
<210> 695
   <211> 12
   <212> PRT
   <213> Artificial Sequence
<220>
   <223> IL-1 ANTAGONIST PEPTIDE
<400> 695
   Glu Leu Val Trp Trp Gln Pro Tyr Ser Leu Gln Leu
   1 5 10
<210> 696
   <211> 12
   <212> PRT
   <213> Artificial Sequence
<220>
   <223> IL-1 ANTAGONIST PEPTIDE
<400> 696
   Asp Leu Val Trp Trp Gln Pro Tyr Ser Val Gln Trp
   1 5 10
<210> 697
   <211> 12
   <212> PRT
   <213> Artificial Sequence
<220>
   <223> IL-1 ANTAGONIST PEPTIDE
<400> 697
   Asn Gly Asn Tyr Trp Gln Pro Tyr Ser Phe Gln Val
   1 5 10
<210> 698
   <211> 12
   <212> PRT
   <213> Artificial Sequence
<220>
   <223> IL-1 ANTAGONIST PEPTIDE
<400> 698
   Glu Leu Val Tyr Trp Gln Pro Tyr Ser Ile Gln Arg
   1 5 10
<210> 699
   <211> 12
   <212> PRT
   <213> Artificial Sequence
<220>
   <223> IL-1 ANTAGONIST PEPTIDE
<400> 699
   Glu Leu Met Tyr Trp Gln Pro Tyr Ser Val Gln Glu
   1 5 10
<210> 700
   <211> 12
   <212> PRT
   <213> Artificial Sequence
<220>
   <223> IL-1 ANTAGONIST PEPTIDE
<400> 700
   Asn Leu Leu Tyr Trp Gln Pro Tyr Ser Met Gln Asp
   1 5 10
<210> 701
   <211> 12
   <212> PRT
   <213> Artificial Sequence
<220>
   <223> IL-1 ANTAGONIST PEPTIDE
<400> 701
   Gly Tyr Glu Trp Tyr Gln Pro Tyr Ser Val Gln Arg
   1 5 10
<210> 702
   <211> 12
   <212> PRT
   <213> Artificial Sequence
<220>
   <223> IL-1 ANTAGONIST PEPTIDE
<400> 702
   Ser Arg Val Trp Tyr Gln Pro Tyr Ser Val Gln Arg
   1 5 10
<210> 703
   <211> 12
   <212> PRT
   <213> Artificial Sequence
<220>
   <223> IL-1 ANTAGONIST PEPTIDE
<400> 703
   Leu Ser Glu Gln Tyr Gln Pro Tyr Ser Val Gln Arg
   1 5 10
<210> 704
   <211> 12
   <212> PRT
   <213> Artificial Sequence
<220>
   <223> IL-1 ANTAGONIST PEPTIDE
<400> 704
   Gly Gly Gly Trp Trp Gln Pro Tyr Ser Val Gln Arg
   1 5 10
<210> 705
   <211> 12
   <212> PRT
   <213> Artificial Sequence
<220>
   <223> IL-1 ANTAGONIST PEPTIDE
<400> 705
   Val Gly Arg Trp Tyr Gln Pro Tyr Ser Val Gln Arg
   1 5 10
<210> 706
   <211> 12
   <212> PRT
   <213> Artificial Sequence
<220>
   <223> IL-1 ANTAGONIST PEPTIDE
<400> 706
   Val His Val Tyr Trp Gln Pro Tyr Ser Val Gln Arg
   1 5 10
<210> 707
   <211> 12
   <212> PRT
   <213> Artificial Sequence
<220>
   <223> IL-1 ANTAGONIST PEPTIDE
<400> 707
   Gln Ala Arg Trp Tyr Gln Pro Tyr Ser Val Gln Arg
   1 5 10
<210> 708
   <211> 12
   <212> PRT
   <213> Artificial Sequence
<220>
   <223> IL-1 ANTAGONIST PEPTIDE
<400> 708
   Val His Val Tyr Trp Gln Pro Tyr Ser Val Gln Thr
   1 5 10
<210> 709
   <211> 12
   <212> PRT
   <213> Artificial Sequence
<220>
   <223> IL-1 ANTAGONIST PEPTIDE
<400> 709
   Arg Ser Val Tyr Trp Gln Pro Tyr Ser Val Gln Arg
   1 5 10
<210> 710
   <211> 12
   <212> PRT
   <213> Artificial Sequence
<220>
   <223> IL-1 ANTAGONIST PEPTIDE
<400> 710
   Thr Arg Val Trp Phe Gln Pro Tyr Ser Val Gln Arg
   1 5 10
<210> 711
   <211> 12
   <212> PRT
   <213> Artificial Sequence
<220>
   <223> IL-1 ANTAGONIST PEPTIDE
<400> 711
   Gly Arg Ile Trp Phe Gln Pro Tyr Ser Val Gln Arg
   1 5 10
<210> 712
   <211> 12
   <212> PRT
   <213> Artificial Sequence
<220>
   <223> IL-1 ANTAGONIST PEPTIDE
<400> 712
   Gly Arg Val Trp Phe Gln Pro Tyr Ser Val Gln Arg
   1 5 10
<210> 713
   <211> 12
   <212> PRT
   <213> Artificial Sequence
<220>
   <223> IL-1 ANTAGONIST PEPTIDE
<400> 713
   Ala Arg Thr Trp Tyr Gln Pro Tyr Ser Val Gln Arg
   1 5 10
<210> 714
   <211> 12
   <212> PRT
   <213> Artificial Sequence
<220>
   <223> IL-1 ANTAGONIST PEPTIDE
<400> 714
   Ala Arg Val Trp Trp Gln Pro Tyr Ser Val Gln Met
   1 5 10
<210> 715
   <211> 12
   <212> PRT
   <213> Artificial Sequence
<220>
   <223> IL-1 ANTAGONIST PEPTIDE
<400> 715
   Arg Leu Met Phe Tyr Gln Pro Tyr Ser Val Gln Arg
   1 5 10
<210> 716
   <211> 12
   <212> PRT
   <213> Artificial Sequence
<220>
   <223> IL-1 ANTAGONIST PEPTIDE
<400> 716
   Glu Ser Met Trp Tyr Gln Pro Tyr Ser Val Gln Arg
   1 5 10
<210> 717
   <211> 12
   <212> PRT
   <213> Artificial Sequence
<220>
   <223> IL-1 ANTAGONIST PEPTIDE
<400> 717
   His Phe Gly Trp Trp Gln Pro Tyr Ser Val His Met
   1 5 10
<210> 718
   <211> 12
   <212> PRT
   <213> Artificial Sequence
<220>
   <223> IL-1 ANTAGONIST PEPTIDE
<400> 718
   Ala Arg Phe Trp Trp Gln Pro Tyr Ser Val Gln Arg
   1               5                   10
<210> 719
   <211> 12
   <212> PRT
   <213> Artificial Sequence
<220>
   <223> IL-1 ANTAGONIST PEPTIDE
<400> 719
   Arg Leu Val Tyr Trp Gln Pro Tyr Ala Pro Ile Tyr
   1               5                   10
<210> 720
   <211> 12
   <212> PRT
   <213> Artificial Sequence
<220>
   <223> IL-1 ANTAGONIST PEPTIDE
<400> 720
   Arg Leu Val Tyr Trp Gln Pro Tyr Ser Tyr Gln Thr
   1               5                   10
<210> 721
   <211> 12
   <212> PRT
   <213> Artificial Sequence
<220>
   <223> IL-1 ANTAGONIST PEPTIDE
<400> 721
   Arg Leu Val Tyr Trp Gln Pro Tyr Ser Leu Pro Ile
   1               5                   10
<210> 722
   <211> 12
   <212> PRT
   <213> Artificial Sequence
<220>
   <223> IL-1 ANTAGONIST PEPTIDE
<400> 722
   Arg Leu Val Tyr Trp Gln Pro Tyr Ser Val Gln Ala
   1               5                   10
<210> 723
   <211> 12
   <212> PRT
   <213> Artificial Sequence
<220>
   <223> IL-1 ANTAGONIST PEPTIDE
<400> 723
   Ser Arg Val Trp Tyr Gln Pro Tyr Ala Lys Gly Leu
   1 5 10
<210> 724
   <211> 12
   <212> PRT
   <213> Artificial Sequence
<220>
   <223> IL-1 ANTAGONIST PEPTIDE
<400> 724
   Ser Arg Val Trp Tyr Gln Pro Tyr Ala Gln Gly Leu
   1 5 10
<210> 725
   <211> 12
   <212> PRT
   <213> Artificial Sequence

<220>
   <223> IL-1 ANTAGONIST PEPTIDE
<400> 725
   Ser Arg Val Trp Tyr Gln Pro Tyr Ala Met Pro Leu
   1 5 10
<210> 726
   <211> 12
   <212> PRT
   <213> Artificial Sequence
<220>
   <223> IL-1 ANTAGONIST PEPTIDE
<400> 726
   Ser Arg Val Trp Tyr Gln Pro Tyr Ser Val Gln Ala
   1 5 10
<210> 727
   <211> 12
   <212> PRT
   <213> Artificial Sequence
<220>
   <223> IL-1 ANTAGONIST PEPTIDE
<400> 727
   Ser Arg Val Trp Tyr Gln Pro Tyr Ser Leu Gly Leu
   1 5 10
<210> 728
   <211> 12
   <212> PRT
   <213> Artificial Sequence
<220>
   <223> IL-1 ANTAGONIST PEPTIDE
<400> 728
   Ser Arg Val Trp Tyr Gln Pro Tyr Ala Arg Glu Leu
   1 5 10
<210> 729
   <211> 12
   <212> PRT
   <213> Artificial Sequence
<220>
   <223> IL-1 ANTAGONIST PEPTIDE
<400> 729
   Ser Arg Val Trp Tyr Gln Pro Tyr Ser Arg Gln Pro
   1 5 10
<210> 730
   <211> 12
   <212> PRT
   <213> Artificial Sequence
<220>
   <223> IL-1 ANTAGONIST PEPTIDE
<400> 730
   Ser Arg Val Trp Tyr Gln Pro Tyr Phe Val Gln Pro
   1 5 10
<210> 731
   <211> 12
   <212> PRT
   <213> Artificial Sequence
<220>
   <223> IL-1 ANTAGONIST PEPTIDE
<400> 731
   Glu Tyr Glu Trp Tyr Gln Pro Tyr Ala Leu Pro Leu
   1 5 10
<210> 732
   <211> 12
   <212> PRT
   <213> Artificial Sequence
<220>
   <223> IL-1 ANTAGONIST PEPTIDE
<400> 732
   Ile Pro Glu Tyr Trp Gln Pro Tyr Ala Leu Pro Leu
   1 5 10
<210> 733
   <211> 12
   <212> PRT
   <213> Artificial Sequence
<220>
   <223> IL-1 ANTAGONIST PEPTIDE
<400> 733
   Ser Arg Ile Trp Trp Gln Pro Tyr Ala Leu Pro Leu
   1 5 10
<210> 734
   <211> 12
   <212> PRT
   <213> Artificial Sequence
<220>
   <223> IL-1 ANTAGONIST PEPTIDE
<400> 734
   Asp Pro Leu Phe Trp Gln Pro Tyr Ala Leu Pro Leu
   1 5 10
<210> 735
   <211> 12
   <212> PRT
   <213> Artificial Sequence
<220>
   <223> IL-1 ANTAGONIST PEPTIDE
<400> 735
   Ser Arg Gln Trp Val Gln Pro Tyr Ala Leu Pro Leu
   1 5 10
<210> 736
   <211> 12
   <212> PRT
   <213> Artificial Sequence
<220>
   <223> IL-1 ANTAGONIST PEPTIDE
<400> 736
   Ile Arg Ser Trp Trp Gln Pro Tyr Ala Leu Pro Leu
   1               5                   10
<210> 737
   <211> 11
   <212> PRT
   <213> Artificial Sequence
<220>
   <223> IL-1 ANTAGONIST PEPTIDE
<400> 737
   Arg Gly Tyr Trp Gln Pro Tyr Ala Leu Pro Leu
   1               5                   10
<210> 738
   <211> 12
   <212> PRT
   <213> Artificial Sequence
<220>
   <223> IL-1 ANTAGONIST PEPTIDE
<400> 738
   Arg Leu Leu Trp Val Gln Pro Tyr Ala Leu Pro Leu
   1               5                   10
<210> 739
   <211> 12
   <212> PRT
   <213> Artificial Sequence
<220>
   <223> IL-1 ANTAGONIST PEPTIDE
<400> 739
   Glu Tyr Arg Trp Phe Gln Pro Tyr Ala Leu Pro Leu
   1               5                   10
<210> 740
   <211> 12
   <212> PRT
   <213> Artificial Sequence
<220>
   <223> IL-1 ANTAGONIST PEPTIDE
<400> 740
   Asp Ala Tyr Trp Val Gln Pro Tyr Ala Leu Pro Leu
   1               5                   10
<210> 741
   <211> 12
   <212> PRT
   <213> Artificial Sequence
<220>
   <223> IL-1 ANTAGONIST PEPTIDE
<400> 741
   Trp Ser Gly Tyr Phe Gln Pro Tyr Ala Leu Pro Leu
   1 5 10
<210> 742
   <211> 12
   <212> PRT
   <213> Artificial Sequence
<220>
   <223> IL-1 ANTAGONIST PEPTIDE
<400> 742
   Asn Ile Glu Phe Trp Gln Pro Tyr Ala Leu Pro Leu
   1 5 10
<210> 743
   <211> 12
   <212> PRT
   <213> Artificial Sequence
<220>
   <223> IL-1 ANTAGONIST PEPTIDE
<400> 743
   Thr Arg Asp Trp Val Gln Pro Tyr Ala Leu Pro Leu
   1 5 10
<210> 744
   <211> 12
   <212> PRT
   <213> Artificial Sequence
<220>
   <223> IL-1 ANTAGONIST PEPTIDE
<400> 744
   Asp Ser Ser Trp Tyr Gln Pro Tyr Ala Leu Pro Leu
   1 5 10
<210> 745
   <211> 12
   <212> PRT
   <213> Artificial Sequence
<220>
   <223> IL-1 ANTAGONIST PEPTIDE
<400> 745
   Ile Gly Asn Trp Tyr Gln Pro Tyr Ala Leu Pro Leu
   1 5 10
<210> 746
   <211> 12
   <212> PRT
   <213> Artificial Sequence
<220>
   <223> IL-1 ANTAGONIST PEPTIDE
<400> 746
   Asn Leu Arg Trp Asp Gln Pro Tyr Ala Leu Pro Leu
   1 5 10
<210> 747
   <211> 12
   <212> PRT
   <213> Artificial Sequence
<220>
   <223> IL-1 ANTAGONIST PEPTIDE
<400> 747
   Leu Pro Glu Phe Trp Gln Pro Tyr Ala Leu Pro Leu
   1 5 10
<210> 748
   <211> 12
   <212> PRT
   <213> Artificial Sequence
<220>
   <223> IL-1 ANTAGONIST PEPTIDE
<400> 748
   Asp Ser Tyr Trp Trp Gln Pro Tyr Ala Leu Pro Leu
   1 5 10
<210> 749
   <211> 12
   <212> PRT
   <213> Artificial Sequence
<220>
   <223> IL-1 ANTAGONIST PEPTIDE
<400> 749
   Arg Ser Gln Tyr Tyr Gln Pro Tyr Ala Leu Pro Leu
   1 5 10
<210> 750
   <211> 12
   <212> PRT
   <213> Artificial Sequence
<220>
   <223> IL-1 ANTAGONIST PEPTIDE
<400> 750
   Ala Arg Phe Trp Leu Gln Pro Tyr Ala Leu Pro Leu
   1 5 10
<210> 751
   <211> 12
   <212> PRT
   <213> Artificial Sequence
<220>
   <223> IL-1 ANTAGONIST PEPTIDE
<400> 751
   Asn Ser Tyr Phe Trp Gln Pro Tyr Ala Leu Pro Leu
   1 5 10
<210> 752
   <211> 12
   <212> PRT
   <213> Artificial Sequence
<220>
   <223> IL-1 ANTAGONIST PEPTIDE
<400> 752
   Arg Phe Met Tyr Trp Gln Pro Tyr Ser Val Gln Arg
   1 5 10
<210> 753
   <211> 12
   <212> PRT
   <213> Artificial Sequence
<220>
   <223> IL-1 ANTAGONIST PEPTIDE
<400> 753
   Ala His Leu Phe Trp Gln Pro Tyr Ser Val Gln Arg
   1 5 10
<210> 754
   <211> 9
   <212> PRT
   <213> Artificial Sequence
<220>
   <223> IL-1 ANTAGONIST PEPTIDE
<400> 754
   Trp Trp Gln Pro Tyr Ala Leu Pro Leu
   1 5
<210> 755
   <211> 9
   <212> PRT
   <213> Artificial Sequence
<220>
   <223> IL-1 ANTAGONIST PEPTIDE
<400> 755
   Tyr Tyr Gln Pro Tyr Ala Leu Pro Leu
   1 5
<210> 756
   <211> 9
   <212> PRT
   <213> Artificial Sequence
<220>
   <223> IL-1 ANTAGONIST PEPTIDE
<400> 756
   Tyr Phe Gln Pro Tyr Ala Leu Gly Leu
   1 5
<210> 757
   <211> 10
   <212> PRT
   <213> Artificial Sequence
<220>
   <223> IL-1 ANTAGONIST PEPTIDE
<400> 757
   Tyr Trp Tyr Gln Pro Tyr Ala Leu Pro Leu
   1 5 10
<210> 758
   <211> 10
   <212> PRT
   <213> Artificial Sequence
<220>
   <223> IL-1 ANTAGONIST PEPTIDE
<400> 758
   Arg Trp Trp Gln Pro Tyr Ala Thr Pro Leu
   1 5 10
<210> 759
   <211> 10
   <212> PRT
   <213> Artificial Sequence
<220>
   <223> IL-1 ANTAGONIST PEPTIDE
<400> 759
   Gly Trp Tyr Gln Pro Tyr Ala Leu Gly Phe
   1 5 10
<210> 760
   <211> 10
   <212> PRT
   <213> Artificial Sequence
<220>
   <223> IL-1 ANTAGONIST PEPTIDE
<400> 760
   Tyr Trp Tyr Gln Pro Tyr Ala Leu Gly Leu
   1 5 10
<210> 761
   <211> 10
   <212> PRT
   <213> Artificial Sequence
<220>
   <223> IL-1 ANTAGONIST PEPTIDE
<400> 761
   Ile Trp Tyr Gln Pro Tyr Ala Met Pro Leu
   1 5 10
<210> 762
   <211> 10
   <212> PRT
   <213> Artificial Sequence
<220>
   <223> IL-1 ANTAGONIST PEPTIDE
<400> 762
   Ser Asn Met Gln Pro Tyr Gln Arg Leu Ser
   1 5 10
<210> 763
   <211> 20
   <212> PRT
   <213> Artificial Sequence
<220>
   <223> IL-1 ANTAGONIST PEPTIDE
<400> 763
   Thr Phe Val Tyr Trp Gln Pro Tyr Ala Val Gly Leu Pro Ala Ala Glu
   1 5 10 15
   Thr Ala Cys Asn
   20
<210> 764
   <211> 20
   <212> PRT
   <213> Artificial Sequence
<220>
   <223> IL-1 ANTAGONIST PEPTIDE
<400> 764
   Thr Phe Val Tyr Trp Gln Pro Tyr Ser Val Gln Met Thr Ile Thr Gly
   1 5 10 15
   Lys Val Thr Met
   20

<210> 765
   <211> 20
   <212> PRT
   <213> Artificial Sequence
<220>
   <223> IL-1 ANTAGONIST PEPTIDE
<220>
   <221> misc_feature
   <222> (12, 13)..(16)
   <223> Xaa = any amino acid
<400> 765
   Thr Phe Val Tyr Trp Gln Pro Tyr Ser Ser His Xaa Xaa Val Pro Xaa
   1 5 10 15
   Gly Phe Pro Leu
   20
<210> 766
   <211> 20
   <212> PRT
   <213> Artificial Sequence
<220>
   <223> IL-1 ANTAGONIST PEPTIDE
<400> 766
   Thr Phe Val Tyr Trp Gln Pro Tyr Tyr Gly Asn Pro Gln Trp Ala Ile
   1 5 10 15
   His Val Arg His
   20
<210> 767
   <211> 20
   <212> PRT
   <213> Artificial Sequence
<220>
   <223> IL-1 ANTAGONIST PEPTIDE
<400> 767
   Thr Phe Val Tyr Trp Gln Pro Tyr Val Leu Leu Glu Leu Pro Glu Gly
   1 5 10 15
   Ala Val Arg Ala
   20
<210> 768
   <211> 20
   <212> PRT
   <213> Artificial Sequence
<220>
   <223> IL-1 ANTAGONIST PEPTIDE
<400> 768
   Thr Phe Val Tyr Trp Gln Pro Tyr Val Asp Tyr Val Trp Pro Ile Pro
   1 5 10 15
   Ile Ala Gln Val
   20
<210> 769
   <211> 11
   <212> PRT
   <213> Artificial Sequence
<220>
   <223> IL-1 ANTAGONIST PEPTIDE
<400> 769
   Gly Trp Tyr Gln Pro Tyr Val Asp Gly Trp Arg
   1 5 10
<210> 770
   <211> 12
   <212> PRT
   <213> Artificial Sequence
<220>
   <223> IL-1 ANTAGONIST PEPTIDE
<400> 770
   Arg Trp Glu Gln Pro Tyr Val Lys Asp Gly Trp Ser
   1 5 10
<210> 771
   <211> 12
   <212> PRT
   <213> Artificial Sequence
<220>
   <223> IL-1 ANTAGONIST PEPTIDE
<400> 771
   Glu Trp Tyr Gln Pro Tyr Ala Leu Gly Trp Ala Arg
   1 5 10
<210> 772
   <211> 10
   <212> PRT
   <213> Artificial Sequence
<220>
   <223> IL-1 ANTAGONIST PEPTIDE
<400> 772
   Gly Trp Trp Gln Pro Tyr Ala Arg Gly Leu
   1 5 10
<210> 773
   <211> 12
   <212> PRT
   <213> Artificial Sequence
<220>
   <223> IL-1 ANTAGONIST PEPTIDE
<400> 773
   Leu Phe Glu Gln Pro Tyr Ala Lys Ala Leu Gly Leu
   1 5 10
<210> 774
   <211> 12
   <212> PRT
   <213> Artificial Sequence
<220>
   <223> IL-1 ANTAGONIST PEPTIDE
<400> 774
   Gly Trp Glu Gln Pro Tyr Ala Arg Gly Leu Ala Gly
   1 5 10
<210> 775
   <211> 12
   <212> PRT
   <213> Artificial Sequence
<220>
   <223> IL-1 ANTAGONIST PEPTIDE
<400> 775
   Ala Trp Val Gln Pro Tyr Ala Thr Pro Leu Asp Glu
   1 5 10
<210> 776
   <211> 12
   <212> PRT
   <213> Artificial Sequence
<220>
   <223> IL-1 ANTAGONIST PEPTIDE
<400> 776
   Met Trp Tyr Gln Pro Tyr Ser Ser Gln Pro Ala Glu
   1 5 10
<210> 777
   <211> 12
   <212> PRT
   <213> Artificial Sequence
<220>
   <223> IL-1 ANTAGONIST PEPTIDE
<400> 777
   Gly Trp Thr Gln Pro Tyr Ser Gln Gln Gly Glu Val
   1 5 10
<210> 778
   <211> 12
   <212> PRT
   <213> Artificial Sequence
<220>
   <223> IL-1 ANTAGONIST PEPTIDE
<400> 778
   Asp Trp Phe Gln Pro Tyr Ser Ile Gln Ser Asp Glu
   1 5 10
<210> 779
   <211> 11
   <212> PRT
   <213> Artificial Sequence
<220>
   <223> IL-1 ANTAGONIST PEPTIDE
<400> 779
   Pro Trp Ile Gln Pro Tyr Ala Arg Gly Phe Gly
   1               5                   10
<210> 780
   <211> 12
   <212> PRT
   <213> Artificial Sequence
<220>
   <223> IL-1 ANTAGONIST PEPTIDE
<400> 780
   Arg Pro Leu Tyr Trp Gln Pro Tyr Ser Val Gln Val
   1               5                   10
<210> 781
   <211> 12
   <212> PRT
   <213> Artificial Sequence
<220>
   <223> IL-1 ANTAGONIST PEPTIDE
<400> 781
   Thr Leu Ile Tyr Trp Gln Pro Tyr Ser Val Gln Ile
   1               5                   10
<210> 782
   <211> 12
   <212> PRT
   <213> Artificial Sequence
<220>
   <223> IL-1 ANTAGONIST PEPTIDE
<400> 782
   Arg Phe Asp Tyr Trp Gln Pro Tyr Ser Asp Gln Thr
   1               5                   10
<210> 783
   <211> 12
   <212> PRT
   <213> Artificial Sequence
<220>
   <223> IL-1 ANTAGONIST PEPTIDE
<400> 783
   Trp His Gln Phe Val Gln Pro Tyr Ala Leu Pro Leu
   1 5 10
<210> 784
   <211> 17
   <212> PRT
   <213> Artificial Sequence
<220>
   <223> IL-1 ANTAGONIST PEPTIDE
<400> 784
   Glu Trp Asp Ser Val Tyr Trp Gln Pro Tyr Ser Val Gln Thr Leu Leu
   1 5 10 15
   Arg
<210> 785
   <211> 17
   <212> PRT
   <213> Artificial Sequence
<220>
   <223> IL-1 ANTAGONIST PEPTIDE
<400> 785
   Trp Glu Gln Asn Val Tyr Trp Gln Pro Tyr Ser Val Gln Ser Phe Ala
   1 5 10 15
   Asp
<210> 786
   <211> 16
   <212> PRT
   <213> Artificial Sequence
<220>
   <223> IL-1 ANTAGONIST PEPTIDE
<400> 786
   Ser Asp Val Val Tyr Trp Gln Pro Tyr Ser Val Gln Ser Leu Glu Met
   1 5 10 15
<210> 787
   <211> 17
   <212> PRT
   <213> Artificial Sequence
<220>
   <223> IL-1 ANTAGONIST PEPTIDE
<400> 787
   Tyr Tyr Asp Gly Val Tyr Trp Gln Pro Tyr Ser Val Gln Val Met Pro
   1 5 10 15
   Ala
<210> 788
   <211> 12
   <212> PRT
   <213> Artificial Sequence
<220>
   <223> IL-1 ANTAGONIST PEPTIDE
<400> 788
   Ser Asp Ile Trp Tyr Gln Pro Tyr Ala Leu Pro Leu
   1 5 10
<210> 789
   <211> 12
   <212> PRT
   <213> Artificial Sequence
<220>
   <223> IL-1 ANTAGONIST PEPTIDE
<400> 789
   Gln Arg Ile Trp Trp Gln Pro Tyr Ala Leu Pro Leu
   1 5 10
<210> 790
   <211> 12
   <212> PRT
   <213> Artificial Sequence
<220>
   <223> IL-1 ANTAGONIST PEPTIDE
<400> 790
   Ser Arg Ile Trp Trp Gln Pro Tyr Ala Leu Pro Leu
   1 5 10
<210> 791
   <211> 12
   <212> PRT
   <213> Artificial Sequence
<220>
   <223> IL-1 ANTAGONIST PEPTIDE
<400> 791
   Arg Ser Leu Tyr Trp Gln Pro Tyr Ala Leu Pro Leu
   1 5 10
<210> 792
   <211> 12
   <212> PRT
   <213> Artificial Sequence
<220>
   <223> IL-1 ANTAGONIST PEPTIDE
<400> 792
   Thr Ile Ile Trp Glu Gln Pro Tyr Ala Leu Pro Leu
   1 5 10
<210> 793
   <211> 12
   <212> PRT
   <213> Artificial Sequence
<220>
   <223> IL-1 ANTAGONIST PEPTIDE
<400> 793
   Trp Glu Thr Trp Tyr Gln Pro Tyr Ala Leu Pro Leu
   1 5 10
<210> 794
   <211> 12
   <212> PRT
   <213> Artificial Sequence
<220>
   <223> IL-1 ANTAGONIST PEPTIDE
<400> 794
   Ser Tyr Asp Trp Glu Gln Pro Tyr Ala Leu Pro Leu
   1 5 10
<210> 795
   <211> 12
   <212> PRT
   <213> Artificial Sequence
<220>
   <223> IL-1 ANTAGONIST PEPTIDE
<400> 795
   Ser Arg Ile Trp Cys Gln Pro Tyr Ala Leu Pro Leu
   1 5 10
<210> 796
   <211> 12
   <212> PRT
   <213> Artificial Sequence
<220>
   <223> IL-1 ANTAGONIST PEPTIDE
<400> 796
   Glu Ile Met Phe Trp Gln Pro Tyr Ala Leu Pro Leu
   1 5 10
<210> 797
   <211> 12
   <212> PRT
   <213> Artificial Sequence
<220>
   <223> IL-1 ANTAGONIST PEPTIDE
<400> 797
   Asp Tyr Val Trp Gln Gln Pro Tyr Ala Leu Pro Leu
   1 5 10
<210> 798
   <211> 15
   <212> PRT
   <213> Artificial Sequence
<220>
   <223> IL-1 ANTAGONIST PEPTIDE
<400> 798
   Met Asp Leu Leu Val Gln Trp Tyr Gln Pro Tyr Ala Leu Pro Leu
   1 5 10 15
<210> 799
   <211> 15
   <212> PRT
   <213> Artificial Sequence
<220>
   <223> IL-1 ANTAGONIST PEPTIDE
<400> 799
   Gly Ser Lys Val Ile Leu Trp Tyr Gln Pro Tyr Ala Leu Pro Leu
   1 5 10 15
<210> 800
   <211> 15
   <212> PRT
   <213> Artificial Sequence
<220>
   <223> IL-1 ANTAGONIST PEPTIDE
<400> 800
   Arg Gln Gly Ala Asn Ile Trp Tyr Gln Pro Tyr Ala Leu Pro Leu
   1 5 10 15
<210> 801
   <211> 15
   <212> PRT
   <213> Artificial Sequence
<220>
   <223> IL-1 ANTAGONIST PEPTIDE
<400> 801
   Gly Gly Gly Asp Glu Pro Trp Tyr Gln Pro Tyr Ala Leu Pro Leu
   1 5 10 15
<210> 802
   <211> 15
   <212> PRT
   <213> Artificial Sequence
<220>
   <223> IL-1 ANTAGONIST PEPTIDE
<400> 802
   Ser Gln Leu Glu Arg Thr Trp Tyr Gln Pro Tyr Ala Leu Pro Leu
   1 5 10 15
<210> 803
   <211> 15
   <212> PRT
   <213> Artificial Sequence
<220>
   <223> IL-1 ANTAGONIST PEPTIDE
<400> 803
   Glu Thr Trp Val Arg Glu Trp Tyr Gln Pro Tyr Ala Leu Pro Leu
   1 5 10 15
<210> 804
   <211> 15
   <212> PRT
   <213> Artificial Sequence
<220>
   <223> IL-1 ANTAGONIST PEPTIDE
<400> 804
   Lys Lys Gly Ser Thr Gln Trp Tyr Gln Pro Tyr Ala Leu Pro Leu
   1 5 10 15
<210> 805
   <211> 15
   <212> PRT
   <213> Artificial Sequence
<220>
   <223> IL-1 ANTAGONIST PEPTIDE
<400> 805
   Leu Gln Ala Arg Met Asn Trp Tyr Gln Pro Tyr Ala Leu Pro Leu
   1 5 10 15
<210> 806
   <211> 15
   <212> PRT
   <213> Artificial Sequence
<220>
   <223> IL-1 ANTAGONIST PEPTIDE
<400> 806
   Glu Pro Arg Ser Gln Lys Trp Tyr Gln Pro Tyr Ala Leu Pro Leu
   1 5 10 15
<210> 807
   <211> 15
   <212> PRT
   <213> Artificial Sequence
<220>
   <223> IL-1 ANTAGONIST PEPTIDE
<400> 807
   Val Lys Gln Lys Trp Arg Trp Tyr Gln Pro Tyr Ala Leu Pro Leu
   1 5 10 15
<210> 808
   <211> 15
   <212> PRT
   <213> Artificial Sequence
<220>
   <223> IL-1 ANTAGONIST PEPTIDE
<400> 808
   Leu Arg Arg His Asp Val Trp Tyr Gln Pro Tyr Ala Leu Pro Leu
   1 5 10 15
<210> 809
   <211> 15
   <212> PRT
   <213> Artificial Sequence
<220>
   <223> IL-1 ANTAGONIST PEPTIDE
<400> 809
   Arg Ser Thr Ala Ser Ile Trp Tyr Gln Pro Tyr Ala Leu Pro Leu
   1 5 10 15
<210> 810
   <211> 15
   <212> PRT
   <213> Artificial Sequence
<220>
   <223> IL-1 ANTAGONIST PEPTIDE
<400> 810
   Glu Ser Lys Glu Asp Gln Trp Tyr Gln Pro Tyr Ala Leu Pro Leu
   1 5 10 15
<210> 811
   <211> 15
   <212> PRT
   <213> Artificial Sequence
<220>
   <223> IL-1 ANTAGONIST PEPTIDE
<400> 811
   Glu Gly Leu Thr Met Lys Trp Tyr Gln Pro Tyr Ala Leu Pro Leu
   1 5 10 15
<210> 812
   <211> 15
   <212> PRT
   <213> Artificial Sequence
<220>
   <223> IL-1 ANTAGONIST PEPTIDE
<400> 812
   Glu Gly Ser Arg Glu Gly Trp Tyr Gln Pro Tyr Ala Leu Pro Leu
   1 5 10 15
<210> 813
   <211> 12
   <212> PRT
   <213> Artificial Sequence
<220>
   <223> IL-1 ANTAGONIST PEPTIDE
<400> 813
   Val Ile Glu Trp Trp Gln Pro Tyr Ala Leu Pro Leu
   1 5 10
<210> 814
   <211> 12
   <212> PRT
   <213> Artificial Sequence
<220>
   <223> IL-1 ANTAGONIST PEPTIDE
<400> 814
   Val Trp Tyr Trp Glu Gln Pro Tyr Ala Leu Pro Leu
   1 5 10
<210> 815
   <211> 12
   <212> PRT
   <213> Artificial Sequence
<220>
   <223> IL-1 ANTAGONIST PEPTIDE
<400> 815
   Ala Ser Glu Trp Trp Gln Pro Tyr Ala Leu Pro Leu
   1 5 10
<210> 816
   <211> 12
   <212> PRT
   <213> Artificial Sequence
<220>
   <223> IL-1 ANTAGONIST PEPTIDE
<400> 816
   Phe Tyr Glu Trp Trp Gln Pro Tyr Ala Leu Pro Leu
   1 5 10
<210> 817
   <211> 12
   <212> PRT
   <213> Artificial Sequence
<220>
   <223> IL-1 ANTAGONIST PEPTIDE
<400> 817
   Glu Gly Trp Trp Val Gln Pro Tyr Ala Leu Pro Leu
   1 5 10
<210> 818
   <211> 12
   <212> PRT
   <213> Artificial Sequence
<220>
   <223> IL-1 ANTAGONIST PEPTIDE
<400> 818
   Trp Gly Glu Trp Leu Gln Pro Tyr Ala Leu Pro Leu
   1 5 10
<210> 819
   <211> 12
   <212> PRT
   <213> Artificial Sequence
<220>
   <223> IL-1 ANTAGONIST PEPTIDE
<400> 819
   Asp Tyr Val Trp Glu Gln Pro Tyr Ala Leu Pro Leu
   1 5 10
<210> 820
   <211> 12
   <212> PRT
   <213> Artificial Sequence
<220>
   <223> IL-1 ANTAGONIST PEPTIDE
<400> 820
   Ala His Thr Trp Trp Gln Pro Tyr Ala Leu Pro Leu
   1 5 10
<210> 821
   <211> 12
   <212> PRT
   <213> Artificial Sequence
<220>
   <223> IL-1 ANTAGONIST PEPTIDE
<400> 821
   Phe Ile Glu Trp Phe Gln Pro Tyr Ala Leu Pro Leu
   1 5 10
<210> 822
   <211> 12
   <212> PRT
   <213> Artificial Sequence
<220>
   <223> IL-1 ANTAGONIST PEPTIDE
<400> 822
   Trp Leu Ala Trp Glu Gln Pro Tyr Ala Leu Pro Leu
   1 5 10
<210> 823
   <211> 12
   <212> PRT
   <213> Artificial Sequence
<220>
   <223> IL-1 ANTAGONIST PEPTIDE
<400> 823
   Val Met Glu Trp Trp Gln Pro Tyr Ala Leu Pro Leu
   1               5                   10
<210> 824
   <211> 11
   <212> PRT
   <213> Artificial Sequence
<220>
   <223> IL-1 ANTAGONIST PEPTIDE
<400> 824
   Glu Arg Met Trp Gln Pro Tyr Ala Leu Pro Leu
   1               5                   10
<210> 825
   <211> 12
   <212> PRT
   <213> Artificial Sequence
<220>
   <223> IL-1 ANTAGONIST PEPTIDE
<220>
   <221> misc_feature
   <222> (2, 3, 5)..(6)
   <223> Xaa = any amino acid
<400> 825
   Asn Xaa Xaa Trp Xaa Xaa Pro Tyr Ala Leu Pro Leu
   1               5                   10
<210> 826
   <211> 12
   <212> PRT
   <213> Artificial Sequence
<220>
   <223> IL-1 ANTAGONIST PEPTIDE
<400> 826
   Trp Gly Asn Trp Tyr Gln Pro Tyr Ala Leu Pro Leu
   1               5                   10
<210> 827
   <211> 12
   <212> PRT
   <213> Artificial Sequence
<220>
   <223> IL-1 ANTAGONIST PEPTIDE
<400> 827
   Thr Leu Tyr Trp Glu Gln Pro Tyr Ala Leu Pro Leu
   1 5 10
<210> 828
   <211> 12
   <212> PRT
   <213> Artificial Sequence
<220>
   <223> IL-1 ANTAGONIST PEPTIDE
<400> 828
   Val Trp Arg Trp Glu Gln Pro Tyr Ala Leu Pro Leu
   1 5 10
<210> 829
   <211> 11
   <212> PRT
   <213> Artificial Sequence
<220>
   <223> IL-1 ANTAGONIST PEPTIDE
<400> 829
   Leu Leu Trp Thr Gln Pro Tyr Ala Leu Pro Leu
   1 5 10
<210> 830
   <211 > 12
   <212> PRT
   <213> Artificial Sequence
<220>
   <223> IL-1 ANTAGONIST PEPTIDE
<220>
   <221> misc_feature
   <222> (5)..(6)
   <223> Xaa = any amino acid
<400> 830
   Ser Arg Ile Trp Xaa Xaa Pro Tyr Ala Leu Pro Leu
   1 5 10
<210> 831
   <211> 12
   <212> PRT
   <213> Artificial Sequence
<220>
   <223> IL-1 ANTAGONIST PEPTIDE
<400> 831
   Ser Asp Ile Trp Tyr Gln Pro Tyr Ala Leu Pro Leu
   1 5 10
<210> 832
   <211> 12
   <212> PRT
   <213> Artificial Sequence
<220>
   <223> IL-1 ANTAGONIST PEPTIDE
<220>
   <221> misc_feature
   <222> (5).. (6)
   <223> Xaa = any amino acid
<400> 832
   Trp Gly Tyr Tyr Xaa Xaa Pro Tyr Ala Leu Pro Leu
   1 5 10
<210> 833
   <211> 12
   <212> PRT
   <213> Artificial Sequence
<220>
   <223> IL-1 ANTAGONIST PEPTIDE
<400> 833
   Thr Ser Gly Trp Tyr Gln Pro Tyr Ala Leu Pro Leu
   1 5 10
<210> 834
   <211> 12
   <212> PRT
   <213> Artificial Sequence
<220>
   <223> IL-1 ANTAGONIST PEPTIDE
<220>
   <221> misc_feature
   <222> (5)..(6)
   <223> Xaa = any amino acid
<400> 834
   Val His Pro Tyr Xaa Xaa Pro Tyr Ala Leu Pro Leu
   1 5 10
<210> 835
   <211> 12
   <212> PRT
   <213> Artificial Sequence
<220>
   <223> IL-1 ANTAGONIST PEPTIDE
<400> 835
   Glu His Ser Tyr Phe Gln Pro Tyr Ala Leu Pro Leu
   1 5 10
<210> 836
   <211> 12
   <212> PRT
   <213> Artificial Sequence
<220>
   <223> IL-1 ANTAGONIST PEPTIDE
<220>
   <221> misc_feature
   <222> (1)..(2)
   <223> Xaa = any amino acid
<400> 836
   Xaa Xaa Ile Trp Tyr Gln Pro Tyr Ala Leu Pro Leu
   1 5 10
<210> 837
   <211> 12
   <212> PRT
   <213> Artificial Sequence
<220>
   <223> IL-1 ANTAGONIST PEPTIDE
<400> 837
   Ala Gln Leu His Ser Gln Pro Tyr Ala Leu Pro Leu
   1 5 10
<210> 838
   <211> 12
   <212> PRT
   <213> Artificial Sequence
<220>
   <223> IL-1 ANTAGONIST PEPTIDE
<400> 838
   Trp Ala Asn Trp Phe Gln Pro Tyr Ala Leu Pro Leu
   1 5 10
<210> 839
   <211> 12
   <212> PRT
   <213> Artificial Sequence
<220>
   <223> IL-1 ANTAGONIST PEPTIDE
<400> 839
   Ser Arg Leu Tyr Ser Gln Pro Tyr Ala Leu Pro Leu
   1 5 10
<210> 840
   <211> 12
   <212> PRT
   <213> Artificial Sequence
<220>
   <223> IL-1 ANTAGONIST PEPTIDE
<400> 840
   Gly Val Thr Phe Ser Gln Pro Tyr Ala Leu Pro Leu
   1 5 10

<210> 841
   <211> 12
   <212> PRT
   <213> Artificial Sequence
<220>
   <223> IL-1 ANTAGONIST PEPTIDE
<400> 841
   Ser Ile Val Trp Ser Gln Pro Tyr Ala Leu Pro Leu
   1 5 10
<210> 842
   <211> 12
   <212> PRT
   <213> Artificial Sequence
<220>
   <223> IL-1 ANTAGONIST PEPTIDE
<400> 842
   Ser Arg Asp Leu Val Gln Pro Tyr Ala Leu Pro Leu
   1 5 10
<210> 843
   <211> 17
   <212> PRT
   <213> Artificial Sequence
<220>
   <223> IL-1 ANTAGONIST PEPTIDE
<400> 843
   His Trp Gly His Val Tyr Trp Gln Pro Tyr Ser Val Gln Asp Asp Leu
   1 5 10 15
   Gly
<210> 844
   <211> 17
   <212> PRT
   <213> Artificial Sequence
<220>
   <223> IL-1 ANTAGONIST PEPTIDE
<400> 844
   Ser Trp His Ser Val Tyr Trp Gln Pro Tyr Ser Val Gln Ser Val Pro
   1 5 10 15
   Glu
<210> 845
   <211> 17
   <212> PRT
   <213> Artificial Sequence
<220>
   <223> IL-1 ANTAGONIST PEPTIDE
<400> 845
   Trp Arg Asp Ser Val Tyr Trp Gln Pro Tyr Ser Val Gln Pro Glu Ser
   1 5 10 15
   Ala
<210> 846
   <211> 17
   <212> PRT
   <213> Artificial Sequence
<220>
   <223> IL-1 ANTAGONIST PEPTIDE
<400> 846
   Thr Trp Asp Ala Val Tyr Trp Gln Pro Tyr Ser Val Gln Lys Trp Leu
   1 5 10 15
   Asp
<210> 847
   <211> 17
   <212> PRT
   <213> Artificial Sequence
<220>
   <223> IL-1 ANTAGONIST PEPTIDE
<400> 847
   Thr Pro Pro Trp Val Tyr Trp Gln Pro Tyr Ser Val Gln Ser Leu Asp
   1 5 10 15
   Pro
<210> 848
   <211> 17
   <212> PRT
   <213> Artificial Sequence
<220>
   <223> IL-1 ANTAGONIST PEPTIDE
<400> 848
   Tyr Trp Ser Ser Val Tyr Trp Gln Pro Tyr Ser Val Gln Ser Val His
   1 5 10 15
   Ser
<210> 849
   <211> 10
   <212> PRT
   <213> Artificial Sequence
<220>
   <223> IL-1 ANTAGONIST PEPTIDE
<400> 849
   Tyr Trp Tyr Gln Pro Tyr Ala Leu Gly Leu
   1 5 10
<210> 850
   <211> 10
   <212> PRT
   <213> Artificial Sequence
<220>
   <223> IL-1 ANTAGONIST PEPTIDE
<400> 850
   Tyr Trp Tyr Gln Pro Tyr Ala Leu Pro Leu
   1               5                   10
<210> 851
   <211> 10
   <212> PRT
   <213> Artificial Sequence
<220>
   <223> IL-1 ANTAGONIST PEPTIDE
<400> 851
   Glu Trp Ile Gln Pro Tyr Ala Thr Gly Leu
   1               5                   10
<210> 852
   <211> 10
   <212> PRT
   <213> Artificial Sequence
<220>
   <223> IL-1 ANTAGONIST PEPTIDE
<400> 852
   Asn Trp Glu Gln Pro Tyr Ala Lys Pro Leu
   1               5                   10
<210> 853
   <211> 10
   <212> PRT
   <213> Artificial Sequence
<220>
   <223> IL-1 ANTAGONIST PEPTIDE
<400> 853
   Ala Phe Tyr Gln Pro Tyr Ala Leu Pro Leu
   1               5                   10
<210> 854
   <211> 10
   <212> PRT
   <213> Artificial Sequence
<220>
   <223> IL-1 ANTAGONIST PEPTIDE
<400> 854
   Phe Leu Tyr Gln Pro Tyr Ala Leu Pro Leu
   1               5                   10
<210> 855
   <211> 10
   <212> PRT
   <213> Artificial Sequence
<220>
   <223> IL-1 ANTAGONIST PEPTIDE
<400> 855
   Val Cys Lys Gln Pro Tyr Leu Glu Trp Cys
   1 5 10
<210> 856
   <211> 21
   <212> PRT
   <213> Artificial Sequence
<220>
   <223> IL-1 ANTAGONIST PEPTIDE
<400> 856
   Glu Thr Pro Phe Thr Trp Glu Glu Ser Asn Ala Tyr Tyr Trp Gln Pro
   1 5 10 15
   Tyr Ala Leu Pro Leu
   20
<210> 857
   <211> 21
   <212> PRT
   <213> Artificial Sequence
<220>
   <223> IL-1 ANTAGONIST PEPTIDE
<400> 857
   Gln Gly Trp Leu Thr Trp Gln Asp Ser Val Asp Met Tyr Trp Gln Pro
   1 5 10 15
   Tyr Ala Leu Pro Leu
   20
<210> 858
   <211> 21
   <212> PRT
   <213> Artificial Sequence
<220>
   <223> IL-1 ANTAGONIST PEPTIDE
<400> 858
   Phe Ser Glu Ala Gly Tyr Thr Trp Pro Glu Asn Thr Tyr Trp Gln Pro
   1 5 10 15
   Tyr Ala Leu Pro Leu
   20
<210> 859
   <211> 21
   <212> PRT
   <213> Artificial Sequence
<220>
   <223> IL-1 ANTAGONIST PEPTIDE
<400> 859
   Thr Glu Ser Pro Gly Gly Leu Asp Trp Ala Lys Ile Tyr Trp Gln Pro
   1 5 10 15
   Tyr Ala Leu Pro Leu
   20
<210> 860
   <211> 21
   <212> PRT
   <213> Artificial Sequence
<220>
   <223> IL-1 ANTAGONIST PEPTIDE
<400> 860
   Asp Gly Tyr Asp Arg Trp Arg Gln Ser Gly Glu Arg Tyr Trp Gln Pro
   1 5 10 15
   Tyr Ala Leu Pro Leu
   20
<210> 861
   <211> 21
   <212> PRT
   <213> Artificial Sequence
<220>
   <223> IL-1 ANTAGONIST PEPTIDE
<400> 861
   Thr Ala Asn Val Ser Ser Phe Glu Trp Thr Pro Gly Tyr Trp Gln Pro
   1 5 10 15
   Tyr Ala Leu Pro Leu
   20
<210> 862
   <211> 21
   <212> PRT
   <213> Artificial Sequence
<220>
   <223> IL-1 ANTAGONIST PEPTIDE
<400> 862
   Ser Val Gly Glu Asp His Asn Phe Trp Thr Ser Glu Tyr Trp Gln Pro
   1 5 10 15
   Tyr Ala Leu Pro Leu
   20
<210> 863
   <211> 21
   <212> PRT
   <213> Artificial Sequence
<220>
   <223> IL-1 ANTAGONIST PEPTIDE
<400> 863
   Met Asn Asp Gln Thr Ser Glu Val Ser Thr Phe Pro Tyr Trp Gln Pro
   1 5 10 15
   Tyr Ala Leu Pro Leu
   20
<210> 864
   <211> 21
   <212> PRT
   <213> Artificial Sequence
<220>
   <223> IL-1 ANTAGONIST PEPTIDE
<400> 864
   Ser Trp Ser Glu Ala Phe Glu Gln Pro Arg Asn Leu Tyr Trp Gln Pro
   1 5 10 15
   Tyr Ala Leu Pro Leu
   20
<210> 865
   <211> 21
   <212> PRT
   <213> Artificial Sequence
<220>
   <223> IL-1 ANTAGONIST PEPTIDE
<400> 865
   Gln Tyr Ala Glu Pro Ser Ala Leu Asn Asp Trp Gly Tyr Trp Gln Pro
   1 5 10 15
   Tyr Ala Leu Pro Leu
   20
<210> 866
   <211 > 21
   <212> PRT
   <213> Artificial Sequence
<220>
   <223> IL-1 ANTAGONIST PEPTIDE
<400> 866
   Asn Gly Asp Trp Ala Thr Ala Asp Trp Ser Asn Tyr Tyr Trp Gln Pro
   1 5 10 15
   Tyr Ala Leu Pro Leu
   20
<210> 867
   <211> 15
   <212> PRT
   <213> Artificial Sequence
<220>
   <223> IL-1 ANTAGONIST PEPTIDE
<400> 867
   Thr His Asp Glu His Ile Tyr Trp Gln Pro Tyr Ala Leu Pro Leu
   1 5 10 15
<210> 868
   <211> 21
   <212> PRT
   <213> Artificial Sequence
<220>
   <223> IL-1 ANTAGONIST PEPTIDE
<400> 868
   Met Leu Glu Lys Thr Tyr Thr Thr Trp Thr Pro Gly Tyr Trp Gln Pro
   1 5 10 15
   Tyr Ala Leu Pro Leu
   20
<210> 869
   <211 20
   <212> PRT
   <213> Artificial Sequence
<220>
   <223> IL-1 ANTAGONIST PEPTIDE
<400> 869
<210> 870
   <211> 21
   <212> PRT
   <213> Artificial Sequence
<220>
   <223> IL-1 ANTAGONIST PEPTIDE
<400> 870
   Ser Asp Ala Phe Thr Thr Gln Asp Ser Gln Ala Met Tyr Trp Gln Pro
   1 5 10 15
   Tyr Ala Leu Pro Leu
   20
<210> 871
   <211> 21
   <212> PRT
   <213> Artificial Sequence
<220>
   <223> IL-1 ANTAGONIST PEPTIDE
<400> 871
   Gly Asp Asp Ala Ala Trp Arg Thr Asp Ser Leu Thr Tyr Trp Gln Pro
   1 5 10 15
   Tyr Ala Leu Pro Leu
   20
<210> 872
   <211> 21
   <212> PRT
   <213> Artificial Sequence
<220>
   <223> IL-1 ANTAGONIST PEPTIDE
<400> 872
   Ala Ile Ile Arg Gln Leu Tyr Arg Trp Ser Glu Met Tyr Trp Gln Pro
   1 5 10 15
   Tyr Ala Leu Pro Leu
   20
<210> 873
   <211> 21
   <212> PRT
   <213> Artificial Sequence
<220>
   <223> IL-1 ANTAGONIST PEPTIDE
<400> 873
   Glu Asn Thr Tyr Ser Pro Asn Trp Ala Asp Ser Met Tyr Trp Gln Pro
   1 5 10 15
   Tyr Ala Leu Pro Leu
   20
<210> 874
   <211> 21
   <212> PRT
   <213> Artificial Sequence
<220>
   <223> IL-1 ANTAGONIST PEPTIDE
<400> 874
   Met Asn Asp Gln Thr Ser Glu Val Ser Thr Phe Pro Tyr Trp Gln Pro
   1 5 10 15
   Tyr Ala Leu Pro Leu
   20
<210> 875
   <211> 21
   <212> PRT
   <213> Artificial Sequence
<220>
   <223> IL-1 ANTAGONIST PEPTIDE
<400> 875
   Ser Val Gly Glu Asp His Asn Phe Trp Thr Ser Glu Tyr Trp Gln Pro
   1 5 10 15
   Tyr Ala Leu Pro Leu
   20
<210> 876
   <211> 21
   <212> PRT
   <213> Artificial Sequence
<220>
   <223> IL-1 ANTAGONIST PEPTIDE
<400> 876
   Gln Thr Pro Phe Thr Trp Glu Glu Ser Asn Ala Tyr Tyr Trp Gln Pro
   1 5 10 15
   Tyr Ala Leu Pro Leu
   20
<210> 877
   <211> 21
   <212> PRT
   <213> Artificial Sequence
<220>
   <223> IL-1 ANTAGONIST PEPTIDE
<400> 877
   Glu Asn Pro Phe Thr Trp Gln Glu Ser Asn Ala Tyr Tyr Trp Gln Pro
   1 5 10 15
   Tyr Ala Leu Pro Leu
   20
<210> 878
   <211> 21
   <212> PRT
   <213> Artificial Sequence
<220>
   <223> IL-1 ANTAGONIST PEPTIDE
<400> 878
   Val Thr Pro Phe Thr Trp Glu Asp Ser Asn Val Phe Tyr Trp Gln Pro
   1 5 10 15
   Tyr Ala Leu Pro Leu
   20
<210> 879
   <211> 21
   <212> PRT
   <213> Artificial Sequence
<220>
   <223> IL-1 ANTAGONIST PEPTIDE
<400> 879
   Gln Ile Pro Phe Thr Trp Glu Gln Ser Asn Ala Tyr Tyr Trp Gln Pro
   1 5 10 15
   Tyr Ala Leu Pro Leu
   20
<210> 880
   <211> 21
   <212> PRT
   <213> Artificial Sequence
<220>
   <223> IL-1 ANTAGONIST PEPTIDE
<400> 880
   Gln Ala Pro Leu Thr Trp Gln Glu Ser Ala Ala Tyr Tyr Trp Gln Pro
   1 5 10 15
   Tyr Ala Leu Pro Leu
   20
<210> 881
   <211> 21
   <212> PRT
   <213> Artificial Sequence
<220>
   <223> IL-1 ANTAGONIST PEPTIDE
<400> 881
   Glu Pro Thr Phe Thr Trp Glu Glu Ser Lys Ala Thr Tyr Trp Gln Pro
   1 5 10 15
   Tyr Ala Leu Pro Leu
   20
<210> 882
   <211> 21
   <212> PRT
   <213> Artificial Sequence
<220>
   <223> IL-1 ANTAGONIST PEPTIDE
<400> 882
   Thr Thr Thr Leu Thr Trp Glu Glu Ser Asn Ala Tyr Tyr Trp Gln Pro
   1 5 10 15
   Tyr Ala Leu Pro Leu
   20
<210> 883
   <211> 21
   <212> PRT
   <213> Artificial Sequence
<220>
   <223> IL-1 ANTAGONIST PEPTIDE
<400> 883
   Glu Ser Pro Leu Thr Trp Glu Glu Ser Ser Ala Leu Tyr Trp Gln Pro
   1 5 10 15
   Tyr Ala Leu Pro Leu
   20
<210> 884
   <211> 21
   <212> PRT
   <213> Artificial Sequence
<220>
   <223> IL-1 ANTAGONIST PEPTIDE
<400> 884
   Glu Thr Pro Leu Thr Trp Glu Glu Ser Asn Ala Tyr Tyr Trp Gln Pro
   1 5 10 15
   Tyr Ala Leu Pro Leu
   20
<210> 885
   <211> 21
   <212> PRT
   <213> Artificial Sequence
<220>
   <223> IL-1 ANTAGONIST PEPTIDE
<400> 885
   Glu Ala Thr Phe Thr Trp Ala Glu Ser Asn Ala Tyr Tyr Trp Gln Pro
   1 5 10 15
   Tyr Ala Leu Pro Leu
   20
<210> 886
   <211> 21
   <212> PRT
   <213> Artificial Sequence
<220>
   <223> IL-1 ANTAGONIST PEPTIDE
<400> 886
   Glu Ala Leu Phe Thr Trp Lys Glu Ser Thr Ala Tyr Tyr Trp Gln Pro
   1 5 10 15
   Tyr Ala Leu Pro Leu
   20
<210> 887
   <211> 20
   <212> PRT
   <213> Artificial Sequence
<220>
   <223> IL-1 ANTAGONIST PEPTIDE
<400> 887
   Ser Thr Pro Thr Trp Glu Glu Ser Asn Ala Tyr Tyr Trp Gln Pro Tyr
   1 5 10 15
   Ala Leu Pro Leu
   20
<210> 888
   <211> 21
   <212> PRT
   <213> Artificial Sequence
<220>
   <223> IL-1 ANTAGONIST PEPTIDE
<400> 888
   Glu Thr Pro Phe Thr Trp Glu Glu Ser Asn Ala Tyr Tyr Trp Gln Pro
   1 5 10 15
   Tyr Ala Leu Pro Leu
   20
<210> 889
   <211> 21
   <212> PRT
   <213> Artificial Sequence
<220>
   <223> IL-1 ANTAGONIST PEPTIDE
<400> 889
   Lys Ala Pro Phe Thr Trp Glu Glu Ser Gln Ala Tyr Tyr Trp Gln Pro
   1 5 10 15
   Tyr Ala Leu Pro Leu
   20
<210> 890
   <211> 21
   <212> PRT
   <213> Artificial Sequence
<220>
   <223> IL-1 ANTAGONIST PEPTIDE
<400> 890
   Ser Thr Ser Phe Thr Trp Glu Glu Ser Asn Ala Tyr Tyr Trp Gln Pro
   1 5 10 15
   Tyr Ala Leu Pro Leu
   20
<210> 891
   <211> 21
   <212> PRT
   <213> Artificial Sequence
<220>
   <223> IL-1 ANTAGONIST PEPTIDE
<400> 891
   Asp Ser Thr Phe Thr Trp Glu Glu Ser Asn Ala Tyr Tyr Trp Gln Pro
   1 5 10 15
   Tyr Ala Leu Pro Leu
   20
<210> 892
   <211> 21
   <212> PRT
   <213> Artificial Sequence
<220>
   <223> IL-1 ANTAGONIST PEPTIDE
<400> 892
   Tyr Ile Pro Phe Thr Trp Glu Glu Ser Asn Ala Tyr Tyr Trp Gln Pro
   1 5 10 15
   Tyr Ala Leu Pro Leu
   20
<210> 893
   <211> 21
   <212> PRT
   <213> Artificial Sequence
<220>
   <223> IL-1 ANTAGONIST PEPTIDE
<400> 893
   Gln Thr Ala Phe Thr Trp Glu Glu Ser Asn Ala Tyr Tyr Trp Gln Pro
   1 5 10 15
   Tyr Ala Leu Pro Leu
   20
<210> 894
   <211> 21
   <212> PRT
   <213> Artificial Sequence
<220>
   <223> IL-1 ANTAGONIST PEPTIDE
<400> 894
   Glu Thr Leu Phe Thr Trp Glu Glu Ser Asn Ala Thr Tyr Trp Gln Pro
   1 5 10 15
   Tyr Ala Leu Pro Leu
   20
<210> 895
   <211> 21
   <212> PRT
   <213> Artificial Sequence
<220>
   <223> IL-1 ANTAGONIST PEPTIDE
<400> 895
   Val Ser Ser Phe Thr Trp Glu Glu Ser Asn Ala Tyr Tyr Trp Gln Pro
   1 5 10 15
   Tyr Ala Leu Pro Leu
   20
<210> 896
   <211> 7
   <212> PRT
   <213> Artificial Sequence
<220>
   <223> IL-1 ANTAGONIST PEPTIDE
<400> 896
   Gln Pro Tyr Ala Leu Pro Leu
   1 5
<210> 897
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa is a phosphotyrosyl residue
<220>
   <221> misc_feature
   <222> (2)..(2)
   <223> Position 2, Xaa is a 1-napthylalanyl residue
<220>
   <221> misc_feature
   <222> (6)..(6)
   <223> Position 6, Xaa is an azetidine residue
<400> 897
   Xaa Xaa Pro Tyr Gln Xaa Tyr Ala Leu Pro Leu
   1 5 10
<210> 898
   <211> 21
   <212> PRT
   <213> Artificial Sequence
<220>
   <223> IL-1 ANTAGONIST PEPTIDE
<400> 898
   Thr Ala Asn Val Ser Ser Phe Glu Trp Thr Pro Gly Tyr Trp Gln Pro
   1 5 10 15
   Tyr Ala Leu Pro Leu
   20
<210> 899
   <211> 15
   <212> PRT
   <213> Artificial Sequence
<220>
   <223> IL-1 ANTAGONIST PEPTIDE
<400> 899
   Phe Glu Trp Thr Pro Gly Tyr Trp Gln Pro Tyr Ala Leu Pro Leu
   1 5 10 15
<210> 900
   <211> 15
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 900
   Phe Glu Trp Thr Pro Gly Tyr Trp Gln Xaa Tyr Ala Leu Pro Leu
   1 5 10 15
<210> 901
   <211> 15
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 901
   Phe Glu Trp Thr Pro Gly Tyr Tyr Gln Xaa Tyr Ala Leu Pro Leu
   1 5 10 15
<210> 902
   <211> 21
   <212> PRT
   <213> Artificial Sequence
<220>
   <223> IL-1 ANTAGONIST PEPTIDE
<400> 902
   Glu Thr Pro Phe Thr Trp Glu Glu Ser Asn Ala Tyr Tyr Trp Gln Pro
   1               5                   10                  15
   Tyr Ala Leu Pro Leu
               20
<210> 903
   <211> 18
   <212> PRT
   <213> Artificial Sequence
<220>
   <223> IL-1 ANTAGONIST PEPTIDE
<220>
   <221> misc_feature
   <222> (13)..(13)
   <223> Position 13, Xaa is an azetidine residue
<400> 903
   Phe Thr Trp Glu Glu Ser Asn Ala Tyr Tyr Trp Gln Xaa Tyr Ala Leu
   1               5                   10                  15
   Pro Leu
<210> 904
   <211> 16
   <212> PRT
   <213> Artificial Sequence
<220>
   <223> IL-1 ANTAGONIST PEPTIDE
<400> 904
   Ala Asp Val Leu Tyr Trp Gln Pro Tyr Ala Pro Val Thr Leu Trp Val
   1               5                   10                  15

<210> 905
   <211> 17
   <212> PRT
   <213> Artificial Sequence
<220>
   <223> IL-1 ANTAGONIST
<400> 905
   Gly Asp Val Ala Glu Tyr Trp Gln Pro Tyr Ala Leu Pro Leu Thr Ser
   1 5 10 15
   Leu
<210> 906
   <211> 18
   <212> PRT
   <213> Artificial Sequence
<220>
   <223> IL-1 ANTAGONIST PEPTIDE
<400> 906
   Ser Trp Thr Asp Tyr Gly Tyr Trp Gln Pro Tyr Ala Leu Pro Ile Ser
   1 5 10 15
   Gly Leu
<210> 907
   <211> 8
   <212> PRT
   <213> Artificial Sequence
<220>
   <223> IL-1 ANTAGONIST PEPTIDE
<220>
   <221> misc_feature
   <222> (1)..(2)
   <223> Xaa is any amino acid
<220>
   <221> misc_feature
   <222> (4).. (4)
   <223> Xaa is prolyl or an azetidine residue
<220>
   <221> mise_feature
   <222> (6)..(6)
   <223> Position 6, Xaa is S, A, V or L
<220>
   <221> misc_feature
   <222> (7)..(8)
   <223> Xaa is any amino acid
<400> 907
   Xaa Xaa Gln Xaa Tyr Xaa Xaa Xaa
   1 5
<210> 908
   <211> 8
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa is Y, W or F
<220>
   <221> misc_feature
   <222> (2, 7)..(8)
   <223> Xaa is any amino acid
<220>
   <221> misc_feature
   <222> (4)..(4)
   <223> Position 4, Xaa is prolyl or an azetidine residue
<220>
   <221> misc_feature
   <222> (6)..(6)
   <223> Position 6, Xaa is S, A, V or L
<400> 908
   Xaa Xaa Gln Xaa Tyr Xaa Xaa Xaa
   1               5
<210> 909
   <211> 8
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa is Y, W or F
<220>
   <221> misc_feature
   <222> (2)..(2)
   <223> Position 2, Xaa is E, F, V, W or Y
<220>
   <221> misc_feature
   <222> (4)..(4)
   <223> Position 4, Xaa is prolyl or an azetidine residue
<220>
   <221> misc_feature
   <222> (6)..(6)
   <223> Position 6, Xaa is S, A, V or L
<220>
   <221> misc_feature
   <222> (7)..(7)
   <223> Position 7, Xaa is M, F, V, R, Q, K, T, S, D, L, I or E
<220>
   <221> misc_feature
   <222> (8)..(8)
   <223> Position 8, Xaa is E, L, W, V, H, I, G, A, D, L, Y, N, Q or P
<400> 909
   Xaa Xaa Gln Xaa Tyr Xaa Xaa Xaa
   1               5
<210> 910
   <211> 9
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa is V, L, I, E, P, G, Y, M, T or D
<220>
   <221> misc_feature
   <222> (2)..(2)
   <223> Position 2, Xaa is Y, W or F
<220>
   <221> misc_feature
   <222> (3)..(3)
   <223> Position 3, Xaa is E, F, V, W or Y
<220>
   <221> misc_feature
   <222> (5)..(5)
   <223> Position 5, Xaa is prolyl or an azetidine residue;
<220>
   <221> mise_feature
   <222> (7)..(7)
   <223> Position 7, Xaa is S, A, V or L
<220>
   <221> misc_feature
   <222> (8)..(8)
   <223> Position 8, Xaa is M, F, V, R, Q, K, T, S, D, L, I or E;
<220>
   <221> misc_feature
   <222> (9)..(9)
   <223> Position 9, Xaa is E, L, W, V, H, I, G, A, D, L, Y, N, Q or P
<400> 910
   Xaa Xaa Xaa Gln Xaa Tyr Xaa Xaa Xaa
   1 5
<210> 911
   <211> 15
   <212> PRT
   <213> Artificial Sequence
<220>
   <223> IL-1 ANTAGONIST PEPTIDE
<400> 911
   Phe Glu Trp Thr Pro Gly Tyr Trp Gln Pro Tyr Ala Leu Pro Leu
   1 5 10 15
<210> 912
   <211> 15
   <212> PRT
   <213> Artificial Sequence
<220>
   <223> IL-1 ANTAGONIST PEPTIDE
<220>
   <221> misc_feature
   <222> (10)..(10)
   <223> Xaa = any amino acid
<400> 912
   Phe Glu Trp Thr Pro Gly Tyr Trp Gln Xaa Tyr Ala Leu Pro Leu
   1 5 10 15
<210> 913
   <211> 15
   <212> PRT
   <213> Artificial Sequence
<220>
   <223> IL-1 ANTAGONIST PEPTIDE
<400> 913
   Phe Glu Trp Thr Pro Gly Trp Tyr Gln Pro Tyr Ala Leu Pro Leu
   1 5 10 15
<210> 914
   <211> 15
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 914
   Phe Glu Trp Thr Pro Gly Trp Tyr Gln Xaa Tyr Ala Leu Pro Leu
   1 5 10 15
<210> 915
   <211> 15
   <212> PRT
   <213> Artificial Sequence
<220>
   <223> IL-1 ANTAGONIST PEPTIDE
<400> 915
   Phe Glu Trp Thr Pro Gly Tyr Tyr Gln Pro Tyr Ala Leu Pro Leu
   1 5 10 15
<210> 916
   <211> 15
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 916
   Phe Glu Trp Thr Pro Gly Tyr Tyr Gln Xaa Tyr Ala Leu Pro Leu
   1 5 10 15
<210> 917
   <211> 21
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa is A, D, E, F, G, K, Q, S, T, V or Y
<220>
   <221> misc_feature
   <222> (2).. (2)
   <223> Position 2, Xaa is A, D, G, I, N, P, S, T, V or W
<220>
   <221> misc_feature
   <222> (3)..(3)
   <223> Position 3, Xaa is A, D, G, L, N, P, S, T, W or Y
<220>
   <221> misc_feature
   <222> (4)..(4)
   <223> Position 4, Xaa is A, D, E, F, L, N, R, V or Y
<220>
   <221> misc_feature
   <222> (5).. (5)
   <223> Position 5, Xaa is A, D, E, Q, R, S or T
<220>
   <221> misc_feature
   <222> (6)..(6)
   <223> Position 6, Xaa is H, I, L, P, S, T or W
<220>
   <221> misc_feature
   <222> (7)..(7)
   <223> Position 7, Xaa is A, E, F, K, N, Q, R, S or Y;
<220>
   <221> misc_feature
   <222> (8)..(8)
   <223> Position 8, Xaa is D, E, F, Q, R, T or W
<220>
   <221> mise_feature
   <222> (9)..(9)
   <223> Position 9, Xaa is A, D, P, S, T or W
<220>
   <221> misc_feature
   <222> (10)..(10)
   <223> Position 10, Xaa is A, D, G, K, N, Q, S or T
<220>
   <221> misc_feature
   <222> (11)..(11)
   <223> Position 11, Xaa is A, E, L, P, S, T, V or Y
<220>
   <221> misc_feature
   <222> (12)..(12)
   <223> Position 12, Xaa is V, L, I, E, P, G, Y, M, T or D
<220>
   <221> mise_feature
   <222> (13)..(13)
   <223> Position 13, Xaa is Y, W or F
<220>
   <221> misc_feature
   <222> (14)..(14)
   <223> Position 14, Xaa is E, F, V, W or Y
<220>
   <221> misc_feature
   <222> (16)..(16)
   <223> Position 16, Xaa is P or an azetidine residue;
<220>
   <221> misc_feature
   <222> (18)..(18)
   <223> Position 18, Xaa is S, A, V or L
<220>
   <221> misc_feature
   <222> (19)..(19)
   <223> Position 19, Xaa is M, F, V, R, Q, K, T, S, D, L, I or E
<220>
   <221> misc_feature
   <222> (20)..(20)
   <223> Position 20, Xaa is Q or P.
<400> 917
   Xaa Xaa Xaa Xaa Xaa Xaa Xaa Xaa Xaa Xaa Xaa Xaa Xaa Xaa Gln Xaa
   1               5                   10                  15
   Tyr Xaa Xaa Xaa Leu
               20
<210> 918
   <211> 21
   <212> PRT
   <213> Artificial Sequence
<220>
   <223> IL-1 ANTAGONIST PEPTIDE
<400> 918
   Thr Ala Asn Val Ser Ser Phe Glu Trp Thr Pro Gly Tyr Trp Gln Pro
   1               5                   10                  15
   Tyr Ala Leu Pro Leu
               20
<210> 919
   <211> 18
   <212> PRT
   <213> Artificial Sequence
<220>
   <223> IL-1 ANTAGONIST PEPTIDE
<400> 919
   Ser Trp Thr Asp Tyr Gly Tyr Trp Gln Pro Tyr Ala Leu Pro Ile Ser
   1 5 10 15
   Gly Leu
<210> 920
   <211> 21
   <212> PRT
   <213> Artificial Sequence
<220>
   <223> IL-1 ANTAGONIST PEPTIDE
<400> 920
   Glu Thr Pro Phe Thr Trp Glu Glu Ser Asn Ala Tyr Tyr Trp Gln Pro
   1 5 10 15
   Tyr Ala Leu Pro Leu
   20
<210> 921
   <211> 21
   <212> PRT
   <213> Artificial Sequence
<220>
   <223> IL-1 ANTAGONIST PEPTIDE
<400> 921
   Glu Asn Thr Tyr Ser Pro Asn Trp Ala Asp Ser Met Tyr Trp Gln Pro
   1 5 10 15
   Tyr Ala Leu Pro Leu
   20
<210> 922
   <211> 21
   <212> PRT
   <213> Artificial Sequence
<220>
   <223> IL-1 ANTAGONIST PEPTIDE
<400> 922
   Ser Val Gly Glu Asp His Asn Phe Trp Thr Ser Glu Tyr Trp Gln Pro
   1 5 10 15
   Tyr Ala Leu Pro Leu
   20
<210> 923
   <211> 21
   <212> PRT
   <213> Artificial Sequence
<220>
   <223> IL-1 ANTAGONIST PEPTIDE
<400> 923
   Asp Gly Tyr Asp Arg Trp Arg Gln Ser Gly Glu Arg Tyr Trp Gln Pro
   1 5 10 15
   Tyr Ala Leu Pro Leu
   20
<210> 924
   <211> 15
   <212> PRT
   <213> Artificial Sequence
<220>
   <223> IL-1 ANTAGONIST PEPTIDE
<400> 924
   Phe Glu Trp Thr Pro Gly Tyr Trp Gln Pro Tyr Ala Leu Pro Leu
   1 5 10 15
<210> 925
   <211> 11
   <212> PRT
   <213> Artificial Sequence
<220>
   <223> IL-1 ANTAGONIST PEPTIDE
<400> 925
   Phe Glu Trp Thr Pro Gly Tyr Trp Gln Pro Tyr
   1 5 10
<210> 926
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 926
   Phe Glu Trp Thr Pro Gly Tyr Trp Gln Xaa Tyr
   1 5 10
<210> 927
   <211> 10
   <212> PRT
   <213> Artificial Sequence
<220>
   <223> IL-1 ANTAGONIST PEPTIDE
<400> 927
   Glu Trp Thr Pro Gly Tyr Trp Gln Pro Tyr
   1 5 10
<210> 928
   <211> 11
   <212> PRT
   <213> Artificial Sequence
<220>
   <223> IL-1 ANTAGONIST PEPTIDE
<220>
   <221> mise_feature
   <222> (10)..(10)
   <223> Position 10, Xaa is an azetidine residue
<400> 928
   Phe Glu Trp Thr Pro Gly Trp Tyr Gln Xaa Tyr
   1 5 10
<210> 929
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 929
   Ala Glu Trp Thr Pro Gly Tyr Trp Gln Xaa Tyr
   1 5 10
<210> 930
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 930
   Phe Ala Trp Thr Pro Gly Tyr Trp Gln Xaa Tyr
   1 5 10
<210> 931
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 931
   Phe Glu Ala Thr Pro Gly Tyr Trp Gln Xaa Tyr
   1 5 10
<210> 932
   <211> 11
   <212> PRT
   <213> Artificial Sequence
<220>
   <223> IL-1 ANTAGONIST PEPTIDE
<220>
   <221> mise_feature
   <222> (10)..(10)
   <223> Position 10, Xaa is an azetidine residue
<400> 932
   Phe Glu Trp Ala Pro Gly Tyr Trp Gln Xaa Tyr
   1 5 10
<210> 933
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 933
   Phe Glu Trp Thr Ala Gly Tyr Trp Gln Xaa Tyr
   1 5 10
<210> 934
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 934
   Phe Glu Trp Thr Pro Ala Tyr Trp Gln Xaa Tyr
   1 5 10
<210> 935
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 935
   Phe Glu Trp Thr Pro Gly Ala Trp Gln Xaa Tyr
   1 5 10
<210> 936
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 936
   Phe Glu Trp Thr Pro Gly Tyr Ala Gln Xaa Tyr
   1 5 10
<210> 937
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 937
   Phe Glu Trp Thr Pro Gly Tyr Trp Gln Xaa Ala
   1 5 10
<210> 938
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 938
   Phe Glu Trp Thr Gly Gly Tyr Trp Gln Xaa Tyr
   1 5 10
<210> 939
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 5, D amino acid residue Position 10, Xaa is an azetidine residue
<400> 939
   Phe Glu Trp Thr Pro Gly Tyr Trp Gln Xaa Tyr
   1 5 10
<210> 940
   <211> 11
   <212> PRT
   <213> Artificial Sequence
<220>
   <223> IL-1 ANTAGONIST PEPTIDE
<220>
   <221> misc_feature
   <222> (5)..(10)
   <223> Position 10, Xaa is an azetidine residue
<400> 940
   Phe Glu Trp Thr Xaa Gly Tyr Trp Gln Xaa Tyr
   1 5 10
<210> 941
   <211> 11
   <212> PRT
   <213> Artificial Sequence
<220>
   <223> IL-1 ANTAGONIST PEPTIDE
<220>
   <221> misc_feature
   <222> (5)..(5)
   <223> Position 5, Xaa is a pipecolic acid residue
<220>
   <221> misc_feature
   <222> (10)..(10)
   <223> Position 10, Xaa is an azetidine residue
<400> 941
   Phe Glu Trp Thr Xaa Gly Tyr Trp Gln Xaa Tyr
   1 5 10
<210> 942
   <211> 11
   <212> PRT
   <213> Artificial Sequence
<220>
   <223> IL-1 ANTAGONIST PEPTIDE
<220>
   <221> misc_feature
   <222> (6)..(6)
   <223> Position 6, Xaa is an aminoisobutyric acid residue
<220>
   <221> misc_feature
   <222> (10)..(10)
   <223> Position 10, Xaa is an azetidine residue
<400> 942
   Phe Glu Trp Thr Pro Xaa Tyr Trp Gln Xaa Tyr
   1 5 10
<210> 943
   <211> 11
   <212> PRT
   <213> Artificial Sequence
<220>
   <223> IL-1 ANTAGONIST PEPTIDE
<220>
   <221> misc_feature
   <222> (6)..(6)
   <223> Position 6, Xaa is a sarcosine residue
<220>
   <221> misc_feature
   <222> (10)..(10)
   <223> Position 10, Xaa is an azetidine residue
<400> 943
   Phe Glu Trp Thr Pro Xaa Trp Tyr Gln Xaa Tyr
   1 5 10
<210> 944
   <211> 11
   <212> PRT
   <213> Artificial Sequence
<220>
   <223> IL-1 ANTAGONIST PEPTIDE
<220>
   <221> misc_feature
   <222> (5)..(5)
   <223> Position 5, Xaa is a sarcosine residue
<220>
   <221> misc_feature
   <222> (10)..(10)
   <223> Position 10, Xaa is an azetidine residue
<400> 944
   Phe Glu Trp Thr Xaa Gly Tyr Trp Gln Xaa Tyr
   1 5 10
<210> 945
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 945
   Phe Glu Trp Thr Pro Asn Tyr Trp Gln Xaa Tyr
   1 5 10
<210> 946
   <211> 11
   <212> PRT
   <213> Artificial Sequence
<220>
   <223> IL-1 ANTAGONIST PEPTIDE
<220>
   <221> misc_feature
   <222> (5)..(5)
   <223> Position 5, D amino acid residue
<220>
   <221> misc_feature
   <222> (10)..(10)
   <223> Position 10, Xaa is an azetidine residue
<400> 946
   Phe Glu Trp Thr Pro Val Tyr Trp Gln Xaa Tyr
   1 5 10
<210> 947
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 947
   Phe Glu Trp Thr Val Pro Tyr Trp Gln Xaa Tyr
   1 5 10
<210> 948
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, acetylated Phe
<220>
   <221> misc_feature
   <222> (10)..(10)
   <223> Position 10, Xaa is an azetidine residue
<400> 948
   Phe Glu Trp Thr Pro Gly Trp Tyr Gln Xaa Tyr
   1               5                   10
<210> 949
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, acetylated Phe
<220>
   <221> mise_feature
   <222> (10)..(10)
   <223> Position 10, Xaa is an azetidine residue
<400> 949
   Phe Glu Trp Thr Pro Gly Tyr Trp Gln Xaa Tyr
   1               5                   10
<210> 950
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa = 1-naphthylalanine
<220>
   <221> misc_feature
   <222> (10)..(10)
   <223> Position 10, Xaa is an azetidine residue
<400> 950
   Xaa Glu Trp Thr Pro Gly Tyr Tyr Gln Xaa Tyr
   1 5 10
<210> 951
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, xaa is an azetidine residue
<400> 951
   Tyr Glu Trp Thr Pro Gly Tyr Tyr Gln Xaa Tyr
   1 5 10
<210> 952
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 952
   Phe Glu Trp Val Pro Gly Tyr Tyr Gln Xaa Tyr
   1 5 10
<210> 953
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 953
   Phe Glu Trp Thr Pro Gly Tyr Tyr Gln Xaa Tyr
   1 5 10
<210> 954
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<400> 954
   Phe Glu Trp Thr Pro Ser Tyr Tyr Gln Xaa Tyr
   1 5 10

<210> 955
   <211> 11
   <212> PRT
   <213> Artificial Sequence
<220>
   <223> IL-1 ANTAGONIST PEPTIDE
<220>
   <221 > misc_feature
   <222> (10)..(10)
   <223> Position 10, Xaa is an azetidine residue
<400> 955
   Phe Glu Trp Thr Pro Asn Tyr Tyr Gln Xaa Tyr
   1 5 10
<210> 956
   <211> 12
   <212> PRT
   <213> Artificial Sequence
<220>
   <223> IL-1 ANTAGONIST PEPTIDE
<220>
   <221> misc_feature
   <222> (5)..(5)
   <223> Position 5, Xaa = naphthylalanine
<400> 956
   Ser His Leu Tyr Xaa Gln Pro Tyr Ser Val Gln Met
   1 5 10
<210> 957
   <211> 12
   <212> PRT
   <213> Artificial Sequence
<220>
   <223> IL-1 ANTAGONIST PEPTIDE
<220>
   <221> mise_feature
   <222> (5)..(5)
   <223> Position 5, Xaa = naphthylalanine
<400> 957
   Thr Leu Val Tyr Xaa Gln Pro Tyr Ser Leu Gln Thr
   1 5 10
<210> 958
   <211 > 12
   <212> PRT
   <213> Artificial Sequence
<220>
   <223> IL-1 ANTAGONIST PEPTIDE
<220>
   <221> misc_feature
   <222> (5)..(5)
   <223> Position 5, Xaa = naphthylalanine
<400> 958
   Arg Gly Asp Tyr Xaa Gln Pro Tyr Ser Val Gln Ser
   1 5 10
<210> 959
   <211> 12
   <212> PRT
   <213> Artificial Sequence
<220>
   <223> IL-1 ANTAGONIST PEPTIDE
<220>
   <221> misc_feature
   <222> (5).. (5)
   <223> Position 5, Xaa = naphthylalanine
<400> 959
   Asn Met Val Tyr Xaa Gln Pro Tyr Ser Ile Gln Thr
   1 5 10
<210> 960
   <211> 9
   <212> PRT
   <213> Artificial Sequence
<220>
   <223> IL-1 ANTAGONIST PEPTIDE
<400> 960
   Val Tyr Trp Gln Pro Tyr Ser Val Gln
   1 5
<210> 961
   <211> 9
   <212> PRT
   <213> Artificial Sequence
<220>
   <223> IL-1 ANTAGONIST PEPTIDE
<220>
   <221> misc_feature
   <222> (3)..(3)
   <223> Position 3, Xaa = naphthylalanine
<400> 961
   Val Tyr Xaa Gln Pro Tyr Ser Val Gln
   1 5
<210> 962
   <211> 12
   <212> PRT
   <213> Artificial Sequence
<220>
   <223> IL-1 ANTAGONIST PEPTIDE
<220>
   <221> misc_feature
   <222> (7)..(7)
   <223> Position 7, Xaa is an azetidine residue
<400> 962
   Thr Phe Val Tyr Trp Gln Xaa Tyr Ala Leu Pro Leu
   1 5 10
<210> 963
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa is an azetidine residue
<220>
   <221> misc_feature
   <222> (11)..(11)
   <223> Position 11, Xaa = p-benzoyl-L-phenylalanine
<400> 963
   Phe Glu Trp Thr Pro Gly Tyr Tyr Gln Xaa Xaa
   1               5                   10
<210> 964
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa = acetylated Phe
<220>
   <221 > misc_feature
   <222> (10)..(10)
   <223> Position 10, Xaa is an azetidine residue
<220>
   <221> misc_feature
   <222> (11)..(11)
   <223> Position 11, Xaa = p-benzoyl-L-phenylalanine.
<400> 964
   Xaa Glu Trp Thr Pro Gly Tyr Tyr Gln Xaa Xaa
   1               5                   10
<210> 965
   <211> 11
   <212> PRT
   <213> Artificial Sequence
<220>
   <223> IL-1 ANTAGONIST PEPTIDE
<220>
   <221> misc_feature
   <222> (8)..(10)
   <223> Position 8, Xaa = p-benzoyl-L-phenylalanine Position 10, Xaa is an azetidine residue
<220>
   <221> misc_feature
   <222> (10)..(10)
   <223> Position 10, Xaa is an azetidine residue
<400> 965
   Phe Glu Trp Thr Pro Gly Tyr Xaa Gln Xaa Tyr
   1               5                   10
<210> 966
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (8).. (8)
   <223> Position 8, Xaa = p-benzoyl-L-phenylalanine
<220>
   <221 > misc_feature
   <222> (10)..(10)
   <223> Position 10, Xaa is an azetidine residue.
<400> 966
   Phe Glu Trp Thr Pro Gly Tyr Xaa Gln Xaa Tyr
   1               5                   10
<210> 967
   <211> 11
   <212> PRT
   <213> Artificial Sequence
<220>
   <223> IL-1 ANTAGONIST PEPTIDE
<220>
   <221> misc_feature
   <222> (7)..(7)
   <223> Position 7, Xaa = p-benzoyl-L-phenylalanine
<220>
   <221> misc_feature
   <222> (10)..(10)
   <223> Position 10, Xaa is an azetidine residue.
<400> 967
   Phe Glu Trp Thr Pro Gly Xaa Tyr Gln Xaa Tyr
   1               5                   10
<210> 968
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (7)..(7)
   <223> Position 7, Xaa = p-benzoyl-L-phenylalanine
<220>
   <221> misc_feature
   <222> (10)..(10)
   <223> Position 10, Xaa is an azetidine residue.
<400> 968
   Phe Glu Trp Thr Pro Gly Xaa Tyr Gln Xaa Tyr
   1               5                   10
<210> 969
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (3)..(3)
   <223> Position 3, Xaa = p-benzoyl-L-phenylalanine
<220>
   <221> misc_feature
   <222> (10)..(10)
   <223> Position 10, Xaa is an azetidine residue.
<400> 969
   Phe Glu Xaa Thr Pro Gly Tyr Tyr Gln Xaa Tyr
   1               5                   10
<210> 970
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (3)..(3)
   <223> Position 3, Xaa = p-benzoyl-L-phenylalanine
<220>
   <221> misc_feature
   <222> (10)..(10)
   <223> Position 10, Xaa is an azetidine residue.
<400> 970
   Phe Glu Xaa Thr Pro Gly Tyr Tyr Gln Xaa Tyr
   1               5                   10
<210> 971
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa = p-benzoyl-L-phenylalanine
<220>
   <221> misc_feature
   <222> (10)..(10)
   <223> Position 10, Xaa is an azetidine residue.
<400> 971
   Xaa Glu Trp Thr Pro Gly Tyr Tyr Gln Xaa Tyr
   1               5                   10
<210> 972
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa = acetylated p-benzoyl-L-phenylalanine
<220>
   <221> MOD_RES
   <222> (1)..(1),
   <223> ACETYLATION
<220>
   <221> misc_feature
   <222> (10)..(10)
   <223> Position 10, Xaa is an azetidine residue.
<400> 972
   Xaa Glu Trp Thr Pro Gly Tyr Tyr Gln Xaa Tyr
   1 5 10
<210> 973
   <211> 9
   <212> PRT
   <213> Artificial Sequence
<220>
   <223> IL-1 ANTAGONIST PEPTIDE
<400> 973
   Val Tyr Trp Gln Pro Tyr Ser Val Gln
   1 5
<210> 974
   <211> 12
   <212> PRT
   <213> Artificial Sequence
<220>
   <223> IL-1 ANTAGONIST PEPTIDE
<400> 974
   Arg Leu Val Tyr Trp Gln Pro Tyr Ser Val Gln Arg
   1 5 10
<210> 975
   <211> 12
   <212> PRT
   <213> Artificial Sequence
<220>
   <223> IL-1 ANTAGONIST PEPTIDE
<220>
   <221> misc_feature
   <222> (5)..(5)
   <223> Position 5, Xaa = naphthylalanine
<400> 975
   Arg Leu Val Tyr Xaa Gln Pro Tyr Ser Val Gln Arg
   1 5 10
<210> 976
   <211> 12
   <212> PRT
   <213> Artificial Sequence
<220>
   <223> IL-1 ANTAGONIST PEPTIDE
<400> 976
   Arg Leu Asp Tyr Trp Gln Pro Tyr Ser Val Gln Arg
   1 5 10
<210> 977
   <211> 12
   <212> PRT
   <213> Artificial Sequence
<220>
   <223> IL-1 ANTAGONIST PEPTIDE
<400> 977
   Arg Leu Val Trp Phe Gln Pro Tyr Ser Val Gln Arg
   1 5 10
<210> 978
   <211> 12
   <212> PRT
   <213> Artificial Sequence
<220>
   <223> IL-1 ANTAGONIST PEPTIDE
<400> 978
   Arg Leu Val Tyr Trp Gln Pro Tyr Ser Ile Gln Arg
   1 5 10
<210> 979
   <211> 11
   <212> PRT
   <213> Artificial Sequence
<220>
   <223> IL-1 ANTAGONIST PEPTIDE
<220>
   <221> misc_feature
   <222> (1)..(1)
   <223> Position 1, Xaa = D or Y
<220>
   <221> misc_feature
   <222> (3)..(3)
   <223> Position 3, Xaa = D or S
<220>
   <221> misc_feature
   <222> (4)..(4)
   <223> Position 4, Xaa = S, T or A
<220>
   <221> misc_feature
   <222> (5)..(5)
   <223> Position 5, Xaa = S or W
<220>
   <221> misc_feature
   <222> (6)..(6)
   <223> Position 6, Xaa = S or Y
<220>
   <221> misc_feature
   <222> (7).. (7)
   <223> Xaa is any amino acid
<220>
   <221> misc_feature
   <222> (8)..(8)
   <223> Position 8, Xaa = N, S, K, H or W
<220>
   <221> misc_feature
   <222> (9)..(9)
   <223> Position 9, Xaa = F or L
<220>
   <221> misc_feature
   <222> (10)..(10)
   <223> Position 10, Xaa = D, N, S or L
<220>
   <221> misc_feature
   <222> (11)..(11)
   <223> Position 11, Xaa = L, I, O, M or A.
<400> 979
   Xaa Asn Xaa Xaa Xaa Xaa Xaa Xaa Xaa Xaa Xaa
   1               5                   10
<210> 980
   <211> 11
   <212> PRT
   <213> Artificial Sequence
<220>
   <223> IL-1 ANTAGONIST PEPTIDE
<400> 980
   Asp Asn Ser Ser Trp Tyr Asp Ser Phe Leu Leu
   1 5 10
<210> 981
   <211> 11
   <212> PRT
   <213> Artificial Sequence
<220>
   <223> IL-1 ANTAGONIST PEPTIDE
<400> 981
   Asp Asn Thr Ala Trp Tyr Glu Ser Phe Leu Ala
   1 5 10
<210> 982
   <211> 11
   <212> PRT
   <213> Artificial Sequence
<220>
   <223> IL-1 ANTAGONIST PEPTIDE
<400> 982
   Asp Asn Thr Ala Trp Tyr Glu Asn Phe Leu Leu
   1 5 10
<210> 983
   <211> 17
   <212> PRT
   <213> Artificial Sequence
<220>
   <223> IL-1 ANTAGONIST PEPTIDE
<400> 983
   Pro Ala Arg Glu Asp Asn Thr Ala Trp Tyr Asp Ser Phe Leu Ile Trp
   1 5 10 15
   Cys
<210> 984
   <211> 17
   <212> PRT
   <213> Artificial Sequence
<220>
   <223> IL-1 ANTAGONIST PEPTIDE
<400> 984
   Thr Ser Glu Tyr Asp Asn Thr Thr Trp Tyr Glu Lys Phe Leu Ala Ser
   1 5 10 15
   Gln
<210> 985
   <211> 17
   <212> PRT
   <213> Artificial Sequence
<220>
   <223> IL-1 ANTAGONIST PEPTIDE
<400> 985
   Ser Gln Ile Pro Asp Asn Thr Ala Trp Tyr Gln Ser Phe Leu Leu His
   1 5 10 15
   Gly
<210> 986
   <211> 17
   <212> PRT
   <213> Artificial Sequence
<220>
   <223> IL-1 ANTAGONIST PEPTIDE
<400> 986
   Ser Pro Phe Ile Asp Asn Thr Ala Trp Tyr Glu Asn Phe Leu Leu Thr
   1 5 10 15
   Tyr
<210> 987
   <211> 17
   <212> PRT
   <213> Artificial Sequence
<220>
   <223> IL-1 ANTAGONIST PEPTIDE
<400> 987
   Glu Gln Ile Tyr Asp Asn Thr Ala Trp Tyr Asp His Phe Leu Leu Ser
   1 5 10 15
   Tyr
<210> 988
   <211> 17
   <212> PRT
   <213> Artificial Sequence
<220>
   <223> IL-1 ANTAGONIST PEPTIDE
<400> 988
   Thr Pro Phe Ile Asp Asn Thr Ala Trp Tyr Glu Asn Phe Leu Leu Thr
   1               5                   10                  15
   Tyr
<210> 989
   <211> 17
   <212> PRT
   <213> Artificial Sequence
<220>
   <223> IL-1 ANTAGONIST PEPTIDE
<400> 989
   Thr Tyr Thr Tyr Asp Asn Thr Ala Trp Tyr Glu Arg Phe Leu Met Ser
   1               5                   10                  15
   Tyr
<210> 990
   <211> 17
   <212> PRT
   <213> Artificial Sequence
<220>
   <223> IL-1 ANTAGONIST PEPTIDE
<400> 990
   Thr Met Thr Gln Asp Asn Thr Ala Trp Tyr Glu Asn Phe Leu Leu Ser
   1               5                   10                  15
   Tyr
<210> 991
   <211> 17
   <212> PRT
   <213> Artificial Sequence
<220>
   <223> IL-1 ANTAGONIST PEPTIDE
<400> 991
   Thr Ile Asp Asn Thr Ala Trp Tyr Ala Asn Leu Val Gln Thr Tyr Pro
   1 5 10 15
   Gln
<210> 992
   <211> 17
   <212> PRT
   <213> Artificial Sequence
<220>
   <223> IL-1 ANTAGONIST PEPTIDE
<400> 992
   Thr Ile Asp Asn Thr Ala Trp Tyr Glu Arg Phe Leu Ala Gln Tyr Pro
   1 5 10 15
   Asp
<210> 993
   <211> 17
   <212> PRT
   <213> Artificial Sequence
<220>
   <223> IL-1 ANTAGONIST PEPTIDE
<400> 993
   His Ile Asp Asn Thr Ala Trp Tyr Glu Asn Phe Leu Leu Thr Tyr Thr
   1 5 10 15
   Pro
<210> 994
   <211> 17
   <212> PRT
   <213> Artificial Sequence
<220>
   <223> IL-1 ANTAGONIST PEPTIDE
<400> 994
   Ser Gln Asp Asn Thr Ala Trp Tyr Glu Asn Phe Leu Leu Ser Tyr Lys
   1 5 10 15
   Ala
<210> 995
   <211> 17
   <212> PRT
   <213> Artificial Sequence
<220>
   <223> IL-1 ANTAGONIST PEPTIDE
<400> 995
   Gln Ile Asp Asn Thr Ala Trp Tyr Glu Arg Phe Leu Leu Gln Tyr Asn
   1 5 10 15
   Ala
<210> 996
   <211> 17
   <212> PRT
   <213> Artificial Sequence
<220>
   <223> IL-1 ANTAGONIST PEPTIDE
<400> 996
   Asn Gln Asp Asn Thr Ala Trp Tyr Glu Ser Phe Leu Leu Gln Tyr Asn
   1 5 10 15
   Thr
<210> 997
   <211> 17
   <212> PRT
   <213> Artificial Sequence
<220>
   <223> IL-1 ANTAGONIST PEPTIDE
<400> 997
   Thr Ile Asp Asn Thr Ala Trp Tyr Glu Asn Phe Leu Leu Asn His Asn
   1 5 10 15
   Leu
<210> 998
   <211> 17
   <212> PRT
   <213> Artificial Sequence
<220>
   <223> IL-1 ANTAGONIST PEPTIDE
<400> 998
   His Tyr Asp Asn Thr Ala Trp Tyr Glu Arg Phe Leu Gln Gln Gly Trp
   1 5 10 15
   His
<210> 999
   <211 > 21
   <212> PRT
   <213> Artificial Sequence
<220>
   <223> IL-1 ANTAGONIST PEPTIDE
<400> 999
   Glu Thr Pro Phe Thr Trp Glu Glu Ser Asn Ala Tyr Tyr Trp Gln Pro
   1 5 10 15
   Tyr Ala Leu Pro Leu
   20
<210> 1000
   <211> 21
   <212> PRT
   <213> Artificial Sequence
<220>
   <223> IL-1 ANTAGONIST PEPTIDE
<400> 1000
   Tyr Ile Pro Phe Thr Trp Glu Glu Ser Asn Ala Tyr Tyr Trp Gln Pro
   1 5 10 15
   Tyr Ala Leu Pro Leu
   20
<210> 1001
   <211> 21
   <212> PRT
   <213> Artificial Sequence
<220>
   <223> IL-1 ANTAGONIST PEPTIDE
<400> 1001
   Asp Gly Tyr Asp Arg Trp Arg Gln Ser Gly Glu Arg Tyr Trp Gln Pro
   1 5 10 15
   Tyr Ala Leu Pro Leu
   20
<210> 1002
   <211> 11
   <212> PRT
   <213> Artificial Sequence
<220>
   <223> IL-1 ANTAGONIST PEPTIDE
<220>
   <221 > misc_feature
   <222> (1)..(1)
   <223> Position 1, Xaa = phosphotyrosine
<220>
   <221> misc_feature
   <222> (2)..(2)
   <223> Position 2, Xaa = naphthylalanine
<220>
   <221> misc_feature
   <222> (3)..(3)
   <223> Position 3, Xaa = phosphotyrosine
<220>
   <221> misc_feature
   <222> (5) . (5)
   <223> Position 5, Xaa is an azetidine residue.
<400> 1002
   Xaa Xaa Xaa Gln Gln Xaa Tyr Ala Leu Pro Leu
   1               5                   10
<210> 1003
   <211> 21
   <212> PRT
   <213> Artificial Sequence
<220>
   <223> IL-1 ANTAGONIST PEPTIDE
<400> 1003
   Thr Ala Asn Val Ser Ser Phe Glu Trp Thr Pro Gly Tyr Trp Gln Pro
   1               5                   10                  15
   Tyr Ala Leu Pro Leu
               20
<210> 1004
   <211> 15
   <212> PRT
   <213> Artificial Sequence
<220>
   <223> IL-1 ANTAGONIST PEPTIDE
<220>
   <221 > misc_feature
   <222> (10)..(10)
   <223> Position 10, Xaa = azetidine
<400> 1004
   Phe Glu Trp Thr Pro Gly Tyr Trp Gln Xaa Tyr Ala Leu Pro Leu
   1 5 10 15
<210> 1005
   <211> 17
   <212> PRT
   <213> Artificial Sequence
<220>
   <223> IL-1 ANTAGONIST PEPTIDE
<400> 1005
   Phe Glu Trp Thr Pro Gly Tyr Trp Gln Pro Tyr Ala Leu Pro Leu Ser
   1 5 10 15
   Asp
<210> 1006
   <211> 15
   <212> PRT
   <213> Artificial Sequence
<220>
   <223> IL-1 ANTAGONIST PEPTIDE
<220>
   <221 > misc_feature
   <222> (10)..(10)
   <223> Position 10, Xaa = azetidine
<400> 1006
   Phe Glu Trp Thr Pro Gly Tyr Tyr Gln Xaa Tyr Ala Leu Pro Leu
   1 5 10 15
<210> 1007
   <211> 11
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 1007
   Phe Glu Trp Thr Pro Gly Tyr Trp Gln Xaa Tyr
   1 5 10
<210> 1008
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (10)..(10)
   <223> Position 10, Xaa = azetidine
<400> 1008
   Phe Glu Trp Thr Pro Gly Tyr Trp Gln Xaa Tyr
   1 5 10
<210> 1009
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (10)..(10)
   <223> Position 10, Xaa = azetidine
<400> 1009
   Phe Glu Trp Thr Pro Gly Trp Tyr Gln Xaa Tyr
   1 5 10
<210> 1010
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION

<220>
   <221 misc_feature
   <222> (10)..(10)
   <223> Position 10, Xaa = azetidine
<400> 1010
   Phe Glu Trp Thr Pro Gly Tyr Tyr Gln Xaa Tyr
   1 5 10
<210> 1011
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221 > misc_feature
   <222> (10)..(10)
   <223> Position 10, Xaa = azetidine
<400> 1011
   Phe Glu Trp Thr Pro Ala Tyr Trp Gln Xaa Tyr
   1 5 10
<210> 1012
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (10)..(10)
   <223> Position 10, Xaa = azetidine
<400> 1012
   Phe Glu Trp Thr Pro Ala Trp Tyr Gln Xaa Tyr
   1 5 10
<210> 1013
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (10)..(10)
   <223> Position 10, Xaa = azetidine
<400> 1013
   Phe Glu Trp Thr Pro Ala Tyr Tyr Gln Xaa Tyr
   1 5 10
<210> 1014
   <211> 15
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 1014
   Phe Glu Trp Thr Pro Gly Tyr Tyr Gln Xaa Tyr Ala Leu Pro Leu
   1 5 10 15
<210> 1015
   <211> 15
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 1015
   Phe Glu Trp Thr Pro Gly Tyr Trp Gln Xaa Tyr Ala Leu Pro Leu
   1 5 10 15
<210> 1016
   <211 > 15
   <212> PRT
   <213> Artificial Sequence
<220>
   <223> IL-1 ANTAGONIST PEPTIDE
<220>
   <221> misc_feature
   <222> (10)..(10)
   <223> Position 10, Xaa = azetidine
<400> 1016
   Phe Glu Trp Thr Pro Gly Trp Tyr Gln Xaa Tyr Ala Leu Pro Leu
   1 5 10 15
<210> 1017
   <211> 21
   <212> PRT
   <213> Artificial Sequence
<220>
   <223> IL-1 ANTAGONIST PEPTIDE
<400> 1017
   Thr Ala Asn Val Ser Ser Phe Glu Trp Thr Pro Gly Tyr Trp Gln Pro
   1 5 10 15
   Tyr Ala Leu Pro Leu
   20
<210> 1018
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (10)..(10)
   <223> Xaa at Position 10 is azetidine
<400> 1018
   Phe Glu Trp Thr Pro Gly Tyr Trp Gln Xaa Tyr
   1 5 10
<210> 1019
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (10)..(10)
   <223> Position 10, Xaa = azetidine
<400>1019
   Phe Glu Trp Thr Pro Gly Trp Tyr Gln Xaa Tyr
   1               5                   10
<210> 1020
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (10)..(10)
   <223> Position 10, Xaa = azetidine
<400> 1020
   Phe Glu Trp Thr Pro Gly Tyr Tyr Gln Xaa Tyr
   1               5                   10
<210> 1021
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221 > misc_feature
   <222> (6)..(6)
   <223> Position 6, D amino acid residue
<220>
   <221> misc_feature
   <222> (10)..(10)
   <223> Position 10, Xaa = azetidine.
<400> 1021
   Phe Glu Trp Thr Pro Ala Tyr Trp Gln Xaa Tyr
   1               5                   10
<210> 1022
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (6)..(6)
   <223> Position 6, D amino acid residue
<220>
   <221> misc_feature
   <222> (10)..(10)
   <223> Position 10, Xaa = azetidine.
<400> 1022
   Phe Glu Trp Thr Pro Ala Trp Tyr Gln Xaa Tyr
   1               5                   10
<210> 1023
   <211> 11
   <212> PRT
   <213> Artificial Sequence
<220>
   <223> IL-1 ANTAGONIST PEPTIDE
<220>
   <221> MOD_RES
   <222> (1)..(1)
   <223> ACETYLATION
<220>
   <221> misc_feature
   <222> (6)..(6)
   <223> Position 6, D amino acid residue
<220>
   <221> misc_feature
   <222> (10)..(10)
   <223> Position 10, Xaa = azetidine.
<400> 1023
   Phe Glu Trp Thr Pro Ala Tyr Tyr Gln Xaa Tyr
   1 5 10
<210> 1024
   <211> 20
   <212> PRT
   <213> Artificial Sequence
<220>
   <223> EPO-MIMETIC PEPTIDE
<400> 1024
   Gly Gly Leu Tyr Leu Cys Arg Phe Gly Pro Val Thr Trp Asp Cys Gly
   1 5 10 15
   Tyr Lys Gly Gly
   20
<210> 1025
   <211> 20
   <212> PRT
   <213> Artificial Sequence
<220>
   <223> EPO-MIMETIC PEPTIDE
<400> 1025
   Gly Gly Thr Tyr Ser Cys His Phe Gly Pro Leu Thr Trp Val Cys Lys
   1 5 10 15
   Pro Gln Gly Gly
   20
<210> 1026
   <211> 20
   <212> PRT
   <213> Artificial Sequence
<220>
   <223> EPO-MIMETIC PEPTIDE
<400> 1026
   Gly Gly Asp Tyr His Cys Arg Met Gly Pro Leu Thr Trp Val Cys Lys
   1 5 10 15
   Pro Leu Gly Gly
   20
<210> 1027
   <211> 19
   <212> PRT
   <213> Artificial Sequence
<220>
   <223> VEGF-ANTAGONIST
<400> 1027
   Val Glu Pro Asn Cys Asp Ile His Val Met Trp Glu Trp Glu Cys Phe
   1 5 10 15
   Glu Arg Leu
<210> 1028
   <211> 10
   <212> PRT
   <213> Artificial Sequence
<220>
   <223> MMP INHIBITOR
<400> 1028
   Cys Thr Thr His Trp Gly Phe Thr Leu Cys
   1 5 10
<210> 1029
   <211> 20
   <212> PRT
   <213> Artificial Sequence
<220>
   <223> EPO-MIMETIC PEPTIDE
<400> 1029
   Val Gly Asn Tyr Met Cys His Phe Gly Pro Ile Thr Trp Val Cys Arg
   1 5 10 15
   Pro Gly Gly Gly
   20
<210> 1030
   <211> 20
   <212> PRT
   <213> Artificial Sequence
<220>
   <223> EPO-MIMETIC PEPTIDE
<400> 1030
   Gly Gly Val Tyr Ala Cys Arg Met Gly Pro Ile Thr Trp Val Cys Ser
   1 5 10 15
   Pro Leu Gly Gly
   20
<210> 1031
   <211> 20
   <212> PRT
   <213> Artificial Sequence
<220>
   <223> VEGF- ANTAGONIST
<400> 1031
   Arg Gly Trp Val Glu Ile Cys Ala Ala Asp Asp Tyr Gly Arg Cys Leu
   1 5 10 15
   Thr Glu Ala Gln
   20
<210> 1032
   <211> 19
   <212> PRT
   <213> Artificial Sequence
<200>
   <223> TPO-MIMETIC
<220>
   <221> misc_feature
   <222> (1)..(1)
   <223> Fc domain attached at Position 1 of the N-terminus
<400> 1032
   Gly Gly Gly Gly Gly Ile Glu Gly Pro Thr Leu Arg Gln Trp Leu Ala
   1 5 10 15
   Ala Arg Ala
<210> 1033
   <211> 19
   <212> PRT
   <213> Artificial Sequence
<220>
   <223> TPO-MIMETIC
<220>
   <221> misc_feature
   <222> (19)..(19)
   <223> Fc domain attached at Position 19 of the C-terminus
<400> 1033
   Ile Glu Gly Pro Thr Leu Arg Gln Trp Leu Ala Ala Arg Ala Gly Gly
   1 5 10 15
   Gly Gly Gly
<210> 1034
   <211> 25
   <212> PRT
   <213> Artificial Sequence
<220>
   <223> EPO-MIMETIC
<220>
   <221> misc_feature
   <222> (25)..(25)
   <223> Fc domain attached at Position 25 of the C-terminus
<400> 1034
   Gly Gly Thr Tyr Ser Cys His Phe Gly Pro Leu Thr Trp Val Cys Lys
   1 5 10 15
   Pro Gln Gly Gly Gly Gly Gly Gly Gly
   20 25
<210> 1035
   <211> 19
   <212> PRT
   <213> Artificial Sequence
<220>
   <223> EPO-MIMETIC PEPTIDE
<400> 1035
   Val Gly Asn Tyr Met Ala His Met Gly Pro Ile Thr Trp Val Cys Arg
   1 5 10 15
   Pro Gly Gly
<210> 1036
   <211> 18
   <212> PRT
   <213> Artificial Sequence
<220>
   <223> EPO-MIMETIC PEPTIDE
<400> 1036
   Gly Gly Thr Tyr Ser Cys His Phe Gly Pro Leu Thr Trp Val Cys Lys
   1 5 10 15
   Pro Gln
<210> 1037
   <211> 20
   <212> PRT
   <213> Artificial Sequence
<220>
   <223> EPO-MIMETIC PEPTIDE
<400> 1037
   Gly Gly Leu Tyr Ala Cys His Met Gly Pro Met Thr Trp Val Cys Gln
   1 5 10 15
   Pro Leu Arg Gly
   20
<210> 1038
   <211> 22
   <212> PRT
   <213> Artificial Sequence
<220>
   <223> EPO-MIMETIC PEPTIDE
<400> 1038
   Thr Ile Ala Gln Tyr Ile Cys Tyr Met Gly Pro Glu Thr Trp Glu Cys
   1 5 10 15
   Arg Pro Ser Pro Lys Ala
   20
<210> 1039
   <211> 13
   <212> PRT
   <213> Artificial Sequence
<220>
   <223> EPO-MIMETIC PEPTIDE
<400> 1039
   Tyr Ser Cys His Phe Gly Pro Leu Thr Trp Val Cys Lys
   1 5 10
<210> 1040
   <211> 11
   <212> PRT
   <213> Artificial Sequence
<220>
   <223> EPO-MIMETIC PEPTIDE
<400> 1040
   Tyr Cys His Phe Gly Pro Leu Thr Trp Val Cys
   1 5 10
<210> 1041
   <211> 12
   <212> PRT
   <213> Artificial Sequence
<220>
   <223> EPO-MIMETIC PEPTIDE
<400> 1041
   Ser Cys His Phe Gly Pro Leu Thr Trp Val Cys Lys
   1 5 10
<210> 1042
   <211> 12
   <212> PRT
   <213> Artificial Sequence
<220>
   <223> EPO-MIMETIC PEPTIDE
<220>
   <221> misc_feature
   <222> (1)..(1)
   <223> Xaa (Pos1) can be any one of the 20 L-amino acids; except Xaa (Pos1) may not be Y
<220>
   <221> misc_feature
   <222> (2)..(2)
   <223> Xaa at position 2 can be any one of the 20 L-amino acids
<220>
   <221> misc_feature
   <222> (3)..(3)
   <223> Xaa (Pos3) can be C, A, a-amino-y-bromobutyric acid or Hoc
<220>
   <221> misc_feature
   <222> (4)..(4)
   <223> Xaa (Pos4) can be R, H, L or W
<220>
   <221> misc_feature
   <222> (5)..(5)
   <223> Xaa (Pos5) can be M, F or I
<220>
   <221> misc_feature
   <222> (8)..(8)
   <223> Xaa at position 8 can be any one of the 20 L-amino acids
<220>
   <221> misc_feature
   <222> (11)..(11)
   <223> Xaa (Pos11) can be D, E, I, L or V
<220>
   <221> misc_feature
   <222> (12)..(12)
   <223> Xaa is C, A, alpha-amino-gama bromobutyric acid or Hoc, provided that Xaa at position 3 or 12 is C or Hoc
<400> 1042
   Xaa Xaa Xaa Xaa Xaa Gly Pro Xaa Thr Trp Xaa Xaa
   1               5                   10
<210> 1043
   <211> 5
   <212> PRT
   <213> Artificial Sequence
<220>
   <223> INTEGRIN-BINDING PEPTIDE
<220>
   <221> misc_feature
   <222> (3)..(4)
   <223> Xaa = any amino acid
<400> 1043
   Asp Leu Xaa Xaa Leu
   1               5
<210> 1044
   <211> 12
   <212> PRT
   <213> Artificial Sequence
<220>
   <223> INTEGRIN-BINDING PEPTIDE
<400> 1044
   Arg Thr Asp Leu Asp Ser Leu Arg Thr Tyr Thr Leu
   1               5                   10
<210> 1045
   <211> 20
   <212> PRT
   <213> Artificial Sequence
<220>
   <223> TNF-ALPHA INHIBITOR
<220>
   <221> misc_feature
   <222> (1)..(1)
   <223> Fc domain attached at Position 1 of the N-terminus
<400> 1045
   Gly Gly Gly Gly Gly Asp Phe Leu Pro His Tyr Lys Asn Thr Ser Leu
   1 5 10 15
   Gly His Arg Pro
   20
<210> 1046
   <211> 20
   <212> PRT
   <213> Artificial Sequence
<220>
   <223> TNF-ALPHA INHIBITOR
<220>
   <221> misc_feature
   <222> (20)..(20)
   <223> Fc domain attached at Position 20 of the C-terminus
<400> 1046
   Asp Phe Leu Pro His Tyr Lys Asn Thr Ser Leu Gly His Arg Pro Gly
   1 5 10 15
   Gly Gly Gly Gly
   20
<210> 1047
   <211> 20
   <212> PRT
   <213> Artificial Sequence
<220>
   <223> IL-1 R ANTAGONIST
<220>
   <221> misc_feature
   <222> (1)..(1)
   <223> Fc domain attached at Position 1 of the N-terminus
<400> 1047
   Gly Gly Gly Gly Gly Phe Glu Trp Thr Pro Gly Tyr Trp Gln Pro Tyr
   1               5                   10                  15
   Ala Leu Pro Leu
               20
<210> 1048
   <211> 20
   <212> PRT
   <213> Artificial Sequence
<220>
   <223> IL-1 R ANTAGONIST
<220>
   <221> misc_feature
   <222> (20)..(20)
   <223> Fc domain attached at Position 20 of the C-terminus
<400> 1048
   Phe Glu Trp Thr Pro Gly Tyr Trp Gln Pro Tyr Ala Leu Pro Leu Gly
   1               5                   10                  15
   Gly Gly Gly Gly
               20
<210> 1049
   <211> 24
   <212> PRT
   <213> Artificial Sequence
<220>
   <223> VEGF-ANTAGONIST
<220>
   <221> misc_feature
   <222> (1)..(1)
   <223> Fc domain attached at Position 1 of the N-terminus
<400> 1049
   Gly Gly Gly Gly Gly Val Glu Pro Asn Cys Asp Ile His Val Met Trp
   1               5                   10                  15
   Glu Trp Glu Cys Phe Glu Arg Leu
               20
<210> 1050
   <211> 24
   <212> PRT
   <213> Artificial Sequence
<220>
   <223> VEGF-ANTAGONIST
<220>
   <221> misc_feature
   <222> (24)..(24)
   <223> Fc domain attached at Position 24 of the C-terminus
<400> 1050
   Val Glu Pro Asn Cys Asp Ile His Val Met Trp Glu Trp Glu Cys Phe
   1               5                   10                  15
   Glu Arg Leu Gly Gly Gly Gly Gly
               20
<210> 1051
   <211> 15
   <212> PRT
   <213> Artificial Sequence
<220>
   <223> MMP INHIBITOR
<220>
   <221> > misc_feature
   <222> (1)..(1)
   <223> Fc domain attached at Position 1 of the N-terminus
<400> 1051
   Gly Gly Gly Gly Gly Cys Thr Thr His Trp Gly Phe Thr Leu Cys
   1               5                   10                  15
<210> 1052
   <211> 15
   <212> PRT
   <213> Artificial Sequence
<220>
   <223> MMP INHIBITOR
<220>
   <221> misc_feature
   <222> (15)..(15)
   <223> Fc domain attached at Position 15 of the C-terminus
<400> 1052
   Cys Thr Thr His Trp Gly Phe Thr Leu Cys Gly Gly Gly Gly Gly
   1               5                   10                  15
<210> 1053
   <211> 10
   <212> PRT
   <213> Artificial Sequence
<220>
   <223> INTEGRIN-BINDING PEPTIDE
<400> 1053
   Arg Thr Asp Leu Asp Ser Leu Arg Thr Tyr
   1 5 10
<210> 1054
   <211> 9
   <212> PRT
   <213> Artificial Sequence
<220>
   <223> INTEGRIN-BINDING PEPTIDE
<400> 1054
   Arg Thr Asp Leu Asp Ser Leu Arg Thr
   1 5
<210> 1055
   <211> 757
   <212> DNA
   <213> Artificial Sequence
<220>
   <223> Fc-TNF-ALPHA INHIBITOR
<220>
   <221> CDS
   <222> (4)..(747)
<400> 1055
   cat atg gac aaa act cac aca tgt cca cct tgt cca gct ccg gaa ctc      48
       Met Asp Lys Thr His Thr Cys Pro Pro Cys Pro Ala Pro Glu Leu
       1               5                   10                  15
   ctg ggg gga ccg tca gtc ttc ctc ttc ccc cca aaa ccc aag gac acc      96
   Leu Gly Gly Pro Ser Val Phe Leu Phe Pro Pro Lys Pro Lys Asp Thr
                   20                  25                  30
   ctc atg atc tcc cgg acc cct gag gtc aca tgc gtg gtg gtg gac gtg     144
   Leu Met Ile Ser Arg Thr Pro Glu Val Thr Cys Val Val Val Asp Val
               35                  40                  45
   agc cac gaa gac cct gag gtc aag ttc aac tgg tac gtg gac ggc gtg     192
   Ser His Glu Asp Pro Glu Val Lys Phe Asn Trp Tyr Val Asp Gly Val
           50                  55                  60
   gag gtg cat aat gcc aag aca aag ccg cgg gag gag cag tac aac agc     240
   Glu Val His Asn Ala Lys Thr Lys Pro Arg Glu Glu Gln Tyr Asn Ser
       65                  70                  75
   acg tac cgt gtg gtc agc gtc ctc acc gtc ctg cac cag gac tgg ctg     288
   Thr Tyr Arg Val Val Ser Val Leu Thr Val Leu His Gln Asp Trp Leu
   80                  85                  90                  95
   aat ggc aag gag tac aag tgc aag gtc tcc aac aaa gcc ctc cca gcc     336
   Asn Gly Lys Glu Tyr Lys Cys Lys Val Ser Asn Lys Ala Leu Pro Ala
                   100                 105                 110
   ccc atc gag aaa acc atc tcc aaa gcc aaa ggg cag ccc cga gaa cca     384
   Pro Ile Glu Lys Thr Ile Ser Lys Ala Lys Gly Gln Pro Arg Glu Pro
               115                 120                 125
   cag gtg tac acc ctg ccc cca tcc cgg gat gag ctg acc aag aac cag     432
   Gln Val Tyr Thr Leu Pro Pro Ser Arg Asp Glu Leu Thr Lys Asn Gln
           130                 135                 140
   gtc agc ctg acc tgc ctg gtc aaa ggc ttc tat ccc agc gac atc gcc     480
   Val Ser Leu Thr Cys Leu Val Lys Gly Phe Tyr Pro Ser Asp Ile Ala
       145                 150                 155
   gtg gag tgg gag agc aat ggg cag ccg gag aac aac tac aag acc acg     528
   Val Glu Trp Glu Ser Asn Gly Gln Pro Glu Asn Asn Tyr Lys Thr Thr
   160                 165                 170                 175
   cct ccc gtg ctg gac tcc gac ggc tcc ttc ttc ctc tac agc aag ctc     576
   Pro Pro Val Leu Asp Ser Asp Gly Ser Phe Phe Leu Tyr Ser Lys Leu
                   180                 185                 190
   acc gtg gac aag agc agg tgg cag cag ggg aac gtc ttc tca tgc tcc     624
   Thr Val Asp Lys Ser Arg Trp Gln Gln Gly Asn Val Phe Ser Cys Ser
               195                 200                 205
   gtg atg cat gag gct ctg cac aac cac tac acg cag aag agc ctc tcc     672
   Val Met His Glu Ala Leu His Asn His Tyr Thr Gln Lys Ser Leu Ser
           210                 215                 220
   ctg tct ccg ggt aaa ggt gga ggt ggt ggt gac ttc ctg ccg cac tac     720
   Leu Ser Pro Gly Lys Gly Gly Gly Gly Gly Asp Phe Leu Pro His Tyr
       225                 230                 235
   aaa aac acc tct ctg ggt cac cgt ccg taatggatcc                      757
   Lys Asn Thr Ser Leu Gly His Arg Pro
   240                 245
<210> 1056
   <211> 248
   <212> PRT
   <213> Artificial Sequence
<220>
   <223> Synthetic Construct
<400> 1056
   Met Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Lys Phe Asn Trp Tyr Val Asp Gly Val Glu
       50                  55                  60
   Val His Asn Ala Lys Thr Lys Pro Arg Glu Glu Gln Tyr Asn Ser Thr
   65                  70                  75                  80
   Tyr Arg Val Val Ser Val Leu Thr Val Leu His Gln Asp Trp Leu Asn
                   85                  90                  95
   Gly Lys Glu Tyr Lys Cys Lys Val Ser Asn Lys Ala Leu Pro Ala Pro
               100                 105                 110
   Ile Glu Lys Thr Ile Ser Lys Ala Lys Gly Gln Pro Arg Glu Pro Gln
           115                 120                 125
   Val Tyr Thr Leu Pro Pro Ser Arg Asp Glu Leu Thr Lys Asn Gln Val
       130                 135                 140
   Ser Leu Thr Cys Leu Val Lys Gly Phe Tyr Pro Ser Asp Ile Ala Val
   145 150 155 160
   Glu Trp Glu Ser Asn Gly Gln Pro Glu Asn Asn Tyr Lys Thr Thr Pro
   165 170 175
   Pro Val Leu Asp Ser Asp Gly Ser Phe Phe Leu Tyr Ser Lys Leu Thr
   180 185 190
   Val Asp Lys Ser Arg Trp Gln Gln Gly Asn Val Phe Ser Cys Ser Val
   195 200 205
   Met His Glu Ala Leu His Asn His Tyr Thr Gln Lys Ser Leu Ser Leu
   210 215 220
   Ser Pro Gly Lys Gly Gly Gly Gly Gly Asp Phe Leu Pro His Tyr Lys
   225 230 235 240
   Asn Thr Ser Leu Gly His Arg Pro
   245
<210> 1057
   <211> 761
   <212> DNA
   <213> Artificial Sequence
<220>
   <223> TNF-ALPHA INHIBITOR-Fc
<220>
   <221> CDS
   <222> (4)..(747)
<400> 1057
   cat atg gac ttc ctg ccg cac tac aaa aac acc tct ctg ggt cac cgt      48
       Met Asp Phe Leu Pro His Tyr Lys Asn Thr Ser Leu Gly His Arg
       1               5                   10                  15
   ccg ggt gga ggc ggt ggg gac aaa act cac aca tgt cca cct tgc cca      96
   Pro Gly Gly Gly Gly Gly Asp Lys Thr His Thr Cys Pro Pro Cys Pro
                   20                  25                  30
   gca cct gaa ctc ctg ggg gga ccg tca gtt ttc ctc ttc ccc cca aaa     144
   Ala Pro Glu Leu Leu Gly Gly Pro Ser Val Phe Leu Phe Pro Pro Lys
               35                  40                  45
   ccc aag gac acc ctc atg atc tcc cgg acc cct gag gtc aca tgc gtg     192
   Pro Lys Asp Thr Leu Met Ile Ser Arg Thr Pro Glu Val Thr Cys Val
           50                  55                  60
   gtg gtg gac gtg agc cac gaa gac cct gag gtc aag ttc aac tgg tac     240
   Val Val Asp Val Ser His Glu Asp Pro Glu Val Lys Phe Asn Trp Tyr
       65                  70                  75
   gtg gac ggc gtg gag gtg cat aat gcc aag aca aag ccg cgg gag gag     288
   Val Asp Gly Val Glu Val His Asn Ala Lys Thr Lys Pro Arg Glu Glu
   80                  85                  90                  95
   cag tac aac agc acg tac cgt gtg gtc agc gtc ctc acc gtc ctg cac     336
   Gln Tyr Asn Ser Thr Tyr Arg Val Val Ser Val Leu Thr Val Leu His
                   100                 105                 110
   cag gac tgg ctg aat ggc aag gag tac aag tgc aag gtc tcc aac aaa     384
   Gln Asp Trp Leu Asn Gly Lys Glu Tyr Lys Cys Lys Val Ser Asn Lys
               115                 120                 125
   gcc ctc cca gcc ccc atc gag aaa acc atc tcc aaa gcc aaa ggg cag     432
   Ala Leu Pro Ala Pro Ile Glu Lys Thr Ile Ser Lys Ala Lys Gly Gln
           130                 135                 140
   ccc cga gaa cca cag gtg tac acc ctg ccc cca tcc cgg gat gag ctg     480
   Pro Arg Glu Pro Gln Val Tyr Thr Leu Pro Pro Ser Arg Asp Glu Leu
       145                 150                 155
   acc aag aac cag gtc agc ctg acc tgc ctg gtc aaa ggc ttc tat ccc     528
   Thr Lys Asn Gln Val Ser Leu Thr Cys Leu Val Lys Gly Phe Tyr Pro
   160                 165                 170                 175
   agc gac atc gcc gtg gag tgg gag agc aat ggg cag ccg gag aac aac     576
   Ser Asp Ile Ala Val Glu Trp Glu Ser Asn Gly Gln Pro Glu Asn Asn
                   180                 185                 190
   tac aag acc acg cct ccc gtg ctg gac tcc gac ggc tcc ttc ttc ctc     624
   Tyr Lys Thr Thr Pro Pro Val Leu Asp Ser Asp Gly Ser Phe Phe Leu
               195                 200                 205
   tac agc aag ctc acc gtg gac aag agc agg tgg cag cag ggg aac gtc     672
   Tyr Ser Lys Leu Thr Val Asp Lys Ser Arg Trp Gln Gln Gly Asn Val
           210                 215                 220
   ttc tca tgc tcc gtg atg cat gag gct ctg cac aac cac tac acg cag     720
   Phe Ser Cys Ser Val Met His Glu Ala Leu His Asn His Tyr Thr Gln
       225                 230                 235
   aag agc ctc tcc ctg tct ccg ggt aaa taatggatcc gcgg                  761
   Lys Ser Leu Ser Leu Ser Pro Gly Lys
   240                 245

<210> 1058
   <211> 248
   <212> PRT
   <213> Artificial Sequence
<220>
   <223> Synthetic Construct
<400> 1058
   Met Asp Phe Leu Pro His Tyr Lys Asn Thr Ser Leu Gly His Arg Pro
   1               5                   10                  15
   Gly Gly Gly Gly Gly Asp Lys Thr His Thr Cys Pro Pro Cys Pro Ala
               20                  25                  30
   Pro Glu Leu Leu Gly Gly Pro Ser Val Phe Leu Phe Pro Pro Lys Pro
           35                  40                  45
   Lys Asp Thr Leu Met Ile Ser Arg Thr Pro Glu Val Thr Cys Val Val
       50                  55                  60
   Val Asp Val Ser His Glu Asp Pro Glu Val Lys Phe Asn Trp Tyr Val
   65                  70                  75                  80
   Asp Gly Val Glu Val His Asn Ala Lys Thr Lys Pro Arg Glu Glu Gln
                   85                  90                  95
   Tyr Asn Ser Thr Tyr Arg Val Val Ser Val Leu Thr Val Leu His Gln
               100                 105                 110
   Asp Trp Leu Asn Gly Lys Glu Tyr Lys Cys Lys Val Ser Asn Lys Ala
           115                 120                 125
   Leu Pro Ala Pro Ile Glu Lys Thr Ile Ser Lys Ala Lys Gly Gln Pro
       130                 135                 140
   Arg Glu Pro Gln Val Tyr Thr Leu Pro Pro Ser Arg Asp Glu Leu Thr
   145                 150                 155                 160
   Lys Asn Gln Val Ser Leu Thr Cys Leu Val Lys Gly Phe Tyr Pro Ser
                   165                 170                 175
   Asp Ile Ala Val Glu Trp Glu Ser Asn Gly Gln Pro Glu Asn Asn Tyr
               180                 185                 190
   Lys Thr Thr Pro Pro Val Leu Asp Ser Asp Gly Ser Phe Phe Leu Tyr
           195                 200                 205
   Ser Lys Leu Thr Val Asp Lys Ser Arg Trp Gln Gln Gly Asn Val Phe
       210                 215                 220
   Ser Cys Ser Val Met His Glu Ala Leu His Asn His Tyr Thr Gln Lys
   225                 230                 235                 240
   Ser Leu Ser Leu Ser Pro Gly Lys
                   245
<210> 1059
   <211> 763
   <212> DNA
   <213> Artificial Sequence
<220>
   <223> FC-IL-1 ANTAGONIST
<220>
   <221> CDS
   <222> (4)..(747)
<400> 1059
   cat atg gac aaa act cac aca tgt cca cct tgt cca gct ccg gaa ctc      48
       Met Asp Lys Thr His Thr Cys Pro Pro Cys Pro Ala Pro Glu Leu
       1               5                   10                  15
   ctg ggg gga ccg tca gtc ttc ctc ttc ccc cca aaa ccc aag gac acc      96
   Leu Gly Gly Pro Ser Val Phe Leu Phe Pro Pro Lys Pro Lys Asp Thr
                   20                  25                  30
   ctc atg atc tcc cgg acc cct gag gtc aca tgc gtg gtg gtg gac gtg     144
   Leu Met Ile Ser Arg Thr Pro Glu Val Thr Cys Val Val Val Asp Val
               35                  40                  45
   agc cac gaa gac cct gag gtc aag ttc aac tgg tac gtg gac ggc gtg     192
   Ser His Glu Asp Pro Glu Val Lys Phe Asn Trp Tyr Val Asp Gly Val
           50                  55                  60
   gag gtg cat aat gcc aag aca aag ccg cgg gag gag cag tac aac agc     240
   Glu Val His Asn Ala Lys Thr Lys Pro Arg Glu Glu Gln Tyr Asn Ser
       65                  70                  75
   acg tac cgt gtg gtc agc gtc ctc acc gtc ctg cac cag gac tgg ctg     288
   Thr Tyr Arg Val Val Ser Val Leu Thr Val Leu His Gln Asp Trp Leu
   80                  85                  90                  95
   aat ggc aag gag tac aag tgc aag gtc tcc aac aaa gcc ctc cca gcc     336
   Asn Gly Lys Glu Tyr Lys Cys Lys Val Ser Asn Lys Ala Leu Pro Ala
                   100                 105                 110
   ccc atc gag aaa acc atc tcc aaa gcc aaa ggg cag ccc cga gaa cca     384
   Pro Ile Glu Lys Thr Ile Ser Lys Ala Lys Gly Gln Pro Arg Glu Pro
               115                 120                 125
   cag gtg tac acc ctg ccc cca tcc cgg gat gag ctg acc aag aac cag     432
   Gln Val Tyr Thr Leu Pro Pro Ser Arg Asp Glu Leu Thr Lys Asn Gln
           130                 135                 140
   gtc agc ctg acc tgc ctg gtc aaa ggc ttc tat ccc agc gac atc gcc     480
   Val Ser Leu Thr Cys Leu Val Lys Gly Phe Tyr Pro Ser Asp Ile Ala
       145                 150                 155
   gtg gag tgg gag agc aat ggg cag ccg gag aac aac tac aag acc acg     528
   Val Glu Trp Glu Ser Asn Gly Gln Pro Glu Asn Asn Tyr Lys Thr Thr
   160                 165                 170                 175
   cct ccc gtg ctg gac tcc gac ggc tcc ttc ttc ctc tac agc aag ctc     576
   Pro Pro Val Leu Asp Ser Asp Gly Ser Phe Phe Leu Tyr Ser Lys Leu
                   180                 185                 190
   acc gtg gac aag agc agg tgg cag cag ggg aac gtc ttc tca tgc tcc     624
   Thr Val Asp Lys Ser Arg Trp Gln Gln Gly Asn Val Phe Ser Cys Ser
               195                 200                 205
   gtg atg cat gag gct ctg cac aac cac tac acg cag aag agc ctc tcc     672
   Val Met His Glu Ala Leu His Asn His Tyr Thr Gln Lys Ser Leu Ser
           210                 215                 220
   ctg tct ccg ggt aaa ggt gga ggt ggt ggt ttc gaa tgg acc ccg ggt     720
   Leu Ser Pro Gly Lys Gly Gly Gly Gly Gly Phe Glu Trp Thr Pro Gly
       225                 230                 235
   tac tgg cag ccg tac gct ctg ccg ctg taatggatcc ctcgag               763
   Tyr Trp Gln Pro Tyr Ala Leu Pro Leu
       240                 245
<210> 1060
   <211> 248
   <212> PRT
   <213> Artificial Sequence
<220>
   <223> Synthetic Construct
<400> 1060
   Met Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Lys Phe Asn Trp Tyr Val Asp Gly Val Glu
       50                  55                  60
   Val His Asn Ala Lys Thr Lys Pro Arg Glu Glu Gln Tyr Asn Ser Thr
   65                  70                  75                  80
   Tyr Arg Val Val Ser Val Leu Thr Val Leu His Gln Asp Trp Leu Asn
                   85                  90                  95
   Gly Lys Glu Tyr Lys Cys Lys Val Ser Asn Lys Ala Leu Pro Ala Pro
               100                 105                 110
   Ile Glu Lys Thr Ile Ser Lys Ala Lys Gly Gln Pro Arg Glu Pro Gln
           115                 120                 125
   Val Tyr Thr Leu Pro Pro Ser Arg Asp Glu Leu Thr Lys Asn Gln Val
       130                 135                 140
   Ser Leu Thr Cys Leu Val Lys Gly Phe Tyr Pro Ser Asp Ile Ala Val
   145                 150                 155                 160
   Glu Trp Glu Ser Asn Gly Gln Pro Glu Asn Asn Tyr Lys Thr Thr Pro
                   165                 170                 175
   Pro Val Leu Asp Ser Asp Gly Ser Phe Phe Leu Tyr Ser Lys Leu Thr
               180                 185                 190
   Val Asp Lys Ser Arg Trp Gln Gln Gly Asn Val Phe Ser Cys Ser Val
           195                 200                 205
   Met His Glu Ala Leu His Asn His Tyr Thr Gln Lys Ser Leu Ser Leu
   210 215 220
   Ser Pro Gly Lys Gly Gly Gly Gly Gly Phe Glu Trp Thr Pro Gly Tyr
   225 230 235 240
   Trp Gln Pro Tyr Ala Leu Pro Leu
   245
<210> 1061
   <211> 757
   <212> DNA
   <213> Artificial Sequence
<220>
   <223> IL-1 ANTAGONIST-FC
<220>
   <221> CDS
   <222> (4)..(747)
<400> 1061
   cat atg ttc gaa tgg acc ccg ggt tac tgg cag ccg tac gct ctg ccg      48
       Met Phe Glu Trp Thr Pro Gly Tyr Trp Gln Pro Tyr Ala Leu Pro
       1               5                   10                  15
   ctg ggt gga ggc ggt ggg gac aaa act cac aca tgt cca cct tgc cca      96
   Leu Gly Gly Gly Gly Gly Asp Lys Thr His Thr Cys Pro Pro Cys Pro
                   20                  25                  30
   gca cct gaa ctc ctg ggg gga ccg tca gtt ttc ctc ttc ccc cca aaa     144
   Ala Pro Glu Leu Leu Gly Gly Pro Ser Val Phe Leu Phe Pro Pro Lys
               35                  40                  45
   ccc aag gac acc ctc atg atc tcc cgg acc cct gag gtc aca tgc gtg     192
   Pro Lys Asp Thr Leu Met Ile Ser Arg Thr Pro Glu Val Thr Cys Val
           50                  55                  60
   gtg gtg gac gtg agc cac gaa gac cct gag gtc aag ttc aac tgg tac     240
   Val Val Asp Val Ser His Glu Asp Pro Glu Val Lys Phe Asn Trp Tyr
       65                  70                  75
   gtg gac ggc gtg gag gtg cat aat gcc aag aca aag ccg cgg gag gag     288
   Val Asp Gly Val Glu Val His Asn Ala Lys Thr Lys Pro Arg Glu Glu
   80                  85                  90                  95
   cag tac aac agc acg tac cgt gtg gtc agc gtc ctc acc gtc ctg cac     336
   Gln Tyr Asn Ser Thr Tyr Arg Val Val Ser Val Leu Thr Val Leu His
                   100                 105                 110
   cag gac tgg ctg aat ggc aag gag tac aag tgc aag gtc tcc aac aaa     384
   Gln Asp Trp Leu Asn Gly Lys Glu Tyr Lys Cys Lys Val Ser Asn Lys
               115                 120                 125
   gcc ctc cca gcc ccc atc gag aaa acc atc tcc aaa gcc aaa ggg cag     432
   Ala Leu Pro Ala Pro Ile Glu Lys Thr Ile Ser Lys Ala Lys Gly Gln
           130                 135                 140
   ccc cga gaa cca cag gtg tac acc ctg ccc cca tcc cgg gat gag ctg     480
   Pro Arg Glu Pro Gln Val Tyr Thr Leu Pro Pro Ser Arg Asp Glu Leu
       145                 150                 155
   acc aag aac cag gtc agc ctg acc tgc ctg gtc aaa ggc ttc tat ccc     528
   Thr Lys Asn Gln Val Ser Leu Thr Cys Leu Val Lys Gly Phe Tyr Pro
   160                 165                 170                 175
   agc gac atc gcc gtg gag tgg gag agc aat ggg cag ccg gag aac aac     576
   Ser Asp Ile Ala Val Glu Trp Glu Ser Asn Gly Gln Pro Glu Asn Asn
                   180                 185                 190
   tac aag acc acg cct ccc gtg ctg gac tcc gac ggc tcc ttc ttc ctc     624
   Tyr Lys Thr Thr Pro Pro Val Leu Asp Ser Asp Gly Ser Phe Phe Leu
               195                 200                 205
   tac agc aag ctc acc gtg gac aag agc agg tgg cag cag ggg aac gtc     672
   Tyr Ser Lys Leu Thr Val Asp Lys Ser Arg Trp Gln Gln Gly Asn Val
           210                 215                 220
   ttc tca tgc tcc gtg atg cat gag gct ctg cac aac cac tac acg cag     720
   Phe Ser Cys Ser Val Met His Glu Ala Leu His Asn His Tyr Thr Gln
       225                 230                 235
   aag agc ctc tcc ctg tct ccg ggt aaa taatggatcc                      757
   Lys Ser Leu Ser Leu Ser Pro Gly Lys
   240                 245
<210> 1062
   <211> 248
   <212> PRT
   <213> Artificial Sequence
<220>
   <223> Synthetic Construct
<400> 1062
   Met Phe Glu Trp Thr Pro Gly Tyr Trp Gln Pro Tyr Ala Leu Pro Leu
   1               5                   10                  15
   Gly Gly Gly Gly Gly Asp Lys Thr His Thr Cys Pro Pro Cys Pro Ala
               20                  25                  30
   Pro Glu Leu Leu Gly Gly Pro Ser Val Phe Leu Phe Pro Pro Lys Pro
           35                  40                  45
   Lys Asp Thr Leu Met Ile Ser Arg Thr Pro Glu Val Thr Cys Val Val
       50                  55                  60
   Val Asp Val Ser His Glu Asp Pro Glu Val Lys Phe Asn Trp Tyr Val
   65                  70                  75                  80
   Asp Gly Val Glu Val His Asn Ala Lys Thr Lys Pro Arg Glu Glu Gln
                   85                  90                  95
   Tyr Asn Ser Thr Tyr Arg Val Val Ser Val Leu Thr Val Leu His Gln
               100                 105                 110
   Asp Trp Leu Asn Gly Lys Glu Tyr Lys Cys Lys Val Ser Asn Lys Ala
           115                 120                 125
   Leu Pro Ala Pro Ile Glu Lys Thr Ile Ser Lys Ala Lys Gly Gln Pro
       130                 135                 140
   Arg Glu Pro Gln Val Tyr Thr Leu Pro Pro Ser Arg Asp Glu Leu Thr
   145                 150                 155                 160
   Lys Asn Gln Val Ser Leu Thr Cys Leu Val Lys Gly Phe Tyr Pro Ser
                   165                 170                 175
   Asp Ile Ala Val Glu Trp Glu Ser Asn Gly Gln Pro Glu Asn Asn Tyr
               180                 185                 190
   Lys Thr Thr Pro Pro Val Leu Asp Ser Asp Gly Ser Phe Phe Leu Tyr
           195                 200                 205
   Ser Lys Leu Thr Val Asp Lys Ser Arg Trp Gln Gln Gly Asn Val Phe
       210                 215                 220
   Ser Cys Ser Val Met His Glu Ala Leu His Asn His Tyr Thr Gln Lys
   225                 230                 235                 240
   Ser Leu Ser Leu Ser Pro Gly Lys
                   245
<210> 1063
   <211> 773
   <212> DNA
   <213> Artificial Sequence
<220>
   <223> Fc-VEGF ANTAGONIST
<220>
   <221> CDS
   <222> (4)..(759)
<400> 1063
   cat atg gac aaa act cac aca tgt cca ccg tgc cca gca cct gaa ctc      48
       Met Asp Lys Thr His Thr Cys Pro Pro Cys Pro Ala Pro Glu Leu
       1               5                   10                  15
   ctg ggg gga ccg tca gtt ttc ctc ttc ccc cca aaa ccc aag gac acc      96
   Leu Gly Gly Pro Ser Val Phe Leu Phe Pro Pro Lys Pro Lys Asp Thr
                   20                  25                  30
   ctc atg atc tcc cgg acc cct gag gtc aca tgc gtg gtg gtg gac gtg     144
   Leu Met Ile Ser Arg Thr Pro Glu Val Thr Cys Val Val Val Asp Val
               35                  40                  45
   agc cac gaa gac cct gag gtc aag ttc aac tgg tac gtg gac ggc gtg      192
   Ser His Glu Asp Pro Glu Val Lys Phe Asn Trp Tyr Val Asp Gly Val
           50                  55                  60
   gag gtg cat aat gcc aag aca aag ccg cgg gag gag cag tac aac agc      240
   Glu Val His Asn Ala Lys Thr Lys Pro Arg Glu Glu Gln Tyr Asn Ser
       65                  70                  75
   acg tac cgt gtg gtc agc gtc ctc acc gtc ctg cac cag gac tgg ctg      288
   Thr Tyr Arg Val Val Ser Val Leu Thr Val Leu His Gln Asp Trp Leu
   80                  85                  90                  95
   aat ggc aag gag tac aag tgc aag gtc tcc aac aaa gcc ctc cca gcc      336
   Asn Gly Lys Glu Tyr Lys Cys Lys Val Ser Asn Lys Ala Leu Pro Ala
                   100                 105                 110
   ccc atc gag aaa acc atc tcc aaa gcc aaa ggg cag ccc cga gaa cca      384
   Pro Ile Glu Lys Thr Ile Ser Lys Ala Lys Gly Gln Pro Arg Glu Pro
               115                 120                 125
   cag gtg tac acc ctg ccc cca tcc cgg gat gag ctg acc aag aac cag      432
   Gln Val Tyr Thr Leu Pro Pro Ser Arg Asp Glu Leu Thr Lys Asn Gln
           130                 135                 140
   gtc agc ctg acc tgc ctg gtc aaa ggc ttc tat ccc agc gac atc gcc      480
   Val Ser Leu Thr Cys Leu Val Lys Gly Phe Tyr Pro Ser Asp Ile Ala
       145                 150                 155
   gtg gag tgg gag agc aat ggg cag ccg gag aac aac tac aag acc acg      528
   Val Glu Trp Glu Ser Asn Gly Gln Pro Glu Asn Asn Tyr Lys Thr Thr
   160                 165                 170                 175
   cct ccc gtg ctg gac tcc gac ggc tcc ttc ttc ctc tac agc aag ctc      576
   Pro Pro Val Leu Asp Ser Asp Gly Ser Phe Phe Leu Tyr Ser Lys Leu
                   180                 185                 190
   acc gtg gac aag agc agg tgg cag cag ggg aac gtc ttc tca tgc tcc      624
   Thr Val Asp Lys Ser Arg Trp Gln Gln Gly Asn Val Phe Ser Cys Ser
               195                 200                 205
   gtg atg cat gag gct ctg cac aac cac tac acg cag aag agc ctc tcc      672
   Val Met His Glu Ala Leu His Asn His Tyr Thr Gln Lys Ser Leu Ser
           210                 215                 220
   ctg tct ccg ggt aaa ggt ggt ggt ggt ggt gtt gaa ccg aac tgt gac      720
   Leu Ser Pro Gly Lys Gly Gly Gly Gly Gly Val Glu Pro Asn Cys Asp
       225                 230                 235
   atc cat gtt atg tgg gaa tgg gaa tgt ttt gaa cgt ctg taactcgagg       769
   Ile His Val Met Trp Glu Trp Glu Cys Phe Glu Arg Leu
   240                 245                 250
   atcc                                                                 773
<210> 1064
   <211> 252
   <212> PRT
   <213> Artificial Sequence
<220>
   <223> Synthetic Construct
<400> 1064
   Met Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Lys Phe Asn Trp Tyr Val Asp Gly Val Glu
       50                  55                  60
   Val His Asn Ala Lys Thr Lys Pro Arg Glu Glu Gln Tyr Asn Ser Thr
   65                  70                  75                  80
   Tyr Arg Val Val Ser Val Leu Thr Val Leu His Gln Asp Trp Leu Asn
                   85                  90                  95
   Gly Lys Glu Tyr Lys Cys Lys Val Ser Asn Lys Ala Leu Pro Ala Pro
               100                 105                 110
   Ile Glu Lys Thr Ile Ser Lys Ala Lys Gly Gln Pro Arg Glu Pro Gln
           115                 120                 125
   Val Tyr Thr Leu Pro Pro Ser Arg Asp Glu Leu Thr Lys Asn Gln Val
       130                 135                 140
   Ser Leu Thr Cys Leu Val Lys Gly Phe Tyr Pro Ser Asp Ile Ala Val
   145                 150                 155                 160
   Glu Trp Glu Ser Asn Gly Gln Pro Glu Asn Asn Tyr Lys Thr Thr Pro
                   165                 170                 175
   Pro Val Leu Asp Ser Asp Gly Ser Phe Phe Leu Tyr Ser Lys Leu Thr
               180                 185                 190
   Val Asp Lys Ser Arg Trp Gln Gln Gly Asn Val Phe Ser Cys Ser Val
           195                 200                 205
   Met His Glu Ala Leu His Asn His Tyr Thr Gln Lys Ser Leu Ser Leu
       210                 215                 220
   Ser Pro Gly Lys Gly Gly Gly Gly Gly Val Glu Pro Asn Cys Asp Ile
   225                 230                 235                 240
   His Val Met Trp Glu Trp Glu Cys Phe Glu Arg Leu
                   245                 250
<210> 1065
   <211> 773
   <212> DNA
   <213> Artificial Sequence
<220>
   <223> VEGF ANTAGONIST-Fc
<220>
   <221> CDS
   <222> (4)..(759)
<400> 1065
   cat atg gtt gaa ccg aac tgt gac atc cat gtt atg tgg gaa tgg gaa       48
       Met Val Glu Pro Asn Cys Asp Ile His Val Met Trp Glu Trp Glu
       1               5                   10                  15
   tgt ttt gaa cgt ctg ggt ggt ggt ggt ggt gac aaa act cac aca tgt       96
   Cys Phe Glu Arg Leu Gly Gly Gly Gly Gly Asp Lys Thr His Thr Cys
                   20                  25                  30
   cca ccg tgc cca gca cct gaa ctc ctg ggg gga ccg tca gtt ttc ctc      144
   Pro Pro Cys Pro Ala Pro Glu Leu Leu Gly Gly Pro Ser Val Phe Leu
               35                  40                  45
   ttc ccc cca aaa ccc aag gac acc ctc atg atc tcc cgg acc cct gag      192
   Phe Pro Pro Lys Pro Lys Asp Thr Leu Met Ile Ser Arg Thr Pro Glu
           50                  55                  60
   gtc aca tgc gtg gtg gtg gac gtg agc cac gaa gac cct gag gtc aag      240
   Val Thr Cys Val Val Val Asp Val Ser His Glu Asp Pro Glu Val Lys
       65                  70                  75
   ttc aac tgg tac gtg gac ggc gtg gag gtg cat aat gcc aag aca aag      288
   Phe Asn Trp Tyr Val Asp Gly Val Glu Val His Asn Ala Lys Thr Lys
   80                  85                  90                  95
   ccg cgg gag gag cag tac aac agc acg tac cgt gtg gtc agc gtc ctc      336
   Pro Arg Glu Glu Gln Tyr Asn Ser Thr Tyr Arg Val Val Ser Val Leu
                   100                 105                 110
   acc gtc ctg cac cag gac tgg ctg aat ggc aag gag tac aag tgc aag      384
   Thr Val Leu His Gln Asp Trp Leu Asn Gly Lys Glu Tyr Lys Cys Lys
               115                 120                 125
   gtc tcc aac aaa gcc ctc cca gcc ccc atc gag aaa acc atc tcc aaa      432
   Val Ser Asn Lys Ala Leu Pro Ala Pro Ile Glu Lys Thr Ile Ser Lys
           130                 135                 140
   gcc aaa ggg cag ccc cga gaa cca cag gtg tac acc ctg ccc cca tcc      480
   Ala Lys Gly Gln Pro Arg Glu Pro Gln Val Tyr Thr Leu Pro Pro Ser
       145                 150                 155
   cgg gat gag ctg acc aag aac cag gtc agc ctg acc tgc ctg gtc aaa      528
   Arg Asp Glu Leu Thr Lys Asn Gln Val Ser Leu Thr Cys Leu Val Lys
   160                 165                 170                 175
   ggc ttc tat ccc agc gac atc gcc gtg gag tgg gag agc aat ggg cag      576
   Gly Phe Tyr Pro Ser Asp Ile Ala Val Glu Trp Glu Ser Asn Gly Gln
                   180                 185                 190
   ccg gag aac aac tac aag acc acg cct ccc gtg ctg gac tcc gac ggc      624
   Pro Glu Asn Asn Tyr Lys Thr Thr Pro Pro Val Leu Asp Ser Asp Gly
           195                 200                 205
   tcc ttc ttc ctc tac agc aag ctc acc gtg gac aag agc agg tgg cag        672
   Ser Phe Phe Leu Tyr Ser Lys Leu Thr Val Asp Lys Ser Arg Trp Gln
       210                 215                 220
   cag ggg aac gtc ttc tca tgc tcc gtg atg cat gag gct ctg cac aac        720
   Gln Gly Asn Val Phe Ser Cys Ser Val Met His Glu Ala Leu His Asn
       225                 230                 235
   cac tac acg cag aag agc ctc tcc ctg tct ccg ggt aaa taactcgagg         769
   His Tyr Thr Gln Lys Ser Leu Ser Leu Ser Pro Gly Lys
   240                 245                 250
   atcc                                                                   773
<210> 1066
   <211> 252
   <212> PRT
   <213> Artificial Sequence
<220>
   <223> Synthetic Construct
<400> 1066
   Met Val Glu Pro Asn Cys Asp Ile His Val Met Trp Glu Trp Glu Cys
   1               5                   10                  15
   Phe Glu Arg Leu Gly Gly Gly Gly Gly Asp Lys Thr His Thr Cys Pro
               20                  25                  30
   Pro Cys Pro Ala Pro Glu Leu Leu Gly Gly Pro Ser Val Phe Leu Phe
           35                  40                  45
   Pro Pro Lys Pro Lys Asp Thr Leu Met Ile Ser Arg Thr Pro Glu Val
       50                  55                  60
   Thr Cys Val Val Val Asp Val Ser His Glu Asp Pro Glu Val Lys Phe
   65                  70                  75                  80
   Asn Trp Tyr Val Asp Gly Val Glu Val His Asn Ala Lys Thr Lys Pro
                   85                  90                  95
   Arg Glu Glu Gln Tyr Asn Ser Thr Tyr Arg Val Val Ser Val Leu Thr
               100                 105                 110
   Val Leu His Gln Asp Trp Leu Asn Gly Lys Glu Tyr Lys Cys Lys Val
           115                 120                 125
   Ser Asn Lys Ala Leu Pro Ala Pro Ile Glu Lys Thr Ile Ser Lys Ala
       130                 135                 140
   Lys Gly Gln Pro Arg Glu Pro Gln Val Tyr Thr Leu Pro Pro Ser Arg
   145 150 155 160
   Asp Glu Leu Thr Lys Asn Gln Val Ser Leu Thr Cys Leu Val Lys Gly
   165 170 175
   Phe Tyr Pro Ser Asp Ile Ala Val Glu Trp Glu Ser Asn Gly Gln Pro
   180 185 190
   Glu Asn Asn Tyr Lys Thr Thr Pro Pro Val Leu Asp Ser Asp Gly Ser
   195 200 205
   Phe Phe Leu Tyr Ser Lys Leu Thr Val Asp Lys Ser Arg Trp Gln Gln
   210 215 220
   Gly Asn Val Phe Ser Cys Ser Val Met His Glu Ala Leu His Asn His
   225 230 235 240
   Tyr Thr Gln Lys Ser Leu Ser Leu Ser Pro Gly Lys
   245 250
<210> 1067
   <211> 748
   <212> DNA
   <213> Artificial Sequence
<220>
   <223> Fc MMP INHIBITOR
<220>
   <221> CDS
   <222> (4)..(732)
<400> 1067
   cat atg gac aaa act cac aca tgt cca cct tgt cca gct ccg gaa ctc        48
       Met Asp Lys Thr His Thr Cys Pro Pro Cys Pro Ala Pro Glu Leu
       1               5                   10                  15
   ctg ggg gga ccg tca gtc ttc ctc ttc ccc cca aaa ccc aag gac acc        96
   Leu Gly Gly Pro Ser Val Phe Leu Phe Pro Pro Lys Pro Lys Asp Thr
                   20                  25                  30
   ctc atg atc tcc cgg acc cct gag gtc aca tgc gtg gtg gtg gac gtg       144
   Leu Met Ile Ser Arg Thr Pro Glu Val Thr Cys Val Val Val Asp Val
               35                  40                  45
   agc cac gaa gac cct gag gtc aag ttc aac tgg tac gtg gac ggc gtg       192
   Ser His Glu Asp Pro Glu Val Lys Phe Asn Trp Tyr Val Asp Gly Val
           50                  55                  60
   gag gtg cat aat gcc aag aca aag ccg cgg gag gag cag tac aac agc       240
   Glu Val His Asn Ala Lys Thr Lys Pro Arg Glu Glu Gln Tyr Asn Ser
       65                  70                  75
   acg tac cgt gtg gtc agc gtc ctc acc gtc ctg cac cag gac tgg ctg       288
   Thr Tyr Arg Val Val Ser Val Leu Thr Val Leu His Gln Asp Trp Leu
   80                  85                  90                  95
   aat ggc aag gag tac aag tgc aag gtc tcc aac aaa gcc ctc cca gcc       336
   Asn Gly Lys Glu Tyr Lys Cys Lys Val Ser Asn Lys Ala Leu Pro Ala
                   100                 105                 110
   ccc atc gag aaa acc atc tcc aaa gcc aaa ggg cag ccc cga gaa cca       384
   Pro Ile Glu Lys Thr Ile Ser Lys Ala Lys Gly Gln Pro Arg Glu Pro
               115                 120                 125
   cag gtg tac acc ctg ccc cca tcc cgg gat gag ctg acc aag aac cag       432
   Gln Val Tyr Thr Leu Pro Pro Ser Arg Asp Glu Leu Thr Lys Asn Gln
           130                 135                 140
   gtc agc ctg acc tgc ctg gtc aaa ggc ttc tat ccc agc gac atc gcc       480
   Val Ser Leu Thr Cys Leu Val Lys Gly Phe Tyr Pro Ser Asp Ile Ala
       145                 150                 155
   gtg gag tgg gag agc aat ggg cag ccg gag aac aac tac aag acc acg       528
   Val Glu Trp Glu Ser Asn Gly Gln Pro Glu Asn Asn Tyr Lys Thr Thr
   160                 165                 170                 175
   cct ccc gtg ctg gac tcc gac ggc tcc ttc ttc ctc tac agc aag ctc       576
   Pro Pro Val Leu Asp Ser Asp Gly Ser Phe Phe Leu Tyr Ser Lys Leu
                   180                 185                 190
   acc gtg gac aag agc agg tgg cag cag ggg aac gtc ttc tca tgc tcc       624
   Thr Val Asp Lys Ser Arg Trp Gln Gln Gly Asn Val Phe Ser Cys Ser
               195                 200                 205
   gtg atg cat gag gct ctg cac aac cac tac acg cag aag agc ctc tcc       672
   Val Met His Glu Ala Leu His Asn His Tyr Thr Gln Lys Ser Leu Ser
           210                 215                 220
   ctg tct ccg ggt aaa ggt gga ggt ggt ggt tgc acc acc cac tgg ggt       720
   Leu Ser Pro Gly Lys Gly Gly Gly Gly Gly Cys Thr Thr His Trp Gly
       225                 230                 235
   ttc acc ctg tgc taatggatcc ctcgag                                     748
   Phe Thr Leu Cys
   240
<210> 1068
   <211> 243
   <212> PRT
   <213> Artificial Sequence
<220>
   <223> Synthetic Construct
<400> 1068
   Met Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Lys Phe Asn Trp Tyr Val Asp Gly Val Glu
   50 55 60
   Val His Asn Ala Lys Thr Lys Pro Arg Glu Glu Gln Tyr Asn Ser Thr
   65 70 75 80
   Tyr Arg Val Val Ser Val Leu Thr Val Leu His Gln Asp Trp Leu Asn
   85 90 95
   Gly Lys Glu Tyr Lys Cys Lys Val Ser Asn Lys Ala Leu Pro Ala Pro
   100 105 110
   Ile Glu Lys Thr Ile Ser Lys Ala Lys Gly Gln Pro Arg Glu Pro Gln
   115 120 125
   Val Tyr Thr Leu Pro Pro Ser Arg Asp Glu Leu Thr Lys Asn Gln Val
   130 135 140
   Ser Leu Thr Cys Leu Val Lys Gly Phe Tyr Pro Ser Asp Ile Ala Val
   145 150 155 160
   Glu Trp Glu Ser Asn Gly Gln Pro Glu Asn Asn Tyr Lys Thr Thr Pro
   165 170 175
   Pro Val Leu Asp Ser Asp Gly Ser Phe Phe Leu Tyr Ser Lys Leu Thr
   180 185 190
   Val Asp Lys Ser Arg Trp Gln Gln Gly Asn Val Phe Ser Cys Ser Val
   195 200 205
   Met His Glu Ala Leu His Asn His Tyr Thr Gln Lys Ser Leu Ser Leu
   210 215 220
   Ser Pro Gly Lys Gly Gly Gly Gly Gly Cys Thr Thr His Trp Gly Phe
   225 230 235 240
   Thr Leu Cys
<210> 1069
   <211> 763
   <212> DNA
   <213> Artificial Sequence
<220>
   <223> MMP INHIBITOR-Fc
<220>
   <221> CDS
   <222> (4)..(753)
<400> 1069
   cat atg tgc acc acc cac tgg ggt ttc acc ctg tgc ggt gga ggc ggt      48
       Met Cys Thr Thr His Trp Gly Phe Thr Leu Cys Gly Gly Gly Gly
       1               5                   10                  15
   ggg gac aaa ggt gga ggc ggt ggg gac aaa act cac aca tgt cca cct      96
   Gly Asp Lys Gly Gly Gly Gly Gly Asp Lys Thr His Thr Cys Pro Pro
                   20                  25                  30
   tgc cca gca cct gaa ctc ctg ggg gga ccg tca gtt ttc ctc ttc ccc     144
   Cys Pro Ala Pro Glu Leu Leu Gly Gly Pro Ser Val Phe Leu Phe Pro
               35                  40                  45
   cca aaa ccc aag gac acc ctc atg atc tcc cgg acc cct gag gtc aca     192
   Pro Lys Pro Lys Asp Thr Leu Met Ile Ser Arg Thr Pro Glu Val Thr
           50                  55                  60
   tgc gtg gtg gtg gac gtg agc cac gaa gac cct gag gtc aag ttc aac     240
   Cys Val Val Val Asp Val Ser His Glu Asp Pro Glu Val Lys Phe Asn
       65                  70                  75
   tgg tac gtg gac ggc gtg gag gtg cat aat gcc aag aca aag ccg cgg     288
   Trp Tyr Val Asp Gly Val Glu Val His Asn Ala Lys Thr Lys Pro Arg
   80                  85                  90                  95
   gag gag cag tac aac agc acg tac cgt gtg gtc agc gtc ctc acc gtc     336
   Glu Glu Gln Tyr Asn Ser Thr Tyr Arg Val Val Ser Val Leu Thr Val
                   100                 105                 110
   ctg cac cag gac tgg ctg aat ggc aag gag tac aag tgc aag gtc tcc     384
   Leu His Gln Asp Trp Leu Asn Gly Lys Glu Tyr Lys Cys Lys Val Ser
               115                 120                 125
   aac aaa gcc ctc cca gcc ccc atc gag aaa acc atc tcc aaa gcc aaa     432
   Asn Lys Ala Leu Pro Ala Pro Ile Glu Lys Thr Ile Ser Lys Ala Lys
           130                 135                 140
   ggg cag ccc cga gaa cca cag gtg tac acc ctg ccc cca tcc cgg gat     480
   Gly Gln Pro Arg Glu Pro Gln Val Tyr Thr Leu Pro Pro Ser Arg Asp
       145                 150                 155
   gag ctg acc aag aac cag gtc agc ctg acc tgc ctg gtc aaa ggc ttc     528
   Glu Leu Thr Lys Asn Gln Val Ser Leu Thr Cys Leu Val Lys Gly Phe
   160                 165                 170                 175
   tat ccc agc gac atc gcc gtg gag tgg gag agc aat ggg cag ccg gag     576
   Tyr Pro Ser Asp Ile Ala Val Glu Trp Glu Ser Asn Gly Gln Pro Glu
                   180                 185                 190
   aac aac tac aag acc acg cct ccc gtg ctg gac tcc gac ggc tcc ttc     624
   Asn Asn Tyr Lys Thr Thr Pro Pro Val Leu Asp Ser Asp Gly Ser Phe
               195                 200                 205
   ttc ctc tac agc aag ctc acc gtg gac aag agc agg tgg cag cag ggg     672
   Phe Leu Tyr Ser Lys Leu Thr Val Asp Lys Ser Arg Trp Gln Gln Gly
           210                 215                 220
   aac gtc ttc tca tgc tcc gtg atg cat gag gct ctg cac aac cac tac     720
   Asn Val Phe Ser Cys Ser Val Met His Glu Ala Leu His Asn His Tyr
       225                 230                 235
   acg cag aag agc ctc tcc ctg tct ccg ggt aaa taatggatcc              763
                   Thr Gln Lys Ser Leu Ser Leu Ser Pro Gly Lys
                   240                 245                 250
<210> 1070
   <211> 250
   <212> PRT
   <213> Artificial Sequence
<220>
   <223> Synthetic Construct
<400> 1070
   Met Cys Thr Thr His Trp Gly Phe Thr Leu Cys Gly Gly Gly Gly Gly
   1               5                   10                  15
   Asp Lys Gly Gly Gly Gly Gly Asp Lys Thr His Thr Cys Pro Pro Cys
               20                  25                  30
   Pro Ala Pro Glu Leu Leu Gly Gly Pro Ser Val Phe Leu Phe Pro Pro
           35                  40                  45
   Lys Pro Lys Asp Thr Leu Met Ile Ser Arg Thr Pro Glu Val Thr Cys
       50                  55                  60
   Val Val Val Asp Val Ser His Glu Asp Pro Glu Val Lys Phe Asn Trp
   65                  70                  75                  80
   Tyr Val Asp Gly Val Glu Val His Asn Ala Lys Thr Lys Pro Arg Glu
                   85                  90                  95
   Glu Gln Tyr Asn Ser Thr Tyr Arg Val Val Ser Val Leu Thr Val Leu
               100                 105                 110
   His Gln Asp Trp Leu Asn Gly Lys Glu Tyr Lys Cys Lys Val Ser Asn
           115                 120                 125
   Lys Ala Leu Pro Ala Pro Ile Glu Lys Thr Ile Ser Lys Ala Lys Gly
       130                 135                 140
   Gln Pro Arg Glu Pro Gln Val Tyr Thr Leu Pro Pro Ser Arg Asp Glu
   145                 150                 155                 160
   Leu Thr Lys Asn Gln Val Ser Leu Thr Cys Leu Val Lys Gly Phe Tyr
                   165                 170                 175
   Pro Ser Asp Ile Ala Val Glu Trp Glu Ser Asn Gly Gln Pro Glu Asn
               180                 185                 190
   Asn Tyr Lys Thr Thr Pro Pro Val Leu Asp Ser Asp Gly Ser Phe Phe
           195                 200                 205
   Leu Tyr Ser Lys Leu Thr Val Asp Lys Ser Arg Trp Gln Gln Gly Asn
       210                 215                 220
   Val Phe Ser Cys Ser Val Met His Glu Ala Leu His Asn His Tyr Thr
   225                 230                 235                 240
   Gln Lys Ser Leu Ser Leu Ser Pro Gly Lys
                   245                 250
<210> 1071
   <211> 13
   <212> PRT
   <213> Artificial Sequence
<220>
   <223> INTEGRIN-BINDING PEPTIDE
<400> 1071
   Cys Gly Arg Glu Cys Pro Arg Leu Cys Gln Ser Ser Cys
   1               5                   10
<210> 1072
   <211> 13
   <212> PRT
   <213> Artificial Sequence
<220>
   <223> INTEGRIN-BINDING PEPTIDE
<400> 1072
   Cys Asn Gly Arg Cys Val Ser Gly Cys Ala Gly Arg Cys
   1               5                   10
<210> 1073
   <211> 8
   <212> PRT
   <213> Artificial Sequence
<220>
   <223> INTEGRIN-BINDING PEPTIDE
<400> 1073
   Cys Leu Ser Gly Ser Leu Ser Cys
   1               5
<210> 1074
   <211> 6
   <212> PRT
   <213> Artificial Sequence

<220>
   <223> INTEGRIN-BINDING PEPTIDE
<400> 1074
   Asn Gly Arg Ala His Ala
   1 5
<210> 1075
   <211> 5
   <212> PRT
   <213> Artificial Sequence
<220>
   <223> INTEGRIN-BINDING PEPTIDE
<400> 1075
   Cys Asn Gly Arg Cys
   1 5
<210> 1076
   <211> 9
   <212> PRT
   <213> Artificial Sequence
<220>
   <223> INTEGRIN-BINDING PEPTIDE
<400> 1076
   Cys Asp Cys Arg Gly Asp Cys Phe Cys
   1 5
<210> 1077
   <211> 7
   <212> PRT
   <213> Artificial Sequence
<220>
   <223> INTEGRIN-BINDING PEPTIDE
<400> 1077
   Cys Gly Ser Leu Val Arg Cys
   1 5
<210> 1078
   <211> 8
   <212> PRT
   <213> Artificial Sequence
<220>
   <223> INTEGRIN-BINDING PEPTIDE
<400> 1078
   Arg Thr Asp Leu Asp Ser Leu Arg
   1 5
<210> 1079
   <211> 12
   <212> PRT
   <213> Artificial Sequence
<220>
   <223> INTEGRIN-BINDING PEPTIDE
<400> 1079
   Gly Asp Leu Asp Leu Leu Lys Leu Arg Leu Thr Leu
   1 5 10
<210> 1080
   <211> 12
   <212> PRT
   <213> Artificial Sequence
<220>
   <223> INTEGRIN-BINDING PEPTIDE
<400> 1080
   Gly Asp Leu His Ser Leu Arg Gln Leu Leu Ser Arg
   1 5 10
<210> 1081
   <211> 12
   <212> PRT
   <213> Artificial Sequence
<220>
   <223> INTEGRIN-BINDING PEPTIDE
<400> 1081
   Arg Asp Asp Leu His Met Leu Arg Leu Gln Leu Trp
   1 5 10
<210> 1082
   <211 > 12
   <212> PRT
   <213> Artificial Sequence
<220>
   <223> INTEGRIN-BINDING PEPTIDE
<400> 1082
   Ser Ser Asp Leu His Ala Leu Lys Lys Arg Tyr Gly
   1 5 10
<210> 1083
   <211> 12
   <212> PRT
   <213> Artificial Sequence
<220>
   <223> INTEGRIN-BINDING PEPTIDE
<400> 1083
   Arg Gly Asp Leu Lys Gln Leu Ser Glu Leu Thr Trp
   1 5 10
<210> 1084
   <211> 12
   <212> PRT
   <213> Artificial Sequence
<220>
   <223> INTEGRIN-BINDING PEPTIDE
<400> 1084
   Arg Gly Asp Leu Ala Ala Leu Ser Ala Pro Pro Val
   1 5 10
<210> 1085
   <211 > 20
   <212> PRT
   <213> Artificial Sequence
<220>
   <223> VEGF-ANTAGONIST
<400> 1085
   Arg Gly Trp Val Glu Ile Cys Val Ala Asp Asp Asn Gly Met Cys Val
   1 5 10 15
   Thr Glu Ala Gln
   20
<210> 1086
   <211> 19
   <212> PRT
   <213> Artificial Sequence
<220>
   <223> VEGF-ANTAGONIST
<400> 1086
   Gly Trp Asp Glu Cys Asp Val Ala Arg Met Trp Glu Trp Glu Cys Phe
   1 5 10 15
   Ala Gly Val
<210> 1087
   <211> 16
   <212> PRT
   <213> Artificial Sequence
<220>
   <223> VEGF-ANTAGONIST
<400> 1087
   Arg Gly Trp Val Glu Ile Cys Glu Ser Asp Val Trp Gly Arg Cys Leu
   1 5 10 15
<210>1088
   <211> 16
   <212> PRT
   <213> Artificial Sequence
<220>
   <223> VEGF-ANTAGONIST
<400> 1088
   Arg Gly Trp Val Glu Ile Cys Glu Ser Asp Val Trp Gly Arg Cys Leu
   1 5 10 15
<210> 1089
   <211> 19
   <212> PRT
   <213> Artificial Sequence
<220>
   <223> VEGF-ANTAGONIST
<400> 1089
   Gly Gly Asn Glu Cys Asp Ile Ala Arg Met Trp Glu Trp Glu Cys Phe
   1 5 10 15
   Glu Arg Leu
<210> 1090
   <211> 16
   <212> PRT
   <213> Artificial Sequence
<220>
   <223> VEGF-ANTAGONIST
<400> 1090
   Arg Gly Trp Val Glu Ile Cys Ala Ala Asp Asp Tyr Gly Arg Cys Leu
   1 5 10 15
<210> 1091
   <211> 8
   <212> PRT
   <213> Artificial Sequence
<220>
   <223> MMP INHIBITOR
<220>
   <221> misc_feature
   <222> (6)..(6)
   <223> Xaa = any amino acid
<400> 1091
   Cys Leu Arg Ser Gly Xaa Gly Cys
   1 5
<210> 1092
   <211> 10
   <212> PRT
   <213> Artificial Sequence
<220>
   <223> MMP INHIBITOR
<220>
   <221> misc_feature
   <222> (2, 3, 8)..(9)
   <223> Xaa = any amino acid.
<400> 1092
   Cys Xaa Xaa His Trp Gly Phe Xaa Xaa Cys
   1 5 10
<210> 1093
   <211> 5
   <212> PRT
   <213> Artificial Sequence
<220>
   <223> MMP INHIBITOR
<220>
   <221> misc_feature
   <222> (2).. (2)
   <223> Xaa = any amino acid
<220>
   <221> > misc_feature
   <222> (4)..(4)
   <223> Xaa = any amino acid
<400> 1093
   Cys Xaa Pro Xaa Cys
   1 5
<210> 1094
   <211> 10
   <212> PRT
   <213> Artificial Sequence
<220>
   <223> MMP INHIBITOR
<400> 1094
   Cys Arg Arg His Trp Gly Phe Glu Phe Cys
   1 5 10
<210> 1095
   <211> 10
   <212> PRT
   <213> Artificial Sequence
<220>
   <223> MMP INHIBITOR
<400> 1095
   Ser Thr Thr His Trp Gly Phe Thr Leu Ser
   1 5 10
<210> 1096
   <211> 10
   <212> PRT
   <213> Artificial Sequence
<220>
   <223> CTLA4-MIMETIC PEPTIDE
<400> 1096
   Cys Ser Leu His Trp Gly Phe Trp Trp Cys
   1 5 10
<210> 1097
   <211> 15
   <212> PRT
   <213> Artificial Sequence
<220>
   <223> CARBOHYDRATE (GD1 ALPHA) MIMETIC PEPTIDE
<400> 1097
   Trp His Trp Arg His Arg Ile Pro Leu Gln Leu Ala Ala Gly Arg
   1 5 10 15
<210> 1098
   <211 6
   <212> PRT
   <213> Artificial Sequence
<220>
   <223> BETA-2GPI AB BINDING PEPTIDE
<400> 1098
<210> 1099
   <211> 8
   <212> PRT
   <213> Artificial Sequence
<220>
   <223> BETA-2GPI AB BINDING PEPTIDE
<400> 1099
   Asn Thr Leu Lys Thr Pro Arg Val
   1 5
<210> 1100
   <211> 11
   <212> PRT
   <213> Artificial Sequence
<220>
   <223> BETA-2GPI AB BINDING PEPTIDE
<400> 1100
   Asn Thr Leu Lys Thr Pro Arg Val Gly Gly Cys
   1 5 10
<210>1101
   <211> 6
   <212> PRT
   <213> Artificial Sequence
<220>
   <223> BETA-2GPI AB BINDING PEPTIDE
<400> 1101
   Lys Asp Lys Ala Thr Phe
   1 5
<210> 1102
   <211> 10
   <212> PRT
   <213> Artificial Sequence
<220>
   <223> BETA-2GPI AB BINDING PEPTIDE
<400> 1102
   Lys Asp Lys Ala Thr Phe Gly Cys His Asp
   1 5 10
<210> 1103
   <211> 12
   <212> PRT
   <213> Artificial Sequence
<220>
   <223> BETA-2GPI AB BINDING PEPTIDE
<400> 1103
   Lys Asp Lys Ala Thr Phe Gly Cys His Asp Gly Cys
   1 5 10
<210> 1104
   <211> 6
   <212> PRT
   <213> Artificial Sequence
<220>
   <223> BETA-2GPI AB BINDING PEPTIDE
<400> 1104
   Thr Leu Arg Val Tyr Lys
   1 5
<210> 1105
   <211> 9
   <212> PRT
   <213> Artificial Sequence
<220>
   <223> BETA-2GPI AB BINDING PEPTIDE
<400> 1105
   Ala Thr Leu Arg Val Tyr Lys Gly Gly
   1 5
<210> 1106
   <211> 10
   <212> PRT
   <213> Artificial Sequence
<220>
   <223> BETA-2GPI AB BINDING PEPTIDE
<400> 1106
   Cys Ala Thr Leu Arg Val Tyr Lys Gly Gly
   1 5 10
<210> 1107
   <211> 14
   <212> PRT
   <213> Artificial Sequence
<220>
   <223> MEMBRANE-TRANSPORTING PEPTIDE
<400> 1107
   Ile Asn Leu Lys Ala Leu Ala Ala Leu Ala Lys Lys Ile Leu
   1 5 10
<210> 1108
   <211> 12
   <212> PRT
   <213> Artificial Sequence
<220>
   <223> MEMBRANE-TRANSPORTING PEPTIDE
<400> 1108
   Gly Trp Thr Leu Asn Ser Ala Gly Tyr Leu Leu Gly
   1 5 10
<210> 1109
   <211> 27
   <212> PRT
   <213> Artificial Sequence
<220>
   <223> MEMBRANE-TRANSPORTING PEPTIDE
<400> 1109
   Gly Trp Thr Leu Asn Ser Ala Gly Tyr Leu Leu Gly Lys Ile Asn Leu
   1 5 10 15
   Lys Ala Leu Ala Ala Leu Ala Lys Lys Ile Leu
   20 25
<210> 1110
   <211>
   <212>
   <213> No Sequence Here
<220>
   <223>
<400>
<210> 1111
   <211>
   <212>
   <213> No Sequence Here
<220>
   <223>
<400>
<210> 1112
   <211> 57
   <212> DNA
   <213> Artificial Sequence
<220>
   <223> OLIGONUCLEOTIDE USED TO CONSTRUCT VEGF MIMETIC PEPTIDE
<400> 1112
   gttgaaccga actgtgacat ccatgttatg tgggaatggg aatgttttga acgtctg 57
<210> 1115
   <211> 57
   <212> DNA
   <213> Artificial Sequence
<220>
   <223> VEGF ANTAGONIST CONSTRUCT
<220>
   <221 > CDS
   <222> (1)..(57)
<400> 1115
   gtt gaa ccg aac tgt gac atc cat gtt atg tgg gaa tgg gaa tgt ttt    48
   Val Glu Pro Asn Cys Asp Ile His Val Met Trp Glu Trp Glu Cys Phe
   1               5                   10                  15
   gaa cgt ctg                                                        57
   Glu Arg Leu
<210> 1116
   <211> 19
   <212> PRT
   <213> Artificial Sequence
<220>
   <223> Synthetic Construct
<400> 1116
   Val Glu Pro Asn Cys Asp Ile His Val Met Trp Glu Trp Glu Cys Phe
   1 5 10 15
   Glu Arg Leu
<210> 1117
   <211>
   <212>
   <213> No Sequence Here
<220>
   <223>
<400>
<210> 1118
   <211 > 63
   <212> DNA
   <213> Artificial Sequence
<220>
   <223> SENSE PCR PRIMER FOR MMP INHIBITORY PEPTIDE
<400> 1118
   gaataacata tgtgcaccac ccactggggt ttcaccctgt gcggtggagg cggtggggac 60
   aaa 63
<210>1119
   <211> 81
   <212> DNA
   <213> Artificial Sequence
<220>
   <223> SENSE PCR PRIMER FOR TNF-alpha INHIBITOR PEPTIDE
<400> 1119
   gaataacata tggacttcct gccgcactac aaaaacacct ctctgggtca ccgtccgggt 60
   ggaggcggtg gggacaaaac t 81
<210> 1120
   <211> 81
   <212> DNA
   <213> Artificial Sequence
<220>
   <223> ANTISENSE PCR PRIMER FOR Fc-LINKER CONSTRUCT
<400> 1120
   ccgcggatcc attacagcgg cagagcgtac ggctgccagt aacccggggt ccattcgaaa 60
   ccaccacctc cacctttacc c 81
<210> 1121
   <211> 81
   <212> DNA
   <213> Artificial Sequence
<220>
   <223> SENSE PCR PRIMER FOR IL-1 ANTAGONIST-Fc
<400> 1121
   gaataacata tgttcgaatg gaccccgggt tactggcagc cgtacgctct gccgctgggt 60
   ggaggcggtg gggacaaaac t 81
<210> 1122
   <211>
   <212>
   <213> No Sequence Here
<220>
   <223>
<400>
<210> 1123
   <211>
   <212>
   <213> No Sequence Here
<220>
   <223>
<400>
<210> 1124
   <211>
   <212>
   <213> No Sequence Here
<220>
   <223>
<400>
<210> 1125
   <211> 48
   <212> DNA
   <213> Artificial Sequence
<220>
   <223> SENSE PCR PRIMER FOR Fc CONSTRUCT
<400> 1125
   amgattct agaaggagga ataacatatg gacaaaactc acacatgt 48
<210> 1126
   <211> 51
   <212> DNA
   <213> Artificial Sequence
<220>
   <223> ANTI-SENSE PCR PRIMER FOR Fc CONSTRUCT
<400> 1126
   gtcacagttc ggttcaacac caccaccacc accmaccc ggagacaggg a 51
<210> 1127
   <211 > 54
   <212> DNA
   <213> Artificial Sequence
<220>
   <223> SENSE PCR PRIMER FOR VEGF ANTAGONIST CONSTRUCT
<400> 1127
   tccctgtctc cgggtaaagg tggtggtggt ggtgttgaac cgaactgtga catc 54
<210> 1128
   <211> 39
   <212> DNA
   <213> Artificial Sequence
<220>
   <223> ANTI-SENSE PCR PRIMER FOR VEGF ANTAGONIST CONSTRUCT
<400> 1128
   ccgcggatcc tcgagttaca gacgttcaaa acattccca 39
<210> 1129
   <211> > 48
   <212> DNA
   <213> Artificial Sequence
<220>
   <223> SENSE PCR PRIMER FOR VEGF ANTAGONIST CONSTRUCT
<400> 1129
   amgattct agaaggagga ataacatatg gttgaaccga actgtgac 48
<210> 1130
   <211> 51
   <212> DNA
   <213> Artificial Sequence
<220>
   <223> ANTI-SENSE PCR PRIMER FOR VEGF ANTAGONIST CONSTRUCT
<400> 1130
   acatgtgtga gttttgtcac caccaccacc acccagacgt tcaaaacatt c 51
<210> 1131
   <211> 51
   <212> DNA
   <213> Artificial Sequence
<220>
   <223> SENSE PCR PRIMER FOR Fc CONSTRUCT
<400> 1131
   gaatgttttg aacgtctggg tggtggtggt ggtgacaaaa ctcacacatg t 51
<210> 1132
   <211> 39
   <212> DNA
   <213> Artificial Sequence
<220>
   <223> ANTI-SENSE PCR PRIMER FOR Fc CONSTRUCT
<400> 1132
   ccgcggatcc tcgagttatt tacccggaga cagggagag 39
<210> 1133
   <211> 36
   <212> PRT
   <213> Artificial Sequence
<220>
   <223> TPO MIMETIC PEPTIDE CONSTRUCT
<220>
   <221> misc_feature
   <222> (1)..(1)
   <223> Butoxycarbonyl group attached to the amino terminus.
<220>
   <221> misc_feature
   <222> (2)..(2)
   <223> Tert-butyl group attached to the sidechain
<220>
   <221> misc_feature
   <222> (5)..(5)
   <223> Tert-butyl group attached to the sidechain
<220>
   <221> > misc_feature
   <222> (7)..(7)
   <223> 2,2,4,6,7-pendamethyldihydrobenzofuran-5-sulfonyl group attached to the sidechain
<220>
   <221> misc_feature
   <222> (8)..(8)
   <223> Trityl group attached to the sidechain.
<220>
   <221> misc_feature
   <222> (9)..(9)
   <223> Butoxycarbonyl group attached to the sidechain.
<220>
   <221> misc feature
   <222> (13)..(13)
   <223> 2,2,4,6,7-PENDAMETHYLDIHYDROBENZOFURAN-5-SULFONYL GROUP ATTACHED TO THE
   SIDECHAIN
<220>
   <221> misc_feature
   <222> (18)..(18)
   <223> 1-(4,4-dimethyl-2,6-dioxo-cyclohexylidene) ethyl group attached to the sidechain
<220>
   <221> misc_feature
   <222> (24)..(24)
   <223> Tert-butyl group attached to the sidechain
<220>
   <221> misc_feature
   <222> (27)..(27)
   <223> Tert-buty) group attached to the sidechain
<220>
   <221> > misc_feature
   <222> (29)..(29)
   <223> 2,2,4,6,7-PENDAMETHYLDIHYDROBENZOFURAN-5-SULFONYL GROUP ATTACHED TO THE
   SIDECHAIN
<220>
   <221> misc_feature
   <222> (30)..(30)
   <223> Trityl group attached to the sidechain
<220>
   <221> misc_feature
   <222> (31)..(31)
   <223> Butoxycarbonyl group attached to the sidechain.
<220>
   <221> misc_feature
   <222> (35)..(35)
   <223> 2,2,4,6,7-PENDAMETHYLDIHYDROBENZOFURAN-5-SULFONYL GROUP ATTACHED TO THE
   SIDECHAIN
<220>
   <221> misc_feature
   <222> (36)..(36)
   <223> Methoxy resin attached to the carboxyl terminus.
<400> 1133
   Ile Glu Gly Pro Thr Leu Arg Gln Trp Leu Ala Ala Arg Ala Gly Gly
   1               5                   10                  15
   Gly Lys Gly Gly Gly Gly Ile Glu Gly Pro Thr Leu Arg Gln Trp Leu
               20                  25                  30
   Ala Ala Arg Ala
           35
<210> 1134
   <211> 36
   <212> PRT
   <213> Artificial Sequence
<220>
   <223> TPO MIMETIC PEPTIDE CONSTRUCT
<220>
   <221> misc_feature
   <222> (1)..(1)
   <223> Butoxycarbonyl group attached to the amino terminus.
<220>
   <221 > misc_feature
   <222> (2)..(2)
   <223> Tert-butyl group attached to the sidechain
<220>
   <221> misc_feature
   <222> (5)..(5)
   <223> Tert-butyl group attached to the sidechain
<220>
   <221> misc_feature
   <222> (7)..(7)
   <223> 2,2,4,6,7-pendamethyldihydrobenzofuran-5-sulfonyl group attached to the sidechain
<220>
   <221> misc_feature
   <222> (8)..(8)
   <223> Trityl group attached to the sidechain.
<220>
   <221> misc_feature
   <222> (9)..(9)
   <223> Butoxycarbonyl group attached to the sidechain.
<220>
   <221> misc_feature
   <222> (13)..(13)
   <223> 2,2,4,6,7-pendamethyldihydrobenzofuran-5-sulfonyl group attached to the sidechain
<220>
   <221> misc_feature
   <222> (24)..(24)
   <223> Tert-butyl group attached to the sidechain
<220>
   <221> misc_feature
   <222> (27)..(27)
   <223> Tert-butyl group attached to the sidechain
<220>
   <221> misc_feature
   <222> (29)..(29)
   <223> 2,2,4,6,7-pendamethyldihydrobenzofuran-5-sulfonyl group attached to the sidechain
<220>
   <221> misc_feature
   <222> (30)..(30)
   <223> Trityl group attached to the sidechain.
<220>
   <221> misc_feature
   <222> (31)..(31)
   <223> Butoxycarbonyl group attached to the sidechain.
<220>
   <221> misc_feature
   <222> (35)..(35)
   <223> 2,2,4,6,7-pendamethyldihydrobenzofuran-5-sulfonyl group attached to the sidechain
<220>
   <221> misc_feature
   <222> (36)..(36)
   <223> Methoxy resin attached to the carboxyl terminus.
<400> 1134
   Ile Glu Gly Pro Thr Leu Arg Gln Trp Leu Ala Ala Arg Ala Gly Gly
   1               5                   10                  15
   Gly Lys Gly Gly Gly Gly Ile Glu Gly Pro Thr Leu Arg Gln Trp Leu
               20                  25                  30
   Ala Ala Arg Ala
           35
<210> 1135
   <211> 36
   <212> PRT
   <213> Artificial Sequence
<220>
   <223> TPO MIMETIC PEPTIDE CONSTRUCT
<220>
   <221> misc_feature
   <222> (1)..(1)
   <223> Butoxycarbonyl group attached to the amino terminus.
<220>
   <221> misc_feature
   <222> (2)..(2)
   <223> Tert-butyl group attached to the sidechain.
<220>
   <221> misc_feature
   <222> (5)..(5)
   <223> Tert-butyl group attached to the sidechain.
<220>
   <221> misc_feature
   <222> (7)..(7)
   <223> 2,2,4,6,7-pendamethyldihydrobenzofuran-5-sulfonyl group attached to the sidechain.
<220>
   <221> misc_feature
   <222> (8)..(8)
   <223> Trityl group attached to the sidechain.
<220>
   <221> misc_feature
   <222> (9)..(9)
   <223> Butoxycarbonyl group attached to the sidechain.
<220>
   <221> misc_feature
   <222> (13)..(13)
   <223> 2,2,4,6,7-pendamethyldihydrobenzofuran-5-sulfonyl group attached to the sidechain.
<220>
   <221> misc_feature
   <222> (18)..(18)
   <223> Bromoacetyl group attached to the sidechain.
<220>
   <221> > misc_feature
   <222> (24)..(24)
   <223> Tert-butyl group attached to the sidechain.
<220>
   <221> misc_feature
   <222> (27)..(27)
   <223> Tert-butyl group attached to the sidechain.
<220>
   <221> misc_feature
   <222> (29)..(29)
   <223> 2,2,4,6,7-pendamethyldihydrobenzofuran-5-sulfonyl group attached to the sidechain.
<220>
   <221> misc_feature
   <222> (30)..(30)
   <223> Trityl group attached to the sidechain.
<220>
   <221> > misc_feature
   <222> (31)..(31)
   <223> Butoxycarbonyl group attached to the sidechain.
<220>
   <221> misc_feature
   <222> (35)..(35)
   <223> 2,2,4,6,7-pendamethyldihydrobenzofuran-5-sulfonyl group attached to the sidechain.
<220>
   <221 > misc_feature
   <222> (36)..(36)
   <223> Methoxy resin attached to the carboxyl terminus.
<400> 1135
   Ile Glu Gly Pro Thr Leu Arg Gln Trp Leu Ala Ala Arg Ala Gly Gly
   1 5 10 15
   Gly Lys Gly Gly Gly Gly Ile Glu Gly Pro Thr Leu Arg Gln Trp Leu
   20 25 30
   Ala Ala Arg Ala
   35
<210> 1136
   <211> 36
   <212> PRT
   <213> Artificial Sequence
<220>
   <223> TPO MIMETIC PEPTIDE CONSTRUCT
<220>
   <221> misc_feature
   <222> (18)..(18)
   <223> Bromoacetyl group attached to the sidechain.
<400> 1136
   Ile Glu Gly Pro Thr Leu Arg Gln Trp Leu Ala Ala Arg Ala Gly Gly
   1 5 10 15
   Gly Lys Gly Gly Gly Gly Ile Glu Gly Pro Thr Leu Arg Gln Trp Leu
   20 25 30
   Ala Ala Arg Ala
   35
<210> 1137
   <211> 36
   <212> PRT
   <213> Artificial Sequence
<220>
   <223> TPO MIMETIC PEPTIDE CONSTRUCT

<220>
   <221> misc_feature
   <222> (2)..(2)
   <223> Tert-butyl group attached to the sidechain.
<220>
   <221> misc_feature
   <222> (5)..(5)
   <223> Tert-butyl group attached to the sidechain.
<220>
   <221> misc_feature
   <222> (7).. (7)
   <223> 2,2,4,6,7-pendamethyldihydrobenzofuran-5-sulfonyl group attached to the sidechain.
<220>
   <221> > misc_feature
   <222> (8)..(8)
   <223> Trityl group attached to the sidechain.
<220>
   <221> misc_feature
   <222> (9)..(9)
   <223> Butoxycarbonyl group attached to the sidechain.
<220> -
   <221> > misc_feature
   <222> (13)..(13)
   <223> 2,2,4,6,7-pendamethyldihydrobenzofuran-5-sulfonyl group attached to the sidechain.
<220>
   <221> > misc_feature
   <222> (18)..(18)
   <223> Trityl group attached to the sidechain.
<220>
   <221> misc_feature
   <222> (24)..(24)
   <223> Tert-butyl group attached to the sidechain.
<220>
   <221> misc_feature
   <222> (27)..(27)
   <223> Tert-butyl group attached to the sidechain.
<220>
   <221> misc_feature
   <222> (29)..(29)
   <223> 2,2,4,6,7-pendamethyldihydrobenzofuran-5-sulfonyl group attached to the sidechain.
<220>
   <221> misc_feature
   <222> (30)..(30)
   <223> Trityl group attached to the sidechain.
<220>
   <221> misc_feature
   <222> (31)..(31)
   <223> Butoxycarbonyl group attached to the sidechain.
<220>
   <221> misc_feature
   <222> (35)..(35)
   <223> 2,2,4,6,7-pendamethyldihydrobenzofuran-5-sulfonyl group attached to the sidechain.
<220>
   <221> misc_feature
   <222> (36)..(36)
   <223> Methoxy resin attached to the carboxyl terminus.
<400> 1137
   Ile Glu Gly Pro Thr Leu Arg Gln Trp Leu Ala Ala Arg Ala Gly Gly
   1               5                   10                  15
   Gly Cys Gly Gly Gly Gly Ile Glu Gly Pro Thr Leu Arg Gln Trp Leu
               20                  25                  30
   Ala Ala Arg Ala
           35
<210> 1138
   <211> 36
   <212> PRT
   <213> Artificial Sequence
<220>
   <223> TPO MIMETIC PEPTIDE CONSTRUCT
<400> 1138
   Ile Glu Gly Pro Thr Leu Arg Gln Trp Leu Ala Ala Arg Ala Gly Gly
   1               5                   10                  15
   Gly Cys Gly Gly Gly Gly Ile Glu Gly Pro Thr Leu Arg Gln Trp Leu
               20                  25                  30
   Ala Ala Arg Ala
           35
<210> 1139
   <211> 13
   <212> PRT
   <213> Artificial Sequence
<220>
   <223> SOMATOSTATIN OR CORTISTATIN MIMETIC PEPTIDE
<220>
   <221> MISC_FEATURE
   <222> (3)..(3)
   <223> Xaa is Arg or Lys
<220>
   <221> MISC_FEATURE
   <222> (11)..(11)
   <223> Xaa is Ser or Thr
<400> 1139
   Pro Cys Xaa Asn Phe Phe Trp Lys Thr Phe Xaa Ser Cys
   1               5                   10
<210> 1140
   <211> 14
   <212> PRT
   <213> Artificial Sequence
<220>
   <223> SOMATOSTATIN OR CORTISTATIN MIMETIC PEPTIDE
<220>
   <221> MISC_FEATURE
   <222> (4)..(4)
   <223> Xaa is Arg or Lys
<220>
   <221> MISC_FEATURE
   <222> (12)..(12)
   <223> Xaa is Ser or Thr
<400> 1140
   Met Pro Cys Xaa Asn Phe Phe Trp Lys Thr Phe Xaa Ser Cys
   1 5 10
<210> 1141
   <211> 15
   <212> PRT
   <213> Artificial Sequence
<220>
   <223> SOMATOSTATIN OR CORTISTATIN MIMETIC PEPTIDE
<220>
   <221> MISC_FEATURE
   <222> (5).. (5)
   <223> Xaa is Arg or Lys
<220>
   <221> MISC_FEATURE
   <222> (13)..(13)
   <223> Xaa is Ser or Thr
<400> 1141
   Arg Met Pro Cys Xaa Asn Phe Phe Trp Lys Thr Phe Xaa Ser Cys
   1 5 10 15
<210> 1142
   <211> 16
   <212> PRT
   <213> Artificial Sequence
<220>
   <223> SOMATOSTATIN OR CORTISTATIN MIMETIC PEPTIDE
<220>
   <221> MISC_FEATURE
   <222> (6)..(6)
   <223> Xaa is Arg or Lys
<220>
   <221> MISC_FEATURE
   <222> (14)..(14)
   <223> Xaa is Ser or Thr
<400> 1142
   Asp Arg Met Pro Cys Xaa Asn Phe Phe Trp Lys Thr Phe Xaa Ser Cys
   1               5                   10                  15
<210> 1143
   <211> 13
   <212> PRT
   <213> Artificial Sequence
<220>
   <223> SOMATOSTATIN OR CORTISTATIN MIMETIC PEPTIDE
<220>
   <221> MISC_FEATURE
   <222> (2)..(2)
   <223> Xaa is Arg or Lys
<220>
   <221> MISC_FEATURE
   <222> (10)..(10)
   <223> Xaa is Ser or Thr
<400> 1143
   Cys Xaa Asn Phe Phe Trp Lys Thr Phe Xaa Ser Cys Lys
   1               5                   10
<210> 1144
   <211> 14
   <212> PRT
   <213> Artificial Sequence
<220>
   <223> SOMATOSTATIN OR CORTISTATIN MIMETIC PEPTIDE
<220>
   <221> MISC_FEATURE
   <222> (3)..(3)
   <223> Xaa is Arg or Lys
<220>
   <221> MISC_FEATURE
   <222> (11)..(11)
   <223> Xaa is Ser or Thr
<400> 1144
   Pro Cys Xaa Asn Phe Phe Trp Lys Thr Phe Xaa Ser Cys Lys
   1               5                   10
<210> 1145
   <211> 15
   <212> PRT
   <213> Artificial Sequence
<220>
   <223> SOMATOSTATIN OR CORTISTATIN MIMETIC PEPTIDE
<220>
   <221> MISC_FEATURE
   <222> (4)..(4)
   <223> Xaa is Arg or Lys
<220>
   <221> MISC_FEATURE
   <222> (12)..(12)
   <223> Xaa is Ser or Thr
<400> 1145
   Met Pro Cys Xaa Asn Phe Phe Trp Lys Thr Phe Xaa Ser Cys Lys
   1 5 10 15
<210> 1146
   <211> 16
   <212> PRT
   <213> Artificial Sequence
<220>
   <223> SOMATOSTATIN OR CORTISTATIN MIMETIC PEPTIDE
<220>
   <221> MISC_FEATURE
   <222> (5).. (5)
   <223> Xaa is Arg or Lys
<220>
   <221> MISC_FEATURE
   <222> (13)..(13)
   <223> Xaa is Ser or Thr
<400> 1146
   Arg Met Pro Cys Xaa Asn Phe Phe Trp Lys Thr Phe Xaa Ser Cys Lys
   1 5 10 15
<210> 1147
   <211> 17
   <212> PRT
   <213> Artificial Sequence
<220>
   <223> SOMATOSTATIN OR CORTISTATIN MIMETIC PEPTIDE
<220>
   <221> MISC_FEATURE
   <222> (6)..(6)
   <223> Xaa is Arg or Lys
<220>
   <221> MISC_FEATURE
   <222> (14)..(14)
   <223> Xaa is Ser or Thr
<400> 1147
   Asp Arg Met Pro Cys Xaa Asn Phe Phe Trp Lys Thr Phe Xaa Ser Cys
   1 5 10 15
   Lys
<210> 1148
   <211> 6
   <212> PRT
   <213> Artificial Sequence
<220>
   <223> SOMATOSTATIN OR CORTISTATIN MIMETIC PEPTIDE
<220>
   <221 > MISC_FEATURE
   <222> (1)..(1)
   <223> Xaa is a hydrophobic aliphatic amino acid residue
<220>
   <221> MISC_FEATURE
   <222> (4).. (4)
   <223> Xaa is a hydrophobic aliphatic amino acid residue
<400> 1148
   Xaa Asn Ser Xaa Leu Asn
   1 5

## Description

### Background of the Invention

Recombinant proteins are an emerging class of therapeutic agents. Such recombinant therapeutics have engendered advances in protein formulation and chemical modification. Such modifications can protect therapeutic proteins, primarily by blocking their exposure to proteolytic enzymes. Protein modifications may also increase the therapeutic protein's stability, circulation time, and biological activity. A review article describing protein modification and fusion proteins is Francis (1992), Focus on Growth Factors 3:4-10 (Mediscript, London), which is hereby incorporated by reference.

One useful modification is combination with the "Fc" domain of an antibody. Antibodies comprise two functionally independent parts, a variable domain known as "Fab", which binds antigen, and a constant domain known as "Fc", which links to such effector functions as complement activation and attack by phagocytic cells. An Fc has a long serum half-life, whereas an Fab is short-lived. Capon et al. (1989), Nature 337: 525-31. When constructed together with a therapeutic protein, an Fc domain can provide longer half-life or incorporate such functions as Fc receptor binding, protein A binding, complement fixation and perhaps even placental transfer. Id. Table 1 summarizes use of Fc fusions known in the art.

**Table 1-Fc fusion with therapeutic proteins**

| **Form of Fc** | **Fusion partner** | **Therapeutic implications** | **Reference** |
|---|---|---|---|
| IgG1 | N-terminus of CD30-L | Hodgkin's disease; anaplastic lymphoma; T-cell leukemia | U.S. Patent No. 5,480,981 |
| Murine Fcγ2a | IL-10 | anti-inflammatory; transplant rejection | Zheng et al. (1995), J. Immunol. 154: 5590-600 |
| IgG1 | TNF receptor | septic shock | Fisher et al. (1996), N. Engl. J. Med. 334:1697-1702; Van Zee, K. et al. (1996), J. Immunol. 156: 2221-30 |
| IgG, IgA, IgM, or IgE (excluding the first domain) | TNF receptor | inflammation, autoimmune disorders | U.S. Pat. No. 5,808,029, issued September 15, 1998 |
| IgG1 | CD4 receptor | AIDS | Capon et al. (1989), Nature 337: 525-31 |
| IgG1, IgG3 | N-terminus of IL-2 | anti-cancer, antiviral | Harvill et al. (1995), Immunotech. 1: 95-105 |
| IgG1 | C-terminus of OPG | osteoarthritis; bone density | WO 97/23614, published July 3, 1997 |
| IgG1 | N-terminus of leptin | anti-obesity | PCT/US 97/23183, filed December 11, 1997 |
| Human Ig Cγ1 | CTLA-4 | autoimmune disorders | Linsley (1991), J. Exp. Med. 174:561-9 |

A much different approach to development of therapeutic agents is peptide library screening. The interaction of a protein ligand with its receptor often takes place at a relatively large interface. However, as demonstrated for human growth hormone and its receptor, only a few key residues at the interface contribute to most of the binding energy. Clackson et al. (1995), Science 267: 383-6. The bulk of the protein ligand merely displays the binding epitopes in the right topology or serves functions unrelated to binding. Thus, molecules of only "peptide" length (2 to 40 amino acids) can bind to the receptor protein of a given large protein ligand. Such peptides may mimic the bioactivity of the large protein ligand ("peptide agonists") or, through competitive binding, inhibit the bioactivity of the large protein ligand ("peptide antagonists").

Phage display peptide libraries have emerged as a powerful method in identifying such peptide agonists and antagonists. See, for example, Scott et al. (1990), Science 249: 386; Devlin et al. (1990), Science 249: 404; U.S. Pat. No. 5,223,409, issued June 29,1993; U.S. Pat. No. 5,733,731, issued March 31,1998; U.S. Pat. No. 5,498,530, issued March 12, 1996; U.S. Pat. No. 5,432,018, issued July 11, 1995; U.S. Pat. No. 5,338,665, issued August 16, 1994; U.S. Pat. No. 5,922,545, issued July 13, 1999; WO 96/40987, published December 19,1996; and WO 98/15833, published April 16,1998 (each of which is incorporated by reference). In such libraries, random peptide sequences are displayed by fusion with coat proteins of filamentous phage. Typically, the displayed peptides are affinity-eluted against an antibody-immobilized extracellular domain of a receptor. The retained phages may be enriched by successive rounds of affinity purification and repropagation. The best binding peptides may be sequenced to identify key residues within one or more structurally related families of peptides. See, e.g., Cwirla et al. (1997), Science 276: 1696-9, in which two distinct families were identified. The peptide sequences may also suggest which residues may be safely replaced by alanine scanning or by mutagenesis at the DNA level. Mutagenesis libraries may be created and screened to further optimize the sequence of the best binders. Lowman (1997), Ann. Rev. Biophys. Biomol. Struct. 26: 401-24.

Structural analysis of protein-protein interaction may also be used to suggest peptides that mimic the binding activity of large protein ligands. In such an analysis, the crystal structure may suggest the identity and relative orientation of critical residues of the large protein ligand, from which a peptide may be designed. See, e.g., Takasaki et al. (1997), Nature Biotech. 15:1266-70. These analytical methods may also be used to investigate the interaction between a receptor protein and peptides selected by phage display, which may suggest further modification of the peptides to increase binding affinity.

Other methods compete with phage display in peptide research. A peptide library can be fused to the carboxyl terminus of the lac repressor and expressed in E. coli. Another E. coli-based method allows display on the cell's outer membrane by fusion with a peptidoglycan-associated lipoprotein (PAL). Hereinafter, these and related methods are collectively referred to as "E. coli display." In another method, translation of random RNA is halted prior to ribosome release, resulting in a library of polypeptides with their associated RNA still attached. Hereinafter, this and related methods are collectively referred to as "ribosome display." Other methods employ chemical linkage of peptides to RNA; see, for example, Roberts & Szostak (1997), Proc. Natl. Acad. Sci. USA, 94:12297-303. Hereinafter, this and related methods are collectively referred to as "RNA-peptide screening." Chemically derived peptide libraries have been developed in which peptides are immobilized on stable, non-biological materials, such as polyethylene rods or solvent-permeable resins. Another chemically derived peptide library uses photolithography to scan peptides immobilized on glass slides. Hereinafter, these and related methods are collectively referred to as "chemical-peptide screening." Chemical-peptide screening may be advantageous in that it allows use of D-amino acids and other unnatural analogues, as well as non-peptide elements. Both biological and chemical methods are reviewed in Wells & Lowman (1992), Curr. Opin. Biotechnol. 3X: 355-62.

Conceptually, one may discover peptide mimetics of any protein using phage display and the other methods mentioned above. These methods have been used for epitope mapping, for identification of critical amino acids in protein-protein interactions, and as leads for the discovery of new therapeutic agents. E.g., Cortese et al. (1996), Curr. Opin. Biotech. 7: 616-21. Peptide libraries are now being used most often in immunological studies, such as epitope mapping. Kreeger (1996), The Scientist 10(13):19-20.

Of particular interest here is use of peptide libraries and other techniques in the discovery of pharmacologically active peptides. A number of such peptides identified in the art are summarized in Table 2. The peptides are described in the listed publications, each of which is hereby incorporated by reference. The pharmacologic activity of the peptides is described, and in many instances is followed by a shorthand term therefor in parentheses. Some of these peptides have been modified (e.g., to form C-terminally cross-linked dimers). Typically, peptide libraries were screened for binding to a receptor for a pharmacologically active protein (e.g., EPO receptor). In at least one instance (CTLA4), the peptide library was screened for binding to a monclonal antibody.

**Table 2-Pharmacologically active peptides**

| **Form of peptide** | **Binding partner/ protein of interest^{a}** | **Pharmacologic activity** | **Reference** |
|---|---|---|---|
| intrapeptide disulfide-bonded | EPO receptor | EPO-mimetic | Wrighton et al. (1996), Science 273: 458-63 ; U.S. Pat. No. 5,773,569, issued June 30, 1998 to Wrighton et al. |
| C-terminally cross-linked dimer | EPO receptor | EPO-mimetic | Livnah et al. (1996), Science 273: 464-71 ; Wrighton et al. (1997), Nature Biotechnology 15: 1261-5; International patent application WO 96/40772, published Dec. 19, 1996 |
| linear | EPO receptor | EPO-mimetic | Naranda et al. (1999), Proc. Natl. Acad. Sci. USA, 96: 7569-74 |
| linear | c-Mpl | TPO-mimetic | Cwiria et al.(1997) Science 276: 1696-9; U.S. Pat. No. 5,869,451, issued Feb. 9, 1999; U.S. Pat. No. 5,932,946, issued Aug. 3, 1999 |
| C-terminally cross-linked dimer | c-Mpl | TPO-mimetic | Cwirla et al. (1997), Science 276: 1696-9 |
| disulfide-linked dimer | | stimulation of hematopoiesis ("G-CSF-mimetic") | Paukovits et al. (1984), Hoppe-Seylers Z. Physiol. Chem. 365: 303- 11; Laerum et al. (1988), Exp. Hemat. 16: 274-80 |
| alkylene-linked dimer | | G-CSF-mimetic | Bhatnagar et al. (1996), J. Med, Chem. 39: 3814-9; Cuthbertson et al. (1997), J. Med. Chem. 40: 2876-82; King et al. (1991), Exp. Hematol. 19:481; King et al. (1995), Blood 86 (Suppl. 1): 309a |
| linear | IL-1 receptor | inflammatory and autoimmune diseases ("IL-1 antagonist" or "IL-1 ra-mimetic") | U.S. Pat. No. 5,608,035; U.S. Pat. No. 5,786,331; U.S.-Pat. No. 5,880,096; Yanofsky et al. (1996),Proc. Natl. Acad. Sci. 93:7381-6; Akeson et al. (1996), J. Biol. Chem. 271: 30517-23; Wiekzorek et al. (1997), Pol. J. Pharmacol. 49: 107-17; Yanofsky (1996), PNAs, 93:7381-7386 |
| linear | Facteur thymique serique (FTS) | stimulation of lymphocytes ("FTS-mimetic") | Inagaki-Ohara et al. (1996), Cellular Immunol. 171: 30-40; Yoshida (1984), Int. J.Immunopharmacol, 6:141-6. |
| intrapeptide disulfide bonded | CTLA4 MAb | CTLA4-mimetic | Fukumoto et al. (1998), Nature Biotech. 16: 267- 70 |
| exocyclic | TNF-α receptor | TNF-α antagonist | Takasaki et al. (1997), Nature Biotech. 15:1266-70; WO 98/53842, published December 3, 1998 |
| linear | TNF-α receptor | TNF-α antagonist | Chirinos-Rojas ( ), J. Imm., 5621-5626. |
| intrapeptide disulfide bonded | C3b | inhibition of complement activation; autoimmune diseases ("C3b-antagonist") | Sahu et al. (1996), J. Immunol. 157:884-91 ; Morikis et al. (1998), Protein Sci. 7: 619-27 |
| linear | vinculin | cell adhesion processes- cell growth, differentiation, wound healing, tumor metastasis ("vinculin binding") | Adey et al. (1997), Biochem. J. 324: 523-8 |
| linear | C4 binding protein (C4BP) | anti-thrombotic | Linse et al. (1997), J. Biol. Chem. 272: 14658-65 |
| linear | urokinase receptor | processes associated with urokinase interaction with | Goodson et al. (1994), Proc. Natl. Acad. Sci. 91: 7129-33; |
| | | its receptor (e.g., angiogenesis, tumor cell invasion and metastasis); ("UKR antagonist") | International application WO 97/35969, published October 2, 1997 |
| linear | Mdm2, Hdm2 | Inhibition of inactivation of p53 mediated by Mdm2 or hdm2; anti-tumor ("Mdm/hdm antagonist") ; | Picksley et al. (1994), Oncogene 9: 2523-9; Bottger et al. (1997) J. Mol, Biol. 269: 744-56 ;Bottger et al. (1996), Oncogene 13: 2141-7 |
| linear | p21 ^{WAF1} | anti-tumor by mimicking the activity of p21^{WAF1} | Ball et al. (1997), Curr. Biol. 7: 71-80 |
| linear | farnesyl transferase | anti-cancer by preventing activation of ras | Gibbs et al. (1994), Cell oncogene 77:175-178 |
| linear | Ras effector domain | anti-cancer by inhibiting biological function of the ras oncogene | Moodie et al. (1994), Trends Genet 10: 44-48 Rodriguez et al. (1994), Nature 370:527-532 |
| linear | SH2/SH3 domains | anti-cancer by inhibiting tumor growth with activated tyrosine kinases | Pawson et al (1993), Curr. Biol. 3:434-432 Yu et al. (1994), Cell 76:933-945 |
| linear | p16^{INK4} | anti-cancer by mimicking activity of p16; e.g., inhibiting cyclin D-Cdk complex ("p16-mimetic") | Fåhraeus et al. (1996), Curr. Biol. 6:84-91 |
| linear | Src, Lyn | inhibition of Mast cell activation, IgE-related conditions, type I hypersensitivity ("Mast cell antagonist") | Stauffer et al. (1997), Biochem. 36: 9388-94 |
| linear | Mast cell protease | treatment of inflammatory disorders mediated by release of tryptase-6 ("Mast cell protease inhibitors") | International application WO 98/33812, published August 6, 1998 |
| linear | SH3 domains | treatment of SH3-mediated disease states ("SH3 antagonist") | Rickles et al. (1994), EMSO J. 13: 5598-5604 ; Sparks et al. (1994), J. Biol. Chem. 269: 23853- 6; Sparks et al. (1996), Proc. Natl. Acad. Sci. 93: 1540-4 |
| linear | HBV core antigen (HBcAg) | treatment of HBV viral infections ("anti-HBV") | Dyson & Muray (1995), Proc. Natl. Acad. Sci. 92 2194-8 : |
| linear | selectins | neutrophil adhesion; inflammatory diseases ("selectin antagonist") | Martens et al. (1995), J. Biol. Chem. 270: 21129-36; European patent application EP 0 714 912, published June 5, 1996 |
| linear, cyclized | calmodulin | calmodulin antagonist | Pierce et al. (1995), Molec. Diversity 1: 259-65; Dedman et al. (1993), J. Biol, Chem. 268: 23025-30; Adey & Kay (1996), Gene 169: 133-4 |
| linear, cyclized- | integrins | tumor-homing; treatment for conditions related to integrin-mediated cellular events, including platelet aggregation, thrombosis, wound healing, osteoporosis, tissue repair, angiogenesis (e.g., for treatment of cancer), and tumor invasion ("integrin-binding") | International applications WO 95/14714, published June 1, 1995; WO 97/08203, published March 6, 1997; WO 98/10795, published March 19, 1998; WO 99/24462, published May 20, 1999; Kraft et al. (1999), J. Biol. Chem. 274:1979-1985 |
| cyclic, linear | fibronectin and extracellular matrix components of T cells and macrophages | treatment of inflammatory and autoimmune conditions | WO 98/09985, published March 12, 1998 |
| linear | somatostatin and cortistatin | treatment or prevention of hormone-producing tumors, acromegaly, giantism, dementia, gastric ulcer, tumor growth, inhibition of hormone secretion, modulation of sleep or neural activity | European patent 0 911 393, application published April 28, 1999 |
| linear | bacterial lipopolysaccharide | antibiotic; septic shock; disorders modulatable by CAP37 | U.S. Pat. No. 5,877,151, issued March 2, 1999 |
| linear or cyclic, including D-amino acids | pardaxin, mellitin | antipathogenic | WO 97/31019, published 28 August 1997 |
| linear, cyclic | VIP | impotence, neurodegenerative disorders | WO 97/40070, published October 30, 1997 |
| linear | CTLs | cancer | EP 0 770 624, published May 2, 1997 |
| linear | THF-gamma2 | | Burnstein (1988), Biochem., 27:4066-71. |
| linear | Amylin | | Cooper (1987), Proc.Natl. Acad. Sci. , 84:8628-32. |
| linear | Adrenomedullin | | Kitamura (1993), BBRC, 192:553-60 . |
| cyclic, linear | VEGF | anti-angiogenic; cancer, rheumatoid arthritis, diabetic retinopathy, psoriasis ("VEGF antagonist") | Fairbrother (1998), Biochem., 37:17754- 17764. |
| cyclic | MMP | inflammation and autoimmune disorders; tumor growth ("MMP inhibitor") | Koivunen (1999), Nature Biotech., 17:768-774. |
| | HGH fragment | | U.S. Pat. No. 5,869,452 |
| | Echistatin | inhibition of platelet aggregation | Gan (1988), J, Biol. Chem., 263:19827-32 . |
| linear | SLE autoantibody | SLE | WO 96/30057, published October 3, 1996 |
| | GD1alpha | suppression of tumor metastasis | Ishikawa et al. (1998), FEBS Lett. 441 (1): 20-4 |
| | antiphospholipid beta-2-glycoprotein-I (β2GPI) antibodies | endothelial cell activation, antiphospholipid syndrome (APS), thromboembolic phenomena, thrombocytopenia, and recurrent fetal loss | Blank et al. (1999), Proc. Natl. Acad. Sci. USA 96: 5164-8 |
| linear | T Cell Receptor beta chain | diabetes | WO 96/11214, published April 18, 1996 |

| | | | |
|---|---|---|---|
| ^{a} The protein listed in this column may be bound by the associated peptide (e.g., EPO receptor, IL-1 receptor) or mimicked by the associated peptide. The references listed for each clarify whether the molecule is bound by or mimicked by the peptides. ^{b} FTS is a thymic hormone mimicked by the molecule of this invention rather than a receptor bound by the molecule of this invention. | | | |

Peptides identified by peptide library screening have been regarded as "leads" in development of therapeutic agents rather than as therapeutic agents themselves. Like other proteins and peptides, they would be rapidly removed in vivo either by renal filtration, cellular clearance mechanisms in the reticuloendothelial system, or proteolytic degradation. Francis (1992), Focus on Growth Factors 3: 4-11. As a result, the art presently uses the identified peptides to validate drug targets or as scaffolds for design of organic compounds that might not have been as easily or as quickly identified through chemical library screening. Lowman (1997), Ann. Rev. Biophys. Biomol. Struct. 26: 401-24; Kay et al. (1998), Drug Disc. Today 3: 370-8. The art would benefit from a process by which such peptides could more readily yield therapeutic agents.

### Summary of the Invention

The present invention concerns a process by which the in vivo half-life of one or more biologically active peptides is increased by fusion with a vehicle. In this invention, pharmacologically active compounds are prepared by a process comprising:
a) selecting at least one randomized peptide that modulates the activity of a protein of interest; and
b) preparing a pharmacologic agent comprising at least one Fc domain covalently linked to at least one amino acid sequence of the selected peptide, wherein "peptide" refers to molecules of 2 to 40 amino acids and wherein "peptide" refers to a randomised EPO-mimetic peptide; a randomised TPO-mimetic peptide; a randomised IL-1 antagonist peptide; a randomised GCSF-mimetic peptide; or a randomised TNF-antagonist peptide.
The peptides screened in step (a) are preferably expressed in a phage display library. The vehicle and the peptide may be linked through the N- or C-terminus of the peptide or the vehicle, as described further below. Derivatives of the above compounds (described below) are also encompassed by this invention.

The compounds of this invention may be prepared by standard synthetic methods, recombinant DNA techniques, or any other methods of preparing peptides and fusion proteins. Compounds of this invention that encompass non-peptide portions may be synthesized by standard organic chemistry reactions, in addition to standard peptide chemistry reactions when applicable.

The primary use contemplated is as therapeutic or prophylactic agents. The vehicle-linked peptide may have activity comparable to-or even greater than-the natural ligand mimicked by the peptide. In addition, certain natural ligand-based therapeutic agents might induce antibodies against the patient's own endogenous ligand; the vehicle-linked peptide avoids this pitfall by having little or typically no sequence identity with the natural ligand.

Although mostly contemplated as therapeutic agents, compounds of this invention may also be useful in screening for such agents. For example, one could use an Fc-peptide (e.g., Fc-SH2 domain peptide) in an assay employing anti-Fc coated plates. The vehicle, Fc, may make insoluble peptides soluble and thus useful in a number of assays.

The compounds of this invention may be used for therapeutic or prophylactic purposes by formulating them with appropriate pharmaceutical carrier materials and administering an effective amount to a patient, such as a human (or other mammal) in need thereof. Other related aspects are also included in the instant invention.

Numerous additional aspects and advantages of the present invention will become apparent upon consideration of the figures and detailed description of the invention.

### Brief Description of the Figures

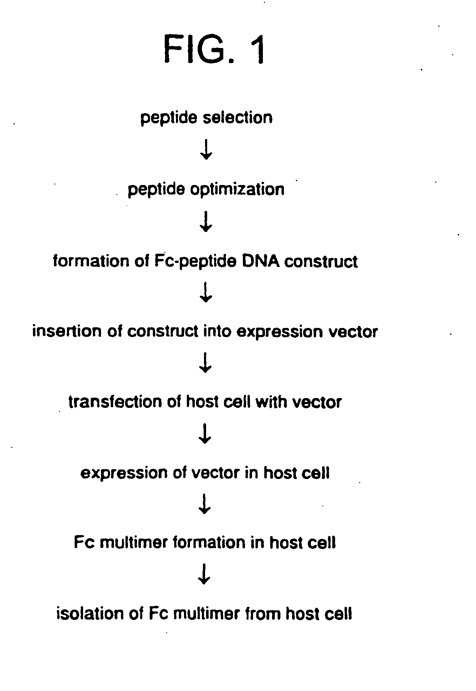

Figure 1 shows a schematic representation of an exemplary process of the invention. In this preferred process, the vehicle is an Fc domain, which is linked to the peptide covalently by expression from a DNA construct encoding both the Fc domain and the peptide. As noted in Figure 1, the Fc domains spontaneously form a dimer in this process.

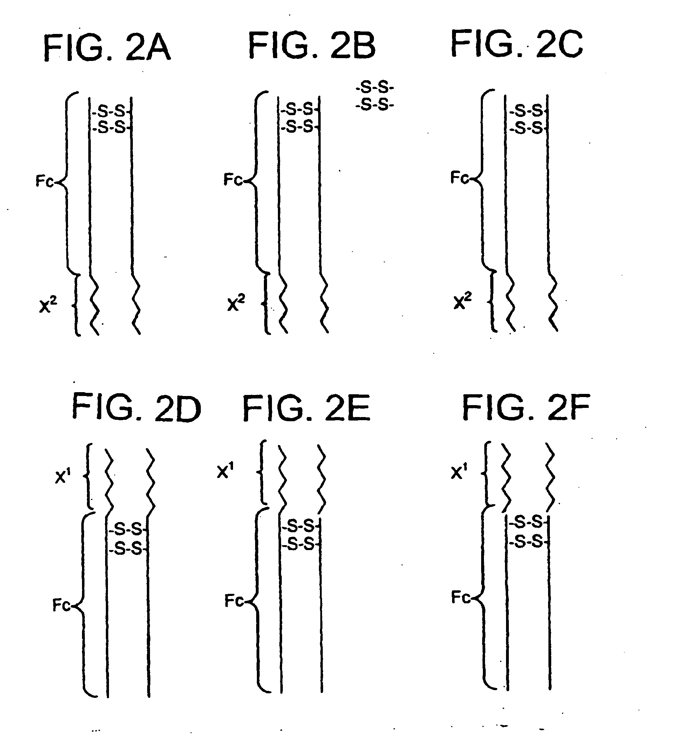

Figure 2 shows exemplary Fc dimers that may be derived from an IgG1 antibody. "Fc" in the figure represents any of the Fc variants within the meaning of "Fc domain" herein. "X¹" and "X²" represent peptides or linker-peptide combinations as defined hereinafter. The specific dimers are as follows:
   A, D: Single disulfide-bonded dimers. IgGl antibodies typically have two disulfide bonds at the hinge region between the constant and variable domains. The Fc domain in Figures 2A and 2 D may be formed by truncation between the two disulfide bond sites or by substitution of a cysteinyl residue with an unreactive residue (e.g., alanyl). In Figure 2A, the Fc domain is linked at the amino terminus of the peptides; in 2D, at the carboxyl terminus.
   B, E: Doubly disulfide-bonded dimers. This Fc domain may be formed by truncation of the parent antibody to retain both cysteinyl residues in the Fc domain chains or by expression from a construct including a sequence encoding such an Fc domain. In Figure 2B, the Fc domain is linked at the amino terminus of the peptides; in 2E, at the carboxyl terminus.
   C, F: Noncovalent dimers. This Fc domain may be formed by elimination of the cysteinyl residues by either truncation or substitution. One may desire to eliminate the cysteinyl residues to avoid impurities formed by reaction of the cysteinyl residue with cysteinyl residues of other proteins present in the host cell. The noncovalent bonding of the Fc domains is sufficient to hold together the dimer.
      Other dimers may be formed by using Fc domains derived from different types of antibodies (e.g., IgG2, IgM).

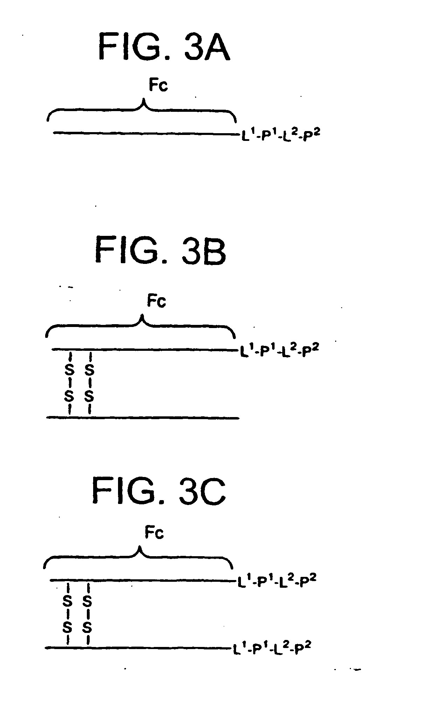

Figure 3 shows the structure of preferred compounds of the invention that feature tandem repeats of the pharmacologically active peptide. Figure 3A shows a single chain molecule and may also represent the DNA construct for the molecule. Figure 3B shows a dimer in which the linker-peptide portion is present on only one chain of the dimer. Figure 3C shows a dimer having the peptide portion on both chains. The dimer of Figure 3C will form spontaneously in certain host cells upon expression of a DNA construct encoding the single chain shown in Figure 3A. In other host cells, the cells could be placed in conditions favoring formation of dimers or the dimers can be formed in vitro.
Figure 4 shows exemplary nucleic acid and amino acid sequences (SEQ ID NOS: 1 and 2, respectively) of human IgG1 Fc that may be used in this invention.
Figure 5 shows a synthetic scheme (SEQ ID NOS:1131-1134) for the preparation of PEGylated peptide 19 (SEQ ID NO: 3).
Figure 6 shows a synthetic scheme (SEQ ID NOS:1135-1136) for the preparation of PEGylated peptide 20 (SEQ ID NO: 4).
Figure 7 shows the nucleotide and amino acid sequences (SEQ ID NOS: 5 and 6, respectively) of the molecule identified as "Fc-TMP" in Example 2 hereinafter.
Figure 8 shows the nucleotide and amino acid sequences (SEQ. ID. NOS: 7 and 8, respectively) of the molecule identified as "Fc-TMP-TMP" in Example 2 hereinafter.
Figure 9 shows the nucleotide and amino acid sequences (SEQ. ID. NOS: 9 and 10, respectively) of the molecule identified as "TMP-TMP-Fc" in Example 2 hereinafter.
Figure 10 shows the nucleotide and amino acid sequences (SEQ. ID. NOS:11 and 12, respectively) of the molecule identified as "TMP-Fc" in Example 2 hereinafter.
Figure 11 shows the number of platelets generated in vivo in normal female BDF1 mice treated with one 100 µg/kg bolus injection of various compounds, with the terms defined as follows.
   PEG-MGDF: 20 kD average molecular weight PEG attached by reductive amination to the N-terminal amino group of amino acids 1-163 of native human TPO, which is expressed in E. coli (so that it is not glycosylated);
   TMP: the TPO-mimetic peptide having the amino acid sequence IEGPTLRQWLAARA (SEQ ID NO: 13);
   TMP-TMP: the TPO-mimetic peptide having the amino acid sequence IEGPTLRQWLAARA-GGGGGGGG-IEGPTLRQWLAARA (SEQ ID NO: 14);
   PEG-TMP-TMP: the peptide of SEQ ID NO: 14, wherein the PEG group is a 5 kD average molecular weight PEG attached as shown in Figure 6;
   Fc-TMP-TMP: the compound of SEQ ID NO: 8 (Figure 8) dimerized with an identical second monomer (i.e., Cys residues 7 and 10 are bound to the corresponding Cys residues in the second monomer to form a dimer, as shown in Figure 2); and
   TMP-TMP-Fc is the compound of SEQ ID NO: 10 (Figure 9) dimerized in the same way as TMP-TMP-Fc except that the Fc domain is attached at the C-terminal end rather than the N-terminal end of the TMP-TMP peptide.

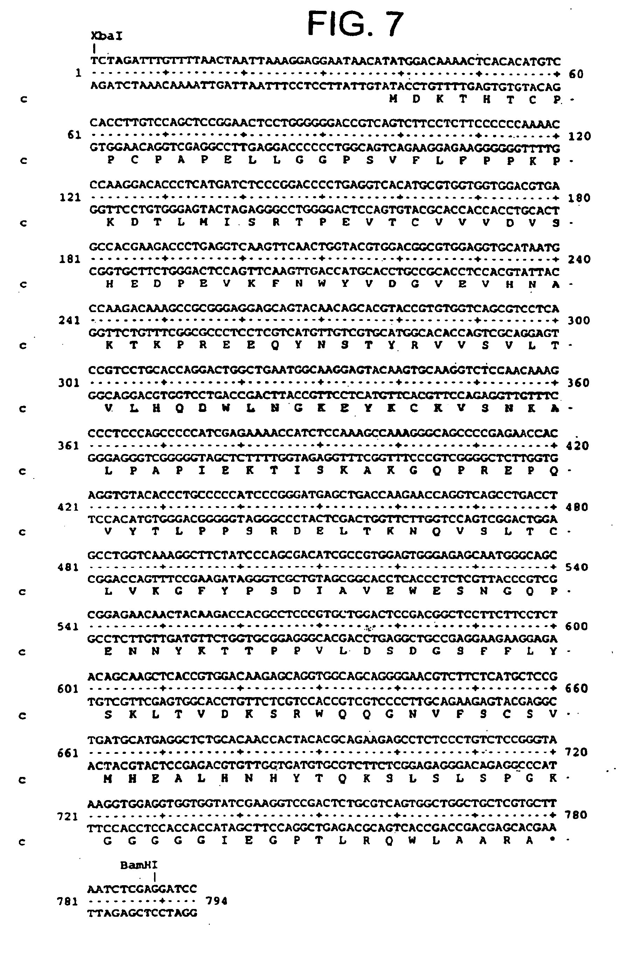

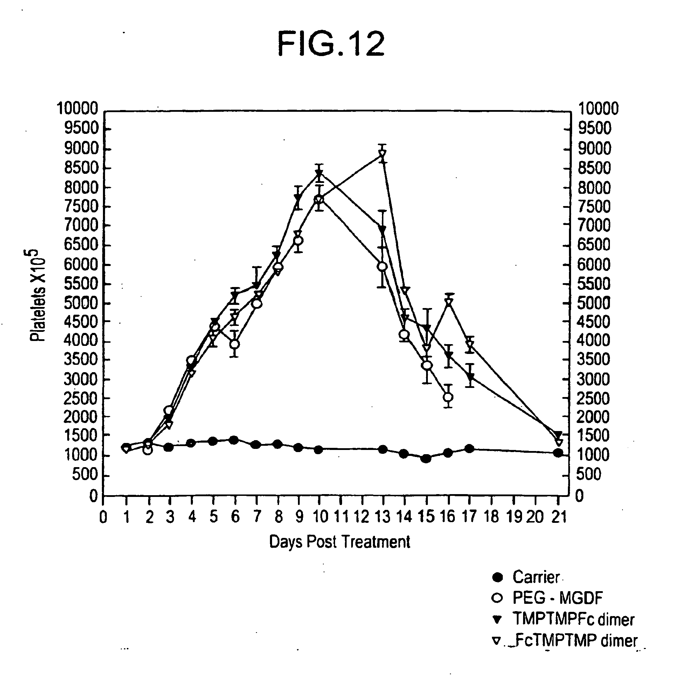

Figure 12 shows the number of platelets generated in vivo in normal BDF1 mice treated with various compounds delivered via implanted osmotic pumps over a 7-day period. The compounds are as defined for Figure 7.

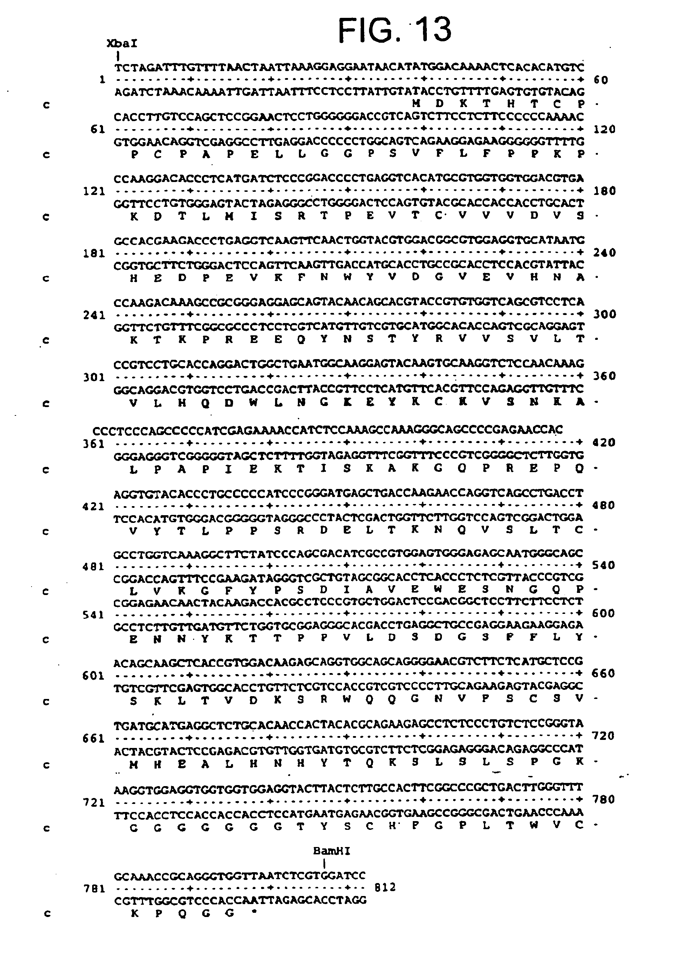

Figure 13 shows the nucleotide and amino acid sequences (SEQ. ID. NOS:15 and 16, respectively) of the molecule identified as "Fc-EMP" in Example 3 hereinafter.

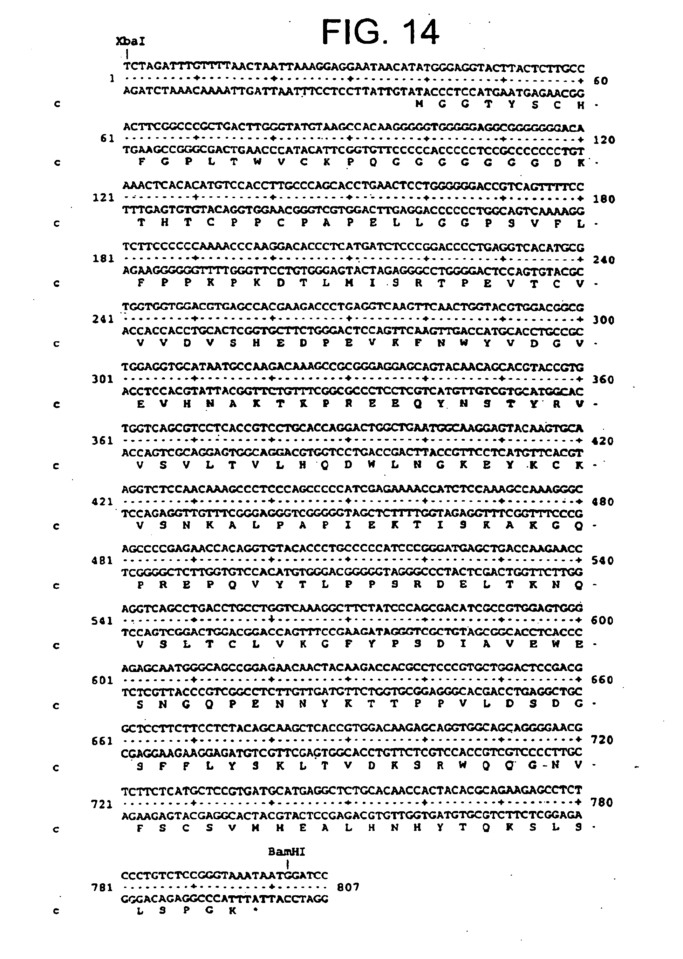

Figure 14 shows the nucleotide and amino acid sequences (SEQ ID NOS:17 and 18, respectively) of the molecule identified as "EMP-Fc" in Example 3 hereinafter.

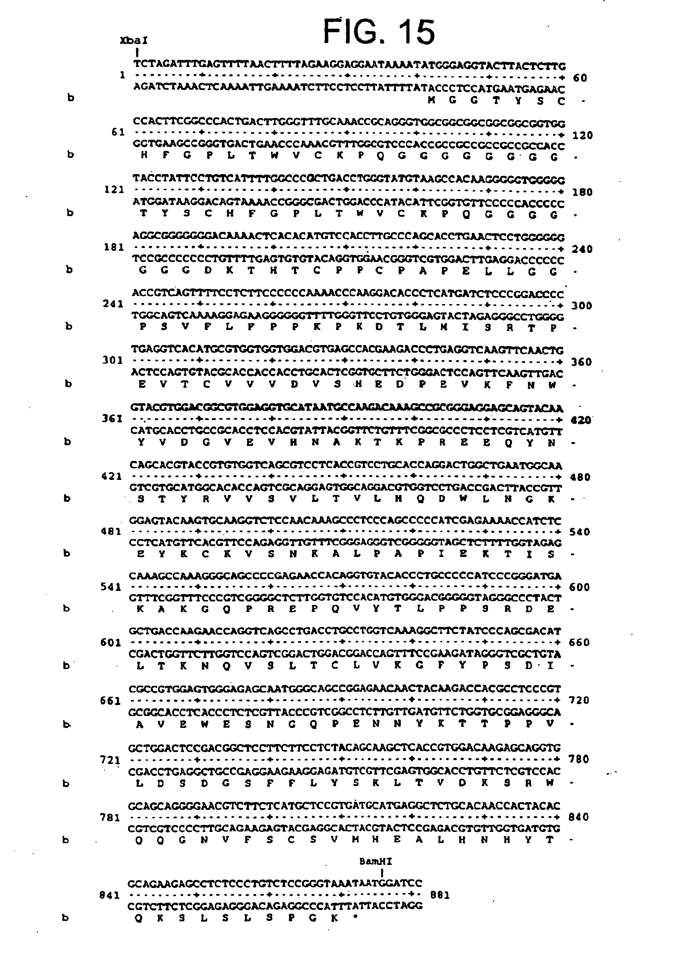

Figure 15 shows the nucleotide and amino acid sequences (SEQ ID NOS:19 and 20, respectively) of the molecule identified as "EMP-EMP-Fc" in Example 3 hereinafter.

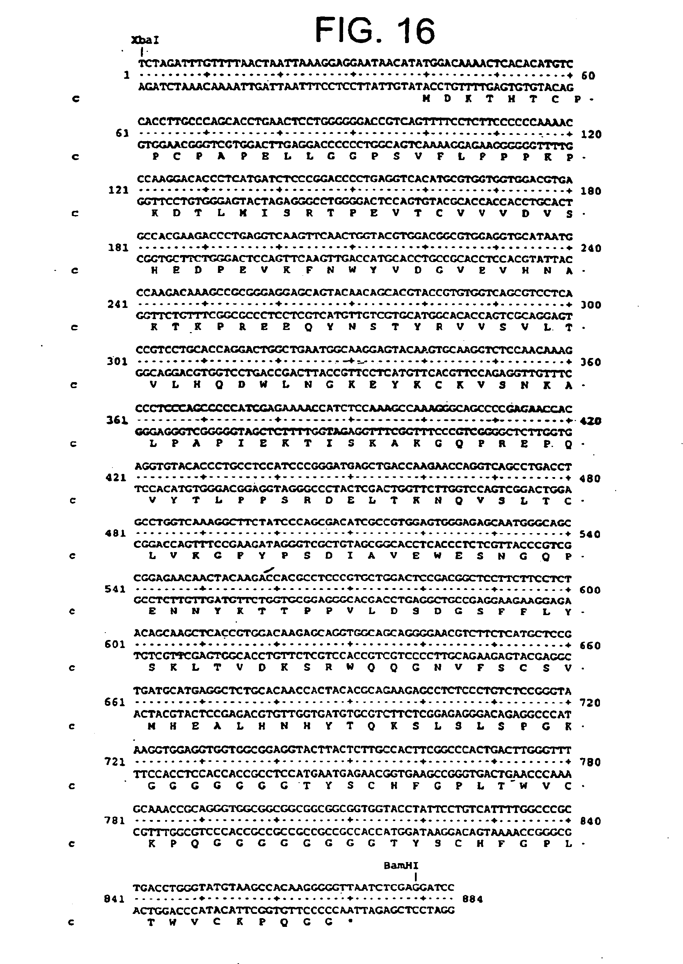

Figure 16 shows the nucleotide and amino acid sequences (SEQ ID NOS: 21 and 22, respectively) of the molecule identified as "Fc-EMP-EMP" in Example 3 hereinafter.

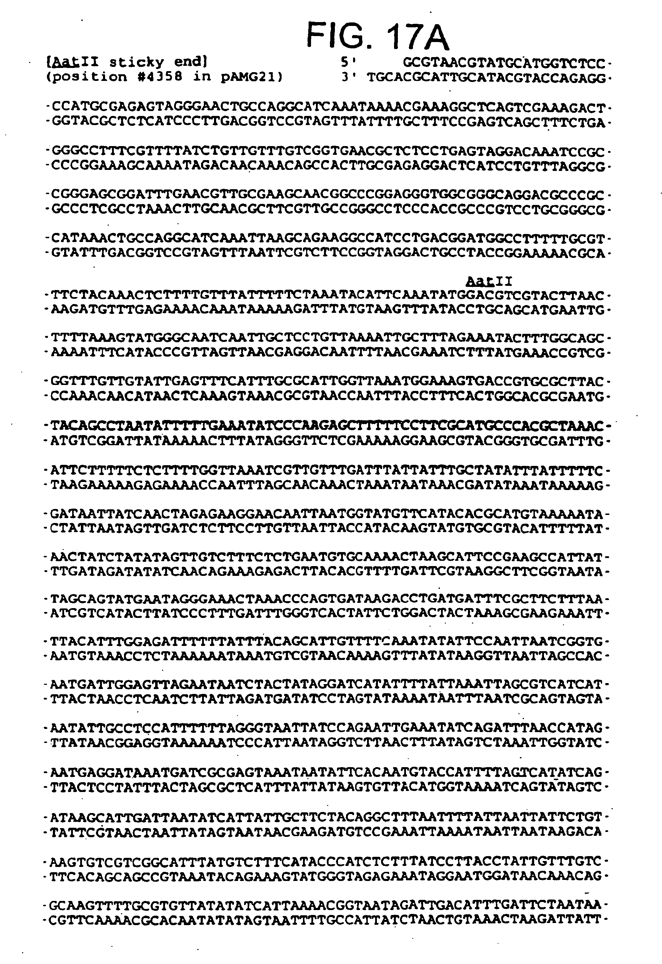

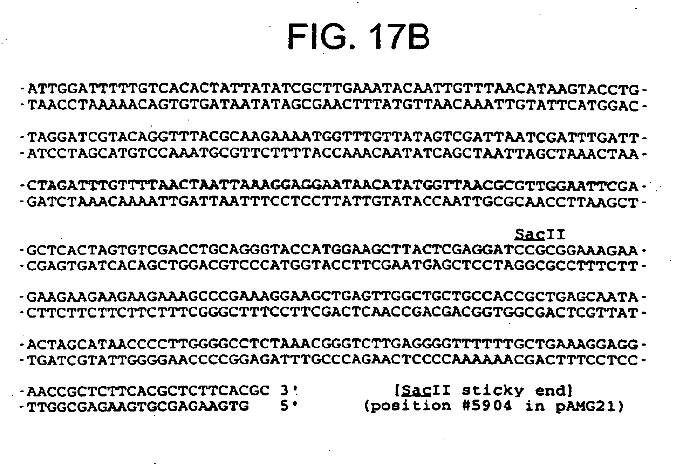

Figures 17A and 17B show the DNA sequence (SEQ ID NO: 23) inserted into pCFM1656 between the unique AatII (position #4364 in pCFM1656) and SacII (position #4585 in pCFM1656) restriction sites to form expression plasmid pAMG21 (ATCC accession no. 98113).

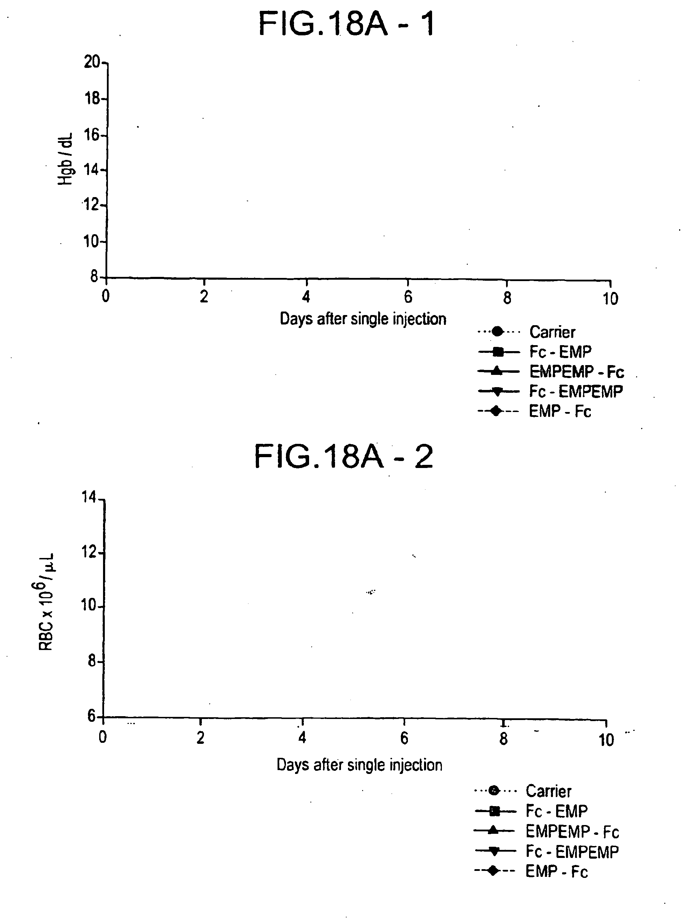

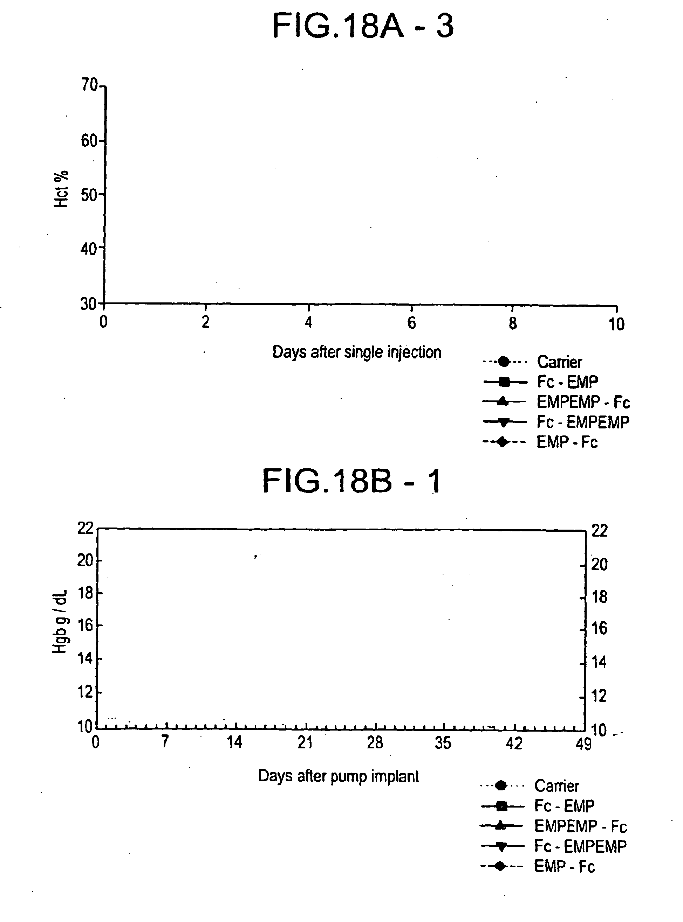

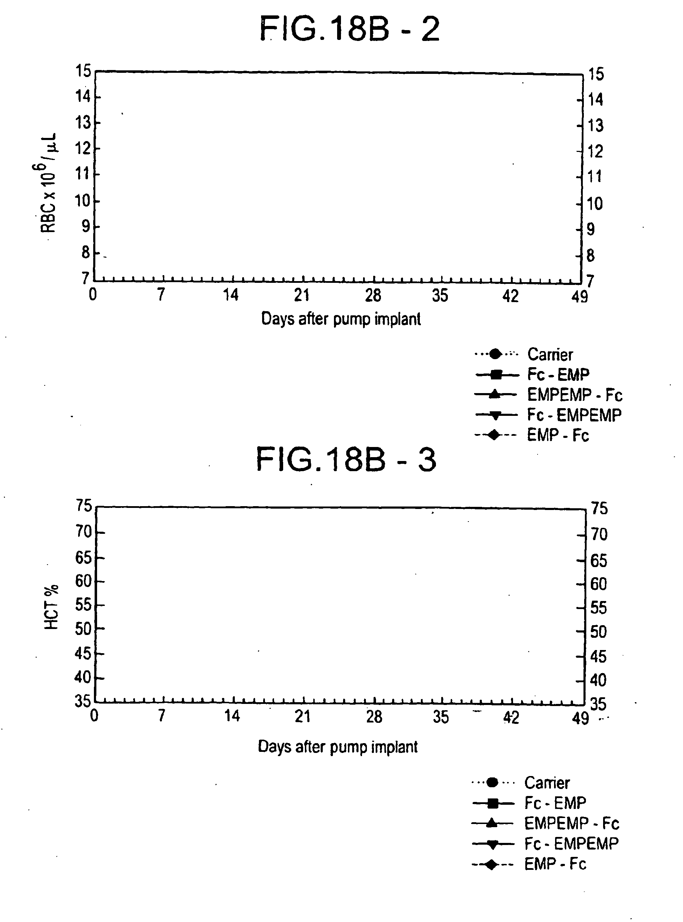

Figure 18A shows the hemoglobin, red blood cells, and hematocrit generated in vivo in normal female BDF1 mice treated with one 100 µg/kg bolus injection of various compounds. Figure 18B shows the same results with mice treated with 100 µg/kg per day delivered by 7-day micro-osmotic pump with the EMPs delivered at 100 µg/kg, rhEPO at 30U/mouse. (In both experiments, neutrophils, lymphocytes, and platelets were unaffected.) In these figures, the terms are defined as follows.
   Fc-EMP: the compound of SEQ ID NO: 16 (Figure 13) dimerized with an identical second monomer (i.e., Cys residues 7 and 10 are bound to the corresponding Cys residues in the second monomer to form a dimer, as shown in Figure 2);
   EMP-Fc: the compound of SEQ ID NO: 18 (Figure 14) dimerized in the same way as Fc-EMP except that the Fc domain is attached at the C-terminal end rather than the N-terminal end of the EMP peptide.
   "EMP-EMP-Fc" refers to a tandem repeat of the same peptide (SEQ ID NO: 20) attached to the same Fc domain by the carboxyl terminus of the peptides. "Fc-EMP-EMP" refers to the same tandem repeat of the peptide but with the same Fc domain attached at the amino terminus of the tandem repeat. All molecules are expressed in E. coli and so are not glycosylated.

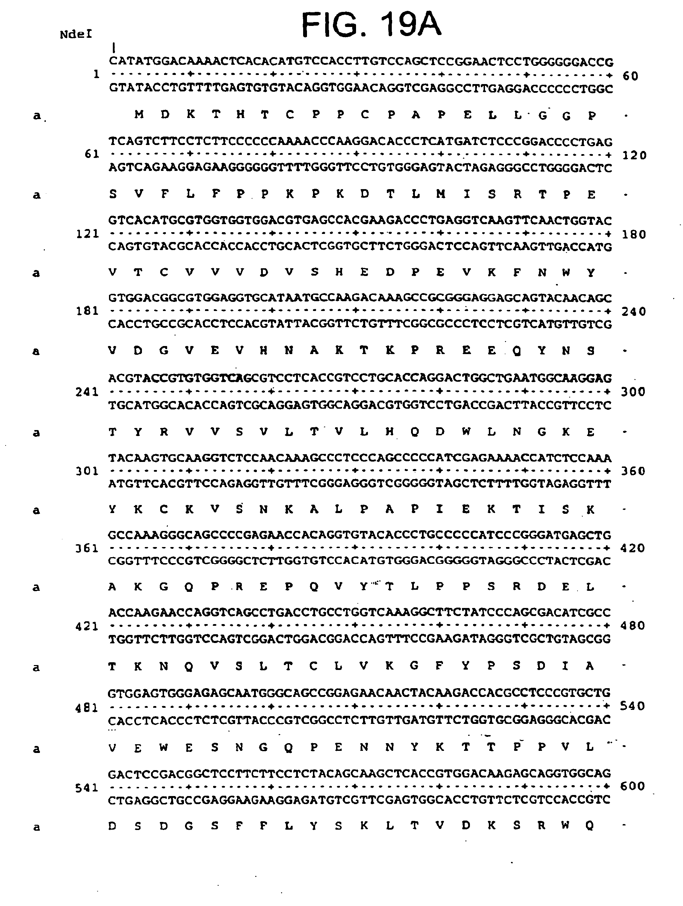

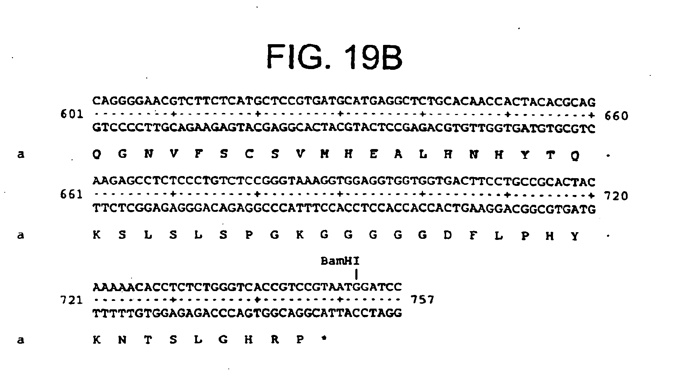

Figures 19A and 19B show the nucleotide and amino acid sequences (SEQ ID NOS: 1055 and 1056) of the Fc-TNF-α inhibitor fusion molecule described in Example 4 hereinafter.

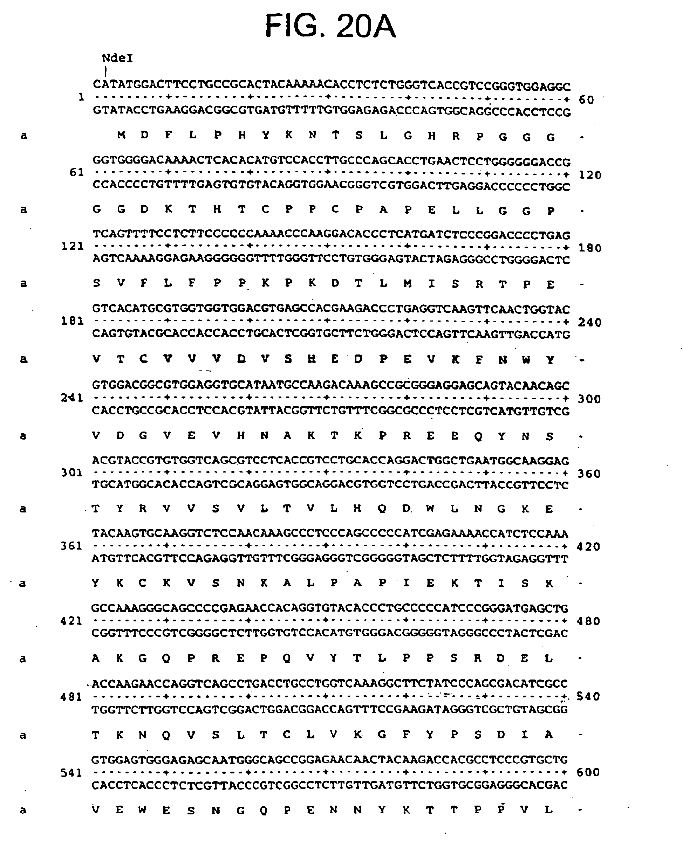

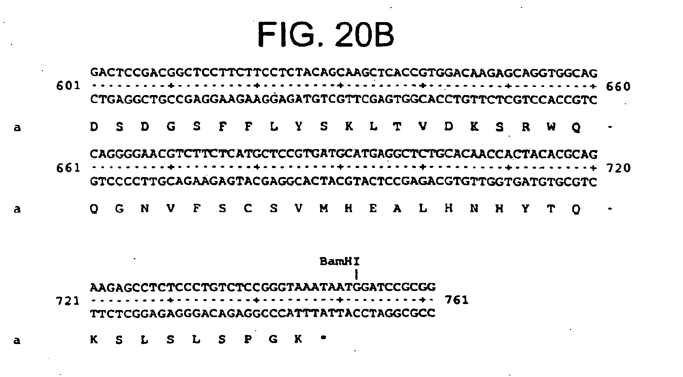

Figures 20A and 20B show the nucleotide and amino acid sequences (SEQ ID NOS: 1057 and 1058) of the TNF-α inhibitor-Fc fusion molecule described in Example 4 hereinafter.

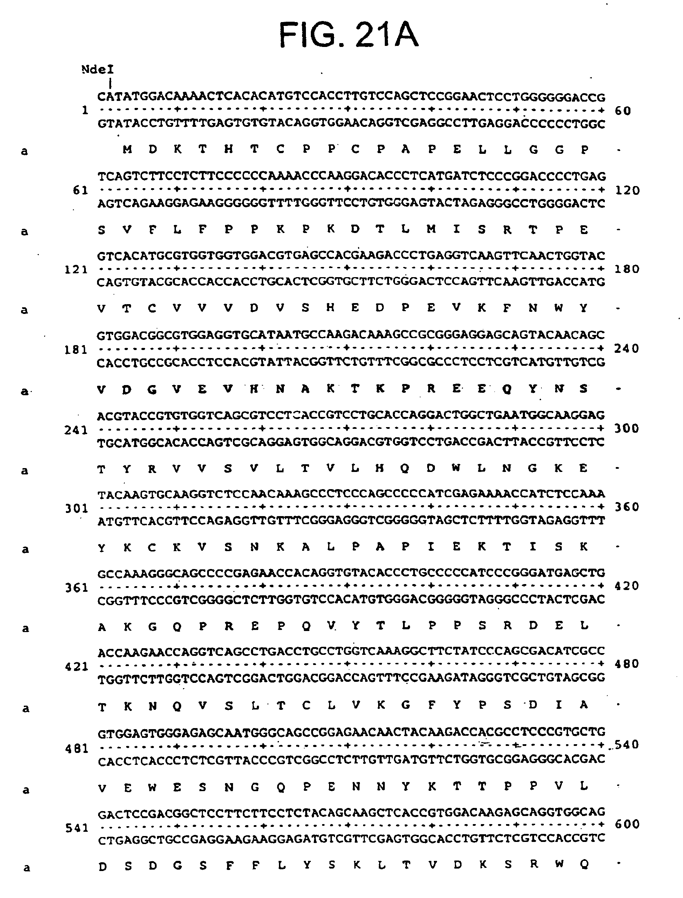

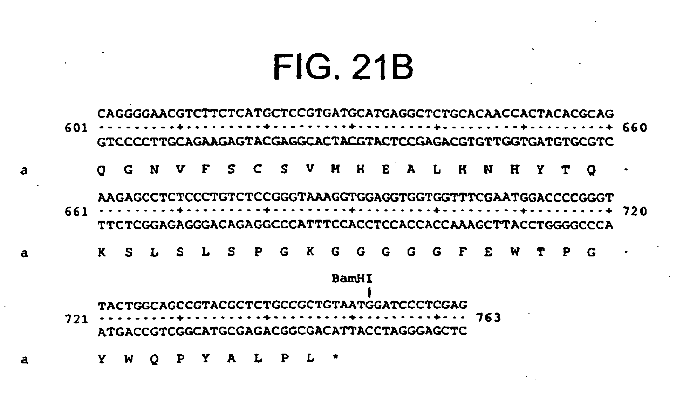

Figures 21A and 21B show the nucleotide and amino acid sequences (SEQ ID NOS: 1059 and 1060) of the Fc-IL-1 antagonist fusion molecule described in Example 5 hereinafter.

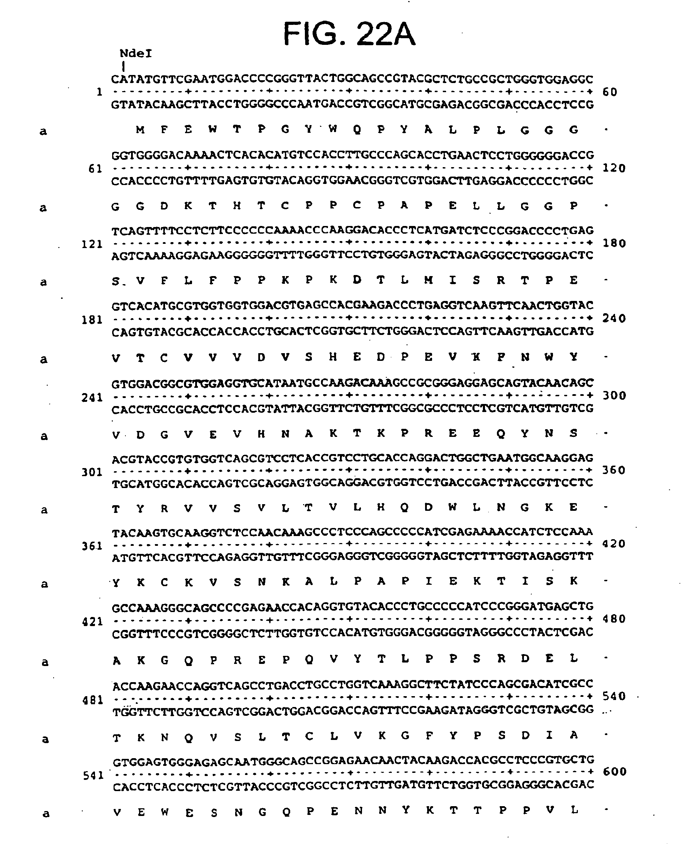

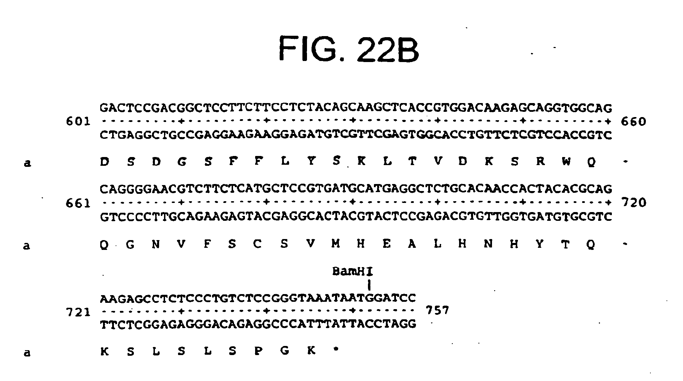

Figures 22A and 22B show the nucleotide and amino acid sequences (SEQ ID NOS: 1061 and 1062) of the IL-1 antagonist-Fc fusion molecule described in Example 5 hereinafter.

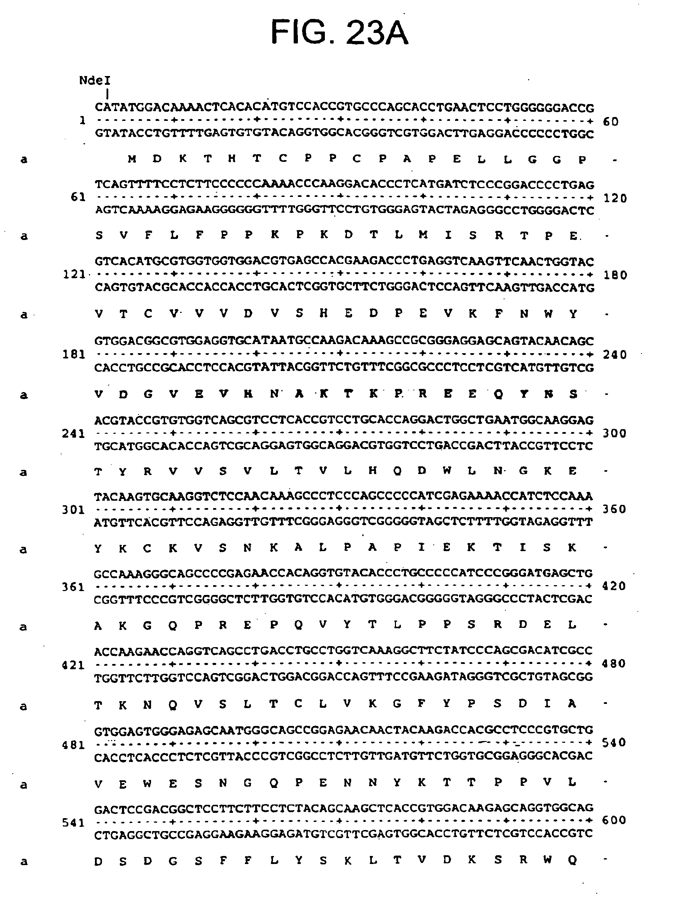

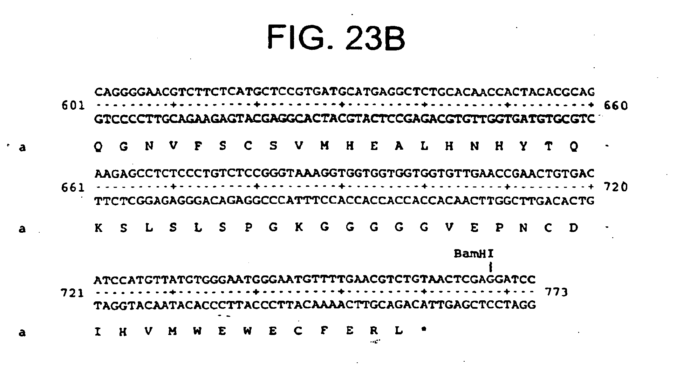

Figures 23A, 23B, and 23C show the nucleotide and amino acid sequences (SEQ ID NOS: 1063 and 1064) of the Fc-VEGF antagonist fusion molecule described in Example 6 hereinafter.

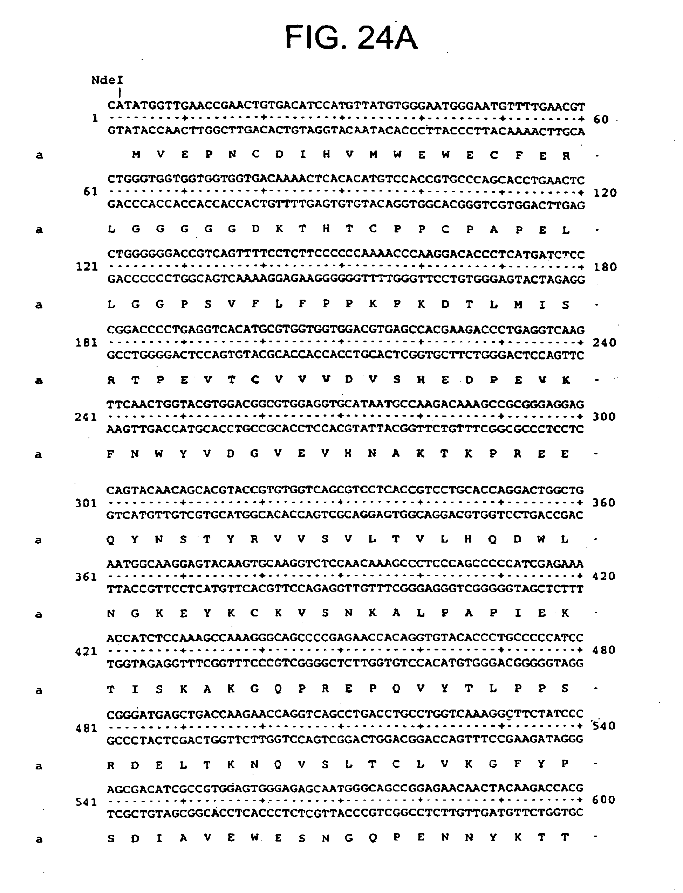

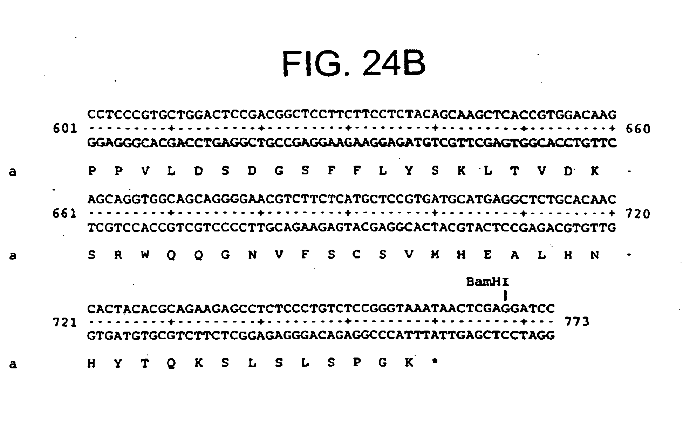

Figures 24A and 24B show the nucleotide and amino acid sequences (SEQ ID NOS: 1065 and 1066) of the VEGF antagonist-Fc fusion molecule described in Example 6 hereinafter.

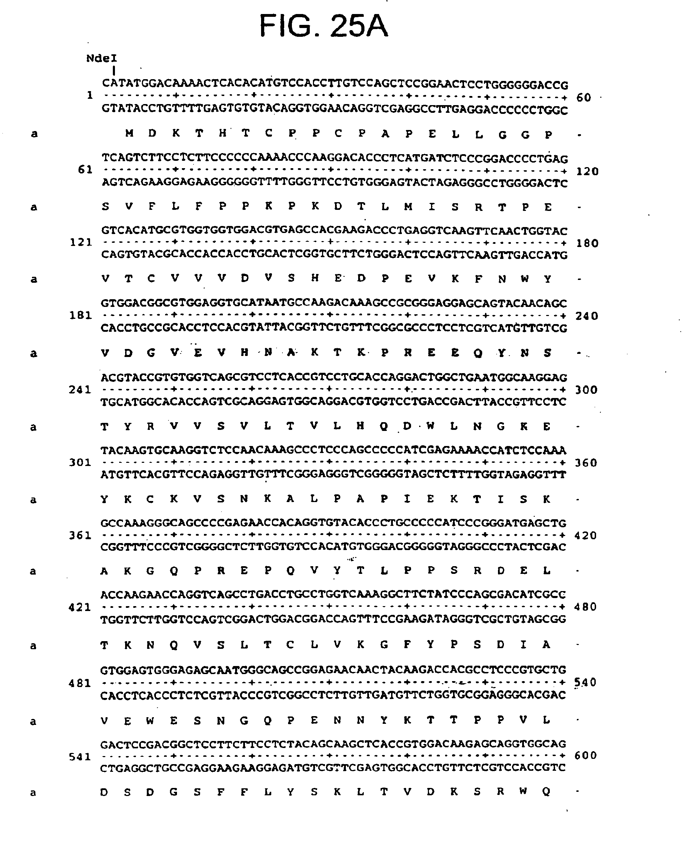

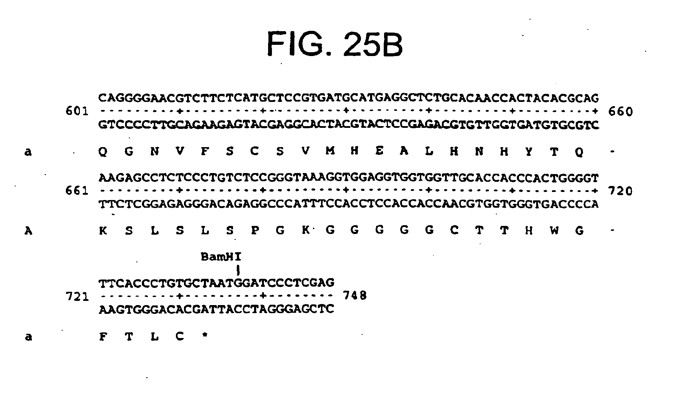

Figures 25A and 25B show the nucleotide and amino acid sequences (SEQ ID NOS: 1067 and 1068) of the Fc-MMP inhibitor fusion molecule described in Example 7 hereinafter.

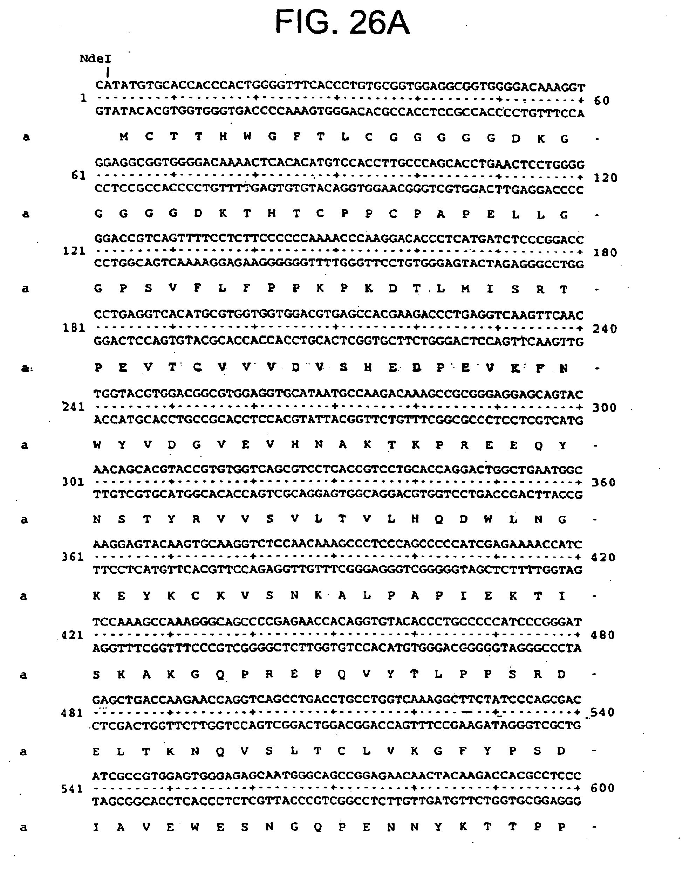

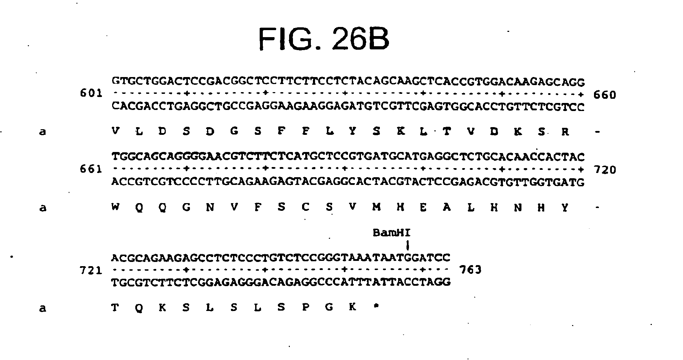

Figures 26A and 26B show the nucleotide and amino acid sequences (SEQ ID NOS: 1069 and 1070) of the MMP inhibitor-Fc fusion molecule described in Example 7 hereinafter.

### Detailed Description of the Invention

### Definition of Terms

The terms used throughout this specification are defined as follows, unless otherwise limited in specific instances.

The term "comprising" means that a compound may include additional amino acids on either or both of the N- or C- termini of the given sequence. Of course, these additional amino acids should not significantly interfere with the activity of the compound.

The term "vehicle" refers to a molecule that prevents degradation and/or increases half-life, reduces toxicity, reduces immunogenicity, or increases biological activity of a therapeutic protein. Exemplary vehicles include an Fc domain (which is preferred) as well as a linear polymer (e.g., polyethylene glycol (PEG), polylysine, dextran, etc.); a branched-chain polymer (see, for example, U.S. Patent No. 4,289,872 to Denkenwalter et al., issued September 15,1981; 5,229,490 to Tam, issued July 20,1993; WO 93/21259 by Frechet et al., published 28 October 1993); a lipid; a cholesterol group (such as a steroid); a carbohydrate or oligosaccharide; or any natural or synthetic protein, polypeptide or peptide that binds to a salvage receptor. Vehicles are further described hereinafter.

The term "native Fc" refers to molecule or sequence comprising the sequence of a non-antigen-binding fragment resulting from digestion of whole antibody, whether in monomeric or multimeric form. The original immunoglobulin source of the native Fc is preferably of human origin and may be any of the immunoglobulins, although IgG1 and IgG2 are preferred. Native Fc's are made up of monomeric polypeptides that may be linked into dimeric or multimeric forms by covalent (i.e., disulfide bonds) and non-covalent association. The number of intermolecular disulfide bonds between monomeric subunits of native Fc molecules ranges from 1 to 4 depending on class (e.g., IgG, IgA, IgE) or subclass (e.g., IgG1, IgG2, IgG3, IgA1 IgGA2). One example of a native Fc is a disulfide-bonded dimer resulting from papain digestion of an IgG (see Ellison et al. (1982), Nucleic Acids Res.10: 4071-9). The term "native Fc" as used herein is generic to the monomeric, dimeric, and multimeric forms.

The term "Fc variant" refers to a molecule or sequence that is modified from a native Fc but still comprises a binding site for the salvage receptor, FcRn. International applications WO 97/34631 (published 25 September 1997) and WO 96/32478 describe exemplary Fc variants, as well as interaction with the salvage receptor, and are hereby incorporated by reference. Thus, the term "Fc variant" comprises a molecule or sequence that is humanized from a non-human native Fc. Furthermore, a native Fc comprises sites that may be removed because they provide structural features or biological activity that are not required for the fusion molecules of the present invention. Thus, the term "Fc variant" comprises a molecule or sequence that lacks one or more native Fc sites or residues that affect or are involved in (1) disulfide bond formation, (2) incompatibility with a selected host cell (3) N-terminal heterogeneity upon expression in a selected host cell, (4) glycosylation, (5) interaction with complement, (6) binding to an Fc receptor other than a salvage receptor, or (7) antibody-dependent cellular cytotoxicity (ADCC). Fc variants are described in further detail hereinafter.

The term "Fc domain" encompasses native Fc and Fc variant molecules and sequences as defined above. As with Fc variants and native Fc's, the term "Fc domain" includes molecules in monomeric or multimeric form, whether digested from whole antibody or produced by other means.

The term "multimer" as applied to Fc domains or molecules comprising Fc domains refers to molecules having two or more polypeptide chains associated covalently, noncovalently, or by both covalent and non-covalent interactions. IgG molecules typically form dimers; IgM, pentamers; IgD, dimers; and IgA, monomers, dimers, trimers, or tetramers. Multimers may be formed by exploiting the sequence and resulting activity of the native Ig source of the Fc or by derivatizing (as defined below) such a native Fc.

The term "dimer" as applied to Fc domains or molecules comprising Fc domains refers to molecules having two polypeptide chains associated covalently or non-covalently. Thus, exemplary dimers within the scope of this invention are as shown in Figure 2.

The terms "derivatizing" and "derivative" or "derivatized" comprise processes and resulting compounds respectively in which (1) the compound has a cyclic portion; for example, cross-linking between cysteinyl residues within the compound; (2) the compound is cross-linked or has a cross-linking site; for example, the compound has a cysteinyl residue and thus forms cross-linked dimers in culture or in vivo; (3) one or more peptidyl linkage is replaced by a non-peptidyl linkage; (4) the N-terminus is replaced by -NRR¹, NRC(O)R¹, -NRC(O)OR¹, -NRS(O)₂R¹,NHC(O)NHR, a succinimide group, or substituted or unsubstituted benzyloxycarbonyl-NH-, wherein R and R¹ and the ring substituents are as defined hereinafter; (5) the C-terminus is replaced by -C(O)R² or -NR³R⁴ wherein R², R³ and R⁴ are as defined hereinafter; and (6) compounds in which individual amino acid moieties are modified through treatment with agents capable of reacting with selected side chains or terminal residues. Derivatives are further described hereinafter.

The term "peptide" refers to molecules of 2 to 40 amino acids, with molecules of 3 to 20 amino acids preferred and those of 6 to 15 amino acids most preferred. Exemplary peptides may be randomly generated by any of the methods cited above, carried in a peptide library (e.g., a phage display library), or derived by digestion of proteins.

The term "randomized" as used to refer to peptide sequences refers to fully random sequences (e.g., selected by phage display methods) and sequences in which one or more residues of a naturally occurring molecule is replaced by an amino acid residue not appearing in that position in the naturally occurring molecule. Exemplary methods for identifying peptide sequences include phage display, E. coli display, ribosome display, RNA-peptide screening, chemical screening, and the like.

The term "pharmacologically active" means that a substance so described is determined to have activity that affects a medical parameter (e.g., blood pressure, blood cell count, cholesterol level) or disease state (e.g., cancer, autoimmune disorders). Thus, pharmacologically active peptides comprise agonistic or mimetic and antagonistic peptides as defined below.

The terms "-mimetic peptide" and "-agonist peptide" refer to a peptide having biological activity comparable to a protein (e.g., EPO, TPO, G-CSF) that interacts with a protein of interest. These terms further include peptides that indirectly mimic the activity of a protein of interest, such as by potentiating the effects of the natural ligand of the protein of interest; see, for example, the G-CSF-mimetic peptides listed in Tables 2 and 7. Thus, the term "EPO-mimetic peptide" comprises any peptides that can be identified or derived as described in Wrighton et al. (1996), Science 273: 458-63, Naranda et al. (1999), Proc. Natl. Acad. Sci. USA 96: 7569-74, or any other reference in Table 2 identified as having EPO-mimetic subject matter. Those of ordinary skill in the art appreciate that each of these references enables one to select different peptides than actually disclosed therein by following the disclosed procedures with different peptide libraries.

The term "TPO-mimetic peptide" comprises peptides that can be identified or derived as described in Cwirla et al. (1997), Science 276: 1696-9, U.S. Pat. Nos. 5,869,451 and 5,932,946 and any other reference in Table 2 identifed as having TPO-mimetic subject matter, as well as the U.S. patent application, "Thrombopoietic Compounds," filed on even date herewith and hereby incorporated by reference. Those of ordinary skill in the art appreciate that each of these references enables one to select different peptides than actually disclosed therein by following the disclosed procedures with different peptide libraries.

The term "G-CSF-mimetic peptide" comprises any peptides that can be identified or described in Paukovits et al. (1984), Hoppe-Seylers Z. Physiol. Chem. 365: 303-11 or any of the references in Table 2 identified as having G-CSF-mimetic subject matter. Those of ordinary skill in the art appreciate that each of these references enables one to select different peptides than actually disclosed therein by following the disclosed procedures with different peptide libraries.

The term "CTLA4-mimetic peptide" comprises any peptides that can be identified or derived as described in Fukumoto et al. (1998), Nature Biotech. 16: 267-70. Those of ordinary skill in the art appreciate-that each of .. these references enables one to select different peptides than actually disclosed therein by following the disclosed procedures with different peptide libraries.

The term "-antagonist peptide" or "inhibitor peptide" refers to a peptide that blocks or in some way interferes with the biological activity of the associated protein of interest, or has biological activity comparable to a known antagonist or inhibitor of the associated protein of interest. Thus, the term "TNF-antagonist peptide" comprises peptides that can be identified or derived as described in Takasaki et al. (1997), Nature Biotech. 15: 1266-70 or any of the references in Table 2 identified as having TNF-antagonistic subject matter. Those of ordinary skill in the art appreciate that each of these references enables one to select different peptides than actually disclosed therein by following the disclosed procedures with different peptide libraries.

The terms "IL-1 antagonist" and "IL-1ra-mimetic peptide" comprises peptides that inhibit or down-regulate activation of the IL-1 receptor by IL-1. IL-1 receptor activation results from formation of a complex among IL-1, IL-1 receptor, and IL-1 receptor accessory protein. IL-1 antagonist or IL-1ra-mimetic peptides bind to IL-1, IL-1 receptor, or IL-1 receptor accessory protein and obstruct complex formation among any two or three components of the complex. Exemplary IL-1 antagonist or IL-1ra-mimetic peptides can be identified or derived as described in U.S. Pat. Nos. 5,608,035, 5,786,331, 5,880,096, or any of the references in Table 2 identified as having IL-1ra-mimetic or IL-1 antagonistic subject matter. Those of ordinary skill in the art appreciate that each of these references enables one to select different peptides than actually disclosed therein by following the disclosed procedures with different peptide libraries.

The term "VEGF-antagonist peptide" comprises peptides that can be identified or derived as described in Fairbrother (1998), Biochem. 37: 17754-64, and in any of the references in Table 2 identified as having VEGF-antagonistic subject matter. Those of ordinary skill in the art appreciate that each of these references enables one to select different peptides than actually disclosed therein by following the disclosed procedures with different peptide libraries.

The term "MMP inhibitor peptide" comprises peptides that can be identified or derived as described in Koiwnen (1999), Nature Biotech.17: 768-74 and in any of the references in Table 2 identified as having MMP inhibitory subject matter. Those of ordinary skill in the art appreciate that each of these references enables one to select different peptides than actually disclosed therein by following the disclosed procedures with different peptide libraries.

Additionally, physiologically acceptable salts of the compounds of this invention are also encompassed herein. By "physiologically acceptable salts" is meant any salts that are known or later discovered to be pharmaceutically acceptable. Some specific examples are: acetate; trifluoroacetate; hydrohalides, such as hydrochloride and hydrobromide; sulfate; citrate; tartrate; glycolate; and oxalate.

### Structure of compounds

In General. In the compositions of matter prepared in accordance with this invention, the peptide may be attached to the vehicle through the peptide's N-terminus or C-terminus. Thus, the vehicle-peptide molecules of this invention may be described by the following formula I:

I (X¹)ₐ-F¹-(X²)_{b}

wherein:
F¹ is an Fc domain;
X¹ and X² are each independently selected from -(L¹)_{c}-P¹, -(L¹)_{c}-P¹-(L²)_{d} -P², -(L¹)_{c}-P¹-(L²)_{d}-P²-(L³)ₑ-P³, and -(L¹)_{c}-P¹-(L²)_{d}-P²-(L³)ₑ -P³-(L⁴)_{f}-P⁴
P¹, P², P³, and P⁴ are each independently sequences of pharmacologically active peptides selected from IL-1 antagonist peptide sequences selected from SEQ ID NOS: 212, 907, 908, 909, 910, 917, and 979 and IL-1 antagonist peptide sequences selected from SEQ ID NOS: 213 to 271, 671 to 906, 911 to 916, and 918 to 1023;
EPO - mimetic peptide sequences selected from Table 5;
TPO - mimetic peptide sequences selected from Table 6;
TNF - antagonist peptide sequences selected from Table 8; and
GCSF - mimetic peptide sequences selected from Table 7;
L¹, L², L³ and L⁴ are each independently linkers; and
a, b, c, d, e, and f, are each independently 0 or 1, provided that at least one of a and b is 1, and wherein "peptide" refers to molecules of 2 to 40 amino acids.

Thus, compound I comprises preferred compounds of the formulae

II ¹-F¹

and multimers thereof wherein F¹ is an Fc domain and is attached at the C-terminus of X¹;

III F¹-X²

and multimers thereof wherein F¹ is an Fc domain and is attached at the N-terminus of X²;

IV F¹-(L¹)_{c}-P¹

and multimers thereof wherein F¹ is an Fc domain and is attached at the N-terminus of -(L¹)_{c}-P¹; and

V F¹-(L¹)_{c}-P¹-(L²)_{d}-P²

and multimers thereof wherein F¹ is an Fc domain and is attached at the N-terminus of -L¹-P¹-L²-P².

Peptides. Any number of peptides may be used in conjunction with the present invention. Of particular interest are peptides that mimic the activity of EPO, TPO, growth hormone, G-CSF, GM-CSF, IL-1ra, leptin, CTLA4, TRAIL, TGF-α, and TGF-β. Peptide antagonists are also of interest, particularly those antagonistic to the activity of TNF, leptin, any of the interleukins (IL-1, 2, 3, ...), and proteins involved in complement activation (e.g., C3b). Targeting peptides are also of interest, including tumor-homing peptides, membrane-transporting peptides, and the like. All of these classes of peptides may be discovered by methods described in the references cited in this specification and other references.

Phage display, in particular, is useful in generating peptides for use in the present invention. It has been stated that affinity selection from libraries of random peptides can be used to identify peptide ligands for any site of any gene product. Dedman et al. (1993), J. Biol. Chem. 268: 23025-30. Phage display is particularly well suited for identifying peptides that bind to such proteins of interest as cell surface receptors or any proteins having linear epitopes. Wilson et al. (1998), Can. J. Microbiol. 44: 313-29; Kay et al. (1998), Drug Disc. Today 3: 370-8. Such proteins are extensively reviewed in Herz et al. (1997), J. Receptor & Signal Transduction Res. 17(5): 671-776, which is hereby incorporated by reference. Such proteins of interest are preferred for use in this invention.

A particularly preferred group of peptides are those that bind to cytokine receptors. Cytokines have recently been classified according to their receptor code. See Inglot (1997), Archivum Immunologiae et Therapiae Experimentalis 45: 353-7, which is hereby incorporated by reference. Among these receptors, most preferred are the CKRs (family I in Table 3). The receptor classification appears in Table 3.

**Table 3-Cytokine Receptors Classified by Receptor Code**

| **Cytokines (ligands)** | | | | **Receptor Type** | | | |
|---|---|---|---|---|---|---|---|
| | **family** | | **subfamily** | | **family** | | **subfamily** |
| I. | Hematopoietic cytokines | 1. | IL-2, IL-4, IL-7, IL-9, IL-13, IL-15 | I. | Cytokine R (CKR) | 1. | shared γCr |
| | | 2. | IL-3, IL-5, GM-CSF | | | 2. | shared GP 140 βR |
| | | 3. | IL-6, IL-11, IL-12, LIF, OSM, CNTF, leptin (OB) | | | 3. | 3.shared RP 130 |
| | | 4. | G-CSF, EPO, TPO, PRL, GH | | | 4. | "single chain" R |
| | | 5. | IL-17, HVS-IL-17 | | | 5. | other R^{c} |
| II. | IL-10 ligands | IL-10, BCRF-1, HSV-IL-10 | | II. | IL-10 R | | |
| III. | Interferons | 1. | IFN-a1, α2, α4, m, t, IFN-β^{d} | III. | Interferon R | 1. | IFNAR |
| | | 2. | IFN-γ | | | 2. | IFNGR |
| IV. | IL-1 ligands | 1. | IL-1α, IL-1β, IL-1Ra | IV. | IL-1R | | |
| V. | TNF ligands | 1. | TNF-α, TNF-β (LT), FAS1, CD40 L, CD30L, CD27 L | V. | NGF/TNF R^{e} | | |
| VI. | Chemokines | 1. | α chemokines: IL-8, GRO α,β,γ, IF-10, PF-4, SDF-1 | VI. | Chemokine R | 1. | CXCR |
| | | 2. | β chemokines: MIP1α, MIP1β, MCP-1,2,3,4, RANTES, eotaxin | | | 2. | CCR |
| | | 3. | γ chemokines: lymphotactin | | | 3. | CR |
| | | | | | | 4. | DARC^{f} |
| VII. | Growth factors | | | VII. | RKF | 1. | TK sub-family |
| | | 1.1 | SCF, M-CSF, | | | 1.1 | IgTK III R |
| | | | PDGF-AA, AB, | | | | |
| | | | BB, FLT-3L, | | | | |
| | | | VEGF, SSV- | | | | |
| | | | PDGF | | | | |
| | | 1.2 | FGFα, FGFβ | | | 1.2 | IgTK IV R |
| | | 1.3 | EGF, TGF-α, | | | 1.3 | Cysteine-rich |
| | | | VV-F19 (EGF- | | | | TK-I |
| | | | like) | | | | |
| | | 1.4 | IGF-I, IGF-II, | | | 1.4 | Cysteine rich |
| | | | Insulin | | | | TK-II |
| | | 1.5 | NGF, BDNF, | | | 1.5 | Cysteine knot |
| | | | NT-3, NT-4^{g} | | | | TK V |
| | | 2. | TGF-β1,β2,β3 | | | 2. | STK subfamily^{h} |

| | | | | | | | |
|---|---|---|---|---|---|---|---|
| ^{c} IL-17R belongs to the CKR family but is not assigned to any of the 4 indicated subjamilies. ^{d} Other IFN type I subtypes remain unassigned. Hematopoietic cytokines, IL-10 ligands and interferons do not possess functional intrinsic protein kinases. The signaling molecules for the cytokines are JAK's, STATs and related non-receptor molecules. IL-14. IL-16 and IL-18 have been cloned but according to the receptor code they remain unassigned. ^{e} TNF receptors use multiple, distinct intracellular molecules for signal transduction including "death domain" of FAS R and 55 kDa TNF-αR that participates in their cytotoxic effects. NGF/TNF R can bind both NGF and related factors as well as TNF ligands. Chemokine receptors are G protein-coupled, seven transmembrane (7TM, serpentine) domain receptors. ^{f}The Duffy blood group antigen (DARC) is an erythrocyte receptor that can bind several different chemokines. It belongs to the immunoglobulin superfamily but characteristics of its signal transduction events remain unclear. ^{g}The neurotrophic cytokines can associate with NGF/TNF receptors also. ^{h}STKS may encompass many other TGF-β-retated factors that remain unassigned. The protein kinases are intrinsic part of the intracellular domain of receptor kinase family (RKF). The enzymes participate in the signals transmission via the receptors. | | | | | | | |

Exemplary peptides for this invention appear in Tables 4 through 20 below. These peptides may be prepared by methods disclosed in the art. Single letter amino acid abbreviations are used. The X in these sequences (and throughout this specification, unless specified otherwise in a particular instance) means that any of the 20 naturally occurring amino acid residues may be present. Any of these peptides may be linked in tandem (i.e., sequentially), with or without linkers, and a few tandem-linked examples are provided in the table. Linkers are listed as "A" and may be any of the linkers described herein. Tandem repeats and linkers are shown separated by dashes for clarity. Any peptide containing a cysteinyl residue may be cross-linked with another Cys-containing peptide, either or both of which may be linked to a vehicle. A few cross-linked examples are provided in the table. Any peptide having more than one Cys residue may form an intrapeptide disulfide bond, as well; see, for example, EPO-mimetic peptides in Table 5. A few examples of intrapeptide disulfide-bonded peptides are specified in the table. Any of these peptides may be derivatized as described herein, and a few derivatized examples are provided in the table. Derivatized peptides in the tables are exemplary rather than limiting, as the associated underivatized peptides may be employed in this invention, as well. For derivatives in which the carboxyl terminus may be capped with an amino group, the capping amino group is shown as -NH₂. For derivatives in which amino acid residues are substituted by moieties other than amino acid residues, the substitutions are denoted by σ, which signifies any of the moieties described in Bhatnagar et al. (1996), J. Med. Chem. 39: 3814-9 and Cuthbertson et al. (1997), J. Med. Chem. 40: 2876-82, which are incorporated by reference. The J substituent and the Z substituents (Z₅, Z₆, ...Z40) are as defined in U.S. Pat. Nos. 5,608,035,5,786,331, and 5,880,096, which are incorporated by reference. For the EPO-mimetic sequences (Table 5), the substituents X₂ through X₁₁ and the integer "n" are as defined in WO 96/40772, which is incorporated by reference. The substituents "ψ," "Θ," and "+" are as defined in Sparks et al. (1996), Proc. Natl. Acad. Sci. 93: 1540-4, which is hereby incorporated by reference. X₄, X₅, X₆, and X₇ are as defined in U.S. Pat. No. 5,773,569, which is hereby incorporated by reference, except that: for integrin-binding peptides, X₁, X₂, X₃, X₄, X₅, X₆, X₇, and X₈ are as defined in International applications WO 95/14714, published June 1, 1995 and WO 97/08203, published March 6,1997, which are also incorporated by reference; and for VIP-mimetic peptides, X₁, X₁', X₁", X₂, X₃, X₄, X₅, X₆ and Z and the integers m and n are as defined in WO 97/40070, published October 30,1997, which is also incorporated by reference. Xaa and Yaa below are as defined in WO 98/09985, published March 12,1998, which is incorporated by reference. AA₁, AA₂, AB₁, AB₂, and AC are as defined in International application WO 98/53842, published December 3, 1998, which is incorporated by reference. X¹, X², X³, and X⁴ in Table 17 only are as defined in European application EP 0 911 393, published April 28, 1999. Residues appearing in boldface are D-amino acids. All peptides are linked through peptide bonds unless otherwise noted. Abbreviations are listed at the end of this specification. In the "SEQ ID NO." column, "NR" means that no sequence listing is required for the given sequence.

**Table 4-IL-1 antagonist peptide sequences**

| **Sequence/structure** | **SEQ ID NO**: |
|---|---|
| Z₁₁Z₇Z₈QZ₅YZ₆Z₉Z₁₀ | 212 |
| XXQZ₅YZ₆XX | 907 |
| Z₇XQZ₅YZ₆XX | 908 |
| Z₇Z₈QZ₅YZ₆Z₉Z₁₀ | 909 |
| Z₁₁Z₇Z₈QZ₅YZ₆Z₉Z₁₀ | 910 |
| Z₁₂Z₁₃Z₁₄Z₁₅Z₁₆Z₁₇Z₁₈Z₁₉Z₂₀Z₂₁Z₂₂Z₁₁Z₇Z₈QZ₅YZ₆Z₉Z₁₀L | 917 |
| Z₂₃NZ₂₄Z₃₉Z₂₅Z₂₆Z₂₇Z₂₈Z₂₉Z₃₀Z₄₀ | 979 |
| TANVSSFEWTPYYWQPYALPL | 213 |
| SWTDYGYWQPYALPISGL | 214 |
| ETPFTWEESNAYYWQPYALPL | 215 |
| ENTYSPNWADSMYWQPYALPL | 216 |
| SVGEDHNFWTSEYWQPYALPL | 217 |
| DGYDRWRQSGERYWQPYALPL | 218 |
| FEWTPGYWQPY | 219 |
| FEWTPGYWQHY | 220 |
| FEWTPGWYQJY | 221 |
| AcFEWTPGWYQJY | 222 |
| FEWTPGWpYQJY | 223 |
| FAWTPGYWQJY | 224 |
| FEWAPGYWQJY | 225 |
| FEWVPGYWQJY | 226 |
| FEWTPGYWQJY | 227 |
| AcFEWTPGYWQJY | 228 |
| FEWTPaWYQJY | 229 |
| FEWTPSarWYQJY | 230 |
| FEWTPGYYQPY | 231 |
| FEWTPGWWOPY | 232 |
| FEWTPNYWQPY | 233 |
| FEWTPvYWQJY | 234 |
| FEWTPecGYWQJY | 235 |
| FEWTPAibYWQJY | 236 |
| FEWTSarGYWQJY | 237 |
| FEWTPGYWQPY | 238 |
| FEWTPGYWQHY | 239 |
| FEWTPGWYQJY | 240 |
| AcFEWTPGWYQJY | 241 |
| FEWTPGW-pY-QJY | 242 |
| FAWTPGYWQJY | 243 |
| FEWAPGYWQJY | 244 |
| FEWVPGYWOJY | 245 |
| FEWTPGYWQJY | 246 |
| AcFEWTPGYWQJY | 247 |
| FEWTPAWYQJY | 248 |
| FEWTPSarWYQJY | 249 |
| FEWTPGYYQPY | 250 |
| FEWTPGWWQPY | 251 |
| FEWTPNYWQPY | 252 |
| FEWTPVYWQJY | 253 |
| FEWTPecGYWQJY | 254 |
| FEWTPAibYWQJY | 255 |
| FEWTSarGYWQJY | 256 |
| FEWTPGYWQPYALPL | 257 |
| 1NapEWTPGYYQJY | 258 |
| YEWTPGYYQJY | 259 |
| FEWVPGYYQJY | 260 |
| FEWTPSYYQJY | 261 |
| FEWTPNYYQJY | 262 |
| TKPR | 263 |
| RKSSK | 264 |
| RKQDK | 265 |
| NRKQDK | 266 |
| RKODKR | 267 |
| ENRKQDKRF | 268 |
| VTKFYF | 269 |
| VTKFY | 270 |
| VTDFY | 271 |
| SHLYWQPYSVQ | 671 |
| TLVYWQPYSLQT | 672 |
| RGDYWQPYSVQS | 673 |
| VHVYWQPYSVQT | 674 |
| RLVYWQPYSVQT | 675 |
| SRVWFQPYSLOS | 676 |
| NMVYWOPYSIOT | 677 |
| SWFWQPYSVOT | 678 |
| TFVYWOPYALPL | 679 |
| TLVYWQPYSIQR | 680 |
| RLVYWQPYSVQR | 681 |
| SPVFWQPYSIQI | 682 |
| WIEWWQPYSVQS | 683 |
| SLIYWQPYSLOM | 684 |
| TRLYWQPYSVQR | 685 |
| RCDYWQPYSVQT | 686 |
| MRVFWQPYSVQN | 687 |
| KIVYWQPYSVOT | 688 |
| RHLYWQPYSVQR | 689 |
| ALVWWQPYSEQI | 690 |
| SRVWFQPYSLQS | 691 |
| WEQPYALPLE | 692 |
| QLVWWQPYSVQR | 693 |
| DLRYWQPYSVQV | 694 |
| ELVWWQPYSLQL | 695 |
| DLVWWQPYSVQW | 696 |
| NGNYWQPYSFQV | 697 |
| ELVYWQPYSIQR | 698 |
| ELMYWQPYSVQE | 699 |
| NLLYWQPYSMQD | 700 |
| GYEWYQPYSVQR | 701 |
| SRVWYQPYSVQR | 702 |
| LSEQYQPYSVQR | 703 |
| GGGWWQPYSVQR | 704 |
| VGRWYQPYSVQR | 705 |
| VHVYWQPYSVQR | 706 |
| QARWYQPYSVQR | 707 |
| VHVYWQPYSVQT | 708 |
| RSVYWQPYSVQR | 709 |
| TRVWFQPYSVQR | 710 |
| GRIWFQPYSVQR | 711 |
| GRVWFQPYSVQR | 712 |
| ARTWYQPYSVOR | 713 |
| ARVWWQPYSVQM | 714 |
| RLMFYQPYSVQR | 715 |
| ESMWYQPYSVQR | 716 |
| HFGWWQPYSVHM | 717 |
| ARFWWQPYSVQR | 718 |
| RLVYWQ PYAPIY | 719 |
| RLVYWQ PYSYQT | 720 |
| RLVYWQ PYSLPI | 721 |
| RLVYWQ PYSVQA | 722 |
| SRVWYQ PYAKGL | 723 |
| SRVWYQ PYAQGL | 724 |
| SRVWYQ PYAMPL | 725 |
| SRVWYQ PYSVQA | 726 |
| SRVWYQ PYSLGL | 727 |
| SRVWYQ PYAREL | 728 |
| SRVWYQ PYSRQP | 729 |
| SRVWYQ PYFVQP | 730 |
| EYEWYQ PYALPL | 731 |
| IPEYWQ PYALPL | 732 |
| SRIWWQ PYALPL | 733 |
| DPLFWQ PYALPL | 734 |
| SRQWVQ PYALPL | 735 |
| IRSWWQ PYALPL | 736 |
| RGYWQ PYALPL | 737 |
| RLLWVQ PYALPL | 738 |
| EYRWFQ PYALPL | 739 |
| DAYWVQ PYALPL | 740 |
| WSGYFQ PYALPL | 741 |
| NIEFWQ PYALPL | 742 |
| TRDWVQ PYALPL | 743 |
| DSSWYQ PYALPL | 744 |
| IGNWYQ PYALPL | 745 |
| NLRWDQ PYALPL | 746 |
| LPEFWQ PYALPL | 747 |
| DSYWWQ PYALPL | 748 |
| RSQYYQ PYALPL | 749 |
| ARFWLQ PYALPL | 750 |
| NSYFWQ PYALPL | 751 |
| RFMYWQPYSVQR | 752 |
| AHLFWQPYSVQR | 753 |
| WWQPYALPL | 754 |
| YYQPYALPL | 755 |
| YFQPYALGL | 756 |
| YWYQPYALPL | 757 |
| RWWQPYATPL | 758 |
| GWYQPYALGF | 759 |
| YWYQPYALGL | 760 |
| IWYQPYAMPL | 761 |
| SNMQPYQRLS | 762 |
| TFVYWQPY AVGLPAAETACN | 763 |
| TFVYWQPY SVQMTITGKVTM | 764 |
| TFVYWQPY SSHXXVPXGFPL | 765 |
| TFVYWQPY YGNPQWAIHVRH | 766 |
| TFVYWQPY VLLELPEGAVRA | 767 |
| TFVYWQPY VDYVWPIPIAQV | 768 |
| GWYQPYVDGWR | 769 |
| RWEQPYVKDGWS | 770 |
| EWYQPYALGWAR | 771 |
| GWWQPYARGL | 772 |
| LFEQPYAKALGL | 773 |
| GWEQPYARGLAG | 774 |
| AWVQPYATPLDE | 775 |
| MWYQPYSSQPAE | 776 |
| GWTQPYSOQGEV | 777 |
| DWFQPYSIQSDE | 778 |
| PWIQPYARGFG | 779 |
| RPLYWQPYSVQV | 780 |
| TLIYWQPYSVQI | 781 |
| RFDYWQPYSDQT | 782 |
| WHQFVQPYALPL | 783 |
| EWDS VYWQPYSVQ TLLR | 784 |
| WEQN VYWOPYSVO SFAD | 785 |
| SDV VYWQPYSVQ SLEM | 786 |
| YYDG VYWOPYSVQ VMPA | 787 |
| SDIWYO PYALPL | 788 |
| QRIWWQ PYALPL | 789 |
| SRIWWQ PYALPL | 790 |
| RSLYWO PYALPL | 791 |
| TIIWEQ PYALPL | 792 |
| WETWYO PYALPL | 793 |
| SYDWEQ PYALPL | 794 |
| SRIWCO PYALPL | 795 |
| EIMFWQ PYALPL | 796 |
| DYVWOO PYALPL | 797 |
| MDLLVO WYOPYALPL | 798 |
| GSKVIL WYOPYALPL | 799 |
| ROGANI WYQPYALPL | 800 |
| GGGDEP WYOPYALPL | 801 |
| SOLERT WYOPYALPL | 802 |
| ETWVRE WYOPYALPL | 803 |
| KKGSTQ WYOPYALPL | 804 |
| LOARMN WYOPYALPL | 805 |
| EPRSOK WYOPYALPL | 806 |
| VKQKWR WYOPYALPL | 807 |
| LRRHDV WYQPYALPL | 808 |
| RSTASI WYOPYALPL | 809 |
| ESKEDQ WYOPYALPL | 810 |
| EGLTMK WYQPYALPL | 811 |
| EGSREG WYQPYALPL | 812 |
| VIEWWQ PYALPL | 813 |
| VWYWEQ PYALPL | 814 |
| ASEWWQ PYALPL | 815 |
| FYEWWO PYALPL | 816 |
| EGWWVQ PYALPL | 817 |
| WGEWLQ PYALPL | 818 |
| DYVWEO PYALPL | 819 |
| AHTWWO PYALPL | 820 |
| FIEWFQ PYALPL | 821 |
| WLAWEQ PYALPL | 822 |
| VMEWWQ PYALPL | 823 |
| ERMWO PYALPL | 824 |
| NXXWXX PYALPL | 825 |
| WGNWYQ PYALPL | 826 |
| TLYWEQ PYALPL | 827 |
| VWRWEQ PYALPL | 828 |
| LLWTQ PYALPL | 829 |
| SRIWXX PYALPL | 830 |
| SDIWYQ PYALPL | 831 |
| WGYYXX PYALPL | 832 |
| TSGWYO PYALPL | 833 |
| VHPYXX PYALPL | 834 |
| EHSYFO PYALPL | 835 |
| XXIWYQ PYALPL | 836 |
| AOLHSO PYALPL | 837 |
| WANWFO PYALPL | 838 |
| SRLYSO PYALPL | 839 |
| GVTFSQ PYALPL | 840 |
| SIVWSQ PYALPL | 841 |
| SRDLVQ PYALPL | 842 |
| HWGH VYWQPYSVQ DDLG | 843 |
| SWHS VYWQPYSVQ SVPE | 844 |
| WRDS VYWQPYSVQ PESA | 845 |
| TWDA VYWQPYSVQ KWLD | 846 |
| TPPW VYWQPYSVQ SLDP | 847 |
| YWSS VYWQPYSVQ SVHS | 848 |
| YWY OPY ALGL | 849 |
| YWY QPY ALPL | 850 |
| EWI QPY ATGL | 851 |
| NWE QPY AKPL | 852 |
| AFY QPY ALPL | 853 |
| FLY QPY ALPL | 854 |
| VCK QPY LEWC | 855 |
| ETPFTWEESNAYYWQPYALPL | 856 |
| QGWLTWQDSVDMYWQPYALPL | 857 |
| FSEAGYTWPENTYWOPYALPL | 858 |
| TESPGGLDWAKIYWQPYALPL | 859 |
| DGYDRWRQSGERYWQPYALPL | 860 |
| TANVSSFEWTPGYWQPYALPL | 861 |
| SVGEDHNFWTSE YWQPYALPL | 862 |
| MNDQTSEVSTFP YWQPYALPL | 863 |
| SWSEAFEQPRNL YWQPYALPL | 864 |
| QYAEPSALNDWG YWQPYALPL | 865 |
| NGDWATADWSNY YWQPYALPL | 866 |
| THDEHI YWQPYALPL | 867 |
| MLEKTYTTWTPG YWQPYALPL | 868 |
| WSDPLTRDADL YWQPYALPL | 869 |
| SDAFTTQDSQAM YWQPYALPL | 870 |
| GDDAAWRTDSLT YWQPYALPL | 871 |
| AIIRQLYRWSEM YWQPYALPL | 872 |
| ENTYSPNWADSM YWQPYALPL | 873 |
| MNDQTSEVSTFP YWQPYALPL | 874 |
| SVGEDHNFWTSE YWQPYALPL | 875 |
| QTPFTWEESNAY YWQPYALPL | 876 |
| ENPFTWQESNAY YWQPYALPL | 877 |
| VTPFTWEDSNVF YWQPYALPL | 878 |
| QIPFTWEQSNAY YWQPYALPL | 879 |
| QAPLTWQESAAY YWQPYALPL | 880 |
| EPTFTWEESKAT YWQPYALPL | 881 |
| TTTLTWEESNAY YWQPYALPL | 882 |
| ESPLTWEESSAL YWQPYALPL | 883 |
| ETPLTWEESNAY YWQPYALPL | 884 |
| EATFTWAESNAY YWQPYALPL | 885 |
| EALFTWKESTAY YWQPYALPL | 886 |
| STP-TWEESNAY YWQPYALPL | 887 |
| ETPFTWEESNAY YWQPYALPL | 888 |
| KAPFTWEESQAY YWQPYALPL | 889 |
| STSFTWEESNAY YWQPYALPL | 890 |
| DSTFTWEESNAY YWQPYALPL | 891 |
| YIPFTWEESNAY YWQPYALPL | 892 |
| QTAFTWEESNAY YWQPYALPL | 893 |
| ETLFTWEESNAT YWQPYALPL | 894 |
| VSSFTWEESNAY YWQPYALPL | 895 |
| QPYALPL | 896 |
| Py-1-NapPYQJYALPL | 897 |
| TANVSSFEWTPG YWQPYALPL | 898 |
| FEWTPGYWQPYALPL | 899 |
| FEWTPGYWQJYALPL | 900 |
| FEWTPGYYQJYALPL | 901 |
| ETPFTWEESNAYYWQPYALPL | 902 |
| FTWEESNAYYWQJYALPL | 903 |
| ADVL YWQPYA PVTLWV | 904 |
| GDVAE YWQPYA LPLTSL | 905 |
| SWTDYG YWQPYA LPISGL | 906 |
| FEWTPGYWQPYALPL | 911 |
| FEWTPGYWQJYALPL | 912 |
| FEWTPGWYQPYALPL | 913 |
| FEWTPGWYOJYALPL | 914 |
| FEWTPGYYQPYALPL | 915 |
| FEWTPGYYQJYALPL | 916 |
| TANVSSFEWTPGYWQPYALPL | 918 |
| SWTDYGYWQPYALPISGL | 919 |
| ETPFTWEESNAYYWQPYALPL | 920 |
| ENTYSPNWADSMYWQPYALPL | 921 |
| SVGEDHNFWTSEYWQPYALPL | 922 |
| DGYDRWRQSGERYWQPYALPL | 923 |
| FEWTPGYWQPYALPL | 924 |
| FEWTPGYWQPY | 925 |
| FEWTPGYWQJY | 926 |
| EWTPGYWQPY | 927 |
| FEWTPGWYQJY | 928 |
| AEWTPGYWQJY | 929 |
| FAWTPGYWQJY | 930 |
| FEATPGYWQJY | 931 |
| FEWAPGYWQJY | 932 |
| FEWTAGYWQJY | 933 |
| FEWTPAYWQJY | 934 |
| FEWTPGAWQJY | 935 |
| FEWTPGYAQJY | 936 |
| FEWTPGYWQJA | 937 |
| FEWTGGYWQJY | 938 |
| FEWTPGYWQJY | 939 |
| FEWTJGYWQJY | 940 |
| FEWTPecGYWQJY | 941 |
| FEWTPAibYWQJY | 942 |
| FEWTPSarWYQJY | 943 |
| FEWTSarGYWQJY | 944 |
| FEWTPNYWQJY | 945 |
| FEWTPVYWQJY | 946 |
| FEWTVPYWQJY | 947 |
| AcFEWTPGWYQJY | 948 |
| AcFEWTPGYWQJY | 949 |
| lNap-EWTPGYYOJY | 950 |
| YEWTPGYYQJY | 951 |
| FEWVPGYYQJY | 952 |
| FEWTPGYYQJY | 953 |
| FEWTPsYYQJY | 954 |
| FEWTPnYYQJY | 955 |
| SHLY-Nap-QPYSVQM | 956 |
| TLVY-Nap-QPYSLQT | 957 |
| RGDY-Nap-QPYSVQS | 958 |
| NMVY-Nap-QPYSIQT | 959 |
| VYWQPYSVQ | 960 |
| VY-Nap-QPYSVQ | 961 |
| TFVYWQJYALPL | 962 |
| FEVVTPGYYQJ-Bpa | 963 |
| XaaFEWTPGYYQJ-Bpa | 964 |
| FEWTPGY-Bpa-QJY | 965 |
| AcFEWTPGY-Bpa-QJY | 966 |
| FEWTPG-Bpa-YQJY | 967 |
| AcFEWTPG-Bpa-YQJY | 968 |
| AcFE-Bpa-TPGYYQJY | 969 |
| AcFE-Bpa-TPGYYQJY | 970 |
| Bpa-EWTPGYYQJY | 971 |
| AcBpa-EWTPGYYQJY | 972 |
| VYWQPYSVQ | 973 |
| RLVYWQPYSVQR | 974 |
| RLVY-Nap-QPYSVQR | 975 |
| RLDYWQPYSVQR | 976 |
| RLVWFQPYSVQR | 977 |
| RLVYWQPYSIQR | 978 |
| DNSSWYDSFLL | 980 |
| DNTAWYESFLA | 981 |
| DNTAWYENFLL | 982 |
| PARE DNTAWYDSFLI WC | 983 |
| TSEY DNTTWYEKFLA SQ | 984 |
| SQIP DNTAWYQSFLL HG | 985 |
| SPFI DNTAWYENFLL TY | 986 |
| EQIY DNTAWYDHFLL SY | 987 |
| TPFI DNTAWYENFLL TY | 988 |
| TYTY DNTAWYERFLM SY | 989 |
| TMTQ DNTAWYENFLL SY | 990 |
| TI DNTAWYANLVQ TYPO | 991 |
| TI DNTAWYERFLA QYPD | 992 |
| HI DNTAWYENFLL TYTP | 993 |
| SQ DNTAWYENFLL SYKA | 994 |
| QI DNTAWYERFLL QYNA | 995 |
| NQ DNTAWYESFLL QYNT | 996 |
| TI DNTAWYENFLL NHNL | 997 |
| HY DNTAWYERFLQ QGWH | 998 |
| ETPFTWEESNAYYWQPYALPL | 999 |
| YIPFTWEESNAYYWQPYALPL | 1000 |
| DGYDRWRQSGERYWQPYALPL | 1001 |
| pY-INap-pY-QJYALPL | 1002 |
| TANVSSFEWTPGYWQPYALPL | 1003 |
| FEWTPGYWQJYALPL | 1004 |
| FEWTPGYWQPYALPLSD | 1005 |
| FEWTPGYYQJYALPL | 1006 |
| FEWTPGYWQJY | 1007 |
| AcFEWTPGYWQJY | 1008 |
| AcFEWrPGWYQJY | 1009 |
| AcFEWTPGYYQJY | 1010 |
| AcFEWTPaYWQJY | 1011 |
| AcFEWTPaWYQJY | 1012 |
| AcFEWTPaYYQJY | 1013 |
| FEWTPGYYQJYALPL | 1014 |
| FEWTPGYWQJYALPL | 1015 |
| FEWTPGWYQJYALPL | 1016 |
| TANVSSFEWTPGYWQPYALPL | 1017 |
| AcFEWTPGYWQJY | 1018 |
| AcFEWTPGWYQJY | 1019 |
| AcFEWTPGYYQJY | 1020 |
| AcFEWTPAYWQJY | 1021 |
| AcFEWTPAWYQJY | 1022 |
| AcFEWTPAYYQJY | 1023 |

**Table 5-EPO-mimetic peptide sequences**

| **Sequence/structure** | **SEQ ID NO:** |
|---|---|
| YXCXXGPXTWXCXP | 83 |
| YXCXXGPXTWXCXP-YXCXXGPXTWXCXP | 84 |
| YXCXXGPXTWXCXP-A-YXCXXGPXTWXCXP | 85 |
| | 86 |
| | 86 |
| GGTYSCHPGPLTWVCKPQGG | 87 |
| GGDYHCRMGPLTWVCKPLGG | 88 |
| GGVYACRMGPITWVCSPLGG | 89 |
| VGNYMCHFGPITWVCRPGGG | 90 |
| GGLYLCRFGPVTWDCGYKGG | 91 |
| GGTYSCHFGPLTWVCKPQGG-GGTYSCHFGPLTWVCKPQGG | 92 |
| GGTYSCHFGPLTWVCKPQGG -A-GGTYSCHFGPLTWVCKPQGG | 93 |
| GGTYSCHFGPLTWVCKPQGGSSK | 94 |
| GGTYSCHFGPLTWVCKPQGGSSK-GGTYSCHFGPLTWVCKPQGGSSK | 95 |
| GGTYSCHFGPLTWVCKPQGGSSK-A- | 96 |
| GGTYSCHFGPLTWVCKPQGGSSK | |
| | 97 |
| | 97 |
| GGTYSCHFGPLTWVCKPQGGSSK(-A-biotin) | 98 |
| CX₄X₅GPX₆TWX₇C | 421 |
| GGTYSCHGPLTWVCKPQGG | 422 |
| VGNYMAHMGPITWVCRPGG | 423 |
| GGPHHVYACRMGPLTWIC | 424 |
| GGTYSCHFGPLTWVCKPQ | 425 |
| GGLYACHMGPMTWVCQPLRG | 426 |
| TIAQYICYMGPETWECRPSPKA | 427 |
| YSCHFGPLTWVCK | 428 |
| YCHFGPLTWVC | 429 |
| X₃X₄X₅GPX₆TWX₇X₈ | 124 |
| YX₂X₃X₄X₅GPX₆TWX₇X₈ | 461 |
| X₁YX₂X₃X₄X₅GPX₆TWX₇X₈X₉X₁₀X₁₁ | 419 |
| X₁YX₂CX₄X₅GPX₆TWX₇CX₉X₁₀X₁₁ | 420 |
| GGLYLCRFGPVTWDCGYKGG | 1024 |
| GGTYSCHFGPLTWVCKPQGG | 1025 |
| GGDYHCRMGPLTWVCKPLGG | 1026 |
| VGNYMCHFGPITWVCRPGGG | 1029 |
| GGVYACRMGPITWVCSPLGG | 1030 |
| VGNYMAHMGPITWVCRPGG | 1035 |
| GGTYSCHFGPLTWVCKPQ | 1036 |
| GGLYACHMGPMTWVCQPLRG | 1037 |
| TIAQYICYMGPETWECRPSPKA | 1038 |
| YSCHFGPLTWVCK | 1039 |
| YCHFGPLTWVC | 1040 |
| SCHFGPLTWVCK | 1041 |
| (AX₂)ₙX₃X₄X₅GPX₆TWX₇X₈ | 1042 |

**Table 6-TPO-mimetic peptide sequences**

| **Sequence/structure** | **SEQ ID NO**: |
|---|---|
| IEGPTLRQWLAARA | 13 |
| IEGPTLRQWLAAKA | 24 |
| IEGPTLREWLAARA | 25 |
| IEGPTLRQWLAARA-A-IEGPTLROWLAARA | 26 |
| IEGPTLROWLAAKA-A-IEGPTLROWLAAKA | 27 |
| | 28 |
| IEGPTLRQWLAARA-A-K(BrAc)-A-IEGPTLRQWLAARA | 29 |
| IEGPTLROWLAARA-A-K(PEG)-A-IEGPTLRQWLAARA | 30 |
| | 31 |
| | 31 |
| | 32 |
| | 32 |
| VRDQIXXXL | 33 |
| TLREWL | 34 |
| GRVRDQVAGW | 35 |
| GRVKDOIAOL | 36 |
| GVRDQVSWAL | 37 |
| ESVREQVMKY | 38 |
| SVRSQISASL | 39 |
| GVRETVYRHM | 40 |
| GVREVIVMHML | 41 |
| GRVRDQIWAAL | 42 |
| AGVRDOILIWL | 43 |
| GRVRDQIMLSL | 44 |
| GRVRDQI(X)₃L | 45 |
| CTLRQWLQGC | 46 |
| CTLQEFLEGC | 47 |
| CTRTEWLHGC | 48 |
| CTLREWLHGGFC | 49 |
| CTLREWVFAGLC | 50 |
| CTLRQWHLLGMC | 51 |
| CTLAEFLASGVEQC | 52 |
| CSLQEFLSHGGYVC | 53 |
| CTLREFLDPTTAVC | 54 |
| CTLKEWLVSHEVWC | 55 |
| CTLREWL(X)₂₋₆C | 56-60 |
| REGPTLRQWM | 61 |
| EGPTLRQWLA | 62 |
| ERGPFWAKAC | 63 |
| REGPRCVMWM | 64 |
| CGTEGPTLSTWLDC | 65 |
| CEQDGPTLLEWLKC | 66 |
| CELVGPSLMSWLTC | 67 |
| CLTGPFVTQWLYEC | 68 |
| CRAGPTLLEWLTLC | 69 |
| CADGPTLREWISFC | 70 |
| C(X)₁₋₂EGPTLREWL(X)₁₋₂C | 71-74 |
| GGCTLREWLHGGFCGG | 75 |
| GGCADGPTLREWISFCGG | 76 |
| GNADGPTLRQWLEGRRPKN | 77 |
| LAIEGPTLROWLHGNGRDT | 78 |
| HGRVGPTLREWKTQVATKK | 79 |
| TIKGPTLROWLKSREHTS | 80 |
| ISDGPTLKEWLSVTRGAS | 81 |
| SIEGPTLREWLTSRTPHS | 82 |

**Table 7-G-CSF-mimetic peptide sequences**

| **Sequence/structure** | **SEQ ID NO:** |
|---|---|
| EEDCK | 99 |
| | 99 |
| | 99 |
| EEDσK | 100 |
| | 100 |
| | 100 |
| pGluEDσK | 101 |
| | 101 |
| | 101 |
| PicSDσK | 102 |
| | 102 |
| | 102 |
| EEDCK-Λ-EEDCK | 103 |
| EEDXK-Λ-EEDXK | 104 |

**Table 8-TNF-antagonist peptide sequences**

| **Sequence/structure** | **SEQ ID NO**: |
|---|---|
| YCFTASENHCY | 106 |
| YCFTNSENHCY | 107 |
| YCFTRSENHCY | 108 |
| FCASENHCY | 109 |
| YCASENHCY | 110 |
| FCNSENHCY | 111 |
| FCNSENRCY | 112 |
| FCNSVENRCY | 113 |
| YCSQSVSNDCF | 114 |
| FCVSNDRCY | 115 |
| YCRKELGQVCY | 116 |
| YCKEPGOCY | 117 |
| YCRKEMGCY | 118 |
| FCRKEMGCY | 119 |
| YCWSQNLCY | 120 |
| YCELSOYLCY | 121 |
| YCWSQNYCY | 122 |
| YCWSQYLCY | 123 |
| DFLPHYKNTSLGHRP | 1085 |
| | NR |

**Table 9-Integrin-binding peptide sequences**

| **Sequence/structure** | **SEQ ID NO**: |
|---|---|
| RX₁ETX₂WX₃ | 441 |
| RX₁ETX₂WX₃ | 442 |
| RGDGX | 443 |
| CRGDGXC | 444 |
| CX₁X₂RLDX₃X₄C | 445 |
| CARRLDAPC | 446 |
| CPSRLDSPC | 447 |
| X₁X₂X₃RGDX₄X₅X₆ | 448 |
| CX₂CRGDCX₅C | 449 |
| CDCRGDCFC | 450 |
| CDCRGDCLC | 451 |
| CLCRGDCIC | 452 |
| X₁X₂DDX₄X₅X₇X₈ | 453 |
| X₁X₂X₃DDX₄X₅X₆X₇X₈ | 454 |
| CWDDGWLC | 455 |
| CWDDLWWLC | 456 |
| CWDDGLMC | 457 |
| CWDDGWMC | 458 |
| CSWDDGWLC | 459 |
| CPDDLWWLC | 460 |
| NGR | NR |
| GSL | NR |
| RGD | NR |
| CGRECPRLCQSSC | 1071 |
| CNGRCVSGCAGRC | 1072 |
| CLSGSLSC | 1073 |
| RGD | NR |
| NGR | NR |
| GSL | NR |
| NGRAHA | 1074 |
| CNGRC | 1075 |
| CDCRGDCFC | 1076 |
| CGSLVRC | 1077 |
| DLXXL | 1043 |
| RTDLDSLRTYTL | 1044 |
| RTDLDSLRTY | 1053 |
| RTDLDSLRT | 1054 |
| RTDLDSLR | 1078 |
| GDLDLLKLRLTL | 1079 |
| GDLHSLRQLLSR | 1080 |
| RDDLHMLRLQLW | 1081 |
| SSDLHALKKRYG | 1082 |
| RGDLKQLSELTW | 1083 |
| RGDLAALSAPPV | 1084 |

**Table 10-Selectin antagonist peptide sequences**

| **Sequence/structure** | **SEQ ID NO:** |
|---|---|
| DITWDQLWDLMK | 147 |
| DITWDELWKIMN | 148 |
| DYTWFELWDMMQ | 149 |
| QITWAQLWNMMK | 150 |
| DMTWHDLWTLMS | 151 |
| DYSWHDLWEMMS | 152 |
| EITWDQLWEVMN | 153 |
| HVSWEQLWDIMN | 154 |
| HITWDQLWRIMT | 155 |
| RNMSWLELWEHMK | 156 |
| AEWTWDQLWHVMNPAESQ | 157 |
| HRAEWLALWEQMSP | 158 |
| KKEDWLALWRIMSV | 159 |
| ITWDQLWDLMK | 160 |
| DITWDQLWDLMK | 161 |
| DITWDOLWDLMK | 162 |
| DITWDOLWDLMK | 163 |
| CQNRYTDLVAIQNKNE | 462 |
| AENWADNEPNNKRNNED | 463 |
| RKNNKTWTWVGTKKALTNE | 464 |
| KKALTNEAENWAD | 465 |
| CQXRYTDLVAIQNKXE | 466 |
| RKXNXXWTWVGTXKXLTEE | 467 |
| AENWADGEPNNKXNXED | 468 |
| CXXXYTXLVAIQNKXE | 469 |
| RKXXXXWXWVGTXKXLTXE | 470 |
| AXNWXXXEPNNXXXED | 471 |
| XKXKTXEAXNWXX | 472 |

**Table 11-Antipathogenic peptide sequences**

| **Sequence/structure** | **SEQ ID NO:** |
|---|---|
| GFFALIPKIISSPLFKTLLSAVGSALSSSGGQQ | 503 |
| GFFALIPKIISSPLFKTLLSAVGSALSSSGGQE | 504 |
| GFFALIPKIISSPLFKTLLSAV | 505 |
| GFFALIPKIISSPLFKTLLSAV | 506 |
| KGFFALIPKIISSPLFKTLLSAV | 507 |
| KKGFFALIPKIISSPLFKTLLSAV | 508 |
| KKGFFALIPKIISSPLFKTLLSAV | 509 |
| GFFALIPKIIS | 510 |
| GIGAVLKVLTTGLPALISWIKRKRQQ | 511 |
| GIGAVLKVLTTGLPALISWIKRKRQQ | 512 |
| GIGAVLKVLTTGLPALISWIKRKRQQ | 513 |
| GIGAVLKVLTTGLPALISWIKR | 514 |
| AVLKVLTTGLPAUSWIKR | 515 |
| KLLLLLKLLLLK | 516 |
| KLLLKLLLKLLK | 517 |
| KLLLKLKLKLLK | 518 |
| KKLLKLKLKLKK | 519 |
| KLLLKLLLKLLK | 520 |
| KLLLKLKLKLLK | 521 |
| KLLLLK | 522 |
| KLLLKLLK | 523 |
| KLLLKLKLKLLK | 524 |
| KLLLKLKLKLLK | 525 |
| KLLLKLKLKLLK | 526 |
| KAAAKAAAKAAK | 527 |
| KVVVKVVVKVVK | 528 |
| KVWKVKVKVVK | 529 |
| KVWKVKVKVK | 530 |
| KVVVKVKVKVVK | 531 |
| KLILKL | 532 |
| KVLHLL | 533 |
| LKLRLL | 534 |
| KPLHLL | 535 |
| KLILKLVR | 536 |
| KVFHLLHL | 537 |
| HKFRILKL | 538 |
| KPFHILHL | 539 |
| KIIIKIKIKIIK | 540 |
| KIIIKIKIKIIK | 541 |
| KIIIKIKIKIIK | 542 |
| KIPIKIKIKIPK | 543 |
| KIPIKIKIKIVK | 544 |
| RIIIRIRIRIIR | 545 |
| RIIIRIRIRIIR | 546 |
| RIIIRIRIRIIR | 547 |
| RIVIRIRIRLIR | 548 |
| RIIVRIRLRIIR | 549 |
| RIGIRLRVRIIR | 550 |
| KIVIRIRIRLIR | 551 |
| RIAVKWRLRFIK | 552 |
| KIGWKLRVRIIR | 553 |
| KKIGWLIIRVRR | 554 |
| RIVIRIRIRLIRIR | 555 |
| RIIVRIRLRIIRVR | 556 |
| RIGIRLRVRIIRRV | 557 |
| KIVIRIRARLIRIRIR | 558 |
| RIIVKIRLRIIKKIRL | 559 |
| KIGIKARVRIIRVKII | 560 |
| RIIVHIRLRIIHHIRL | 561 |
| HIGIKAHVRIIRVHII | 562 |
| RIYVKIHLRYIKKIRL | 563 |
| KIGHKARVHIIRYKII | 564 |
| RIYVKPHPRYIKKIRL | 565 |
| KPGHKARPHIIRYKII | 566 |
| KIVIRIRIRLIRIRIRKIV | 567 |
| RIIVKIRLRIIKKIRLIKK | 568 |
| KIGWKLRVRIIRVKIGRLR | 569 |
| KIVIRIRIRLIRIRIRKIVKVKRIR | 570 |
| RFAVKIRLRIIKKIRLIKKIRKRVIK | 571 |
| KAGWKLRVRIIRVKIGRLRKIGWKKRVRIK | 572 |
| RIYVKPHPRYIKKIRL | 573 |
| KPGHKARPHIIRYKII | 574 |
| KIVIRIRIRLIRIRIRKIV | 575 |
| RIIVKIRLRIIKKIRLIKK | 576 |
| RIYVSKISIYIKKIRL | 577 |
| KIVIFTRIRLTSIRIRSIV | 578 |
| KPIHKARPTIIRYKMI | 579 |
| cyclicCKGFFALIPKIISSPLFKTLLSAVC | 580 |
| CKKGFFALIPKIISSPLFKTLLSAVC | 581 |
| CKKKGFFALIPKIISSPLFKTLLSAVC | 582 |
| CyclicCRIVIRIRIRLIRIRC | 583 |
| CyclicCKPGHKARPHIIRYKIIC | 584 |
| CyclicCRFAVKIRLRIIKKIRLIKKIRKRVIKC | 585 |
| KLLLKLLL KLLKC | 586 |
| KLLLKLLLKLLK | 587 |
| KLLLKLKLKLLKC | 588 |
| KLLLKLLLKLLK | 589 |

**Table 12-VIP-mimetic peptide sequences**

| **Sequence/structure** | **SEQ ID NO:** |
|---|---|
| HSDAVFYDNYTR LRKQMAVKKYLN SILN | 590 |
| Nle HSDAVFYDNYTR LRKQMAVKKYLN SILN | 591 |
| X₁ X₁' X₁" X₂ | 592 |
| X₃SX₄LN | 593 |
| | 594 |
| KKYL | 595 |
| NSILN | 596 |
| KKYL | 597 |
| KKYA | 598 |
| AVKKYL | 599 |
| NSILN | 600 |
| KKYV | 601 |
| SILauN | 602 |
| KKYLNle | 603 |
| NSYLN | 604 |
| NSIYN | 605 |
| KKYLPPNSILN | 606 |
| LauKKYL | 607 |
| CapKKYL | 608 |
| KYL | NR |
| KKYNIe | 609 |
| VKKYL | 610 |
| LNSILN | 611 |
| YLNSILN | 612 |
| KKYLN | 613 |
| KKYLNS | 614 |
| KKYLNSI | 615 |
| KKYLNSIL | 616 |
| KKYL | 617 |
| KKYDA | 618 |
| AVKKYL | 619 |
| NSILN | 620 |
| KKYV | 621 |
| SILauN | 622 |
| NSYLN | 623 |
| NSIYN | 624 |
| KKYLNle | 625 |
| KKYLPPNSILN | 626 |
| KKYL | 627 |
| KKYDA | 628 |
| AVKKYL | 629 |
| NSILN | 630 |
| KKYV | 631 |
| SILauN | 632 |
| LauKKYL | 633 |
| CapKKYL | 634 |
| KYL | NR |
| KYL | NR |
| KKYNIe | 635 |
| VKKYL | 636 |
| LNSILN | 637 |
| YLNSILN | 638 |
| KKYLNle | 639 |
| KKYLN | 640 |
| KKYLNS | 641 |
| KKYLNSI | 642 |
| KKYLNSIL | 643 |
| KKKYLD | 644 |
| cyclicCKKYLC | 645 |
| | 646 |
| KKYA | 647 |
| WWTDTGLW | 648 |
| WWTDDGLW | 649 |
| WWDTRGLWVWTI | 650 |
| FWGNDGIWLESG | 651 |
| DWDQFGLWRGAA | 652 |
| RWDDNGLWVWL | 653 |
| SGMWSHYGIWMG | 654 |
| GGRWDQAGLWVA | 655 |
| KLWSEQGIWMGE | 656 |
| CWSMHGLWLC | 657 |
| GCWDNTGIWVPC | 658 |
| DWDTRGLWVY | 659 |
| SLWDENGAWI | 660 |
| KWDDRGLWMH | 661 |
| QAWNERGLWT | 662 |
| QWDTRGLWVA | 663 |
| WNVHGIWQE | 664 |
| SWDTRGLWVE | 665 |
| DWDTRGLWVA | 666 |
| SWGRDGLWIE | 667 |
| EWTDNGLWAL | 668 |
| SWDEKGLWSA | 669 |
| SWDSSGLWMD | 670 |

**Table 13-Mdm/hdm antagonist peptide sequences**

| **Sequence/structure** | **SEQ ID NO:** |
|---|---|
| TFSDLW | 130 |
| QETFSDLWKLLP | 131 |
| QPTFSDLWKLLP | 132 |
| QETFSDYWKLLP | 133 |
| QPTFSDYWKLLP | 134 |
| MPRFMDYWEGLN | 135 |
| VQNFIDYWTQQF | 136 |
| TGPAFTHYWATF | 137 |
| IDRAPTFRDHWFALV | 138 |
| PRPALVFADYWETLY | 139 |
| PAFSRFWSDLSAGAH | 140 |
| PAFSRFWSKLSAGAH | 141 |
| PXFXDYWXXL | 142 |
| QETFSDLWKLLP | 143 |
| QPTFSDLWKLLP | 144 |
| QETFSDYWKLLP | 145 |
| QPTFSDYWKLLP | 146 |

**Table 14-Calmodulin antagonist peptide sequences**

| **Sequence/structure** | **SEQ ID NO:** |
|---|---|
| SCVKWGKKEFCGS | 164 |
| SCWKYWGKECGS | 165 |
| SCYEWGKLRWCGS | 166 |
| SCLRWGKWSNCGS | 167 |
| SCWRWGKYQICGS | 168 |
| SCVSWGALKLCGS | 169 |
| SCIRWGQNTFCGS | 170 |
| SCWQWGNLKICGS | 171 |
| SCVRWGQLSICGS | 172 |
| LKKFNARRKLKGAILTTMLAK | 173 |
| RRWKKNFIAVSAANRFKK | 174 |
| RKWQKTGHAVRAIGRLSS | 175 |
| INLKALAALAKKIL | 176 |
| KIWSILAPLG17LVKLVA | 177 |
| LKKLLKLLKKLLKL | 178 |
| LKWKKLLKLLKKLLKKLL | 179 |
| AEWPSLTEIKTLSHFSV | 180 |
| AEWPSPTRVISTTYFGS | 181 |
| AELAHWPPVKTVLRSFT | 182 |
| AEGSWLQLLNLMKQMNN | 183 |
| AEWPSLTEIK | 184 |

**Table 15-Mast cell antagonists/Mast cell protease inhibitor peptide sequences**

| **Sequence/structure** | **SEQ ID NO:** |
|---|---|
| SGSGVLKRPLPILPVTR | 272 |
| RWLSSRPLPPLPLPPRT | 273 |
| GSGSYDTLALPSLPLHPMSS | 274 |
| GSGSYDTRALPSLPLHPMSS | 275 |
| GSGSSGVTMYPKLPPHWSMA | 276 |
| GSGSSGVRMYPKLPPHWSMA | 277 |
| GSGSSSMRMVPTIPGSAKHG | 278 |
| RNR | NR |
| QT | NR |
| RQK | NR |
| NRQ | NR |
| RQK | NR |
| RNRQKT | 436 |
| RNRQ | 437 |
| RNRQK | 438 |
| NRQKT | 439 |
| RQKT | 440 |

**Table 16-SH3 antagonist peptide sequences**

| **Sequence/structure** | **SEQ ID NO**: |
|---|---|
| RPLPPLP | 282 |
| RELPPLP | 283 |
| SPLPPLP | 284 |
| GPLPPLP | 285 |
| RPLPIPP | 286 |
| RPLPIPP | 287 |
| RRLPPTP | 288 |
| ROLPPTP | 289 |
| RPLPSRP | 290 |
| RPLPTRP | 291 |
| SRLPPLP | 292 |
| RALPSPP | 293 |
| RRLPRTP | 294 |
| RPVPPIT | 295 |
| ILAPPVP | 296 |
| RPLPMLP | 297 |
| RPLPILP | 298 |
| RPLPSLP | 299 |
| RPLPSLP | 300 |
| RPLPMIP | 301 |
| RPLPLIP | 302 |
| RPLPPTP | 303 |
| RSLPPLP | 304 |
| RPOPPPP | 305 |
| RQLPIPP | 306 |
| XXXRPLPPLPXP | 307 |
| XXXRPLPPIPXX | 308 |
| XXXRPLPPLPXX | 309 |
| RXXRPLPPLPXP | 310 |
| RXXRPLPPLPPP | 311 |
| PPPYPPPPIPXX | 312 |
| PPPYPPPPVPXX | 313 |
| LXXRPLPXΨP | 314 |
| ΨXXRPLPXLP | 315 |
| PPXΘXPPPΨP | 316 |
| +PPΨPXKPXWL | 317 |
| RPXΨPΨR+SXP | 318 |
| PPVPPRPXXTL | 319 |
| ΨPΨLPΨK | 320 |
| +ΘDXPLPXLP | 321 |

**Table 17-Somatostatin or cortistatin mimetic peptide sequences**

| **Sequence/structure** | **SEQ ID NO:** |
|---|---|
| X¹-X²-Asn-Phe-Phe-Trp-Lys-Thr-Phe-X³-Ser-X⁴ | 473, 1137-1146 |
| Asp Arg Met Pro Cys Arg Asn Phe Phe Trp Lys Thr Phe Ser Ser Cys Lys | 474 |
| Met Pro Cys Arg Asn Phe Phe Trp Lys Thr Phe Ser Ser Cys Lys | 475 |
| Cys Arg Asn Phe Phe Trp Lys Thr Phe Ser Ser Cys Lys | 476 |
| Asp Arg Met Pro Cys Arg Asn Phe Phe Trp Lys Thr Phe Ser Ser Cys | 477 |
| Met Pro Cys Arg Asn Phe Phe Trp Lys Thr Phe Ser Ser Cys | 478 |
| Cys Arg Asn Phe Phe Trp Lys Thr Phe Ser Ser Cys | 479 |
| Asp Arg Met Pro Cys Lys Asn Phe Phe Trp Lys Thr Phe Ser Ser Cys | 480 |
| Met Pro Cys Lys Asn Phe Phe Trp Lys Thr Phe Ser Ser Cys Lys | 481 |
| Cys Lys Asn Phe Phe Trp Lys Thr Phe Ser Ser Cys Lys | 482 |
| Asp Arg Met Pro Cys Lys Asn Phe Phe Trp Lys Thr Phe Ser Ser Cys | 483 |
| Met Pro Cys Lys Asn Phe Phe Trp Lys Thr Phe Ser Ser Cys | 484 |
| Cys Lys Asn Phe Phe Trp Lys Thr Phe Ser Ser Cys | 485 |
| Asp Arg Met Pro Cys Arg Asn Phe Phe Trp Lys Thr Phe Thr Ser Cys Lys | 486 |
| Met Pro Cys Arg Asn Phe Phe Trp Lys Thr Phe Thr Ser Cys Lys | 487 |
| Cys Arg Asn Phe Phe Trp Lys Thr Phe Thr Ser Cys Lys | 488 |
| Asp Arg Met Pro Cys Arg Asn Phe Phe Trp Lys Thr Phe Thr Ser Cys | 489 |
| Met Pro Cys Arg Asn Phe Phe Trp Lys Thr Phe Thr Ser Cys | 490 |
| Cys Arg Asn Phe Phe Trp Lys Thr Phe Thr Ser Cys | 491 |
| Asp Arg Met Pro Cys Lys Asn Phe Phe Trp Lys Thr Phe Thr Ser Cys Lys | 492 |
| Met Pro Cys Lys Asn Phe Phe Trp Lys Thr Phe Thr Ser Cys Lys | 493 |
| Cys Lys Asn Phe Phe Trp Lys Thr Phe Thr Ser Cys Lys | 494 |
| Asp Arg Met Pro Cys Lys Asn Phe Phe Trp Lys Thr Phe Thr Ser Cys | 495 |
| Met Pro Cys Lys Asn Phe Phe Trp Lys Thr Phe Thr Ser Cys | 496 |
| Cys Lys Asn Phe Phe Trp Lys Thr Phe Thr Ser Cys | 497 |

**Table 18-UKR antagonist peptide sequences**

| **Sequence/structure** | **SEQ ID NO**: |
|---|---|
| AEPMPHSLNFSOYLWYT | 196 |
| AEHTYSSLWDTYSPLAF | 197 |
| AELDLWMRHYPLSFSNR | 198 |
| AESSLWTRYAWPSMPSY | 199 |
| AEWHPGLSFGSYLWSKT | 200 |
| AEPALLNWSFFFNPGLH | 201 |
| AEWSFYNLHLPEPQTIF | 202 |
| AEPLDLWSLYSLPPLAM | 203 |
| AEPTLWQLYQFPLRLSG | 204 |
| AEISFSELMWLRSTPAF | 205 |
| AELSEADLWTTWFGMGS | 206 |
| AESSLWRIFSPSALMMS | 207 |
| AESLPTLTSILWGKESV | 208 |
| AETLFMDLWHDKHILLT | 209 |
| AEILNFPLWHEPLWSTE | 210 |
| AESOTGTLNTLFWNTLR | 211 |
| AEPVYQYELDSYLRSYY | 430 |
| AELDLSTFYDIQYLLRT | 431 |
| AEFFKLGPNGYVYLHSA | 432 |
| FKLXXXGYVYL | 433 |
| AESTYHHLSLGYMYTLN | 434 |
| YHXLXXGYMYT | 435 |

**Table 19-Macrophage and/or T-cell inhibiting peptide sequences**

| **Sequence/structure** | **SEQ ID NO**: |
|---|---|
| Xaa-Yaa-Arg | NR |
| Arg-Yaa-Xaa | NR |
| Xaa-Arg-Yaa | NR |
| Yaa-Arg-Xaa | NR |
| Ala-Arg | NR |
| Arg-Arg | NR |
| Asn-Arg | NR |
| Asp-Arg | NR |
| Cys-Arg | NR |
| Gln-Arg | NR |
| Glu-Arg | NR |
| Gly-Arg | NR |
| His-arg | NR |
| lle-Arg | NR |
| Leu-Arg | NR |
| Lys-Arg | NR |
| Met-Arg | NR |
| Phe-Arg | NR |
| Ser-Arg | NR |
| Thr-Arg | NR |
| Trp-Arg | NR |
| Tyr-Arg | NR |
| Val-Arg | NR |
| Ala-Glu-Arg | NR |
| Arg-Glu-Arg | NR |
| Asn-Glu-Arg | NR |
| Asp-Glu-Arg | NR |
| Cys-Glu-Arg | NR |
| Gln-Glu-Arg | NR |
| Glu-Glu-Arg | NR |
| Gly-Glu-Arg | NR |
| His-Glu-Arg | NR |
| Ile-Glu-Arg | NR |
| Leu-Glu-Arg | NR |
| Lys-Glu-Arg | NR |
| Met-Glu-Arg | NR |
| Phe-Glu-Arg | NR |
| Pro-Glu-Arg | NR |
| Ser-Glu-Arg | NR |
| Thr-Glu-Arg | NR |
| Trp-Glu-Arg | NR |
| Tyr-Glu-Arg | NR |
| Val-Glu-Arg | NR |
| Arg-Ala | NR |
| Arg-Asp | NR |
| Arg-Cys | NR |
| Arg-Gln | NR |
| Arg-Glu | NR |
| Arg-Gly | NR |
| Arg-His | NR |
| Arg-lle | NR |
| Arg-Leu | NR |
| Arg-Lys | NR |
| Arg-Met | NR |
| Arg-Phe | NR |
| Arg-Pro | NR |
| Arg-Ser | NR |
| Arg-Thr | NR |
| Arg-Trp | NR |
| Arg-Tyr | NR |
| Arg-Val | NR |
| Arg-Glu-Ala | NR |
| Arg-Glu-Asn | NR |
| Arg-Glu-Asp | NR |
| Arg-Glu-Cys | NR |
| Arg-Glu-Gin | NR |
| Arg-Glu-Glu | NR |
| Arg-Glu-Gly | NR |
| Arg-Glu-His | NR |
| Arg-Glu-Ile | NR |
| Arg-Glu-Leu | NR |
| Arg-Glu-Lys | NR |
| Arg-Glu-Met | NR |
| Arg-Glu-Phe | NR |
| Arg-Glu-Pro | NR |
| Arg-Glu-Ser | NR |
| Arg-Glu-Thr | NR |
| Arg-Glu-Trp | NR |
| Arg-Glu-Tyr | NR |
| Arg-Glu-Val | NR |
| Ala-Arg-Glu | NR |
| Arg-Arg-Glu | NR |
| Asn-Arg-Glu | NR |
| Asp-Arg-Glu | NR |
| Cys-Arg-Glu | NR |
| Gin-Arg-Glu | NR |
| Glu-Arg-Glu | NR |
| Gly-Arg-Glu | NR |
| His-Arg-Glu | NR |
| Ile-Arg-Glu | NR |
| Leu-Arg-Glu | NR |
| Lys-Arg-Glu | NR |
| Met-Arg-Glu | NR |
| Phe-Arg-Glu | NR |
| Pro-Arg-Glu | NR |
| Ser-Arg-Glu | NR |
| Thr-Arg-Glu | NR |
| Trp-Arg-Glu | NR |
| Tyr-Arg-Glu | NR |
| Val-Arg-Glu | NR |
| Glu-Arg-Ala, | NR |
| Glu-Arg-Arg | NR |
| Glu-Arg-Asn | NR |
| Glu-Arg-Asp | NR |
| Glu-Arg-Cys | NR |
| Glu-Arg-Gln | NR |
| Glu-Arg-Gly | NR |
| Glu-Arg-His | NR |
| Glu-Arg-Ile | NR |
| Glu-Arg-Leu | NR |
| Glu-Arg-Lys | NR |
| Glu-Arg-Met | NR |
| Glu-Arg-Phe | NR |
| Glu-Arg-Pro | NR |
| Glu-Arg-Ser | NR |
| Glu-Arg-Thr | NR |
| Glu-Arg-Trp | NR |
| Glu-Arg-Tyr | NR |
| Glu-Arg-Val | NR |

**Table 20-Additional Exemplary Pharmacologically Active Peptides**

| **Sequence/structure** | **SEQ ID NO**: | **Activity** |
|---|---|---|
| VEPNCDIHVMWEWECFERL | 1027 | VEGF-antagonist |
| GERWCFDGPLTWVCGEES | 398 | VEGF-antagonist |
| RGWVEICVADDNGMCVTEAQ | 1085 | VEGF-antagonist |
| GWDECDVARMWEWECFAGV | 1086 | VEGF- antagonist |
| GERWCFDGPRAWVCGWEI | 501 | VEGF- antagonist |
| EELWCFDGPRAWVCGYVK | 502 | VEGF- antagonist |
| RGWVEICAADDYGRCLTEAQ | 1031 | VEGF- antagonist |
| RGWVEICESDVWGRCL | 1087 | VEGF- antagonist |
| RGWVEICESDVWGRCL | 1088 | VEGF- antagonist |
| GGNECDIARMWEWECFERL | 1089 | VEGF- antagonist |
| RGWVEICAADDYGRCL | 1090 | VEGF-antagonist |
| CTTHWGFTLC | 1028 | MMP inhibitor |
| CLRSGXGC | 1091 | MMP inhibitor |
| CXXHWGFXXC | 1092 | MMP inhibitor |
| CXPXC | 1093 | MMP inhibitor |
| CRRHWGFEFC | 1094 | MMP inhibitor |
| STTHWGFTLS | 1095 | MMP inhibitor |
| CSLHWGFWWC | 1096 | CTLA4-mimetic |
| GFVCSGIFAVGVGRC | 125 | CTLA4-mimetic |
| APGVRLGCAVLGRYC | 126 | CTLA4-mimetic |
| LLGRMK | 105 | Antiviral (HBV) |
| ICVVQDWGHHRCTAGHMANLTSHASAI | 127 | C3b antagonist |
| ICVVQDWGHHRCT | 128 | C3b antagonist |
| CVVQDWGHHAC | 129 | C3b antagonist |
| STGGFDDVYDWARGVSSALTTTLVATR | 185 | Vinculin-binding |
| STGGFDDVYDWARRVSSALTTTLVATR | 186 | Vinculin-binding |
| SRGVNFSEWLYDMSAAMKEASNVPPSRRSR | 187 | Vinculin-binding |
| SSQNWDMEAGVEDLTAAMLGLLSTIHSSSR | 188 | Vinculin-binding |
| SSPSLYTQFLVNYESAATRIQDLLIASRPSR | 189 | Vinculin-binding |
| SSTGWVDLLGALORAADATRTSIPPSLONSR | 190 | Vinculin-binding |
| DVYTKKELIECARRVSEK | 191 | Vinculin-binding |
| EKGSYYPGSGIAQFHIDYNNVS | 192 | C4BP-binding |
| SGIAQFHIDYNNVSSAEGWHVN | 193 | C4BP-binding |
| LVTVEKGSYYPGSGIAQFHIDYNNVSSAEGWHVN | 194 | C4BP-binding |
| SGIAQFHIDYNNVS | 195 | C4BP-binding |
| LLGRMK | 279 | anti-HBV |
| ALLGRMKG | 280 | anti-HBV |
| LDPAFR | 281 | anti-HBV |
| CXXRGDC | 322 | inhibition of platelet aggregation |
| RPLPPLP | 323 | Src antagonist |
| PPVPPR | 324 | Src antagonist |
| XFXDXWXXLXX | 325 | Anti-cancer (particularly for sarcomas) |
| KACRRLFGPVDSEOLSRDCD | 326 | p16-mimetic |
| RERWNFDFVTETPLEGDFAW | 327 | p16-mimetic |
| KRRQTSMTDFYHSKRRLIFS | 328 | p16-mimetic |
| TSMTDFYHSKRRLIFSKRKP | 329 | p16-mimetic |
| RRLIF | 330 | p16-mimetic |
| KRRQTSATDFYHSKRRLIFSRQIKIWFQNRRMKWKK | 331 | p16-mimetic |
| KRRLIFSKRQIKIWFONRRMKWKK | 332 | p16-mimetic |
| | 498 | CAP37 mimetic/LPS binding |
| | 499 | CAP37 mimetic/LPS binding |
| | 500 | CAP37 mimetic/LPS binding |
| WHWRHRIPLQLAAGR | 1097 | carbohydrate (GD1 alpha) mimetic |
| LKTPRV | 1098 | β2GPI Ab binding |
| NTLKTPRV | 1099 | β2GPI Ab binding |
| NTLKTPRVGGC | 1100 | β2GPI Ab binding |
| KDKATF | 1101 | β2GPI Ab binding |
| KDKATFGCHD | 1102 | β2GPI Ab binding |
| KDKATFGCHDGC | 1103 | β2GPIAb binding |
| TLRVYK | 1104 | β2GPI Ab binding |
| ATLRVYKGG | 1105 | β2GPI Ab binding |
| CATLRVYKGG | 1106 | β2GPI Ab binding |
| INLKALAALAKKIL | 1107 | Membrane-transporting |
| GWT | NR | Membrane-transporting |
| GWTLNSAGYLLG | 1108 | Membrane-transporting |
| GWTLNSAGYLLGKINLKALAALAKKIL | 1109 | Membrane-transporting |

The present invention is also particularly useful with peptides having activity in treatment of:
- cancer, wherein the peptide is a VEGF-mimetic or a VEGF receptor antagonist, a HER2 agonist or antagonist, a CD20 antagonist and the like;
- asthma, wherein the protein of interest is a CKR3 antagonist, an IL-5 receptor antagonist, and the like;
- thrombosis, wherein the protein of interest is a GPIIb antagonist, a GPIIIa antagonist, and the like;
- autoimmune diseases and other conditions involving immune modulation, wherein the protein of interest is an IL-2 receptor antagonist, a CD40 agonist or antagonist, a CD40L agonist or antagonist, a thymopoietin mimetic and the like.

Vehicles. This invention requires the presence of at least one vehicle (F¹, F²) attached to a peptide through the N-terminus, C-terminus or a sidechain of one of the amino acid residues. Multiple vehicles may also be used; e.g., Fc's at each terminus or an Fc at a terminus and a PEG group at the other terminus or a sidechain.

An Fc domain is the preferred vehicle. The Fc domain may be fused to the N or C termini of the peptides or at both the N and C termini. For the TPO-mimetic peptides, molecules having the Fc domain fused to the N terminus of the peptide portion of the molecule are more bioactive than other such fusions, so fusion to the N terminus is preferred.

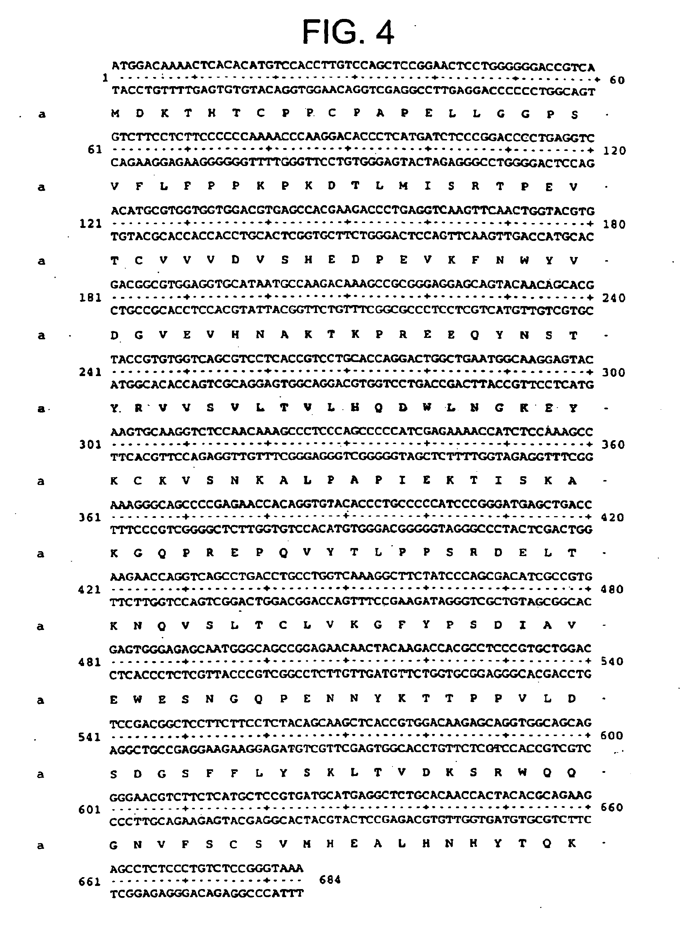

As noted above, Fc variants are suitable vehicles within the scope of this invention. A native Fc may be extensively modified to form an Fc variant in accordance with this invention, provided binding to the salvage receptor is maintained; see, for example WO 97/34631 and WO 96/32478. In such Fc variants, one may remove one or more sites of a native Fc that provide structural features or functional activity not required by the fusion molecules of this invention. One may remove these sites by, for example, substituting or deleting residues, inserting residues into the site, or truncating portions containing the site. The inserted or substituted residues may also be altered amino acids, such as peptidomimetics or D-amino acids. Fc variants may be desirable for a number of reasons, several of which are described below. Exemplary Fc variants include molecules and sequences in which:
1. Sites involved in disulfide bond formation are removed. Such removal may avoid reaction with other cysteine-containing proteins present in the host cell used to produce the molecules of the invention. For this purpose, the cysteine-containing segment at the N-terminus may be truncated or cysteine residues may be deleted or substituted with other amino acids (e.g., alanyl, seryl). In particular, one may truncate the N-terminal 20-amino acid segment of SEQ ID NO: 2 or delete or substitute the cysteine residues at positions 7 and 10 of SEQ ID NO: 2. Even when cysteine residues are removed, the single chain Fc domains can still form a dimeric Fc domain that is held together non-covalently.
2. A native Fc is modified to make it more compatible with a selected host cell. For example, one may remove the PA sequence near the N-terminus of a typical native Fc, which may be recognized by a digestive enzyme in E. coli such as proline iminopeptidase. One may also add an N-terminal methionine residue, especially when the molecule is expressed recombinantly in a bacterial cell such as E. coli. The Fc domain of SEQ ID NO: 2 (Figure 4) is one such Fc variant.
3. A portion of the N-terminus of a native Fc is removed to prevent N-terminal heterogeneity when expressed in a selected host cell. For this purpose, one may delete any of the first 20 amino acid residues at the N-terminus, particularly those at positions 1, 2, 3, 4 and 5.
4. One or more glycosylation sites are removed. Residues that are typically glycosylated (e.g., asparagine) may confer cytolytic response. Such residues may be deleted or substituted with unglycosylated residues (e.g., alanine).
5. Sites involved in interaction with complement, such as the C1q binding site, are removed. For example, one may delete or substitute the EKK sequence of human IgG1. Complement recruitment may not be advantageous for the molecules of this invention and so may be avoided with such an Fc variant.
6. Sites are removed that affect binding to Fc receptors other than a salvage receptor. A native Fc may have sites for interaction with certain white blood cells that are not required for the fusion molecules of the present invention and so may be removed.
7. The ADCC site is removed. ADCC sites are known in the art; see, for example, Molec. Immunol. 29 (5): 633-9 (1992) with regard to ADCC sites in IgG1. These sites, as well, are not required for the fusion molecules of the present invention and so may be removed.
8. When the native Fc is derived from a non-human antibody, the native Fc may be humanized. Typically, to humanize a native Fc, one will substitute selected residues in the non-human native Fc with residues that are normally found in human native Fc. Techniques for antibody humanization are well known in the art.

Preferred Fc variants include the following. In SEQ ID NO: 2 (Figure 4) the leucine at position 15 may be substituted with glutamate; the glutamate at position 99, with alanine; and the lysines at positions 101 and 103, with alanines. In addition, one or more tyrosine residues can be replaced by phenyalanine residues.

An alternative vehicle would be a protein, polypeptide, peptide, antibody, antibody fragment, , or small molecule (e.g., a peptidomimetic compound) capable of binding to a salvage receptor. For example, one could use as a vehicle a polypeptide as described in U.S. Pat. No. 5,739,277, issued April 14, 1998 to Presta et al. Peptides could also be selected by phage display for binding to the FcRn salvage receptor. Such salvage receptor-binding compounds are also included within the meaning of "vehicle" and are within the scope of this invention. Such vehicles should be selected for increased half-life (e.g., by avoiding sequences recognized by proteases) and decreased immunogenicity (e.g., by favoring non-immunogenic sequences, as discovered in antibody humanization).

As noted above, polymer vehicles may also be used for F¹ and F². Various means for attaching chemical moieties useful as vehicles are currently available, see, e.g., Patent Cooperation Treaty ("PCT") International Publication No. WO 96/11953, entitled "N-Terminally Chemically Modified Protein Compositions and Methods," herein incorporated by reference in its entirety. This PCT publication discloses, among other things, the selective attachment of water soluble polymers to the N-terminus of proteins.

A preferred polymer vehicle is polyethylene glycol (PEG). The PEG group may be of any convenient molecular weight and may be linear or branched. The average molecular weight of the PEG will preferably range from about 2 kiloDalton ("kD") to about 100 kDa, more preferably from about 5 kDa to about 50 kDa, most preferably from about 5 kDa to about 10 kDa. The PEG groups will generally be attached to the compounds of the invention via acylation or reductive alkylation through a reactive group on the PEG moiety (e.g., an aldehyde, amino, thiol, or ester group) to a reactive group on the inventive compound (e.g., an aldehyde, amino, or ester group).

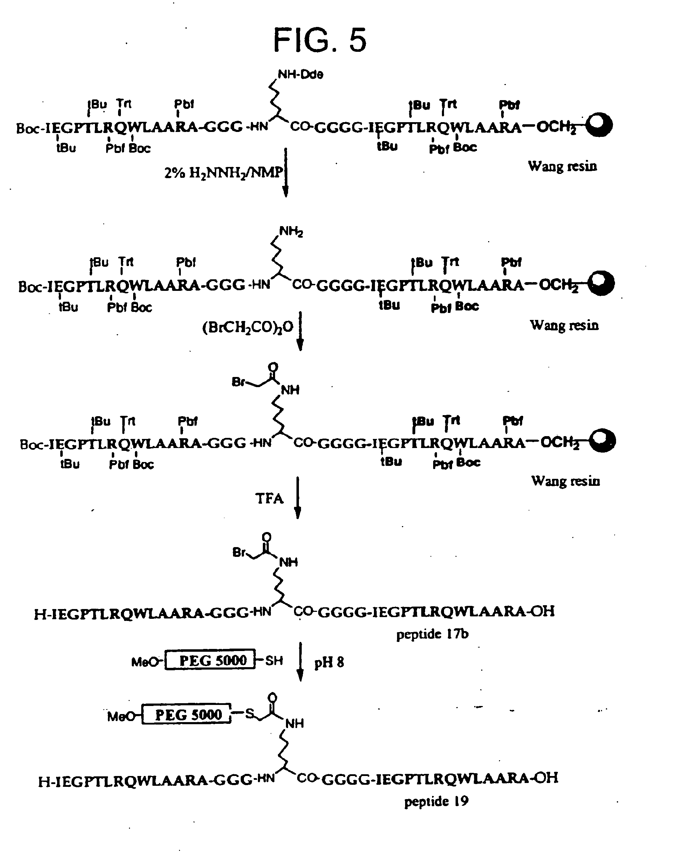

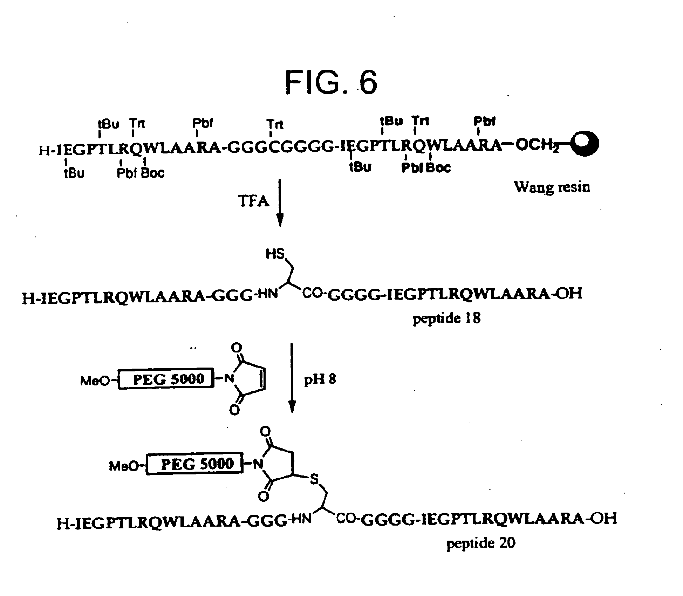

A useful strategy for the PEGylation of synthetic peptides consists of combining, through forming a conjugate linkage in solution, a peptide and a PEG moiety, each bearing a special functionality that is mutually reactive toward the other. The peptides can be easily prepared with conventional solid phase synthesis (see, for example, Figures 5 and 6 and the accompanying text herein). The peptides are "preactivated" with an appropriate functional group at a specific site. The precursors are purified and fully characterized prior to reacting with the PEG moiety. Ligation of the peptide with PEG usually takes place in aqueous phase and can be easily monitored by reverse phase analytical HPLC. The PEGylated peptides can be easily purified by preparative HPLC and characterized by analytical HPLC, amino acid analysis and laser desorption mass spectrometry.

Polysaccharide polymers are another type of water soluble polymer which may be used for protein modification. Dextrans are polysaccharide polymers comprised of individual subunits of glucose predominantly linked by α1-6 linkages. The dextran itself is available in many molecular weight ranges, and is readily available in molecular weights from about 1 kD to about 70 kD. Dextran is a suitable water soluble polymer for use in the present invention as a vehicle by itself or in combination with another vehicle (e.g., Fc). See, for example, WO 96/11953 and WO 96/05309. The use of dextran conjugated to therapeutic or diagnostic immunoglobulins has been reported; see, for example, European Patent Publication No. 0 315 456, which is hereby incorporated by reference. Dextran of about 1 kD to about 20 kD is preferred when dextran is used as a vehicle in accordance with the present invention.

Linkers. Any "linker" group is optional. When present, its chemical structure is not critical, since it serves primarily as a spacer. The linker is preferably made up of amino acids linked together by peptide bonds. Thus, in preferred embodiments, the linker is made up of from 1 to 20 amino acids linked by peptide bonds, wherein the amino acids are selected from the 20 naturally occurring amino acids. Some of these amino acids may be glycosylated, as is well understood by those in the art. In a more preferred embodiment, the 1 to 20 amino acids are selected from glycine, alanine, proline, asparagine, glutamine, and lysine. Even more preferably, a linker is made up of a majority of amino acids that are sterically unhindered, such as glycine and alanine. Thus, preferred linkers are polyglycines (particularly (Gly)₄, (Gly)₅), poly(Gly-Ala), and polyalanines. Other specific examples of linkers are:
(Gly)₃Lys(Gly)₄ (SEQ ID NO: 333);
(Gly)₃AsnGlySer(Gly)₂ (SEQ ID NO: 334);
(Gly)₃Cys(Gly)₄ (SEQ ID NO: 335); and
GlyProAsnGlyGly (SEQ ID NO: 336).
To explain the above nomenclature, for example, (Gly)₃Lys(Gly)₄ means Gly-Gly-Gly-Lys-Gly-Gly-Gly-Gly. Combinations of Gly and Ala are also preferred. The linkers shown here are exemplary; linkers within the scope of this invention may be much longer and may include other residues.

Non-peptide linkers are also possible. For example, alkyl linkers such as -NH-(CH₂)ₛ-C(O)-, wherein s = 2-20 could be used. These alkyl linkers may further be substituted by any non-sterically hindering group such as lower alkyl (e.g., C₁-C₆) lower acyl, halogen (e.g., Cl, Br), CN, NH₂, phenyl, etc. An exemplary non-peptide linker is a PEG linker, 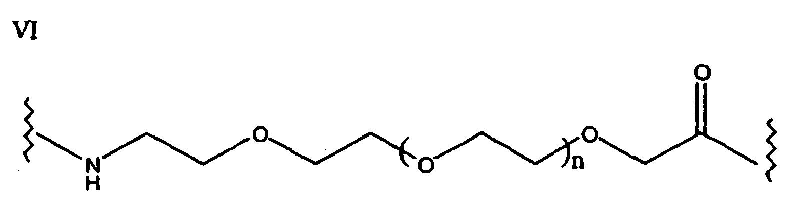 wherein n is such that the linker has a molecular weight of 100 to 5000 kD, preferably 100 to 500 kD. The peptide linkers may be altered to form derivatives in the same manner as described above.

Derivatives. The inventors also contemplate derivatizing the peptide and/or vehicle portion of the compounds. Such derivatives may improve the solubility, absorption, biological half life, and the like of the compounds. The moieties may alternatively eliminate or attenuate any undesirable side-effect of the compounds and the like. Exemplary derivatives include compounds in which:
1. The compound or some portion thereof is cyclic. For example, the peptide portion may be modified to contain two or more Cys residues (e.g., in the linker), which could cyclize by disulfide bond formation. For citations to references on preparation of cyclized derivatives, see Table 2.
2. The compound is cross-linked or is rendered capable of cross-linking between molecules. For example, the peptide portion may be modified to contain one Cys residue and thereby be able to form an intermolecular disulfide bond with a like molecule. The compound may also be cross-linked through its C-terminus, as in the molecule shown below.
3. 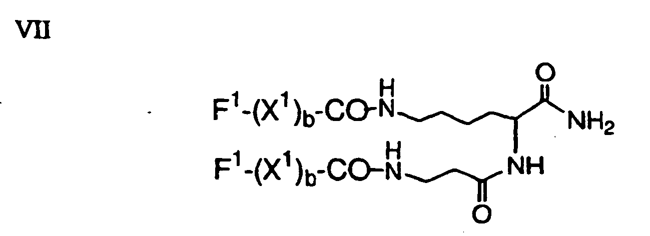
4. One or more peptidyl [-C(O)NR-] linkages (bonds) is replaced by a non-peptidyl linkage. Exemplary non-peptidyl linkages are -CH₂-carbamate [-CH₂-OC(O)NR-], phosphonate, -CH₂-sulfonamide [-CH₂-S(O)₂NR-], urea [-NHC(O)NH-], -CH₂-secondary amine, and alkylated peptide [-C(O)NR⁶- wherein R⁶ is lower alkyl].
5. The N-terminus is derivatized. Typically, the N-terminus may be acylated or modified to a substituted amine. Exemplary N-terminal derivative groups include -NRR¹ (other than -NH₂), -NRC(O)R¹, -NRC(O)OR¹, -NRS(O)₂R¹, -NHC(O)NHR¹, succinimide, or benzyloxycarbonyl-NH- (CBZ-NH-), wherein R and R¹ are each independently hydrogen or lower alkyl and wherein the phenyl ring may be substituted with 1 to 3 substituents selected from the group consisting of C₁-C₄ alkyl, C₁-C₄ alkoxy, chloro, and bromo.
6. The free C-terminus is derivatized. Typically, the C-terminus is esterified or amidated. For example, one may use methods described in the art to add (NH-CH₂-CH₂-NH₂)₂ to compounds of this invention having any of SEQ ID NOS: 504 to 508 at the C-terminus. Likewise, one may use methods described in the art to add -NH₂ to compounds of this invention having any of SEQ ID NOS: 924 to 955, 963 to 972, 1005 to 1013, or 1018 to 1023 at the C-terminus. Exemplary C-terminal derivative groups include, for example, -C(O)R² wherein R² is lower alkoxy or -NR³R⁴ wherein R³ and R⁴ are independently hydrogen or C₁-C₈ alkyl (preferably C₁-C₄ alkyl).
7. A disulfide bond is replaced with another, preferably more stable, cross-linking moiety (e.g., an alkylene). See, e.g., Bhatnagar et al. (1996), J. Med. Chem. 39: 3814-9; Alberts et al. (1993) Thirteenth Am. Pep. Symp., 357-9.
8. One or more individual amino acid residues is modified. Various derivatizing agents are known to react specifically with selected sidechains or terminal residues, as described in detail below.

Lysinyl residues and amino terminal residues may be reacted with succinic or other carboxylic acid anhydrides, which reverse the charge of the lysinyl residues. Other suitable reagents for derivatizing alpha-amino-containing residues include imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate.

Arginyl residues may be modified by reaction with any one or combination of several conventional reagents, including phenylglyoxal, 2,3-butanedione, 1,2-cyclohexanedione, and ninhydrin. Derivatization of arginyl residues requires that the reaction be performed in alkaline conditions because of the high pKa of the guanidine functional group. Furthermore, these reagents may react with the groups of lysine as well as the arginine epsilon-amino group.

Specific modification of tyrosyl residues has been studied extensively, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively.

Carboxyl sidechain groups (aspartyl or glutamyl) may be selectively modified by reaction with carbodiimides (R'-N=C=N-R') such as 1-cyclohexyl-3-(2-morpholinyl-(4-ethyl) carbodiimide or 1-ethyl-3-(4-azonia-4,4-dimethylpentyl) carbodiimide. Furthermore, aspartyl and glutamyl residues may be converted to asparaginyl and glutaminyl residues by reaction with ammonium ions.

Glutaminyl and asparaginyl residues may be deamidated to the corresponding glutamyl and aspartyl residues. Alternatively, these residues are deamidated under mildly acidic conditions. Either form of these residues falls within the scope of this invention.

Cysteinyl residues can be replaced by amino acid residues or other moieties either to eliminate disulfide bonding or, conversely, to stabilize cross-linking. See, e.g., Bhatnagar et al. (1996), J. Med. Chem. 39: 3814-9.

Derivatization with bifunctional agents is useful for cross-linking the peptides or their functional derivatives to a water-insoluble support matrix or to other macromolecular vehicles. Commonly used cross-linking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophenyl)dithio]propioiniidate yield photoactivatable intermediates that are capable of forming crosslinks in the presence of light. Alternatively, reactive water-insoluble matrices such as cyanogen bromide-activated carbohydrates and the reactive substrates described in U.S. Pat. Nos. 3,969,287; 3,691,016; 4,195,128; 4,247,642; 4,229,537; and 4,330,440 are employed for protein immobilization.

Carbohydrate (oligosaccharide) groups may conveniently be attached to sites that are known to be glycosylation sites in proteins. Generally, O-linked oligosaccharides are attached to serine (Ser) or threonine (Thr) residues while N-linked oligosaccharides are attached to asparagine (Asn) residues when they are part of the sequence Asn-X-Ser/Thr, where X can be any amino acid except proline. X is preferably one of the 19 naturally occurring amino acids other than proline. The structures of N-linked and O-linked oligosaccharides and the sugar residues found in each type are different. One type of sugar that is commonly found on both is N-acetylneuraminic acid (referred to as sialic acid). Sialic acid is usually the terminal residue of both N-linked and O-linked oligosaccharides and, by virtue of its negative charge, may confer acidic properties to the glycosylated compound. Such site(s) may be incorporated in the linker of the compounds of this invention and are preferably glycosylated by a cell during recombinant production of the polypeptide compounds (e.g., in mammalian cells such as CHO, BHK, COS). However, such sites may further be glycosylated by synthetic or semi-synthetic procedures known in the art.

Other possible modifications include hydroxylation of proline and lysine, phosphorylation of hydroxyl groups of seryl or threonyl residues, oxidation of the sulfur atom in Cys, methylation of the alpha-amino groups of lysine, arginine, and histidine side chains. Creighton, Proteins: Structure and Molecule Properties (W. H. Freeman & Co., San Francisco), pp. 79-86 (1983).

Compounds of the present invention may be changed at the DNA level, as well. The DNA sequence of any portion of the compound may be changed to codons more compatible with the chosen host cell. For E. coli, which is the preferred host cell, optimized codons are known in the art. Codons may be substituted to eliminate restriction sites or to include silent restriction sites, which may aid in processing of the DNA in the selected host cell. The vehicle, linker and peptide DNA sequences may be modified to include any of the foregoing sequence changes.

### Methods of Making

The compounds of this invention largely may be made in transformed host cells using recombinant DNA techniques. To do so, a recombinant DNA molecule coding for the peptide is prepared. Methods of preparing such DNA molecules are well known in the art. For instance, sequences coding for the peptides could be excised from DNA using suitable restriction enzymes. Alternatively, the DNA molecule could be synthesized using chemical synthesis techniques, such as the phosphoramidate method. Also, a combination of these techniques could be used.

The invention also includes a vector capable of expressing the peptides in an appropriate host. The vector comprises the DNA molecule that codes for the peptides operatively linked to appropriate expression control sequences. Methods of effecting this operative linking, either before or after the DNA molecule is inserted into the vector, are well known. Expression control sequences include promoters, activators, enhancers, operators, ribosomal binding sites, start signals, stop signals, cap signals, polyadenylation signals, and other signals involved with the control of transcription or translation.

The resulting vector having the DNA molecule thereon is used to transform an appropriate host. This transformation may be performed using methods well known in the art.

Any of a large number of available and well-known host cells may be used in the practice of this invention. The selection of a particular host is dependent upon a number of factors recognized by the art. These include, for example, compatibility with the chosen expression vector, toxicity of the peptides encoded by the DNA molecule, rate of transformation, ease of recovery of the peptides, expression characteristics, bio-safety and costs. A balance of these factors must be struck with the understanding that not all hosts may be equally effective for the expression of a particular DNA sequence. Within these general guidelines, useful microbial hosts include bacteria (such as E. coli sp.), yeast (such as Saccharomyces sp.) and other fungi, insects, plants, mammalian (including human) cells in culture, or other hosts known in the art.

Next, the transformed host is cultured and purified. Host cells may be cultured under conventional fermentation conditions so that the desired compounds are expressed. Such fermentation conditions are well known in the art. Finally, the peptides are purified from culture by methods well known in the art.

The compounds may also be made by synthetic methods. For example, solid phase synthesis techniques may be used. Suitable techniques are well known in the art, and include those described in Merrifield (1973), Chem. Polypeptides, pp. 335-61 (Katsoyannis and Panayotis eds.); Merrifield (1963), J. Am. Chem. Soc. 85: 2149; Davis et al. (1985), Biochem. Intl. 10: 394-414; Stewart and Young (1969), Solid Phase Peptide Synthesis; U.S. Pat. No. 3,941,763; Finn et al. (1976), The Proteins (3rd ed.) 2:105-253; and Erickson et al. (1976), The Proteins (3rd ed.) 2: 257-527. Solid phase synthesis is the preferred technique of making individual peptides since it is the most cost-effective method of making small peptides.

Compounds that contain derivatized peptides or which contain non-peptide groups may be synthesized by well-known organic chemistry techniques.

### Uses of the Compounds

In general. The compounds of this invention have pharmacologic activity resulting from their ability to bind to proteins of interest as agonists, mimetics or antagonists of the native ligands of such proteins of interest. The utility of specific compounds is shown in Table 2. The activity of these compounds can be measured by assays known in the art. For the TPO-mimetic and EPO-mimetic compounds, in vivo assays are further described in the Examples section herein.

In addition to therapeutic uses, the compounds of the present invention are useful in diagnosing diseases characterized by dysfunction of their associated protein of interest. In one embodiment, a method of detecting in a biological sample a protein of interest (e.g., a receptor) that is capable of being activated comprising the steps of: (a) contacting the sample with a compound of this invention; and (b) detecting activation of the protein of interest by the compound. The biological samples include tissue specimens, intact cells, or extracts thereof. The compounds of this invention may be used as part of a diagnostic kit to detect the presence of their associated proteins of interest in a biological sample. Such kits employ the compounds of the invention having an attached label to allow for detection. The compounds are useful for identifying normal or abnormal proteins of interest. For the EPO-mimetic compounds, for example, presence of abnormal protein of interest in a biological sample may be indicative of such disorders as Diamond Blackfan anemia, where it is believed that the EPO receptor is dysfunctional.

Therapeutic uses of EPO-mimetic compounds. The EPO-mimetic compounds of the invention are useful for treating disorders characterized by low red blood cell levels. Included in the invention are methods of modulating the endogenous activity of an EPO receptor in a mammal, preferably methods of increasing the activity of an EPO receptor. In general, any condition treatable by erythropoietin, such as anemia, may also be treated by the EPO-mimetic compounds of the invention. These compounds are administered by an amount and route of delivery that is appropriate for the nature and severity of the condition being treated and may be ascertained by one skilled in the art. Preferably, administration is by injection, either subcutaneous, intramuscular, or intravenous.

Therapeutic uses of TPO-mimetic compounds. For the TPO-mimetic compounds, one can utilize such standard assays as those described in WO95/26746 entitled "Compositions and Methods for Stimulating Megakaryocyte Growth and Differentiation". In vivo assays also appear in the Examples hereinafter.

The conditions to be treated are generally those that involve an existing megakaryocyte/platelet deficiency or an expected megakaryocyte/platelet deficiency (e.g., because of planned surgery or platelet donation). Such conditions will usually be the result of a deficiency (temporary or permanent) of active Mpl ligand in vivo. The generic term for platelet deficiency is thrombocytopenia, and hence the methods and compositions of the present invention are generally available for treating thrombocytopenia in patients in need thereof.

Thrombocytopenia (platelet deficiencies) may be present for various reasons, including chemotherapy and other therapy with a variety of drugs, radiation therapy, surgery, accidental blood loss, and other specific disease conditions. Exemplary specific disease conditions that involve thrombocytopenia and may be treated in accordance with this invention are: aplastic anemia, idiopathic thrombocytopenia, metastatic tumors which result in thrombocytopenia, systemic lupus erythematosus, splenomegaly, Fanconi's syndrome, vitamin B12 deficiency; folic acid deficiency, May-Hegglin anomaly, Wiskott-Aldrich syndrome, and paroxysmal nocturnal hemoglobinuria. Also, certain treatments for AIDS result in thrombocytopenia (e.g., AZT). Certain wound healing disorders might also benefit from an increase in platelet numbers.

With regard to anticipated platelet deficiencies, e.g., due to future surgery, a compound of the present invention could be administered several days to several hours prior to the need for platelets. With regard to acute situations, e.g., accidental and massive blood loss, a compound of this invention could be administered along with blood or purified platelets.

The TPO-mimetic compounds of this invention may also be useful in stimulating certain cell types other than megakaryocytes if such cells are found to express Mpl receptor. Conditions associated with such cells that express the Mpl receptor, which are responsive to stimulation by the Mpl ligand, are also within the scope of this invention.

The TPO-mimetic compounds of this invention may be used in any situation in which production of platelets or platelet precursor cells is desired, or in which stimulation of the c-Mpl receptor is desired. Thus, for example, the compounds of this invention may be used to treat any condition in a mammal wherein there is a need of platelets, megakaryocytes, and the like. Such conditions are described in detail in the following exemplary sources: WO95/26746; WO95/21919; WO95/18858; WO95/21920 and are incorporated herein.

The TPO-mimetic compounds of this invention may also be useful in maintaining the viability or storage life of platelets and/or megakaryocytes and related cells. Accordingly, it could be useful to include an effective amount of one or more such compounds in a composition containing such cells.

The therapeutic methods, compositions and compounds of the present invention may also be employed, alone or in combination with other cytokines, soluble Mpl receptor, hematopoietic factors, interleukins, growth factors or antibodies in the treatment of disease states characterized by other symptoms as well as platelet deficiencies. It is anticipated that the inventive compound will prove useful in treating some forms of thrombocytopenia in combination with general stimulators of hematopoiesis, such as IL-3 or GM-CSF. Other megakaryocytic stimulatory factors, i.e., meg-CSF, stem cell factor (SCF), leukemia inhibitory factor (LIF), oncostatin M (OSM), or other molecules with megakaryocyte stimulating activity may also be employed with Mpl ligand. Additional exemplary cytokines or hematopoietic factors for such co-administration include IL-1 alpha, IL-1 beta, IL-2, IL-3, IL-4, IL-5, IL-6, IL-11, colony stimulating factor-1 (CSF-1), SCF, GM-CSF, granulocyte colony stimulating factor (G-CSF), EPO, interferon-alpha (IFN-alpha), consensus interferon, IFN-beta, or IFN-gamma. It may further be useful to administer, either simultaneously or sequentially, an effective amount of a soluble mammalian Mpl receptor, which appears to have an effect of causing megakaryocytes to fragment into platelets once the megakaryocytes have reached mature form. Thus, administration of an inventive compound (to enhance the number of mature megakaryocytes) followed by administration of the soluble Mpl receptor (to inactivate the ligand and allow the mature megakaryocytes to produce platelets) is expected to be a particularly effective means of stimulating platelet production. The dosage recited above would be adjusted to compensate for such additional components in the therapeutic composition. Progress of the treated patient can be monitored by conventional methods.

In cases where the inventive compounds are added to compositions of platelets and/or megakaryocytes and related cells, the amount to be included will generally be ascertained experimentally by techniques and assays known in the art. An exemplary range of amounts is-0.1 µg-1 mg inventive compound per 10⁶ cells.

### Pharmaceutical Compositions

In General. The present invention also provides methods of using pharmaceutical compositions of the inventive compounds. Such pharmaceutical compositions may be for administration for injection, or for oral, pulmonary, nasal, transdermal or other forms of administration. In general, the invention encompasses pharmaceutical compositions comprising effective amounts of a compound of the invention together with pharmaceutically acceptable diluents, preservatives, solubilizers, emulsifiers, adjuvants and/or carriers. Such compositions include diluents of various buffer content (*e.g*., Tris-HCl, acetate, phosphate), pH and ionic strength; additives such as detergents and solubilizing agents (*e.g*., Tween 80, Polysorbate 80), anti-oxidants (*e.g*., ascorbic acid, sodium metabisulfite), preservatives (*e.g*., Thimersol, benzyl alcohol) and bulking substances (*e.g.*, lactose, mannitol); incorporation of the material into particulate preparations of polymeric compounds such as polylactic acid, polyglycolic acid, etc. or into liposomes. Hyaluronic acid may also be used, and this may have the effect of promoting sustained duration in the circulation. Such compositions may influence the physical state, stability, rate of in vivo release, and rate of in vivo clearance of the present proteins and derivatives. See, e.g., Remington's Pharmaceutical Sciences, 18th Ed. (1990, Mack Publishing Co., Easton, PA 18042) pages 1435-1712 which are herein incorporated by reference. The compositions may be prepared in liquid form, or may be in dried powder, such as lyophilized form. Implantable sustained release formulations are also contemplated, as are transdermal formulations.

Oral dosage forms. Contemplated for use herein are oral solid dosage forms, which are described generally in Chapter 89 of Remington's Pharmaceutical Sciences (1990),18th Ed., Mack Publishing Co. Easton PA 18042, which is herein incorporated by reference. Solid dosage forms include tablets, capsules, pills, troches or lozenges, cachets or pellets. Also, liposomal or proteinoid encapsulation may be used to formulate the present compositions (as, for example, proteinoid microspheres reported in U.S. Patent No. 4,925,673). Liposomal encapsulation may be used and the liposomes may be derivatized with various polymers (e.g., U.S. Patent No. 5,013,556). A description of possible solid dosage forms for the therapeutic is given in Chapter 10 of Marshall, K., Modern Pharmaceutics (1979), edited by G. S. Banker and C. T. Rhodes, herein incorporated by reference. In general, the formulation will include the inventive compound, and inert ingredients which allow for protection against the stomach environment, and release of the biologically active material in the intestine.

Also specifically contemplated are oral dosage forms of the above inventive compounds. If necessary, the compounds may be chemically modified so that oral delivery is efficacious. Generally, the chemical modification contemplated is the attachment of at least one moiety to the compound molecule itself, where said moiety permits (a) inhibition of proteolysis; and (b) uptake into the blood stream from the stomach or intestine. Also desired is the increase in overall stability of the compound and increase in circulation time in the body. Moieties useful as covalently attached vehicles in this invention may also be used for this purpose. Examples of such moieties include: PEG, copolymers of ethylene glycol and propylene glycol, carboxymethyl cellulose, dextran, polyvinyl alcohol, polyvinyl pyrrolidone and polyproline. See, for example, Abuchowski and Davis, Soluble Polymer-Enzyme Adducts, Enzymes as Drugs (1981), Hocenberg and Roberts, eds., Wiley-Interscience, New York, NY, , pp 367-83; Newmark, et al. (1982), J. Appl. Biochem. 4:185-9. Other polymers that could be used are poly-1,3-dioxolane and poly-1,3,6-tioxocane. Preferred for pharmaceutical usage, as indicated above, are PEG moieties.

For oral delivery dosage forms, it is also possible to use a salt of a modified aliphatic amino acid, such as sodium N-(8-[2-hydroxybenzoyl] amino) caprylate (SNAC), as a carrier to enhance absorption of the therapeutic compounds of this invention. The clinical efficacy of a heparin formulation using SNAC has been demonstrated in a Phase II trial conducted by Emisphere Technologies. See US Patent No. 5,792,451, "Oral drug delivery composition and methods".

The compounds of this invention can be included in the formulation as fine multiparticulates in the form of granules or pellets of particle size about 1 mm. The formulation of the material for capsule administration could also be as a powder, lightly compressed plugs or even as tablets. The therapeutic could be prepared by compression.

Colorants and flavoring agents may all be included. For example, the protein (or derivative) may be formulated (such as by liposome or microsphere encapsulation) and then further contained within an edible product, such as a refrigerated beverage containing colorants and flavoring agents.

One may dilute or increase the volume of the compound of the invention with an inert material. These diluents could include carbohydrates, especially mannitol, α-lactose, anhydrous lactose, cellulose, sucrose, modified dextrans and starch. Certain inorganic salts may also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrants include but are not limited to starch including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An antifrictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the compound of this invention into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethonium chloride. The list of potential nonionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the protein or derivative either alone or as a mixture in different ratios.

Additives may also be included in the formulation to enhance uptake of the compound. Additives potentially having this property are for instance the fatty acids oleic acid, linoleic acid and linolenic acid.

Controlled release formulation may be desirable. The compound of this invention could be incorporated into an inert matrix which permits release by either diffusion or leaching mechanisms e.g., gums. Slowly degenerating matrices may also be incorporated into the formulation, e.g., alginates, polysaccharides. Another form of a controlled release of the compounds of this invention is by a method based on the Oros therapeutic system (Alza Corp.), i.e., the drug is enclosed in a semipermeable membrane which allows water to enter and push drug out through a single small opening due to osmotic effects. Some enteric coatings also have a delayed release effect.

Other coatings may be used for the formulation. These include a variety of sugars which could be applied in a coating pan. The therapeutic agent could also be given in a film coated tablet and the materials used in this instance are divided into 2 groups. The first are the nonenteric materials and include methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, providone and the polyethylene glycols. The second group consists of the enteric materials that are commonly esters of phthalic acid.

A mix of materials might be used to provide the optimum film coating. Film coating may be carried out in a pan coater or in a fluidized bed or by compression coating.

Pulmonary delivery forms. Also contemplated herein is.pulmonary delivery of the present protein (or derivatives thereof). The protein (or derivative) is delivered to the lungs of a mammal while inhaling and traverses across the lung epithelial lining to the blood stream. (Other reports of this include Adjei et al., Pharma. Res. (1990) 7: 565-9; Adjei et al. (1990), Internatl. J. Pharmaceutics 63: 135-44 (leuprolide acetate); Braquet et al. (1989), J. Cardiovasc. Pharmacol. 13 (suppl.5): s.143-146 (endothelin-1); Hubbard et al. (1989), Annals Int. Med. 3: 206-12 (α1-antitrypsin); Smith et al. (1989), J. Clin. Invest. 84:1145-6 (α1-proteinase); Oswein et al. (March 1990), "Aerosolization of Proteins", Proc. Symp. Resp. Drug Delivery II, Keystone, Colorado (recombinant human growth hormone); Debs et al. (1988), J. Immunol. 140: 3482-8 (interferon-γ and tumor necrosis factor α) and Platz et al., U.S. Patent No. 5,284,656 (granulocyte colony stimulating factor).

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art. Some specific examples of commercially available devices suitable for the practice of this invention are the Ultravent nebulizer, manufactured by Mallinckrodt, Inc., St. Louis, Missouri; the Acorn II nebulizer, manufactured by Marquest Medical Products, Englewood, Colorado; the Ventolin metered dose inhaler, manufactured by Glaxo Inc., Research Triangle Park, North Carolina; and the Spinhaler powder inhaler, manufactured by Fisons Corp., Bedford, Massachusetts.

All such devices require the use of formulations suitable for the dispensing of the inventive compound. Typically, each formulation is specific to the type of device employed and may involve the use of an appropriate propellant material, in addition to diluents, adjuvants and/or carriers useful in therapy.

The inventive compound should most advantageously be prepared in particulate form with an average particle size of less than 10 µm (or microns), most preferably 0.5 to 5 µm, for most effective delivery to the distal lung.

Pharmaceutically acceptable carriers include carbohydrates such as trehalose, mannitol, xylitol, sucrose, lactose, and sorbitol. Other ingredients for use in formulations may include DPPC, DOPE, DSPC and DOPC. Natural or synthetic surfactants may be used. PEG may be used (even apart from its use in derivatizing the protein or analog). Dextrans, such as cyclodextran, may be used. Bile salts and other related enhancers may be used. Cellulose and cellulose derivatives may be used. Amino acids may be used, such as use in a buffer formulation.

Also, the use of liposomes, microcapsules or microspheres, inclusion complexes, or other types of carriers is contemplated.

Formulations suitable for use with a nebulizer, either jet or ultrasonic, will typically comprise the inventive compound dissolved in water at a concentration of about 0.1 to 25 mg of biologically active protein per mL of solution. The formulation may also include a buffer and a simple sugar (e.g., for protein stabilization and regulation of osmotic pressure). The nebulizer formulation may also contain a surfactant, to reduce or prevent surface induced aggregation of the protein caused by atomization of the solution in forming the aerosol.

Formulations for use with a metered-dose inhaler device will generally comprise a finely divided powder containing the inventive compound suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing the inventive compound and may also include a bulking agent, such as lactose, sorbitol, sucrose, mannitol, trehalose, or xylitol in amounts which facilitate dispersal of the powder from the device, e.g., 50 to 90% by weight of the formulation.

Nasal delivery forms. Nasal delivery of the inventive compound is also contemplated. Nasal delivery allows the passage of the protein to the blood stream directly after administering the therapeutic product to the nose, without the necessity for deposition of the product in the lung. Formulations for nasal delivery include those with dextran or cyclodextran. Delivery via transport across other mucous membranes is also contemplated.

Dosages. The dosage regimen involved in a method for treating the above-described conditions will be determined by the attending physician, considering various factors which modify the action of drugs, e.g. the age, condition, body weight, sex and diet of the patient, the severity of any infection, time of administration and other clinical factors. Generally, the daily regimen should be in the range of 0.1-1000 micrograms of the inventive compound per kilogram of body weight, preferably 0.1-150 micrograms per kilogram.

### Specific preferred embodiments

The inventors have determined preferred peptide sequences for molecules having many different kinds of activity. The inventors have further determined preferred structures of these preferred peptides combined with preferred linkers and vehicles. Preferred structures for these preferred peptides listed in Table 21 below.

**Table 21-Preferred embodiments**

| **Sequence/structure** | **SEQ ID NO:** | **Activity** |
|---|---|---|
| F¹-(G)₅-IEGPTLRQWLAARA-(G)₈-IEGPTLRQWLAARA | 337 | TPO-mimetic |
| IEGPTLRQWLAARA-(G)₈-IEGPTLRQWLAARA-(G)₅-F¹ | 338 | TPO-mimetic |
| F¹-(G)₅-IEGPTLRQWLAARA | 1032 | TPO-mimetic |
| IEGPTLRQWLAARA -(G)₅- F¹ | 1033 | TPO-mimetic |
| | 339 | EPO-mimetic |
| | 340 | EPO-mimetic |
| GGTYSCHFGPLTWVCKPQGG-(G)₅-F¹ | 1034 | EPO-mimetic |
| F¹-(G)₅-DFLPHYKNTSLGHRP | 1045 | TNF-α inhibitor |
| DFLPHYKNTSLGHRP-(G)₅-F¹ | 1046 | TNF-α inhibitor |
| F¹-(G)₅- FEWTPGYWQPYALPL | 1047 | IL-1 R antagonist |
| FEWTPGYWQPYALPL-(G)₅-F¹ | 1048 | IL-1 R antagonist |
| F¹-(G)₅-VEPNCDIHVMWEWECFERL | 1049 | VEGF-antagonist |
| VEPNCDIHVMWEWECFERL-(G)₅-F¹ | 1050 | VEGF-antagonist |
| F¹-(G)₅-CTTHWGFTLC | 1051 | MMP inhibitor |
| CTTHWGFTLC-(G)₅-F¹ | 1052 | MMP inhibitor |

| | | |
|---|---|---|
| "F¹" is an Fc domain as defined previously herein. | | |

### Working examples

The compounds described above may be prepared as described below. These examples comprise preferred embodiments of the invention and are illustrative rather than limiting.

### Example 1

### TPO-Mimetics

The following example uses peptides identified by the numbers appearing in Table A hereinafter.

Preparation of peptide 19. Peptide 17b (12 mg) and MeO-PEG-SH 5000 (30 mg, 2 equiv.) were dissolved in 1 ml aqueous buffer (pH 8). The mixture was incubated at RT for about 30 minutes and the reaction was checked by analytical HPLC, which showed a > 80% completion of the reaction. The pegylated material was isolated by preparative HPLC.

Preparation of peptide 20. Peptide 18 (14 mg) and MeO-PEG-maleimide (25 mg) were dissolved in about 1.5 ml aqueous buffer (pH 8). The mixture was incubated at RT for about 30 minutes, at which time about 70% transformation was complete as monitored with analytical HPLC by applying an aliquot of sample to the HPLC column. The pegylated material was purified by preparative HPLC.

Bioactivity assay. The TPO in vitro bioassay is a mitogenic assay utilizing an IL-3 dependent clone of murine 32D cells that have been transfected with human mpl receptor. This assay is described in greater detail in WO 95/26746. Cells are maintained in MEM medium containing 10% Fetal Clone II and 1 ng/ml mIL-3. Prior to sample addition, cells are prepared by rinsing twice with growth medium lacking mIL-3. An extended twelve point TPO standard curve is prepared, ranging from 33 to 39 pg/ml. Four dilutions, estimated to fall within the linear portion of the standard curve, (100 to 125 pg/ml), are prepared for each sample and run in triplicate. A volume of 100 µl of each dilution of sample or standard is added to appropriate wells of a 96 well microtiter plate containing 10,000 cells/well. After forty-four hours at 37°C and 10% CO₂, MTS (a tetrazolium compound which is bioreduced by cells to a formazan) is added to each well. Approximately six hours later, the optical density is read on a plate reader at 490 nm. A dose response curve (log TPO concentration vs. O.D.- Background) is generated and linear regression analysis of points which fall in the linear portion of the standard curve is performed. Concentrations of unknown test samples are determined using the resulting linear equation and a correction for the dilution factor.

TMP tandem repeats with polyglycine linkers. Our design of sequentially linked TMP repeats was based on the assumption that a dimeric form of TMP was required for its effective interaction with c-Mpl (the TPO receptor) and that depending on how they were wound up against each other in the receptor context, the two TMP molecules could be tethered together in the C- to N-terminus configuration in a way that would not perturb the global dimeric conformation. Clearly, the success of the design of tandem linked repeats depends on proper selection of the length and composition of the linker that joins the C- and N-termini of the two sequentially aligned TMP monomers. Since no structural information of the TMP bound to c-Mpl was available, a series of repeated peptides with linkers composed of 0 to 10 and 14 glycine residues (Table A) were synthesized. Glycine was chosen because of its simplicity and flexibility, based on the rationale that a flexible polyglycine peptide chain might allow for the free folding of the two tethered TMP repeats into the required conformation, while other amino acid sequences may adopt undesired secondary structures whose rigidity might disrupt the correct packing of the repeated peptide in the receptor context.

The resulting peptides are readily accessible by conventional solid phase peptide synthesis methods (Merrifield (1963), J. Amer. Chem. Soc. 85: 2149) with either Fmoc or t-Boc chemistry. Unlike the synthesis of the C-terminally linked parallel dimer which required the use of an orthogonally protected lysine residue as the initial branch point to build the two peptide chains in a pseudosymmetrical way (Cwirla et al. (1997), Science 276:1696-9), the synthesis of these tandem repeats was a straightforward, stepwise assembly of the continuous peptide chains from the C- to N-terminus. Since dimerization of TMP had a more dramatic effect on the proliferative activity than binding affinity as shown for the C-terminal dimer (Cwirla et al. (1997)), the synthetic peptides were tested directly for biological activity in a TPO-dependent cell-proliferation assay using an IL-3 dependent clone of murine 32D cells transfected with the full-length c-Mpl (Palacios et al., Cell 41:727 (1985)). As the test results showed, all the polyglycine linked tandem repeats demonstrated >1000 fold increases in potency as compared to the monomer, and were even more potent than the C-terminal dimer in this cell proliferation assay. The absolute activity of the C-terminal dimer in our assay was lower than that of the native TPO protein, which is different from the previously reported findings in which the C-terminal dimer was found to be as active as the natural ligand (Cwirla et al. (1997)). This might be due to differences in the conditions used in the two assays. Nevertheless, the difference in activity between tandem (C terminal of first monomer linked to N terminal of second monomer) and C-terminal (C terminal of first monomer linked to C terminal of second monomer; also referred to as parallel) dimers in the same assay clearly demonstrated the superiority of tandem repeat strategy over parallel peptide dimerization. It is interesting to note that a wide range of length is tolerated by the linker. The optimal linker between tandem peptides with the selected TMP monomers apparently is composed of 8 glycines.

Other tandem repeats. Subsequent to this first series of TMP tandem repeats, several other molecules were designed either with different linkers or containing modifications within the monomer itself. The first of these molecules, peptide 13, has a linker composed of GPNG, a sequence known to have a high propensity to form a β-turn-type secondary structure. Although still about 100-fold more potent than the monomer, this peptide was found to be >10-fold less active than the equivalent GGGG-linked analog. Thus, introduction of a relatively rigid β-turn at the linker region seemed to have caused a slight distortion of the optimal agonist conformation in this short linker form.

The Trp9 in the TMP sequence is a highly conserved residue among the active peptides isolated from random peptide libraries. There is also a highly conserved Trp in the consensus sequences of EPO mimetic peptides and this Trp residue was found to be involved in the formation of a hydrophobic core between the two EMPs and contributed to hydrophobic interactions with the EPO receptor. Livnah et al. (1996), Science 273: 464-71). By analogy, the Trp9 residue in TMP might have a similar function in dimerization of the peptide ligand, and as an attempt to modulate and estimate the effects of noncovalent hydrophobic forces exerted by the two indole rings, several analogs were made resulting from mutations at the Trp. So in peptide 14, the Trp residue was replaced in each of the two TMP monomers with a Cys, and an intramolecular disulfide bond was formed between the two cysteines by oxidation which was envisioned to mimic the hydrophobic interactions between the two Trp residues in peptide dimerization. Peptide 15 is the reduced form of peptide 14. In peptide 16, the two Trp residues were replaced by Ala. As the assay data show, all three analogs were inactive. These data further demonstrated that Trp is critical for the activity of the TPO mimetic peptide, not just for dimer formation.

The next two peptides (peptide 17a, and 18) each contain in their 8-amino acid linker a Lys or Cys residue. These two compounds are precursors to the two PEGylated peptides (peptide 19 and 20) in which the side chain of the Lys or Cys is modified by a PEG moiety. A PEG moiety was introduced at the middle of a relatively long linker, so that the large PEG component (5 kDa) is far enough away from the critical binding sites in the peptide molecule. PEG is a known biocompatible polymer which is increasingly used as a covalent modifier to improve the pharmacokinetic profiles of peptide- and protein-based therapeutics.

A modular, solution-based method was devised for convenient PEGylation of synthetic or recombinant peptides. The method is based on the now well established chemoselective ligation strategy which utilizes the specific reaction between a pair of mutually reactive functionalities. So, for pegylated peptide 19, the lysine side chain was preactivated with a bromoacetyl group to give peptide 17b to accommodate reaction with a thiol-derivatized PEG. To do that, an orthogonal protecting group, Dde, was employed for the protection of the lysine ε-amine. Once the whole peptide chain was assembled, the N-terminal amine was reprotected with t-Boc. Dde was then removed to allow for the bromoacetylation. This strategy gave a high quality crude peptide which was easily purified using conventional reverse phase HPLC. Ligation of the peptide with the thiol-modified PEG took place in aqueous buffer at pH 8 and the reaction completed within 30 minutes. MALDI-MS analysis of the purified, pegylated material revealed a characteristic, bell-shaped spectrum with an increment of 44 Da between the adjacent peaks. For PEG-peptide 20, a cysteine residue was placed in the linker region and its side chain thiol group would serve as an attachment site for a maleimide-containing PEG. Similar conditions were used for the pegylation of this peptide. As the assay data revealed, these two pegylated peptides had even higher in vitro bioactivity as compared to their unpegylated counterparts.

Peptide 21 has in its 8-amino acid linker a potential glycosylation motif, NGS. Since our exemplary tandem repeats are made up of natural amino acids linked by peptide bonds, expression of such a molecule in an appropriate eukaryotic cell system should produce a glycopeptide with the carbohydrate moiety added on the side chain carboxyamide of Asn. Glycosylation is a common post-translational modification process which can have many positive impacts on the biological activity of a given protein by increasing its aqueous solubility and in vivo stability. As the assay data show, incorporation of this glycosylation motif into the linker maintained high bioactivity. The synthetic precursor of the potential glycopeptide had in effect an activity comparable to that of the -(G)₈-linked analog. Once glycosylated, this peptide is expected to have the same order of activity as the pegylated peptides, because of the similar chemophysical properties exhibited by a PEG and a carbohydrate moiety.

The last peptide is a dimer of a tandem repeat. It was prepared by oxidizing peptide 18, which formed an intermolecular disulfide bond between the two cysteine residues located at the linker. This peptide was designed to address the possibility that TMP was active as a tetramer. The assay data showed that this peptide was not more active than an average tandem repeat on an adjusted molar basis, which indirectly supports the idea that the active form of TMP is indeed a dimer, otherwise dimerization of a tandem repeat would have a further impact on the bioactivity.

In order to confirm the in vitro data in animals, one pegylated TMP tandem repeat (compound 20 in Table A) was delivered subcutaneously to normal mice via osmotic pumps. Time and dose-dependent increases were seen in platelet numbers for the duration of treatment. Peak platelet levels over 4-fold baseline were seen on day 8. A dose of 10 µg/kg/day of the pegylated TMP repeat produced a similar response to rHuMGDF (non-pegylated) at 100 µg/kg/day delivered by the same route.

**Table A-TPO-mimetic Peptides**

| **Peptide No.** | **Compound** | **SEQ ID NO:** | **RelativePotency** |
|---|---|---|---|
| | TPO | | ++++ |
| | TMP monomer | 13 | + |
| | TMP C-C dimer | | +++- |
| TMP-(G)ₙ-TMP: | | | |
| 1 | n = 0 | 341 | ++++- |
| 2 | n=1 | 342 | ++++ |
| 3 | n=2 | 343 | ++++ |
| 4 | n=3 | 344 | ++++ |
| 5 | n=4 | 345 | ++++ |
| 6 | n = 5 | 346 | ++++ |
| 7 | n=6 | 347 | ++++ |
| 8 | n=7 | 348 | ++++ |
| 9 | n=8 | 349 | ++++- |
| 10 | n = 9 | 350 | ++++ |
| 11 | n = 10 | 351 | ++++ |
| 12 | n = 14 | 352 | ++++ |
| 13 | TMP-GPNG-TMP | 353 | +++ |
| 14 | 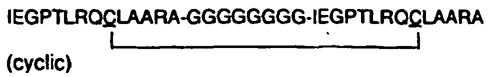 | 354 | - |
| 15 | 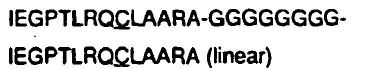 | 355 | - |
| 16 | 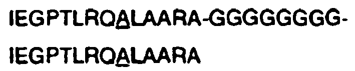 | 356 | - |
| 17a | TMP-GGGKGGGG-TMP | 357 | ++++ |
| 17b | TMP-GGGK(BrAc)GGGG-TMP | 358 | ND |
| 18 | TMP-GGGCGGGG-TMP | 359 | ++++ |
| 19 | TMP-GGGK(PEG)GGGG-TMP | 360 | +++++ |
| 20 | TMP-GGGC(PEG)GGGG-TMP | 361 | +++++ |
| 21 | TMP-GGGN*GSGG-TMP | 362 | ++++ |
| 22 | 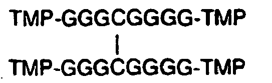 | 363 | |
| | | 363 | ++++ |

Discussion. It is well accepted that MGDF acts in a way similar to hGH, i.e., one molecule of the protein ligand binds two molecules of the receptor for its activation. Wells et al.(1996), Ann. Rev. Biochem. 65: 609-34. Now, this interaction is mimicked by the action of a much smaller peptide, TMP. However, the present studies suggest that this mimicry requires the concerted action of two TMP molecules, as covalent dimerization of TMP in either a C-C parallel or C-N sequential fashion increased the in vitro biological potency of the original monomer by a factor of greater than 10³. The relatively low biopotency of the monomer is probably due to inefficient formation of the noncovalent dimer. A preformed covalent repeat has the ability to eliminate the entropy barrier for the formation of a noncovalent dimer which is exclusively driven by weak, noncovalent interactions between two molecules of the small, 14-residue peptide.

It is intriguing that this tandem repeat approach had a similar effect on enhancing bioactivity as the reported C-C dimerization is intriguing. These two strategies brought about two very different molecular configurations. The C-C dimer is a quasi-symmetrical molecule, while the tandem repeats have no such symmetry in their linear structures. Despite this difference in their primary structures, these two types of molecules appeared able to fold effectively into a similar biologically active conformation and cause the dimerization and activation of c-Mpl. These experimental observations provide a number of insights into how the two TMP molecules may interact with one another in binding to c-Mpl. First, the two C-termini of the two bound TMP molecules must be in relatively close proximity with each other, as suggested by data on the C-terminal dimer. Second, the respective N- and C-termini of the two TMP molecules in the receptor complex must also be very closely aligned with each other, such that they can be directly tethered together with a single peptide bond to realize the near maximum activity-enhancing effect brought about by the tandem repeat strategy. Insertion of one or more (up to 14) glycine residues at the junction did not increase (or decrease) significantly the activity any further. This may be due to the fact that a flexible polyglycine peptide chain is able to loop out easily from the junction without causing any significant changes in the overall conformation. This flexibility seems to provide the freedom of orientation for the TMP peptide chains to fold into the required conformation in interacting with the receptor and validate it as a site of modification. Indirect evidence supporting this came from the study on peptide 13, in which a much more rigid b-turn-forming sequence as the linker apparently forced a deviation of the backbone alignment around the linker which might have resulted in a slight distortion of the optimal conformation, thus resulting in a moderate (10-fold) decrease in activity as compared with the analogous compound with a 4-Gly linker. Third, Trp9 in TMP plays a similar role as Trp13 in EMP, which is involved not only in peptide:peptide interaction for the formation of dimers but also is important for contributing hydrophobic forces in peptide:receptor interaction. Results obtained with the W to C mutant analog, peptide 14, suggest that a covalent disulfide linkage is not sufficient to approximate the hydrophobic interactions provided by the Trp pair and that, being a short linkage, it might bring the two TMP monomers too close, therefore perturbing the overall conformation of the optimal dimeric structure.

An analysis of the possible secondary structure of the TMP peptide can provide further understanding on the interaction between TMP and c-Mpl. This can be facilitated by making reference to the reported structure of the EPO mimetic peptide. Livnah et al. (1996), Science 273:464-75 The receptor-bound EMP has a b-hairpin structure with a b-turn formed by the highly consensus Gly-Pro-Leu-Thr at the center of its sequence. Instead of GPLT, TMP has a highly selected GPTL sequence which is likely to form a similar turn. However, this turn-like motif is located near the N-terminal part in TMP. Secondary structure prediction using Chau-Fasman method suggests that the C-terminal half of the peptide has a tendency to adopt a helical conformation. Together with the highly conserved Trp at position 9, this C-terminal helix may contribute to the stabilization of the dimeric structure. It is interesting to note that most of our tandem repeats are more potent than the C-terminal parallel dimer. Tandem repeats seem to give the molecule a better fit conformation than does the C-C parallel dimerization. The seemingly asymmetric feature of a tandem repeat might have brought it closer to the natural ligand which, as an asymmetric molecule, uses two different sites to bind two identical receptor molecules.

Introduction of a PEG moiety was envisaged to enhance the in vivo activity of the modified peptide by providing it a protection against proteolytic degradation and by slowing down its clearance through renal filtration. It was unexpected that pegylation could further increase the in vitro bioactivity of a tandem repeated TMP peptide in the cell-based proliferation assay.

### Example 2

### Fc-TMP fusions

TMPs (and EMPs as described in Example 3) were expressed in either monomeric or dimeric form as either N-terminal or C-terminal fusions to the Fc region of human IgG1. In all cases, the expression construct utilized the luxPR promoter promoter in the plasmid expression vector pAMG21.

Fc-TMP. A DNA sequence coding for the Fc region of human IgG1 fused in-frame to a monomer of the TPO-mimetic peptide was constructed using standard PCR technology. Templates for PCR reactions were the pFc-A3 vector and a synthetic TMP gene. The synthetic gene was constructed from the 3 overlapping oligonucleotides (SEQ ID NOS: 364, 365, and 366, respectively) shown below:

| | |
|---|---|
| 1842-97 | 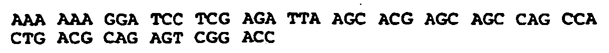 |
| 1842-98 | AAA GGT GGA GGT GGT GGT ATC GAA GGT CCG ACT CTG CGT |
| 1842-99 | 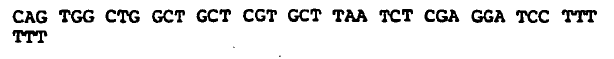 |

These oligonucleotides were annealed to form the duplex encoding an amino acid sequence (SEQ ID NOS: 367 and 368, respectively) shown below: 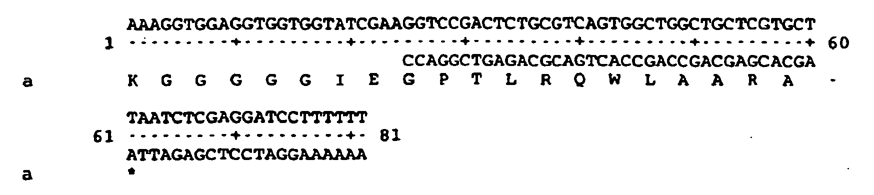 This duplex was amplified in a PCR reaction using 1842-98 and 1842-97 as the sense and antisense primers.

The Fc portion of the molecule was generated in a PCR reaction with pFc-A3 using the primers shown below (SEQ ID NOS: 369 and 370):

| | |
|---|---|
| 1216-52 | AAC ATA AGT ACC TGT AGG ATC G |
| 1830-51 | TTCGATACCA CCACCTCCAC CTTTACCCGG AGACAGGGAG AGGCTCTTCTGC |

The oligonucleotides 1830-51 and 1842-98 contain an overlap of 24 nucleotides, allowing the two genes to be fused together in the correct reading frame by combining the above PCR products in a third reaction using the outside primers, 1216-52 and 1842-97.

The final PCR gene product (the full length fusion gene) was digested with restriction endonucleases XbaI and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such clone was selected and designated Amgen strain #3728.

The nucleotide and amino acid sequences (SEQ ID NOS: 5 and 6) of the fusion protein are shown in Figure 7.

Fc-TMP-TMP. A DNA sequence coding for the Fc region of human IgG1 fused in-frame to a dimer of the TPO-mimetic peptide was constructed using standard PCR technology. Templates for PCR reactions were the pFc-A3 vector and a synthetic TMP-TMP gene. The synthetic gene was constructed from the 4 overlapping oligonucleotides (SEQ ID NOS: 371 to 374, respectively) shown below:

| | |
|---|---|
| 1830-52 | |
| 1830-53 | |
| 1830-54 | |
| 1830-55 | |

The 4 oligonucleotides were annealed to form the duplex encoding an amino acid sequence (SEQ ID NOS: 375 and 376, respectively) shown below: This duplex was amplified in a PCR reaction using 1830-52 and 1830-55 as the sense and antisense primers.

The Fc portion of the molecule was generated in a PCR reaction with pFc-A3 using the primers 1216-52 and 1830-51 as described above for Fc-TMP. The full length fusion gene was obtained from a third PCR reaction using the outside primers 1216-52 and 1830-55.

The final PCR gene product (the full length fusion gene) was digested with restriction endonucleases XbaI and BamHI, and then ligated into the vector pAMG21 and transformed into competent E. coli strain 2596 cells as described in example 1. Clones were screened for the ability to produce the recombinant protein product and to possess the gene fusion having the correct nucleotide sequence. A single such clone was selected and designated Amgen strain #3727.

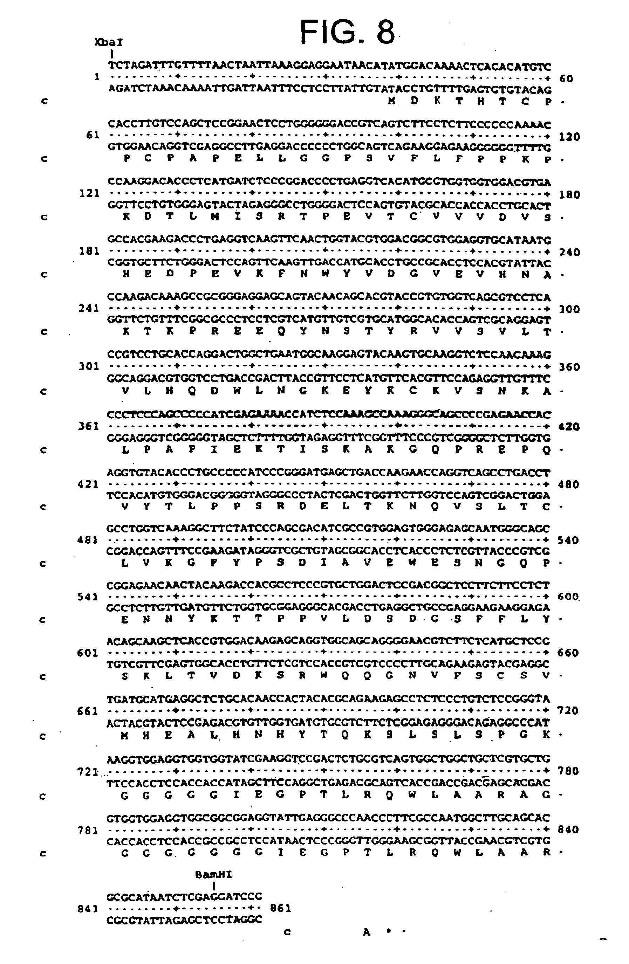

The nucleotide and amino acid sequences (SEQ ID NOS: 7 and 8) of the fusion protein are shown in Figure 8.

TMP-TMP-Fc. A DNA sequence coding for a tandem repeat of the TPO-mimetic peptide fused in-frame to the Fc region of human IgG1 was constructed using standard PCR technology. Templates for PCR reactions were the EMP-Fc plasmid from strain #3688 (see Example 3) and a synthetic gene encoding the TMP dimer. The synthetic gene for the tandem repeat was constructed from the 7 overlapping oligonucleotides shown below (SEQ ID NOS: 377 to 383, respectively):

| | |
|---|---|
| 1885-52 | TTT TTT CAT ATG ATC GAA GGT CCG ACT CTG CGT CAG TGG |
| 1885-53 | |
| 1885-54 | |
| 1885-55 | |
| 1885-56 | |
| 1885-57 | |
| 1885-58 | CCC ACC GCC TCC CCC TGC GCG TGC TGC |

These oligonucleotides were annealed to form the duplex shown encoding an amino acid sequence shown below (SEQ ID NOS 384 and 385): This duplex was amplified in a PCR reaction using 1885-52 and 1885-58 as the sense and antisense primers.

The Fc portion of the molecule was generated in a PCR reaction with DNA from the EMP-Fc fusion strain #3688 (see Example 3) using the primers 1885-54 and 1200-54. The full length fusion gene was obtained from a third PCR reaction using the outside primers 1885-52 and 1200-54.

The final PCR gene product (the full length fusion gene) was digested with restriction endonucleases XbaI and BamHI, and then ligated into the vector pAMG21 and transformed into competent E. coli strain 2596 cells as described for Fc-EMP herein. Clones were screened for the ability to produce the recombinant protein product and to possess the gene fusion having the correct nucleotide sequence. A single such clone was selected and designated Amgen strain #3798.

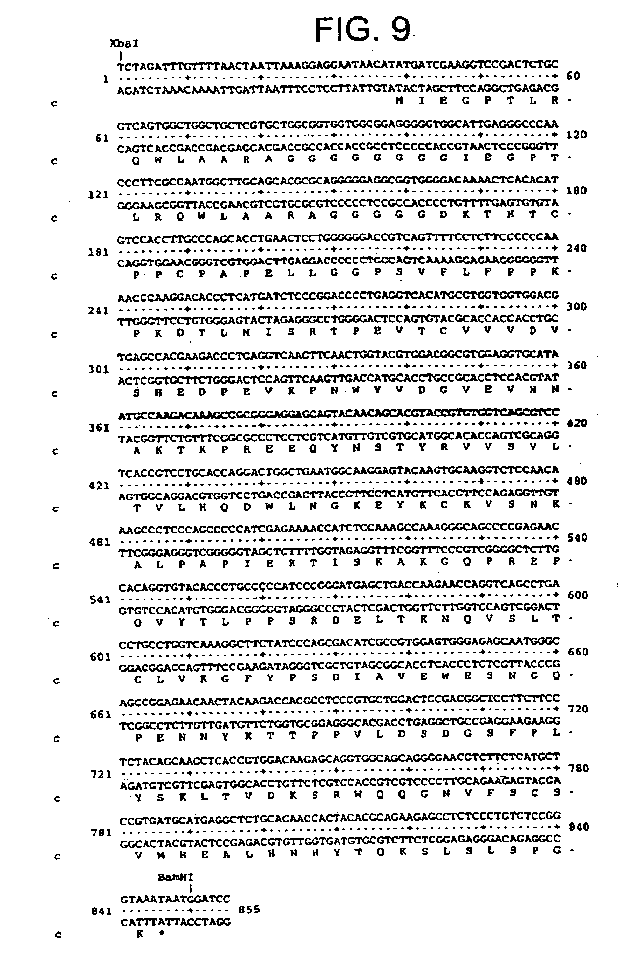

The nucelotide and amino acid sequences (SEQ ID NOS: 9 and 10) of the fusion protein are shown in Figure 9.

TMP-Fc. A DNA sequence coding for a monomer of the TPO-mimetic peptide fused in-frame to the Fc region of human IgG1 was obtained fortuitously in the ligation in TMP-TMP-Fc, presumably due to the ability of primer 1885-54 to anneal to 1885-53 as well as to 1885-58. A single clone having the correct nucleotide sequence for the TMP-Fc construct was selected and designated Amgen strain #3788.

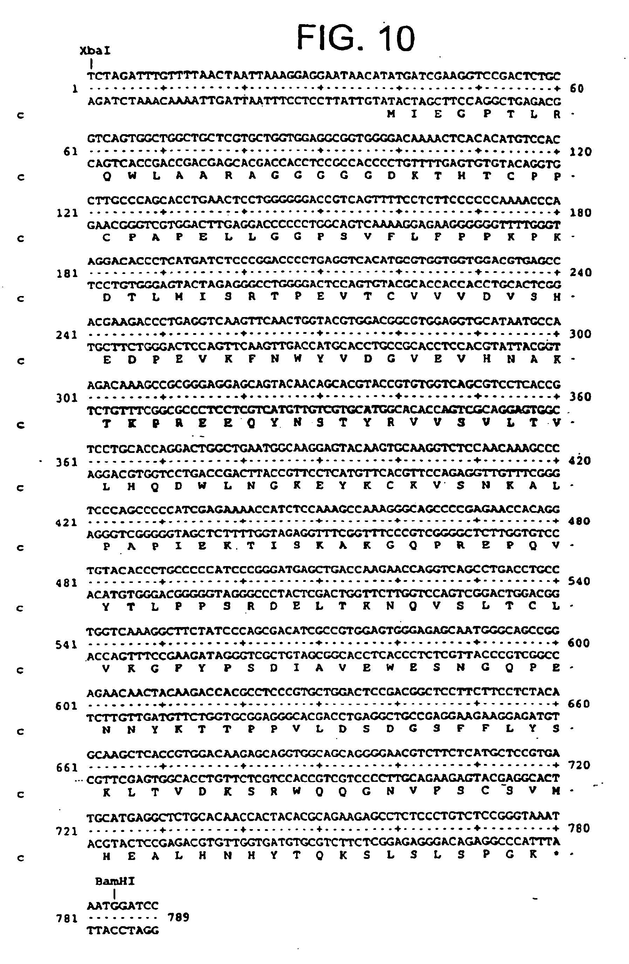

The nucleotide and amino acid sequences (SEQ ID NOS:11 and 12) of the fusion protein are shown in Figure 10.

Expression in E. coli. Cultures of each of the pAMG21-Fc-fusion constructs in E. coli GM221 were grown at 37 °C in Luria Broth medium containing 50 mg/ml kanamycin. Induction of gene product expression from the luxPR promoter was achieved following the addition of the synthetic autoinducer N-(3-oxohexanoyl)-DL-homoserine lactone to the culture media to a final concentration of 20 ng/ml. Cultures were incubated at 37 °C for a further 3 hours. After 3 hours, the bacterial cultures were examined by microscopy for the presence of inclusion bodies and were then collected by centrifugation. Refractile inclusion bodies were observed in induced cultures indicating that the Fc-fusions were most likely produced in the insoluble fraction in E. coli. Cell pellets were lysed directly by resuspension in Laemmli sample buffer containing 10% b-mercaptoethanol and were analyzed by SDS-PAGE. In each case, an intense coomassie-stained band of the appropriate molecular weight was observed on an SDS-PAGE gel.

pAMG21. The expression plasmid pAMG21 can be derived from the Amgen expression vector pCFM1656 (ATCC #69576) which in turn be derived from the Amgen expression vector system described in US Patent No. 4,710,473. The pCFM1656 plasmid can be derived from the described pCFM836 plasmid (Patent No. 4,710,473) by:
(a) destroying the two endogenous Ndel restriction sites by end filling with T4 polymerase enzyme followed by blunt end ligation;
(b) replacing the DNA sequence between the unique AatII and ClaI restriction sites containing the synthetic P_{L} promoter with a similar fragment obtained from pCFM636 (patent No. 4,710,473) containing the PL promoter (see SEQ ID NO: 386 below); and
(c) substituting the small DNA sequence between the unique ClaI and KpnI restriction sites with the oligonucleotide having the sequence of SEQ ID NO: 388.
The expression plasmid pAMG21 can then be derived from pCFM1656 by making a series of site-directed base changes by PCR overlapping oligo mutagenesis and DNA sequence substitutions. Starting with the BglII site (plasmid bp # 180) immediately 5' to the plasmid replication promoter P_{copB} and proceeding toward the plasmid replication genes, the base pair changes are as shown in Table B below.

**Table B-Base pair changes resulting in pAMG21**

| pAMG21 bp # | bp in pCFM1656 | bp changed to in pAMG21 |
|---|---|---|
| # 204 | T/A | C/G |
| # 428 | A/T | G/C |
| # 509 | G/C | A/T |
| # 617 | - - | insert two G/C bp |
| # 679 | G/C | T/A |
| # 980 | T/A | C/G |
| # 994 | G/C | A/T |
| # 1004 | A/T | C/G |
| # 1007 | C/G | T/A |
| # 1028 | A/T | T/A |
| # 1047 | C/G | T/A |
| # 1178 | G/C | T/A |
| # 1466 | G/C | T/A |
| # 2028 | G/C | bp deletion |
| # 2187 | C/G | T/A |
| # 2480 | A/T | T/A |
| | | |
| # 2499-2502 | AGTG | GTCA |
| | TCAC | CAGT |
| # 2642 | TCCGAGC | 7 bp deletion |
| | AGGCTCG | |
| | | |
| # 3435 | G/C | A/T |
| # 3446 | G/C | A/T |
| # 3643 | A/T | T/A |

The DNA sequence between the unique AatII (position #4364 in pCFM1656) and SacII (position #4585 in pCFM1656) restriction sites is substituted with the DNA sequence (SEQ ID NO: 23) shown in Figures 17A and 17B. During the ligation of the sticky ends of this substitution DNA sequence, the outside AatII and SacII sites are destroyed. There are unique AatII and SacII sites in the substituted DNA.

GM221 (Amgen #2596). The Amgen host strain #2596 is an E.coli K-12 strain derived from Amgen strain #393. It has been modified to contain both the temperature sensitive lambda repressor cI857s7 in the early ebg region and the lacI^{Q} repressor in the late ebg region (68 minutes). The presence of these two repressor genes allows the use of this host with a variety of expression systems, however both of these repressors are irrelevant to the expression from luxP_{R}. The untransformed host has no antibiotic resistances.

The ribosome binding site of the cI857s7 gene has been modified to include an enhanced RBS. It has been inserted into the ebg operon between nucleotide position 1170 and 1411 as numbered in Genbank accession number M64441Gb_Ba with deletion of the intervening ebg sequence. The sequence of the insert is shown below with lower case letters representing the ebg sequences flanking the insert shown below (SEQ ID NO: 388):

The construct was delivered to the chromosome using a recombinant phage called MMebg-cI857s7enhanced RBS #4 into F'tet/393. After recombination and resolution only the chromosomal insert described above remains in the cell. It was renamed F'tet/GM101. F'tet/GM101 was then modified by the delivery of a lacI^{Q} construct into the ebg operon between nucleotide position 2493 and 2937 as numbered in the Genbank accession number M64441Gb_Ba with the deletion of the intervening ebg sequence. The sequence of the insert is shown below with the lower case letters representing the ebg sequences flanking the insert (SEQ ID NO: 389) shown below:

The construct was delivered to the chromosome using a recombinant phage called AGebg-LacIQ#5 into F'tet/GM101. After recombination and resolution only the chromosomal insert described above remains in the cell. It was renamed F'tet/GM221. The F'tet episome was cured from the strain using acridine orange at a concentration of 25 µg/ml in LB. The cured strain was identified as tetracyline sensitive and was stored as GM221.

Expression. Cultures of pAMG21-Fc-TMP-TMP in *E*. *coli* GM221 in Luria Broth medium containing 50 µg/ml kanamycin were incubated at 37°C prior to induction. Induction of Fc-TMP-TMP gene product expression from the luxPR promoter was achieved following the addition of the synthetic autoinducer N-(3-oxohexanoyl)-DL-homoserme lactone to the culture media to a final concentration of 20 ng/ml and cultures were incubated at 37°C for a further 3 hours. After 3 hours, the bacterial cultures were examined by microscopy for the presence of inclusion bodies and were then collected by centrifugation. Refractile inclusion bodies were observed in induced cultures indicating that the Fc-TMP-TMP was most likely produced in the insoluble fraction in *E*. *coli.* Cell pellets were lysed directly by resuspension in Laemmli sample buffer containing 10% •-mercaptoethanol and were analyzed by SDS-PAGE. An intense Coomassie stained band of approximately 30kDa was observed on an SDS-PAGE gel. The expected gene product would be 269 amino acids in length and have an expected molecular weight of about 29.5 kDa. Fermentation was also carried out under standard batch conditions at the 10 L scale, resulting in similar expression levels of the Fc-TMP-TMP to those obtained at bench scale.

Purification of Fc-TMP-TMP. Cells are broken in water (1/10) by high pressure homogenization (2 passes at 14,000 PSI) and inclusion bodies are harvested by centrifugation (4200 RPM in J-6B for 1 hour). Inclusion bodies are solubilized in 6M guanidine, 50mM Tris, 8mM DTT, pH 8.7 for 1 hour at a 1 /10 ratio. The solubilized mixture is diluted 20 times into 2M urea, 50 mM tris, 160mM arginine, 3mM cysteine, pH 8.5. The mixture is stirred overnight in the cold and then concentrated about 10 fold by ultafiltration. It is then diluted 3 fold with 10mM Tris, 1.5M urea, pH 9. The pH of this mixture is then adjusted to pH 5 with acetic acid. The precipitate is removed by centrifugation and the supernatant is loaded onto a SP-Sepharose Fast Flow column equilibrated in 20mM NaAc, 100 mM NaCl, pH 5(10mg/ml protein load, room temperature). The protein is eluted off using a 20 column volume gradient in the same buffer ranging from 100mM NaCl to 500mM NaCl. The pool from the column is diluted 3 fold and loaded onto a SP-Sepharose HP column in 20 mM NaAc, 150 mM NaCl, pH 5(10 mg/ml protein load, room temperature). The protein is eluted off using a 20 column volume gradient in the same buffer ranging from 150 mM NaCl to 400 mM NaCl. The peak is pooled and filtered.

Characterization of Fc-TMP activity. The following is a summary of in vivo data in mice with various compounds of this invention.

Mice: Normal female BDF1 approximately 10-12 weeks of age.

Bleed schedule: Ten mice per group treated on day 0, two groups started 4 days apart for a total of 20 mice per group. Five mice bled at each time point, mice were bled a minimum of three times a week. Mice were anesthetized with isoflurane and a total volume of 140-160 µl of blood was obtained by puncture of the orbital sinus. Blood was counted on a Technicon H1E blood analyzer running software for murine blood. Parameters measured were white blood cells, red blood cells, hematocrit, hemoglobin, platelets, neutrophils.

Treatments: Mice were either injected subcutaneously for a bolus treatment or implanted with 7-day micro-osmotic pumps for continuous delivery. Subcutaneous injections were delivered in a volume of 0.2 ml. Osmotic pumps were inserted into a subcutaneous incision made in the skin between the scapulae of anesthetized mice. Compounds were diluted in PBS with 0.1% BSA. All experiments included one control group, labeled "carrier" that were treated with this diluent only. The concentration of the test articles in the pumps was adjusted so that the calibrated flow rate from the pumps gave the treatment levels indicated in the graphs.

Compounds: A dose titration of the compound was delivered to mice in 7 day micro-osmotic pumps. Mice were treated with various compounds at a single dose of 100 µg/kg in 7 day osmotic pumps. Some of the same compounds were then given to mice as a single bolus injection.

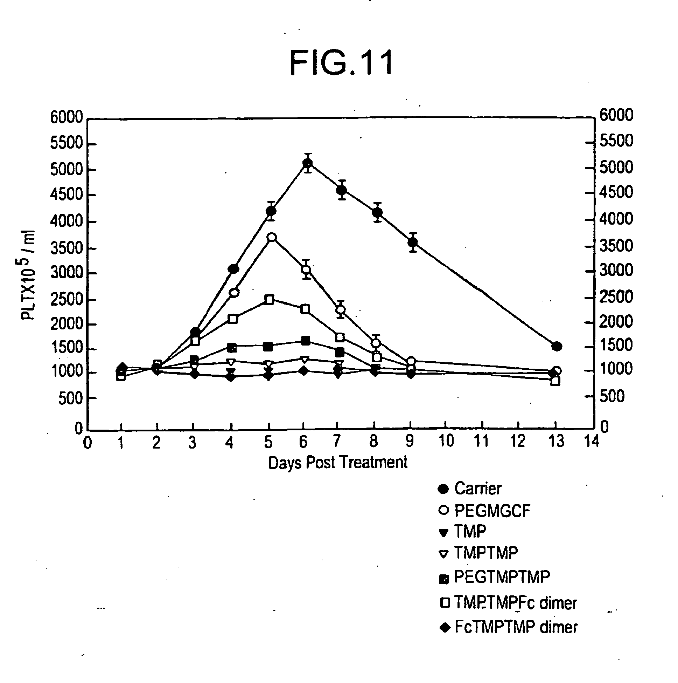

Activity test results: The results of the activity experiments are shown in Figures 11 and 12. In dose response assays using 7-day micro-osmotic pumps, the maximum effect was seen with the compound of SEQ ID NO: 18 was at 100 µg/kg/day; the 10 µg/kg/day dose was about 50% maximally active and 1 µg/kg/day was the lowest dose at which activity could be seen in this assay system. The compound at 10 µg/kg/day dose was about equally active as 100 µg/kg/day unpegylated rHu-MGDF in the same experiment.

### Example 3

### Fc-EMP fusions

Fc-EMP. A DNA sequence coding for the Fc region of human IgG1 fused in-frame to a monomer of the EPO-mimetic peptide was constructed using standard PCR technology. Templates for PCR reactions were a vector containing the Fc sequence (pFc-A3, described in International application WO 97/23614, published July 3,1997) and a synthetic gene encoding EPO monomer. The synthetic gene for the monomer was constructed from the 4 overlapping oligonucleotides (SEQ ID NOS: 390 to 393, respectively) shown below:

| | |
|---|---|
| 1798-2 | |
| 1798-3 | |
| 1798-4 | |
| 1798-5 | |

The 4 oligonucleotides were annealed to form the duplex encoding an amino acid sequence (SEQ ID NOS: 394 and 395, respectively) shown below:

This duplex was amplified in a PCR reaction using

| | |
|---|---|
| 1798-18 | |

and

| | |
|---|---|
| 1798-19 | |

as the sense and antisense primers (SEQ ID NOS: 396 and 397, respectively).

The Fc portion of the molecule was generated in a PCR reaction with pFc-A3 using the primers

| | |
|---|---|
| 1216-52 | AAC ATA AGT ACC TGT AGG ATC G |
| 1798-17 | |

which are SEQ ID NOS: 369 and 399, respectively. The oligonucleotides 1798-17 and 1798-18 contain an overlap of 61 nucleotides, allowing the two genes to be fused together in the correct reading frame by combining the above PCR products in a third reaction using the outside primers, 1216-52 and 1798-19.

The final PCR gene product (the full length fusion gene) was digested with restriction endonucleases XbaI and BamHI, and then ligated into the vector pAMG21 (described below), also digested with XbaI and BamHI. Ligated DNA was transformed into competent host cells of E. coli strain 2596 (GM221, described herein). Clones were screened for the ability to produce the recombinant protein product and to possess the gene fusion having the correct nucleotide sequence. A single such clone was selected and designated Amgen strain #3718.

The nucleotide and amino acid sequence of the resulting fusion protein (SEQ ID NOS: 15 and 16) are shown in Figure 13.

EMP-Fc. A DNA sequence coding for a monomer of the EPO-mimetic peptide fused in-frame to the Fc region of human IgG1 was constructed using standard PCR technology. Templates for PCR reactions were the pFC-A3a vector and a synthetic gene encoding EPO monomer. The synthetic gene for the monomer was constructed from the 4 overlapping oligonucleotides 1798-4 and 1798-5 (above) and 1798-6 and 1798-7 (SEQ ID NOS: 400 and 401, respectively) shown below:

| | |
|---|---|
| 1798-6 | |
| 1798-7 | |

The 4 oligonucleotides were annealed to form the duplex encoding an amino acid sequence (SEQ ID NOS: 402 and 403, respectively) shown below:

This duplex was amplified in a PCR reaction using

| | |
|---|---|
| 1798-21 | TTA TTT CAT ATG AAA GGT GGT AAC TAT TCC TGT CAT TTT |

and

| | |
|---|---|
| 1798-22 | |

as the sense and antisense primers (SEQ ID NOS: 404 and 405, respectively).

The Fc portion of the molecule was generated in a PCR reaction with pFc-A3 using the primers

| | |
|---|---|
| 1798-23 | |

and

| | |
|---|---|
| 1200-54 | GTT ATT GCT CAG CGG TGG CA |

which are SEQ ID NOS: 406 and 407, respectively. The oligonucleotides 1798-22 and 1798-23 contain an overlap of 43 nucleotides, allowing the two genes to be fused together in the correct reading frame by combining the above PCR products in a third reaction using the outside primers, 1787-21 and 1200-54.

The final PCR gene product (the full length fusion gene) was digested with restriction endonucleases XbaI and BamHI, and then ligated into the vector pAMG21 and transformed into competent E. coli strain 2596 cells as described above. Clones were screened for the ability to produce the recombinant protein product and to possess the gene fusion having the correct nucleotide sequence. A single such clone was selected and designated Amgen strain #3688.

The nucleotide and amino acid sequences (SEQ ID NOS: 17 and 18) of the resulting fusion protein are shown in Figure 14.

EMP-EMP-Fc. A DNA sequence coding for a dimer of the EPO-mimetic peptide fused in-frame to the Fc region of human IgG1 was constructed using standard PCR technology. Templates for PCR reactions were the EMP-Fc plasmid from strain #3688 above and a synthetic gene encoding the EPO dimer. The synthetic gene for the dimer was constructed from the 8 overlapping oligonucleotides (SEQ ID NOS:408 to 415, respectively) shown below:

| | |
|---|---|
| 1869-23 | |
| 1869-48 | |
| 1871-72 | |
| 1871-73 | |
| 1871-74 | |
| 1871-75 | |
| 1871-78 | |
| 1871-79 | |

The 8 oligonucleotides were annealed to form the duplex encoding an amino acid sequence (SEQ ID NOS: 416 and 417, respectively) shown below:

This duplex was amplified in a PCR reaction using 1869-23 and 1871-79 (shown above) as the sense and antisense primers.

The Fc portion of the molecule was generated in a PCR reaction with strain 3688 DNA using the primers 1798-23 and 1200-54 (shown above).

The oligonucleotides 1871-79 and 1798-23 contain an overlap of 31 nucleotides, allowing the two genes to be fused together in the correct reading frame by combining the above PCR products in a third reaction using the outside primers, 1869-23 and 1200-54.

The final PCR gene product (the full length fusion gene) was digested with restriction endonucleases XbaI and BamHI, and then ligated into the vector pAMG21 and transformed into competent E. coli strain 2596 cells as described for Fc-EMP. Clones were screened for ability to produce the recombinant protein product and possession of the gene fusion having the correct nucleotide sequence. A single such clone was selected and designated Amgen strain #3813.

The nucleotide and amino acid sequences (SEQ ID NOS: 19 and 20, respectively) of the resulting fusion protein are shown in Figure 15. There is a silent mutation at position 145 (A to G, shown in boldface) such that the final construct has a different nucleotide sequence_than the oligonucleotide 1871-72 from which it was derived.

Fc-EMP-EMP. A DNA sequence coding for the Fc region of human IgG1 fused in-frame to a dimer of the EPO-mimetic peptide was constructed using standard PCR technology. Templates for PCR reactions were the plasmids from strains 3688 and 3813 above.

The Fc portion of the molecule was generated in a PCR reaction with strain 3688 DNA using the primers 1216-52 and 1798-17 (shown above). The EMP dimer portion of the molecule was the product of a second PCR reaction with strain 3813 DNA using the primers 1798-18 (also shown above) and SEQ ID NO: 418, shown below:

| | |
|---|---|
| 1798-20 | CTA ATT GGA TCC TCG AGA TTA ACC CCC TTG TGG CTT ACAT |

The oligonucleotides 1798-17 and 1798-18 contain an overlap of 61 nucleotides, allowing the two genes to be fused together in the correct reading frame by combining the above PCR products in a third reaction using the outside primers, 1216-52 and 1798-20.

The final PCR gene product (the full length fusion gene) was digested with restriction endonucleases XbaI and BamHI, and then ligated into the vector pAMG21 and transformed into competent E. coli strain 2596 cells as described for Fc-EMP. Clones were screened for the ability to produce the recombinant protein product and to possess the gene fusion having the correct nucleotide sequence. A single such clone was selected and designated Amgen strain #3822.

The nucleotide and amino acid sequences (SEQ ID NOS:21 and 22 respectively) of the fusion protein are shown in Figure 16.

Characterization of Fc-EMP activity. Characterization was carried out in vivo as follows.

Mice: Normal female BDF1 approximately 10-12 weeks of age.

Bleed schedule: Ten mice per group treated on day 0, two groups started 4 days apart for a total of 20 mice per group. Five mice bled at each time point, mice were bled a maximum of three times a week. Mice were anesthetized with isoflurane and a total volume of 140-160 ml of blood was obtained by puncture of the orbital sinus. Blood was counted on a Technicon H1E blood analyzer running software for murine blood. Parameters measured were WBC, RBC, HCT, HGB, PLT, NEUT, LYMPH.

Treatments: Mice were either injected subcutaneously for a bolus treatment or implanted with 7 day micro-osmotic pumps for continuous delivery. Subcutaneous injections were delivered in a volume of 0.2 ml. Osmotic pumps were inserted into a subcutaneous incision made in the skin between the scapulae of anesthetized mice. Compounds were diluted in PBS with 0.1% BSA. All experiments included one control group, labeled "carrier" that were treated with this diluent only. The concentration of the test articles in the pumps was adjusted so that the calibrated flow rate from the pumps gave the treatment levels indicated in the graphs.

Experiments: Various Fc-conjugated EPO mimetic peptides (EMPs) were delivered to mice as a single bolus injection at a dose of 100 µg/kg. Fc-EMPs were delivered to mice in 7-day micro-osmotic pumps. The pumps were not replaced at the end of 7 days. Mice were bled until day 51 when HGB and HCT returned to baseline levels.

### Example 4

### TNF-α inhibitors

Fc-TNF-α inhibitors. A DNA sequence coding for the. Fc region of human IgG1 fused in-frame to a monomer of the TNF-α inhibitory peptide was constructed using standard PCR technology. The Fc and 5 glycine linker portion of the molecule was generated in a PCR reaction with DNA from the Fc-EMP fusion strain #3718 (see Example 3) using the sense primer 1216-52 and the antisense primer 2295-89 (SEQ ID NOS: 369 and 1112, respectively). The nucleotides encoding the TNF-α inhibitory peptide were provided by the PCR primer 2295-89 shown below:

| | |
|---|---|
| 1216-52 | AAC ATA AGT ACC TGT AGG ATC G |
| 2295-89 | |

The oligonucleotide 2295-89 overlaps the glycine linker and Fc portion of the template by 22 nucleotides, with the PCR resulting in the two genes being fused together in the correct reading frame.

The PCR gene product (the full length fusion gene) was digested with restriction endonucleases Ndel and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such clone was selected and designated Amgen strain #4544.

The nucleotide and amino acid sequences (SEQ ID NOS: 1055 and 1056) of the fusion protein are shown in Figures 19A and 19B.

TNF-α inhibitor-Fc. A DNA sequence coding for a TNF-α inhibitory peptide fused in-frame to the Fc region of human IgG1 was constructed using standard PCR technology. The template for the PCR reaction was a plasmid containing an unrelated peptide fused via a five glycine linker to Fc. The nucleotides encoding the TNF-α inhibitory peptide were provided by the sense PCR primer 2295-88, with primer 1200-54 serving as the antisense primer (SEQ ID NOS:1117 and 407, respectively). The primer sequences are shown below:

| | |
|---|---|
| 2295-88 | |
| 1200-54 | GTT ATT GCT CAG CGG TGG CA |

The oligonucleotide 2295-88 overlaps the glycine linker and Fc portion of the template by 24 nucleotides, with the PCR resulting in the two genes being fused together in the correct reading frame.

The PCR gene product (the full length fusion gene) was digested with restriction endonucleases NdeI and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such clone was selected and designated Amgen strain #4543.
The nucleotide and amino acid sequences (SEQ ID NOS: 1057 and 1058) of the fusion protein are shown in Figures 20A and 20B.

Expression in E. coli. Cultures of each of the pAMG21-Fc-fusion constructs in E. coli GM221 were grown at 37 °C in Luria Broth medium containing 50 mg/ml kanamycin. Induction of gene product expression from the luxPR promoter was achieved following the addition of the synthetic autoinducer N-(3-oxohexanoyl)-DL-homoserine lactone to the culture media to a final concentration of 20 ng/ml. Cultures were incubated at 37 °C for a further 3 hours. After 3 hours, the bacterial cultures were examined by microscopy for the presence of inclusion bodies and were then collected by centrifugation. Refractile inclusion bodies were observed in induced cultures indicating that the Fc-fusions were most likely produced in the insoluble fraction in E. coli. Cell pellets were lysed directly by resuspension in Laemmli sample buffer containing 10% β-mercaptoethanol and were analyzed by SDS-PAGE. In each case, an intense coomassie-stained band of the appropriate molecular weight was observed on an SDS-PAGE gel.

Purification of Fc-peptide fusion proteins. Cells are broken in water (1/10) by high pressure homogenization (2 passes at 14,000 PSI) and inclusion bodies are harvested by centrifugation (4200 RPM in J-6B for 1 hour). Inclusion bodies are solubilized in 6M guanidine, 50mM Tris, 8mM DTT, pH 8.7 for 1 hour at a 1/10 ratio. The solubilized mixture is diluted 20 times into 2M urea, 50 mM tris, 160mM arginine, 3mM cysteine, pH 8.5. The mixture is stirred overnight in the cold and then concentrated about 10 fold by ultafiltration. It is then diluted 3 fold with 10mM Tris, 1.5M urea, pH 9. The pH of this mixture is then adjusted to pH 5 with acetic acid. The precipitate is removed by centrifugation and the supernatant is loaded onto a SP-Sepharose Fast Flow column equilibrated in 20mM NaAc, 100 mM NaCl, pH 5 (10mg/ml protein load, room temperature). The protein is eluted from the column using a 20 column volume gradient in the same buffer ranging from 100mM NaCI to 500mM NaCl. The pool from the column is diluted 3 fold and loaded onto a SP-Sepharose HP column in 20mM NaAc,150mM NaCl, pH 5(10mg/ml protein load, room temperature). The protein is eluted using a 20 column volume gradient in the same buffer ranging from 150mM NaCl to 400mM NaCl. The peak is pooled and filtered.

Characterization of activity of Fc-TNF-α inhibitor and TNF-α inhibitor -Fc. Binding of these peptide fusion proteins to TNF-α can be characterized by BIAcore by methods available to one of ordinary skill in the art who is armed with the teachings of the present specification.

### Example 5

### IL-1 Antagonists

Fc-IL-1 antagonist. A DNA sequence coding for the Fc region of human IgG1 fused in-frame to a monomer of an IL-1 antagonist peptide was constructed using standard PCR technology. The Fc and 5 glycine linker portion of the molecule was generated in a PCR reaction with DNA from the Fc-EMP fusion strain #3718 (see Example 3) using the sense primer 1216-52 and the antisense primer 2269-70 (SEQ ID NOS: 369 and 1118, respectively). The nucleotides encoding the IL-1 antagonist peptide were provided by the PCR primer 2269-70 shown below:

| | |
|---|---|
| 1216-52 | AAC ATA AGT ACC TGT AGG ATC G |
| 2269-70 | |

The oligonucleotide 2269-70 overlaps the glycine linker and Fc portion of the template by 22 nucleotides, with the PCR resulting in the two genes being fused together in the correct reading frame.

The PCR gene product (the full length fusion gene) was digested with restriction endonucleases NdeI and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such clone was selected and designated Amgen strain #4506.

The nucleotide and amino acid sequences (SEQ ID NOS: 1059 and 1060) of the fusion protein are shown in Figures 21A and 21B.

IL-1 antagonist-Fc. A DNA sequence coding for an IL-1 antagonist peptide fused in-frame to the Fc region of human IgG1 was constructed using standard PCR technology. The template for the PCR reaction was a plasmid containing an unrelated peptide fused via a five glycine linker to Fc. The nucleotides encoding the IL-1 antagonist peptide were provided by the sense PCR primer 2269-69, with primer 1200-54 serving as the antisense primer (SEQ ID NOS:1119 and 407, respectively). The primer sequences are shown below:

| | |
|---|---|
| 2269-69 | |
| 1200-54 | GTT ATT GCT CAG CGG TGG CA |

The oligonucleotide 2269-69 overlaps the glycine linker and Fc portion of the template by 24 nucleotides, with the PCR resulting in the two genes being fused together in the correct reading frame.

The PCR gene product (the full length fusion gene) was digested with restriction endonucleases NdeI and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such clone was selected and designated Amgen strain #4505.

The nucleotide and amino acid sequences (SEQ ID NOS: 1061 and 1062) of the fusion protein are shown in Figures 22A and 22B. Expression and purification were carried out as in previous examples.

Characterization of Fc-IL-1 antagonist peptide and IL-1 antagonist peptide-Fc activity. IL-1 Receptor Binding competition between IL-1β, IL-1RA and Fc-conjugated IL-1 peptide sequences was carried out using the IGEN system. Reactions contained 0.4 nM biotin-IL-1R + 15 nM IL-1-TAG + 3 uM competitor + 20 ug/ml streptavidin-conjugate beads, where competitors were IL-1RA, Fc-IL-1 antagonist, IL-1 antagonist-Fc). Competition was assayed over a range of competitor concentrations from 3 uM to 1.5 pM. The results are shown in Table C below:

**Table C-Results from IL-1 Receptor Binding Competition Assay**

| | ***IL-1pep-Fc*** | ***Fc-IL-1pep*** | ***IL-1ra*** |
|---|---|---|---|
| **KI** | 281.5 | 59.58 | 1.405 |
| **EC50** | 530.0 | 112.2 | 2.645 |

| **95% Confidence Intervals** | | | |
|---|---|---|---|
| **EC50** | 280.2 to 1002 | 54.75 to 229.8 | 1.149 to |
| | | | 6.086 |
| **KI** | 148.9 to 532.5 | 29.08 to 122.1 | 0.6106 to |
| | | | 3.233 |

| **Goodness of Fit** | | | |
|---|---|---|---|
| **R**² | 0.9790 | 0.9687 | 0.9602 |

### Example 6

### VEGF-Antagonists

Fc-VEGF Antagonist. A DNA sequence coding for the Fc region of human IgG1 fused in-frame to a monomer of the VEGF mimetic peptide was constructed using standard PCR technology. The templates for the PCR reaction were the pFc-A3 plasmid and a synthetic VEGF mimetic peptide gene. The synthetic gene was assembled by annealing the following two oligonucleotides primer (SEQ ID NOS: 1110 and 1111, respectively):

| | |
|---|---|
| 2293-11 | |
| 2293-12 | |

The two oligonucleotides anneal to form the following duplex encoding an amino add sequence shown below (SEQ ID NOS 1113 and 1114): This duplex was amplified in a PCR reaction using 2293-05 and 2293-06 as the sense and antisense primers (SEQ ID NOS. 1111 and 1112).

The Fc portion of the molecule was generated in a PCR reaction with the pFc-A3 plasmid using the primers 2293-03 and 2293-04 as the sense and antisense primers (SEQ ID NOS. 1120 and 1121, respectively). The full length fusion gene was obtained from a third PCR reaction using the outside primers 2293-03 and 2293-06 (SEQ ID NO 1112). These primers are shown below:

| | |
|---|---|
| 2293-03 | |
| 2293-04 | |
| 2293-05 | |
| 2293-06 | CCG CGG ATC CTC GAG TTA CAG ACG TTC AAA ACA TTC CCA |

The PCR gene product (the full length fusion gene) was digested with restriction endonucleases NdeI and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such clone was selected and designated Amgen strain #4523.

The nucleotide and amino acid sequences (SEQ ID NOS: 1063 and 1064) of the fusion protein are shown in Figures 23A and 23B.

VEGF antagonist -Fc. A DNA sequence coding for a VEGF mimetic peptide fused in-frame to the Fc region of human IgG1 was constructed using standard PCR technology. The templates for the PCR reaction were the pFc-A3 plasmid and the synthetic VEGF mimetic peptide gene described above. The synthetic duplex was amplified in a PCR reaction using 2293-07 and 2293-08 as the sense and antisense primers (SEQ ID NOS. 1124 and 1125, respectively).

The Fc portion of the molecule was generated in a PCR reaction with the pFc-A3 plasmid using the primers 2293-09 and 2293-10 as the sense and antisense primers (SEQ ID NOS. 1126 and 1127, respectively). The full length fusion gene was obtained from a third PCR reaction using the outside primers 2293-07 and 2293-10. These primers are shown below:

| | |
|---|---|
| 2293-07 | |
| 2293-08 | |
| 2293-09 | |
| 2293-10 | CCG CGG ATC CTC GAG TTA TTT ACC CGG AGA CAG GGA GAG |

The PCR gene product (the full length fusion gene) was digested with restriction endonucleases NdeI and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such done was selected and designated Amgen strain #4524.

The nucleotide and amino acid sequences (SEQ ID NOS: 1065 and 1066) of the fusion protein are shown in Figures 24A and 24B. Expression and purification were carried out as in previous examples.

### Example 7

### MMP Inhibitors

Fc-MMP inhibitor. A DNA sequence coding for the Fc region of human IgG1 fused in-frame to a monomer of an MMP inhibitory peptide was constructed using standard PCR technology. The Fc and 5 glycine linker portion of the molecule was generated in a PCR reaction with DNA from the Fc-TNF-α inhibitor fusion strain #4544 (see Example 4) using the sense primer 1216-52 and the antisense primer 2308-67 (SEQ ID NOS: 369 and 1115, respectively). The nucleotides encoding the MMP inhibitor peptide were provided by the PCR primer 2308-67 shown below:

| | |
|---|---|
| 1216-52 | AAC ATA AGT ACC TGT AGG ATC G |
| 2308-67 | |

The oligonucleotide 2308-67 overlaps the glycine linker and Fc portion of the template by 22 nucleotides, with the PCR resulting in the two genes being fused together in the correct reading frame.

The PCR gene product (the full length fusion gene) was digested with restriction endonucleases NdeI and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such clone was selected and designated Amgen strain #4597.

The nucleotide and amino acid sequences (SEQ ID NOS: 1067 and 1068) of the fusion protein are shown in Figures 25A and 25B. Expression and purification were carried out as in previous examples.

MMP Inhibitor-Fc. A DNA sequence coding for an MMP inhibitory peptide fused in-frame to the Fc region of human IgG1 was constructed using standard PCR technology. The Fc and 5 glycine linker portion of the molecule was generated in a PCR reaction with DNA from the Fc-TNF-α inhibitor fusion strain #4543 (see Example 4). The nucleotides encoding the MMP inhibitory peptide were provided by the sense PCR primer 2308-66, with primer 1200-54 serving as the antisense primer (SEQ ID NOS: 1116 and 407, respectively). The primer sequences are shown below:

| | |
|---|---|
| 2308-66 | |
| 1200-54 | GTT ATT GCT CAG CGG TGG CA |

The oligonucleotide 2269-69 overlaps the glycine linker and Fc portion of the template by 24 nucleotides, with the PCR resulting in the two genes being fused together in the correct reading frame.

The PCR gene product (the full length fusion gene) was digested with restriction endonucleases NdeI and BamHI, and then ligated into the vector pAMG21 and transformed into competent E. coli strain 2596 cells as described for EMP-Fc herein. Clones were screened for the ability to produce the recombinant protein product and to possess the gene fusion having the correct nucleotide sequence. A single such clone was selected and designated Amgen strain #4598.

The nucleotide and amino acid sequences (SEQ ID NOS: 1069 and 1070) of the fusion protein are shown in Figures 26A and 26B.

The invention now being fully described, it will be apparent to one of ordinary skill in the art that many changes and modifications can be made thereto, without departing from the spirit and scope of the invention as set forth herein.

### Abbreviations

Abbreviations used throughout this specification are as defined below, unless otherwise defined in specific circumstances.
- Ac: acetyl (used to refer to acetylated residues)
- AcBpa: acetylated p-benzoyl-L-phenylalanine
- ADCC: antibody-dependent cellular cytotoxicity
- Aib: aminoisobutyric acid
- bA: beta-alanine
- Bpa: p-benzoyl-L-phenylalanine
- BrAc: bromoacetyl (BrCH₂C(O)
- BSA: Bovine serum albumin
- Bzl: Benzyl
- Cap: Caproic acid
- CTL: Cytotoxic T lymphocytes
- CTLA4: Cytotoxic T lymphocyte antigen 4
- DARC: Duffy blood group antigen receptor
- DCC: Dicylcohexylcarbodiimide
- Dde: 1-(4,4-dimethyl-2,6-dioxo-cyclohexylidene)ethyl
- EMP: Erythropoietin-mimetic peptide
- ESI-MS: Electron spray ionization mass spectrometry
- EPO: Erythropoietin
- Fmoc: fluorenylmethoxycarbonyl
- G-CSF: Granulocyte colony stimulating factor
- GH: Growth hormone
- HCT: hematocrit
- HGB: hemoglobin
- hGH: Human growth hormone
- HOBt: 1-Hydroxybenzotriazole
- HPLC: high performance liquid chromatography
- IL: interleukin
- IL-R: interleukin receptor
- IL-1R: interleukin-1 receptor
- IL-1ra: interleukin-1 receptor antagonist
- Lau: Lauric acid
- LPS: lipopolysaccharide
- LYMPH: lymphocytes
- MALDI-MS: Matrix-assisted laser desorption ionization mass spectrometry
- Me: methyl
- MeO: methoxy
- MHC: major histocompatibility complex
- MMP: matrix metalloproteinase
- MMPI: matrix metalloproteinase inhibitor
- 1-Nap: 1-napthylalanine
- NEUT: neutrophils
- NGF: nerve growth factor
- Nle: norleucine
- NMP: N-methyl-2-pyrrolidinone
- PAGE: polyacrylamide gel electrophoresis
- - PBS: Phosphate-buffered saline
- Pbf: 2,2,4,6,7-pendamethyldihydrobenzofuran-5-sulfonyl
- PCR: polymerase chain reaction
- Pec: pipecolic acid
- PEG: Poly(ethylene glycol)
- pGlu: pyroglutamic acid
- Pic: picolinic acid
- PLT: platelets
- pY: phosphotyrosine
- RBC: red blood cells
- RBS: ribosome binding site
- RT: room temperature (25 °C)
- Sar: sarcosine
- SDS: sodium dodecyl sulfate
- STK: serine-threonine kinases
- t-Boc: tert-Butoxycarbonyl
- tBu: tert-Butyl
- TGF: tissue growth factor
- THF: thymic humoral factor
- TK: tyrosine kinase
- TMP: Thrombopoietin-mimetic peptide
- TNF: Tissue necrosis factor
- TPO: Thrombopoietin
- TRAIL: TNF-related apoptosis-inducing ligand
- Trt: trityl
- UK: urokinase
- UKR: urokinase receptor
- VEGF: vascular endothelial cell growth factor
- VIP: vasoactive intestinal peptide
- WBC: white blood cells

## Claims

1. A composition of matter of the formula
(X¹)ₐ-F¹-(X²)_{b}
and multimers thereof, wherein:
F¹ is an Fc domain;
X¹ and X² are each independently selected from
-(L¹)_{c}-P¹.
-(L¹)_{c}-P¹-(L²)_{d}-P².
-(L¹)_{c}-P¹-(L²)_{d}-P²-(L³)ₑ-P³, and
-(L¹)_{c}-P¹-(L²)_{d}-P²-(L³)ₑ-P³-(L⁴)_{f}-P⁴
P¹, P², P³, and P⁴ are each independently randomised sequences of pharmacologically active peptides selected from
IL-1 antagonist peptide sequences selected from SEQ ID NOs: 212, 907, 908, 909, 910, 917, and 979 and IL-1 antagonist peptide sequences selected from SEQ ID NOs: 213 to 271, 671 to 906, 911 to 916, and 918 to 1023;
EPO-mimetic peptide sequences selected from Table 5
TPO-mimetic peptide sequences selected from Table 6
TNF-antagonist peptide sequences selected from Table 8;
GCSF-mimetic peptide sequences selected from Table 7
L¹, L², L³, and L⁴ are each independently linkers; and
a, b, c, d, e, and f, are each independently 0 or 1, provided that at least one of a and b is 1, and wherein "peptide" refers to molecules of 2 to 40 amino acids.

2. The composition of matter of Claim 1 of the formulae
X¹-F¹
or
F¹-X².

3. The composition of matter of Claim 1 of the formula
F¹-(L¹)_{c}-P¹.

4. The composition of matter of Claim 1 of the formula
F¹-(L¹)_{c}-P¹-(L²)_{d}-P².

5. The composition of matter of any one of Claims 1 to 4, wherein F¹ is an IgG Fc domain.

6. The composition of matter of any one of Claims 1 to 4, wherein F¹ is an IgG1 Fc domain.

7. The composition of matter of any one of Claims 1 to 4, wherein F¹ comprises the sequence of SEQ ID NO: 2.

8. The composition of matter of Claim 1 comprising a sequence selected from SEQ ID NOs: 339 and 340.

9. The composition of matter of Claim 1 comprising a sequence selected from SEQ ID NOs: 20 and 22.

10. The composition of matter of Claim 1 having a sequence selected from SEQ ID NOs: 6 and 12.

11. A DNA encoding a composition of matter of any one of Claims 1 to 10.

12. An expression vector comprising the DNA of Claim 11.

13. A host cell comprising the expression vector of Claim 12.

14. The cell of Claim 13, wherein the cell is an E.coli cell.

15. A pharmaceutical composition comprising a composition of matter according to any one of Claims 1 to 10 together with a pharmaceutically acceptable diluent, preservative, solubiliser, emulsifier, adjuvant and/or carrier.

16. A composition of matter according to any one of Claims 1 to 10 for use as a therapeutic or prophylactic agent.

17. A process for preparing a pharmacologically active compound, which comprises
a) selecting at least one randomized peptide that modulates the activity of a protein of interest; and
b) preparing a pharmacologic agent comprising at least one Fc domain covalently linked to at least one amino acid sequence of the selected peptide or peptides,
wherein "peptide" referst to molecules of 2 to 40 amino acids, and wherein "peptide" refers to a randomised EPO-mimetic peptide, a randomised TPO-mimetic peptide, a randomised IL-1 antagonist peptide, a randomised GCSF-mimetic peptide or a randomised TNF-antagonist peptide.

18. The process of Claim 17, wherein the peptide is selected in a process comprising screening of a phage display library, and E.coli display library, a ribosomal library, or a chemical peptide library.

19. The process of Claims 17 or 18, wherein the peptide is selected from Tables 4 to 20.

20. The process of Claim 19, wherein the compound prepared is of the formula
(X¹)ₐ-F¹-(X²)_{b}
and multimers thereof, wherein:
F¹ is an Fc domain;
X¹ and X² are each independently selected from
-(L¹)_{c}-P¹,
-(L¹)_{c}-P-(L¹)_{d}-P²,
-(L¹)_{c}-P¹-(L²)_{d}-P²-(L³)ₑ-P³, and
-(L¹)_{c}-P¹-(L²)_{d}-P²-(L³)ₑ-P³-(L⁴)_{f}-P⁴
P¹, P², P³, and P⁴ are each independently randomised sequences of pharmacologically active peptides;
L¹, L², L³, and L⁴ are each independently linkers; and
a, b, c, d, e, and f, are each independently 0 or 1, provide that at least one of a and b is 1.

21. The process of Claim 20, wherein the compound prepared is of the formulae
X¹-F¹
or
F¹-X².

22. The process of Claim 20, wherein the compound prepared is of the formulae
F¹-(L¹)_{c}-P¹
or
-(L¹)_{c}-P¹-(L²)_{d}-P².

23. The process of any one of Claims 17 to 22, wherein F¹ is an IgG Fc domain.

24. The process of any one of Claims 17 to 22, wherein F¹ is an IgG1 Fc domain.

25. The process of any one of Claims 17 to 22, wherein F¹ comprises the sequence of SEQ ID NO: 2.

## Patentansprüche

1. Stoffzusammensetzung der Formel
(X¹)ₐ-F¹-(X²)_{b}
und Multimere davon, wobei:
F¹ eine Fc-Domäne ist;
X¹ und X² jeweils unabhängig aus -(L¹)_{c}-P¹,-(L¹)_{c}-P¹-(L²)_{d}-P²,-(L¹)_{c}-P¹-(L²)_{d}-P²-(L³)ₑ-P³ und -(L¹)_{c}-P¹-(L²)_{d}-P²-(L³)ₑ-P³-(L⁴)_{f}-P⁴ ausgewählt sind;
P¹, P², P³ und P⁴ jeweils unabhängig randomisierte Sequenzen pharmakologisch aktiver Peptide, ausgewählt aus IL-1-Antagonist-Peptidsequenzen, ausgewählt aus den SEQ ID NOs: 212, 907, 908, 909, 910, 917 und 979, und IL-1-Antagonist-Peptidsequenzen, ausgewählt aus den SEQ ID NOs: 213 bis 271, 671 bis 906, 911 bis 916 und 918 bis 1023,
EPO-nachahmenden Peptidsequenzen, ausgewählt aus Tabelle 5, TPO-nachahmenden Peptidsequenzen, ausgewählt aus Tabelle 6, TNF-Antagonist-Peptidsequenzen, ausgewählt aus Tabelle 8, GCSF-nachahmenden Peptidsequenzen, ausgewählt aus Tabelle 7, sind;
L¹, L², L³ und L⁴ jeweils unabhängige Linker sind; und
a, b, c, d, e und f jeweils unabhängig 0 oder 1 sind, vorausgesetzt, dass wenigstens a oder b 1 ist, und wobei "Peptid" Moleküle mit 2 bis 40 Aminosäuren bezeichnet.

2. Stoffzusammensetzung nach Anspruch 1 mit der Formel
X¹-F¹
oder
F¹-X².

3. Stoffzusammensetzung nach Anspruch 1 mit der Formel
F¹-(L¹)_{c}-P¹.

4. Stoffzusammensetzung nach Anspruch 1 mit der Formel
F¹-(L¹)_{c}-P¹-(L²)_{d}-P².

5. Stoffzusammensetzung nach einem jeden der Ansprüche 1 bis 4, wobei F¹ eine IgG-Fc-Domäne ist.

6. Stoffzusammensetzung nach einem jeden der Ansprüche 1 bis 4, wobei F¹ eine IgG1-Fc-Domäne ist.

7. Stoffzusammensetzung nach einem jeden der Ansprüche 1 bis 4, wobei F¹ die Sequenz von SEQ ID NO: 2 umfasst.

8. Stoffzusammensetzung nach Anspruch 1, die eine aus den SEQ ID NOs: 339 und 340 ausgewählte Sequenz umfasst.

9. Stoffzusammensetzung nach Anspruch 1, die eine aus den SEQ ID NOs: 20 und 22 ausgewählte Sequenz umfasst.

10. Stoffzusammensetzung nach Anspruch 1, die eine aus den SEQ ID NOs: 6 und 12 ausgewählte Sequenz aufweist.

11. DNA, die für eine Stoffzusammensetzung nach einem jeden der Ansprüche 1 bis 10 kodiert.

12. Expressionsvektor, der die DNA nach Anspruch 11 umfasst.

13. Wirtszelle, die den Expressionsvektor nach Anspruch 12 umfasst.

14. Zelle nach Anspruch 13, wobei die Zelle eine E. coli-Zelle ist.

15. Pharmazeutische Zusammensetzung, die eine Stoffzusammensetzung nach einem jeden der Ansprüche 1 bis 10 zusammen mit einem pharmazeutisch akzeptablen Verdünnungsmittel, Konservierungsmittel, Lösungsverbesserer, Emulgierungsmittel, Adjuvans und/oder Träger umfasst.

16. Stoffzusammensetzung gemäß einem jeden der Ansprüche 1 bis 10 zur Verwendung als therapeutisches oder prophylaktisches Agens.

17. Verfahren zum Herstellen einer pharmakologisch aktiven Verbindung, das
a) Auswählen wenigstens eines randomisierten Peptides, das die Aktivität eines interessierenden Proteins moduliert; und
b) Herstellen eines pharmakologischen Agens, das wenigstens eine Fc-Domäne umfasst, die kovalent mit wenigstens einer Aminosäuresequenz des ausgewählten Peptides oder der ausgewählten Peptide verbunden ist,
umfasst, wobei "Peptid" Moleküle mit 2 bis 40 Aminosäuren bezeichnet und wobei "Peptid" ein randomisiertes EPO-nachahmendes Peptid, ein randomisiertes TPO-nachahmendes Peptid, ein randomisiertes IL-1-Antagonist-Peptid, ein randomisiertes GCSF-nachahmendes Peptid oder ein randomisiertes TNF-Antagonist-Peptid bezeichnet.

18. Verfahren nach Anspruch 17, wobei das Peptid in einem Verfahren ausgewählt wird, das Screenen einer Phage-Display-Bibliothek, einer E. coli-Display-Bibliothek, einer ribosomalen Bibliothek oder einer chemischen Peptidbibliothek umfasst.

19. Verfahren nach Anspruch 17 oder 18, wobei das Peptid aus den Tabellen 4 bis 20 ausgewählt ist.

20. Verfahren nach Anspruch 19, wobei die hergestellte Verbindung die Formel
(X¹)ₐ-F¹-(X²)_{b}
und Multimere davon aufweist, wobei:
F¹ eine Fc-Domäne ist;
X¹ und X² jeweils unabhängig aus -(L¹)_{c}-P¹, -(L¹)_{c}-P¹-(L²)_{d}-P², -(L¹)_{c}-P¹-(L²)_{d}-P²-(L³)ₑ-P³ und -(L¹)_{c}-P¹-(L²)_{d}-P²-(L³)ₑ-P³-(4)_{f}-P⁴ ausgewählt sind;
P¹, P², P³ und P⁴ jeweils unabhängig randomisierte Sequenzen pharmakologisch aktiver Peptide sind;
L¹, L², L³ und L⁴ jeweils unabhängige Linker sind; und
a, b, c, d, e und f jeweils unabhängig 0 oder 1 sind, vorausgesetzt, dass wenigstens a oder b 1 ist.

21. Verfahren nach Anspruch 20, wobei die hergestellte Verbindung die Formeln
X¹-F¹
oder
F¹-X²
aufweist.

22. Verfahren nach Anspruch 20, wobei die hergestellte Verbindung die Formeln
F¹-(L¹)_{c}-P¹
oder
F¹-(L¹)_{c}-P¹-(L²)_{d}-P²
aufweist.

23. Verfahren nach einem jeden der Ansprüche 17 bis 22, wobei F¹ eine IgG-Fc-Domäne ist.

24. Verfahren nach einem jeden der Ansprüche 17 bis 22, wobei F¹ eine IgG1-Fc-Domäne ist.

25. Verfahren nach einem jeden der Ansprüche 17 bis 22, wobei F¹ die Sequenz von SEQ ID NO: 2 umfasst.

## Revendications

1. Composition de matière de formule :
(X¹)ₐ-F¹-(X²)_{b}
et de ses multimères, dans laquelle :
F¹ est un domaine de Fc ;
X¹ et X² sont chacun indépendamment choisis parmi -(L¹)_{c}-P¹, -(L¹)_{c}-P¹-(L²)_{d}-P², -(L¹)_{c}-P¹-(L²)_{d}-P²-(L³)ₑ -P³ et -(L¹)_{c}-P¹-(L²)_{d}-P²-(L³)ₑ-P³-(L⁴)_{f}-P⁴;
P¹, P², P³ et P⁴ sont chacun indépendamment des séquences randomisées de peptides actifs pharmacologiquement choisis parmi les séquences peptidiques antagonistes de IL-1 choisies parmi les SEQ ID n° 212, 907, 908, 909, 910, 917 et 979, et les séquences peptidiques antagonistes de l'IL-1 choisies parmi les SEQ ID n° 213 à 271, 671 à 906, 911 à 916 et 918 à 1023, les séquences peptidiques mimétiques de l'EPO choisies dans le tableau 5, les séquences peptidiques mimétiques de la TPO choisies dans le tableau 6, les séquences peptidiques antagonistes de la TNF choisies dans le tableau 8 et les séquences peptidiques mimétiques du GCSF choisies dans le tableau 7 ;
L¹, L², L³ et L⁴ sont chacun indépendamment des lieurs ; et
a, b, c, d, e et f sont chacun indépendamment 0 ou 1, pourvu qu'au moins l'un de a et b soit 1 et dans laquelle le terme « peptide » se réfère à des molécules de 2 à 40 acides aminés.

2. Composition de matière selon la revendication 1 de formule :
X¹-F¹
ou
F¹-X².

3. Composition de matière selon la revendication 1 de formule :
F¹-(L¹)_{c}-P¹.

4. Composition de matière selon la revendication 1 de formule :
F¹-(L¹)_{c}-p¹-(L²)_{d}-P².

5. Composition de matière selon l'une quelconque des revendications 1 à 4, dans laquelle F¹ est un domaine Fc d'IgG.

6. Composition de matière selon l'une quelconque des revendications 1 à 4, dans laquelle F¹ est un domaine Fc d'IgG 1.

7. Composition de matière selon l'une quelconque des revendications 1 à 4, dans laquelle F¹ comprend la séquence de SEQ ID n° 2.

8. Composition de matière selon la revendication 1, comprenant une séquence choisie parmi les SEQ ID n° 339 et 340.

9. Composition de matière selon la revendication 1, comprenant une séquence choisie parmi les SEQ ID n° 20 et 22.

10. Composition de matière selon la revendication 1, ayant une séquence choisie parmi les SEQ ID n° 6 et 12.

11. ADN codant pour une composition de matière selon l'une quelconque des revendications 1 à 10.

12. Vecteur d'expression comprenant l'ADN de la revendication 11.

13. Cellule hôte comprenant le vecteur d'expression de la revendication 12.

14. Cellule selon la revendication 13, dans laquelle la cellule est une cellule de E. coli.

15. Composition pharmaceutique comprenant une composition de matière selon l'une quelconque des revendications 1 à 10 conjointement avec un diluant, un conservateur, un solubilisant, un émulsionnant, un adjuvant et/ou un véhicule acceptables au plan pharmaceutique.

16. Composition de matière selon l'une quelconque des revendications 1 à 10 pour usage comme agent thérapeutique ou prophylactique.

17. Procédé de préparation d'un composé actif au plan pharmacologique qui comprend :
a) la sélection d'au moins un peptide randomisé qui module l'activité d'une protéine intéressante ; et
b) la préparation d'un agent pharmacologique comprenant au moins un domaine de Fc lié par covalence à au moins une séquence d'acides aminés du ou des peptides choisis,
dans lequel le terme « peptide » se réfère à des molécules de 2 à 40 acides aminés et dans lequel le terme « peptide » se réfère à un peptide mimétique de l'EPO randomisé, un peptide mimétique de la TPO randomisé, un peptide antagoniste de l'IL-1 randomisé, un peptide mimétique du GCSF randomisé et d'un peptide antagoniste de la TNF randomisé.

18. Procédé selon la revendication 17, dans lequel le peptide est choisi dans un procédé comprenant le criblage d'une bibliothèque d'affichage de phages, d'une bibliothèque d'affichage de E. coli, d'une bibliothèque de ribosomes ou d'une bibliothèque de peptides chimiques.

19. Procédé selon la revendication 17 ou la revendication 18, dans lequel le peptide est choisi parmi les tableaux 4 à 20.

20. Procédé selon la revendication 19, dans lequel le composé préparé a pour formule :
(X¹)ₐ-F¹-(X²)_{b}
et de ses multimères, dans laquelle :
F¹ est un domaine de Fc ;
X¹ et X² sont chacun indépendamment choisis parmi -(L¹)_{c}-P¹, -(L¹)_{c}-P¹-(L²)_{d}-P², -(L¹)_{c}-P¹-(L²)_{d}-P²-(L3)ₑ -P³ et -(L¹)_{c}-P¹-(L²)_{d}-P²-(L³)ₑ-P³-(L⁴)_{f}-P⁴;
P¹, P², P³ et P⁴ sont chacun indépendamment des séquences randomisées de peptides actifs pharmacologiquement ;
L¹, L², L³ et L⁴ sont chacun indépendamment des lieurs ; et
a, b, c, d, e et f sont chacun indépendamment 0 ou 1, pourvu qu'au moins l'un de a et b soit 1.

21. Procédé selon la revendication 20, dans lequel le composé préparé a pour formule :
X¹-F¹
ou
F¹-X².

22. Procédé selon la revendication 20, dans lequel le composé préparé a pour formule :
F¹-(L¹)_{c}-P¹
ou
F¹-(L¹)_{c}-P¹-(L²)_{d}-P².

23. Procédé selon l'une quelconque des revendications 17 à 22, dans laquelle F¹ est un domaine Fc d'IgG.

24. Procédé selon l'une quelconque des revendications 17 à 22, dans laquelle F¹ est un domaine. Fc d'IgG 1.

25. Procédé selon l'une quelconque des revendications 17 à 22, dans laquelle F¹ comprend la séquence de SEQ ID n° 2.
